# EUROPÄISCHE PATENTSCHRIFT

(11) **EP 4 240 726 B1**
(45) Veröffentlichungstag und Bekanntmachung des Hinweises auf die Patenterteilung: **08.01.2025**
(21) Anmeldenummer: 21799070.4
(22) Anmeldetag: 02.11.2021
(51) Int. Cl.: C07D 401/04, C07D 405/14, A01N 43/653

(54) **[(1-PHENYL-5-(HETEROARYL)-1H-1,2,4-TRIAZOL-3-YL)OXY]ESSIGSÄURE-DERIVATE ALS SAFENER ZUM SCHUTZ VON NUTZ- UND KULTURPFLANZEN**
DERIVATIVES OF [(1-PHENYL-5-(HETEROARYL)-1H-1,2,4-TRIAZOL-3-YL)OXY]ACETIC ACID AS SAFENERS FOR THE PROTECTION OF CULTIVATED PLANTS
DERIVÉS D' ACIDE [(1-PHÉNYLE-5-(HÉTÉROARYLE)-1H-1,2,4-TRIAZOL-3-YL)OXY]ACETIQUE COMME PHYTOPROTECTEURS POUR LA PROTECTION DE PLANTES CULTIVÉES

(30) Priorität: 05.11.2020 EP 20206033
(43) Veröffentlichungstag der Anmeldung: 13.09.2023
(73) Patentinhaber: Bayer Aktiengesellschaft, 51373 Leverkusen (DE)
(72) Erfinder: MÜLLER, Thomas, 60323 Frankfurt (DE); REINGRUBER, Anna Maria, 64646 Heppenheim (DE); FRACKENPOHL, Jens, 60439 Frankfurt (DE); HELMKE, Hendrik, 65835 Liederbach (DE); DITTGEN, Jan, 60316 Frankfurt (DE)
(74) Vertreter: BIP Patents
(86) Internationale Anmeldenummer: PCT/EP2021/080358
(87) Internationale Veröffentlichungsnummer: WO 2022/096446

(56) Entgegenhaltungen:
- WO-A1-2006/040016
- "Modern Crop Protection Compounds", 23 February 2007, WILEY.VCH, USA, ISBN: 978-3-527-31496-6, article CHRIS ROSINGER ET AL: "Safener for Herbicides", pages: 259 - 281, XP055741771, DOI: 10.1002/9783527619580.ch5

## Beschreibung

Die vorliegende Erfindung betrifft nutzpflanzenschützende Verbindungen und Mittel, welche spezielle Verbindungen als Safener zur Reduktion phytotoxischer Wirkungen von Agrochemikalien, insbesondere von Herbiziden, enthalten. Insbesondere betrifft die Erfindung bestimmte substituierte [(1-Phenyl-5-(Heteroaryl)-1H-1,2,4-triazol-3-yl)oxy]essigsäure-Derivate sowie deren Salze als Safener und Verfahren zu ihrer Herstellung.

Bei der Bekämpfung unerwünschter Organismen in land- und forstwirtschaftlichen Nutzpflanzenkulturen mit Pestiziden werden häufig auch die Nutzpflanzen durch die verwendeten Pestizide, wie Herbizide, Insektizide, Fungizide und andere, mehr oder weniger stark geschädigt. Dieser unerwünschte phytotoxische Nebeneffekt tritt in besonderem Maße bei der Verwendung von zahlreichen Herbiziden - und dort in erster Linie bei der Applikation im Nachauflauf - in Nutzpflanzenkulturen wie beispielsweise Mais, Reis oder Getreide auf. Durch den Einsatz sogenannter "Safener" oder "Antidots" können in manchen Fällen die Nutzpflanzen gegen die phytotoxischen Eigenschaften der Pestizide geschützt werden, ohne dass die pestizide Wirkung gegenüber den Schadorganismen geschmälert oder wesentlich beeinträchtigt wird. In manchen Fällen ist in Anwesenheit von Safenern sogar eine verbesserte pestizide Wirkung gegen Schadorganismen wie Unkräutern beobachtet worden.

Die bislang als Safener bekannt gewordenen Verbindungen gehören zu einer großen Zahl unterschiedlicher chemischer Strukturklassen, wobei deren Eignung für die Safeneranwendung in der Regel auch von den chemischen Strukturen der Pestizide und von den Nutzpflanzenkulturen abhängig ist.

Lange bekannt sind Safenerwirkungen von Verbindungen aus der Gruppe der Derivate von Phenoxy- oder Heteroaryloxyalkancarbonsäuren, wenn diese Verbindungen in Kombination mit Herbiziden angewandt werden. Beispiele für solche Verbindungen sind MCPA und ähnliche Verbindungen, welche zugleich gegen Schadpflanzen noch herbizid wirksam sind, oder Cloquintocet-mexyl.

Weiterhin bekannt sind Safener aus der Gruppe der Derivate von N-phenylsubstituierten Heteroarylcarbonsäureestern mit mehreren Heteroatomen im Heteroaromaten. Beispiele für solche Safener sind die in Handelsprodukten verwendeten Safener Mefenpyr-diethyl und Isoxadifen-ethyl.

Aus WO 2004/084631 ist die Verwendung von hydroxy-substituierten aromatischen Carbonsäurederivaten bekannt. In WO 2005/015994 sind spezielle Derivate der Salicylsäure als Safener beschrieben. Diese eignen sich besonders für die Anwendung als Safener in Mais- und Sojakulturen. Weiterhin sind aus WO 2005/112630 1,2-Dihydrochinoxalin-2-on-Derivate und aus WO 2008/131860 Pyridoncarboxamide als Safener bekannt. WO2006/040016 betrifft die Verwendung von Safenern zur Wirkungssteigerung von Fungiziden, Kombinationen von Fungiziden und Safenern und deren Verwendung zur Bekämpfung von unerwünschten Mikroorganismen.

Wirkstoffe aus der chemischen Klasse der substituierten [(1-Phenyl-5-(Heteroaryl)-1H-1,2,4-triazol-3-yl)oxy]essigsäure-Derivate mit pflanzenwirksamen Eigenschaften sind aus der Literatur nicht bekannt. Verschiedene Schriften beschreiben [(1,5-Diphenyl-1H-1,2,4-triazol-3-yl)oxy]essigsäure-Derivate mit medizinischen Eigenschaften. Aus Polish J. Chem. 2006, 80, 889-897 und Bioorganic & Medicinal Chemistry 2018, 26, 3321-3344 sind [(1,5-Diphenyl-1H-1,2,4-triazol-3-yl)oxy]essigsäure-Derivate bekannt.

Bei der Anwendung von Safenern zum Schutz von Nutzpflanzen vor Schädigungen durch Pestizide hat sich gezeigt, dass die bekannten Safener in vielen Fällen Nachteile aufweisen können. Dazu zählen:
- die nutzpflanzenschützenden Eigenschaften sind nicht ausreichend,
- in Kombination mit einem bestimmten Herbizid ist das Spektrum der Nutzpflanzen, in denen der Safener/Herbizid-Einsatz erfolgen soll, nicht ausreichend groß,
- ein bestimmter Safener ist nur mit wenigen Herbiziden kombinierbar,
- Die Verwendung von Safenern erhöht die zu applizierende Aufwandmenge und Menge an Formulierungshilfsstoffen und kann damit anwendungstechnische Probleme verursachen.

Aus den genannten Gründen besteht ein gesteigerter Bedarf an der Bereitstellung alternativer Verbindungen mit Safener-Wirkung.

Gegenstand der Erfindung sind neue nutzpflanzenschützende Verbindungen der allgemeinen Formel (I) oder deren Salze, 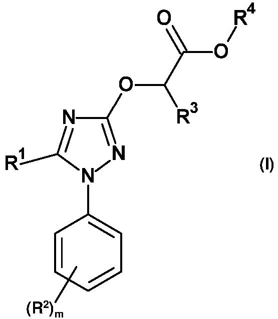 zur Reduktion phytotoxischer Wirkungen von Pestiziden, insbesondere von Herbiziden, auf Nutz- oder Kulturpflanzen, worin
- R¹: für Heteroaryl steht, wobei der Heteroaryl-Rest unsubstituiert oder durch Halogen, Cyano, Nitro, (C₁-C₆)Alkyl, (C₂-C₆)Alkenyl, (C₂-C₆)Alkinyl, (C₃-C₈)Cycloalkyl, (C₃-C₈)Cycloalkenyl, (C₁-C₆)Alkoxy und (C₁-C₆)AlkylS(O)ₚ, wobei die letztgenannten sieben Reste unsubstituiert sind, oder durch einen oder mehrere Reste aus der Gruppe Halogen, Cyano, (C₁-C₆)Alkoxy und (C₁-C₆)AlkylS(O)ₚ substituiert sind, bedeuten,
- R²: für Wasserstoff, Halogen, Cyano, Nitro, (C₁-C₆)Alkyl, (C₂-C₆)Alkenyl, (C₂-C₆)Alkinyl, (C₃-C₈)Cycloalkyl, (C₃-C₈)Cycloalkenyl, (C₁-C₆)Alkoxy und (C₁-C₆)AlkylS(O)ₚ, wobei die letztgenannten sieben Reste unsubstituiert oder durch einen schon mehrere Reste aus der Gruppe Halogen, Cyano, (C₁-C₆)Alkoxy und (C₁-C₆)AlkylS(O)ₚ substituiert sind, bedeuten,
- R³: für Wasserstoff und (C₁-C₆)Alkyl steht,
- R⁴: für Wasserstoff, (C₁-C₁₈)Alkyl, (C₁-C₁₈)Haloalkyl, (C₁-C₁₈)Cyanoalkyl, (C₂-C₁₈)Alkenyl, (C₂-C₁₈)Alkinyl, (C₃-C₁₂)Cycloalkyl, (C₃-C₁₂)Cycloalkenyl, Aryl, Heteroaryl, (C₁-C₁₈)Alkoxy-(C₁-C₁₈)alkyl, (C₁-C₁₈)Haloalkoxy-(C₁-C₁₈)alkyl, (C₁-C₁₈)Alkoxy-(C₁-C₁₈)haloalkyl, (C₁-C₁₈)Alkylthio-(C₁-C₁₈)alkyl, (C₁-C₁₈)Haloalkylthio-(C₁-C₁₈)alkyl, (C₂-C₁₈)Haloalkenyl, (C₂-C₁₈)Haloalkinyl, Heterocyclyl-(C₁-C₁₈)alkyl, Aryl-(C₁-C₁₈)alkyl, (C₃-C₁₂)Cycloalkyl-(C₁-C₁₈)alkyl, (C₁-C₁₈)Alkoxycarbonyl-(C₁-C₁₈)alkyl, und (C₁-C₁₈)Alkoxycarbonyl-(C₃-C₁₂)cycloalkyl-(C₁-C₁₈)alkyl, steht, oder
einen Rest der Formel -NR^{a}R^{b} oder -N=CR^{c}R^{d},
wobei in den vorgenannten 2 Resten jeder der Reste R^{a}, R^{b}, R^{c} und R^{d} unabhängig voneinander Wasserstoff, (C₁-C₄)Alkyl, (C₂-C₄)Alkenyl, (C₂-C₄)Alkinyl, Benzyl, substituiertes Benzyl, Phenyl oder substituiertes Phenyl bedeutet,
oder R^{a} und R^{b} zusammen mit dem N-Atom einen 3-bis 8-gliedrigen Heterocyclus, welcher zusätzlich zum N-Atom ein oder zwei weitere Heteroringatome aus der Gruppe N, O und S enthalten kann, und welcher unsubstituiert oder durch einen oder mehrere Reste aus der Gruppe (C₁-C₄)Alkyl und (C₁-C₄)Haloalkyl substituiert ist, bilden können,
oder R^{c} und R^{d} zusammen mit dem C-Atom einen 3- bis 8-gliedrigen carbocyclischen oder heterocyclischen Rest, welcher 1 bis 3 Heteroringatome aus der Gruppe N, O und S enthalten kann, wobei der carbocyclische oder heterocyclische Rest unsubstituiert oder durch einen oder mehrere Reste aus der Gruppe (C₁-C₄)Alkyl und (C₁-C₄)Haloalkyl substituiert ist, bedeutet,
- m: für eine Zahl von 0 bis 5 steht,
und
- p: 0, 1 oder 2 bedeutet.

Die Verbindungen der allgemeinen Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Piperidino, Morpholino oder Pyridino, Salze bilden. Diese Salze enthalten dann die konjugierte Base der Säure als Anion. Geeignete Substituenten, die in deprotonierter Form, wie z.B. Sulfonsäuren, bestimmte Sulfonsäureamide oder Carbonsäuren, vorliegen, können innere Salze mit ihrerseits protonierbaren Gruppen, wie Aminogruppen bilden. Salzbildung kann auch durch Einwirkung einer Base auf Verbindungen der allgemeinen Formel (I) erfolgen. Geeignete Basen sind beispielsweise organische Amine, wie Trialkylamine, Morpholin, Piperidin und Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der azide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre Ammoniumsalze, zum Beispiel mit Kationen der Formel [NRⁱRⁱⁱRⁱⁱⁱR^{iv}]⁺, worin Rⁱ bis R^{iv} jeweils unabhängig voneinander einen organischen Rest, insbesondere Alkyl, Aryl, Arylalkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Im Folgenden werden die erfindungsgemäß verwendeten Verbindungen der Formel (I) und ihre Salze als "Verbindungen der allgemeinen Formel (I)" bezeichnet.

Bevorzugter Erfindungsgegenstand sind Verbindungen der allgemeinen Formel (I), worin
- R¹: für Heteroaryl steht, wobei der Heteroaryl-Rest unsubstituiert oder durch Halogen, Cyano, Nitro, (C₁-C₆)Alkyl, (C₂-C₆)Alkenyl, (C₂-C₄)Alkinyl, (C₃-C₇)Cycloalkyl, (C₃-C₇)Cycloalkenyl, (C₁-C₄)Alkoxy und (C₁-C₄)AlkylS(O)ₚ, wobei die letztgenannten sieben Reste unsubstituiert sind, oder durch einen oder mehrere Reste aus der Gruppe Halogen, Cyano, (C₁-C₄)Alkoxy und (C₁-C₄)AlkylS(O)ₚ substituiert sind, bedeuten,
- R²: für Wasserstoff, Halogen, Cyano, Nitro, (C₁-C₄)Alkyl, (C₂-C₄)Alkenyl, (C₂-C₄)Alkinyl, (C₃-C₆)Cycloalkyl, (C₃-C₆)Cycloalkenyl, (C₁-C₄)Alkoxy und (C₁-C₄)AlkylS(O)ₚ,wobei die letztgenannten sieben Reste unsubstituiert sind, oder durch einen schon mehrere Reste aus der Gruppe Halogen, Cyano, (C₁-C₄)Alkoxy und (C₁-C₄)AlkylS(O)ₚ substituiert sind, bedeuten,
- R³: für Wasserstoff und (C₁-C₄)Alkyl steht,
- R⁴: für Wasserstoff, (C₁-C₁₆)Alkyl, (C₁-C₁₆)Haloalkyl, (C₁-C₁₆)Cyanoalkyl, (C₂-C₁₆)Alkenyl, (C₂-C₁₆)Alkinyl, (C₃-C₁₂)Cycloalkyl, (C₃-C₁₂)Cycloalkenyl, Aryl, Heteroaryl, (C₁-C₁₆)Alkoxy-(C₁-C₁₆)alkyl, (C₁-C₁₆)Haloalkoxy-(C₁-C₁₆)alkyl, (C₁-C₁₆)Alkoxy-(C₁-C₁₆)haloalkyl, (C₁-C₁₆)Alkylthio-(C₁-C₁₆)alkyl, (C₁-C₁₆)Haloalkylthio-(C₁-C₁₆)alkyl, (C₂-C₁₆)Haloalkenyl, (C₂-C₁₆)Haloalkinyl, Heterocyclyl-(C₁-C₁₆)alkyl, Aryl-(C₁-C₁₆)alkyl, (C₃-C₁₂)Cycloalkyl-(C₁-C₁₆)alkyl, (C₁-C₁₆)Alkoxycarbonyl-(C₁-C₁₆)alkyl, und (C₁-C₁₆)Alkoxycarbonyl-(C₃-C₁₂)cycloalkyl-(C₁-C₁₆)alkyl steht,
- m: für eine Zahl von 0 bis 4 steht,
und
- p: 0,1 oder 2 bedeutet.

Ganz besonders bevorzugter Erfindungsgegenstand sind Verbindungen der allgemeinen Formel (I), worin
- R¹: für Heteroaryl steht, wobei der Heteroaryl-Rest unsubstituiert ist, oder einfach oder mehrfach durch Halogen, Cyano, Methyl, Ethyl, CF₃, CF₂Cl, CH₂F, CHF₂, OCH₃, OCF₃, SCH₃, SOCH₃, SO₂CH₃ und SCF₃ substituiert ist,
- R²: für Wasserstoff, Halogen, Cyano, Methyl, Ethyl, CF₃, CF₂Cl, CH₂F, CHF₂, OCH₃, OCF₃, SCH₃, SOCH₃, SO₂CH₃ und SCF₃ steht,
- R³: für Wasserstoff, CH₂CH₃ und CH₃, steht,
- R⁴: für Wasserstoff, (C₁-C₁₂)Alkyl, (C₁-C₁₂)Haloalkyl, (C₁-C₁₂)Cyanoalkyl, (C₂-C₁₂)Alkenyl, (C₂-C₁₂)Alkinyl, (C₃-C₁₂)Cycloalkyl, (C₃-C₁₂)Cycloalkenyl, Aryl, Heteroaryl, (C₁-C₁₂)Alkoxy-(C₁-C₁₂)alkyl, (C₁-C₁₂)Haloalkoxy-(C₁-C₁₂)alkyl, (C₁-C₁₂)Alkoxy-(C₁-C₁₂)haloalkyl, (C₁-C₁₂)Alkylthio-(C₁-C₁₂)alkyl, (C₁-C₁₂)Haloalkylthio-(C₁-C₁₂)alkyl, (C₂-C₁₂)Haloalkenyl, (C₂-C₁₂)Haloalkinyl, Heterocyclyl-(C₁-C₁₂)alkyl, Aryl-(C₁-C₁₂)alkyl, (C₃-C₁₂)Cycloalkyl-(C₁-C₁₂)alkyl, (C₁-C₁₂)Alkoxycarbonyl-(C₁-C₁₂)alkyl, und (C₁-C₁₂)Alkoxycarbonyl-(C₃-C₁₂)cycloalkyl-(C₁-C₁₂)alkyl steht.
und
- m: für 0, 1, 2 oder 3 steht.

Im Speziellen bevorzugter Erfindungsgegenstand sind Verbindungen der allgemeinen Formel (I), worin
- R¹: für Pyridin-2-yl, Pyridin-3-yl, Pyridin-4-yl, Pyrazin-2-yl, Pyrazin-3-yl, Pyrimidin-2-yl, Pyrimidin-4-yl, Pyrimidin-5-yl, Pyridazin-3-yl, Pyridazin-4-yl, 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl, 1,2,4-, 1,3,2-, 1,3,6- und 1,2,6-Oxdiazinyl, Isoxazol-3-yl, Isoxazol-4-yl, Isoxazol-5-yl, 1,3-Oxazol-2-yl, 1,3-Oxazol-4-yl, 1,3-Oxazol-5-yl, Isothiazol-3-yl, Isothiazol-4-yl, Isothiazol-5-yl, 1,3-Thiazol-2-yl, 1,3-Thiazol-4-yl, 1,3-Thiazol-5-yl, 1H-Pyrrol-1-yl, 1H-Pyrrol-2-yl, 1H-Pyrrol-3-yl, Furan-2-yl, Furan-3-yl, Thien-2-yl; Thien-3-yl, 1H-Imidazol-1-yl, 1H-Imidazol-2-yl, 1H-Imidazol-4-yl, 1H-Imidazol-5-yl, 1H-Pyrazol-1-yl, 1H-Pyrazol-3-yl, 1H-Pyrazol-4-yl, 1H-Pyrazol-5-yl, 1H-1,2,3-Triazol-1-yl, 1H-1,2,3-Triazol-4-yl, 1H-1,2,3-Triazol-5-yl, 2H-1,2,3-Triazol-2-yl, 2H-1,2,3-Triazol-4-yl, 1H-1,2,4-Triazol-1-yl, 1H-1,2,4-Triazol-5-yl, 4H-1,2,4-Triazol-3-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, 1,3,4-Thiadiazol-2-yl, 1,3,4-Thiadiazol-2-yl, 1,2,4- Thiadiazol-5-yl, 1,2,4-Thiadiazol-3-yl, 1,2,5-Thiadiazol-3-yl, 1,2,5-Thiadiazol-3-yl, 1,2,3-Thiadiazol-4-yl, 1,2,3-Thiadiazol-3-yl, welcher unsubstituiert ist, oder einfach oder mehrfach durch Halogen, Cyano, Methyl, CF₃, CF₂Cl, CH₂F, CHF₂, OCH₃, OCF₃, SCH₃, SOCH₃, SO₂CH₃ und SCF₃ substituiert ist, steht,
- R²: für Wasserstoff, Fluor, Chlor, Brom, Iod, CN, Methyl, CF₃, CF₂Cl, CH₂F, CHF₂, OCH₃, OCF₃, SCH₃, SOCH₃, SO₂CH₃ und SCF₃ steht,
- R³: für Wasserstoff und CH₃ steht,
- R⁴: für Wasserstoff, (C₁-C₁₀)Alkyl, (C₁-C₁₀)Haloalkyl, (C₁-C₁₀)Cyanoalkyl, (C₂-C₁₀)Alkenyl, (C₂-C₁₀)Alkinyl, (C₃-C₉)Cycloalkyl, (C₃-C₉)Cycloalkenyl, Aryl, Heteroaryl, (C₁-C₁₀)Alkoxy-(C₁-C₁₀)alkyl, (C₁-C₁₀)Haloalkoxy-(C₁-C₁₀)alkyl, (C₁-C₁₀)Alkoxy-(C₁-C₁₀)haloalkyl, (C₁-C₁₀)Alkylthio-(C₁-C₁₀)alkyl, (C₁-C₁₀)Haloalkylthio-(C₁-C₁₀)alkyl, (C₂-C₁₈)Haloalkenyl, (C₂-C₁₈)Haloalkinyl, Heterocyclyl-(C₁-C₁₀)alkyl, Aryl-(C₁-C₁₀)alkyl, (C₃-C₉)Cycloalkyl-(C₁-C₁₀)alkyl, (C₁-C₁₀)Alkoxycarbonyl-(C₁-C₁₀)alkyl und (C₁-C₁₀)Alkoxycarbonyl-(C₃-C₉)cycloalkyl-(C₁-C₁₀)alkyl steht,
und
- m: für 0, 1, 2 oder 3 steht.

Im ganz Speziellen bevorzugter Erfindungsgegenstand sind Verbindungen der allgemeinen Formel (I), worin
R¹ für die Gruppen Q-1.1 bis Q-1.59

| | | | | |
|---|---|---|---|---|
| 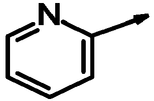 | 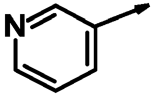 | 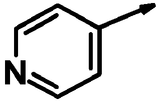 | 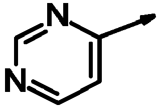 | 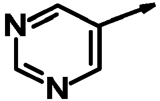 |
| Q-1.1 | Q-1.2 | Q-1.3 | Q-1.4 | Q-1.5 |
| 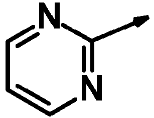 | 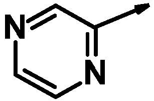 | 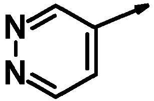 | 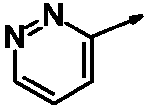 | 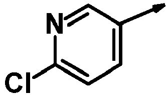 |
| Q-1.6 | Q-1.7 | Q-1.8 | Q-1.9 | Q-1.10 |
| 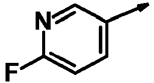 | 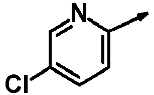 | 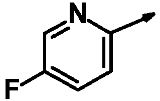 | 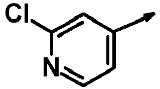 | 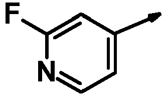 |
| Q-1.11 | Q-1.12 | Q-1.13 | Q-1.14 | Q-1.15 |
| 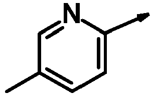 | 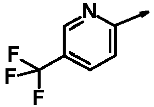 | 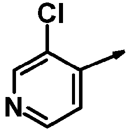 | 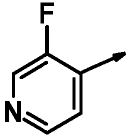 | 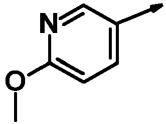 |
| Q-1.16 | Q-1.17 | Q-1.18 | Q-1.19 | Q-1.20 |
| 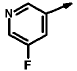 | 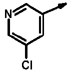 | 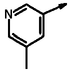 | 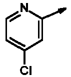 | 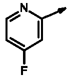 |
| Q-1.21 | Q-1.22 | Q-1.23 | Q-1.24 | Q-1.25 |
| 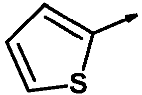 | 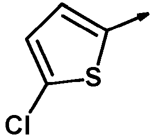 | 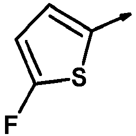 | 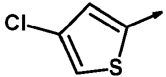 | 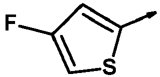 |
| Q-1.26 | Q-1.27 | Q-1.28 | Q-1.29 | Q-1.30 |
| 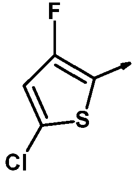 | 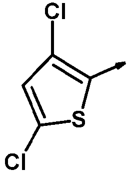 | 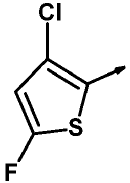 | 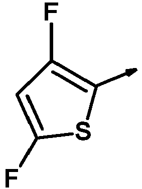 | 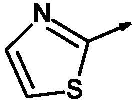 |
| Q-1.31 | Q-1.32 | Q-1.33 | Q-1.34 | Q-1.35 |
| 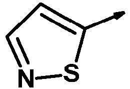 | 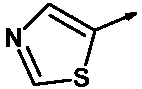 | 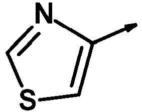 | 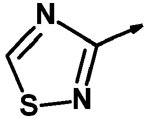 | 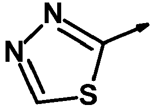 |
| Q-1.36 | Q-1.37 | Q-1.38 | Q-1.39 | Q-1.40 |
| 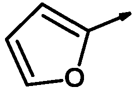 | 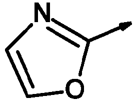 | 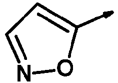 | 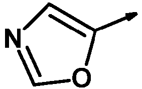 | 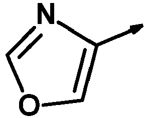 |
| Q-1.41 | Q-1.42 | Q-1.43 | Q-1.44 | Q-1.45 |
| 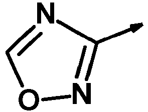 | 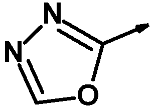 | 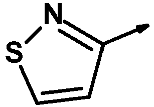 | 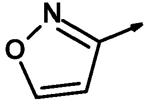 | 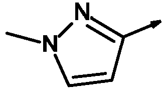 |
| Q-1.46 | Q-1.47 | Q-1.48 | Q-1.49 | Q-1.50 |
| 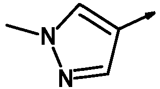 | 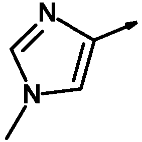 | 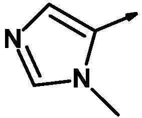 | 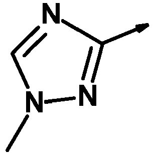 | 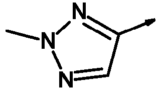 |
| Q-1.51 | Q-1.52 | Q-1.53 | Q-1.54 | Q-1.55 |
| 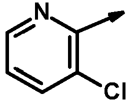 | 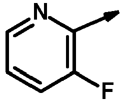 | 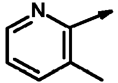 | 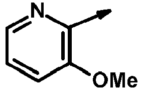 | |
| Q-1.56 | Q-1.57 | Q-1.58 | Q-1.59 | |

- R²: für Wasserstoff, Fluor, Chlor, Brom, Iod, Cyano, Methyl, CF₃, CH₂F, CHF₂, OCH₃, OCF₃, SCH₃, SOCH₃, SO₂CH₃ und SCF₃ steht,
- R³: für Wasserstoff steht,
- R⁴: für Wasserstoff, Methyl, Ethyl, n-Propyl, i-Propyl, n-Butyl, n-Pentyl, Phenyl, Benzyl, CHz(4-Cl-Ph), CH₂(4-F-Ph), CH₂(4-MeO-Ph), 2-Methoxyethyl, Tetrahydrofuran-2-yl-methyl, Tetrahydrofuran-3-yl-methyl, Tetrahydropyran-2-yl-methyl, Tetrahydropyran-3-yl-methyl, Tetrahydropyran-4-yl-methyl, Methylpropionat-3-yl, Ethylpropionat-3-yl, Methylacetat-2-yl, Ethylacetat-2-yl, Methylpivalat-2-yl, Ethylpivalat-3-yl, Methyl-2-methylpropanoat-3-yl, Methyl-2,2-dimethylpropanoat-3-yl, Ethyl-2-methylpropanoat-3-yl, Methyl-2-propanoat-2-yl, Ethyl-2-propanoat-2-yl, Methyl-acet-2-yl, Ethyl-acetat-2-yl, Methyl-1-methylcyclopropancarboxylat-2-yl, Ethyl-1-methylcyclopropancarboxylat-2-yl, 2-(Dimethylamino)ethyl, Oxetan-3-yl, (3-Methyloxetan-3-yl)methyl, 2,2,2-Trifluorethyl, 2,2-Difluorethyl, 2-Fluorethyl, 2,2,3,3,3-Pentafluorpropyl, Cyclopropylmethyl, 1-Cyclopropyl-ethyl, (1-Methyl-cyclopropyl)-methyl, (2,2-Dichlorcyclopropyl)-methyl, (2,2-Dimethyl-cyclopropyl)-methyl, Allyl, Propargyl, (Prop-2-in-1-yl), 2-Chlorprop-2-en-1-yl, 3-Phenylprop-2-in-1-yl, 3,3-Dichlorprop-2-en-1-yl, 3,3-Dichlor-2-fluor-prop-2-en-1-yl, Methylprop-2-in-1-yl, 2- Methylprop-2-en-1-yl, But-2-en-1-yl, But-3-en-1-yl, But-2-in-1-yl, But-3-in-1-yl, 4-Chlor-but-2-in-1-yl, 3-Methyl-but-2-en-1-yl, 3-Methyl-but-1-en-1-yl, (2E)-1-Methylbut-2-en-1-yl, (E)-Pent-3-en-2-yl oder (Z)-Pent-3-en-2-yl, Cyclobutylmethyl, Cyclopentylmethyl, Cyclohexylmethyl, Heptan-2-yl, iso-Butyl, 1,3-Dioxolan-2-ylmethyl oder 1-Ethyl-5-methyl-1H-pyrazol-4-methyl steht,
- m: für 0, 1, 2 oder 3 steht.

Im äußerst Speziellen bevorzugtem Erfindungsgegenstand sind Verbindungen der allgemeinen Formel (I), worin
R¹ für die Gruppen Q-1.1 bis Q-1.59

| | | | | |
|---|---|---|---|---|
| 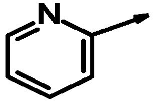 | 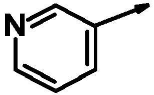 | 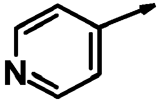 | 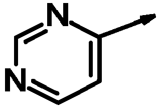 | 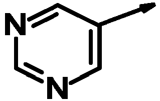 |
| Q-1.1 | Q-1.2 | Q-1.3 | Q-1.4 | Q-1.5 |
| 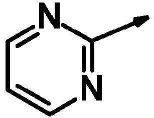 | 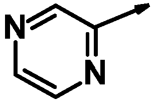 | 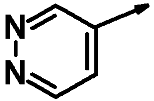 | 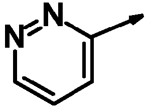 | 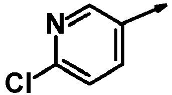 |
| Q-1.6 | Q-1.7 | Q-1.8 | Q-1.9 | Q-1.10 |
| 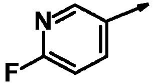 | 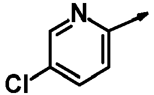 | 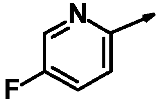 | 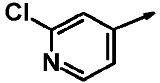 | 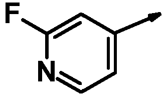 |
| Q-1.11 | Q-1.12 | Q-1.13 | Q-1.14 | Q-1.15 |
| 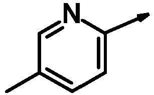 | 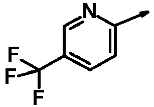 | 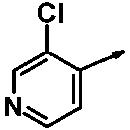 | 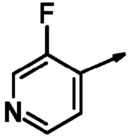 | 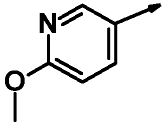 |
| Q-1.16 | Q-1.17 | Q-1.18 | Q-1.19 | Q-1.20 |
| 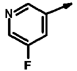 | 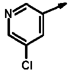 | 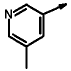 | 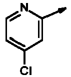 | 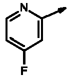 |
| Q-1.21 | Q-1.22 | Q-1.23 | Q-1.24 | Q-1.25 |
| 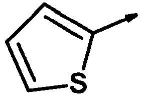 | 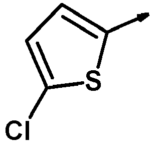 | 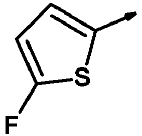 | 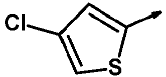 | 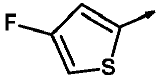 |
| Q-1.26 | Q-1.27 | Q-1.28 | Q-1.29 | Q-1.30 |
| 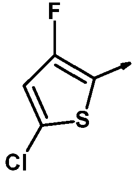 | 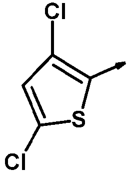 | 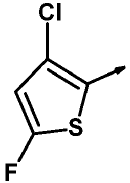 | 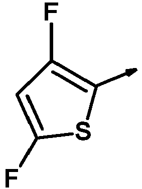 | 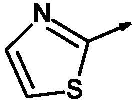 |
| Q-1.31 | Q-1.32 | Q-1.33 | Q-1.34 | Q-1.35 |
| 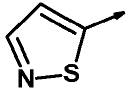 | 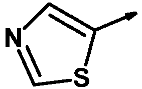 | 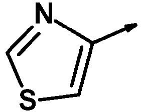 | 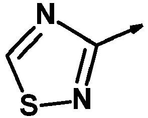 | 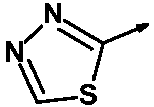 |
| Q-1.36 | Q-1.37 | Q-1.38 | Q-1.39 | Q-1.40 |
| 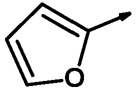 | 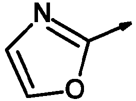 | 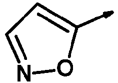 | 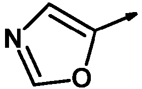 | 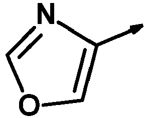 |
| Q-1.41 | Q-1.42 | Q-1.43 | Q-1.44 | Q-1.45 |
| 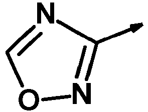 | 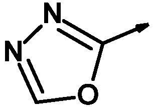 | 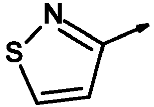 | 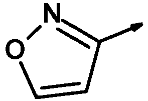 | 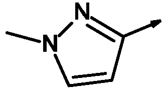 |
| Q-1.46 | Q-1.47 | Q-1.48 | Q-1.49 | Q-1.50 |
| 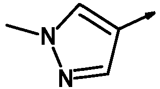 | 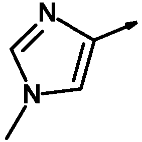 | 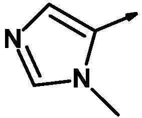 | 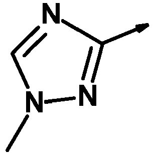 | 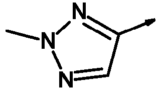 |
| Q-1.51 | Q-1.52 | Q-1.53 | Q-1.54 | Q-1.55 |
| 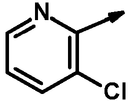 | 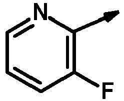 | 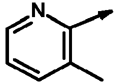 | | 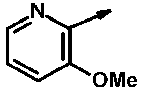 |
| Q-1.56 | Q-1.57 | Q-1.58 | | Q-1.59 |

steht,
und (R²)ₘ-Phenyl für die Gruppen Q-2.1 bis Q-2.53

| | | | | |
|---|---|---|---|---|
| 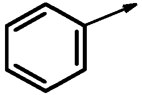 | 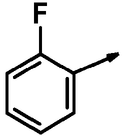 | 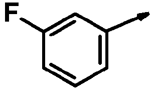 | 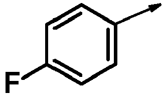 | 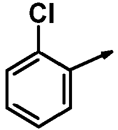 |
| Q-2.1 | Q-2.2 | Q-2.3 | Q-2.4 | Q-2.5 |
| 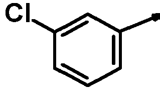 | 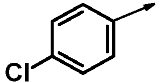 | 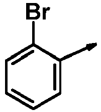 | 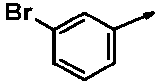 | 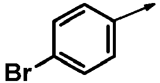 |
| Q-2.6 | Q-2.7 | Q-2.8 | Q-2.9 | Q-2.10 |
| 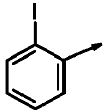 | 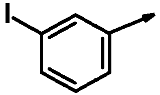 | 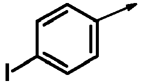 | 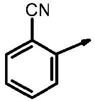 | 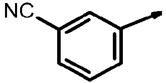 |
| Q-2.11 | Q-2.12 | Q-2.13 | Q-2.14 | Q-2.15 |
| 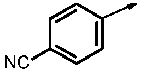 | 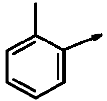 | 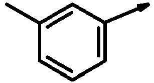 | 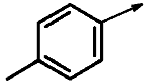 | 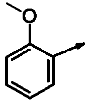 |
| Q-2.16 | Q-2.17 | Q-2.18 | Q-2.19 | Q-2.20 |
| 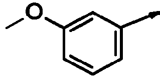 | 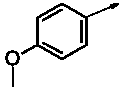 | 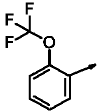 | 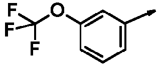 | 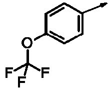 |
| Q-2.21 | Q-2.22 | Q-2.23 | Q-2.24 | Q-2.25 |
| 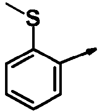 | 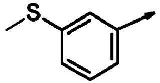 | 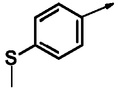 | 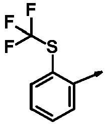 | 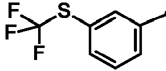 |
| Q-2.26 | Q-2.27 | Q-2.28 | Q-2.29 | Q-2.30 |
| 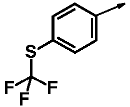 | 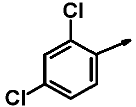 | 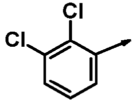 | 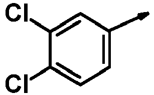 | 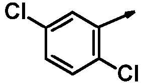 |
| Q-2.31 | Q-2.32 | Q-2.33 | Q-2.34 | Q-2.35 |
| 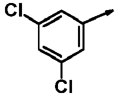 | 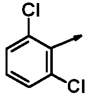 | 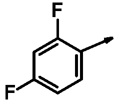 | 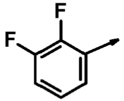 | 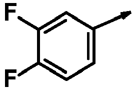 |
| Q-2.36 | Q-2.37 | Q-2.38 | Q-2.39 | Q-2.40 |
| 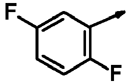 | 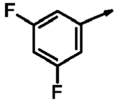 | 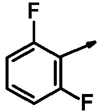 | 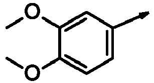 | 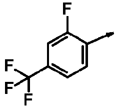 |
| Q-2.41 | Q-2.42 | Q-2.43 | Q-2.44 | Q-2.45 |
| 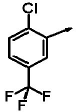 | 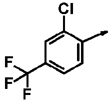 | 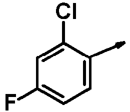 | 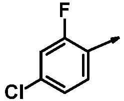 | 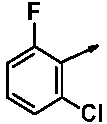 |
| Q-2.46 | Q-2.47 | Q-2.48 | Q-2.49 | Q-2.50 |
| 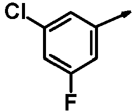 | 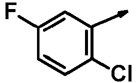 | 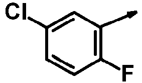 | | |
| Q-2.51 | Q-2.52 | Q-2.53 | | |

steht.
- R³: für Wasserstoff steht,
- R⁴: für Wasserstoff, Methyl, Ethyl, n-Propyl, i-Propyl, n-Butyl, n-Pentyl, Phenyl, Benzyl, CHz(4-Cl-Ph), CH₂(4-F-Ph), CH₂(4-MeO-Ph), 2-Methoxyethyl, Tetrahydrofuran-2-yl-methyl, Tetrahydrofuran-3-yl-methyl, Tetrahydropyran-2-yl-methyl, Tetrahydropyran-3-yl-methyl, Tetrahydropyran-4-yl-methyl, Methylpropionat-3-yl, Ethylpropionat-3-yl, Methylacetat-2-yl, Ethylacetat-2-yl, Methylpivalat-2-yl, Ethylpivalat-3-yl, Methyl-2-methylpropanoat-3-yl, Methyl-2,2-dimethylpropanoat-3-yl, Ethyl-2-methylpropanoat-3-yl, Methyl-2-propanoat-2-yl, Ethyl-2-propanoat-2-yl, Methyl-acetat-2-yl, Ethyl-acetat-2-yl, Methyl-1-methylcyclopropan-carboxylat-2yl, Ethyl-1-methylcyclopropancarboxylat-2-yl, 2-(Dimethylamino)ethyl, Oxetan-3-yl, (3-Methyloxetan-3-yl)methyl, 2,2,2-Trifluorethyl, 2,2-Difluorethyl, 2-Fluorethyl, 2,2,3,3,3-Pentafluorpropyl, Cyclopropylmethyl, 1-Cyclopropyl-ethyl, (1-Methyl-cyclopropyl)-methyl, (2,2-Dichlorcyclopropyl)-methyl, (2,2-Dimethyl-cyclopropyl)-methyl, Allyl, Propargyl (Prop-2-in-1-yl), 2-Chlorprop-2-en-1-yl, 3-Phenylprop-2-in-1-yl, 3,3-Dichlorprop-2-en-1-yl, 3,3-Dichlor-2-fluor-prop-2-en-1-yl, Methylprop-2-in-1-yl, 2-Methylprop-2-en-1-yl, But-2-en-1-yl, But-3-en-1-yl, But-2-in-1-yl, But-3-in-1-yl, 4-Chlor-but-2-in-1-yl, 3-Methyl-but-2-en-1-yl, 3-Methyl-but-1-en-1-yl, (2E)-1-Methylbut-2-en-1-yl, (E)-Pent-3-en-2-yl oder (Z)-Pent-3-en-2-yl, Cyclobutylmethyl, Cyclopentylmethyl, Cyclohexylmethyl, Heptan-2-yl, iso-Butyl, 1,3-Dioxolan-2-ylmethyl oder 1-Ethyl-5-methyl-1H-pyrazol-4-methyl steht.

Im besonders äußerst Speziellen bevorzugtem Erfindungsgegenstand sind Verbindungen der allgemeinen Formel (I), worin
R¹ für Q-1.1 bis Q-1.59

| | | | | |
|---|---|---|---|---|
| 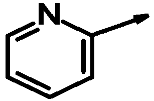 | 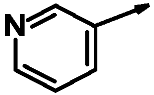 | 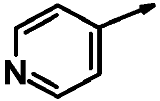 | 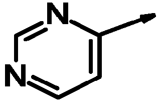 | 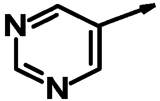 |
| Q-1.1 | Q-1.2 | Q-1.3 | Q-1.4 | Q-1.5 |
| 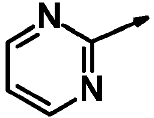 | 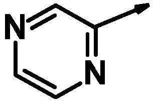 | 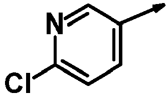 | 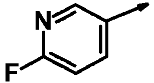 | 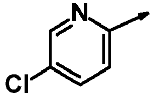 |
| Q-1.6 | Q-1.7 | Q-1.10 | Q-1.11 | Q-1.12 |
| 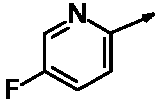 | 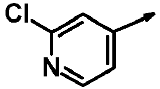 | 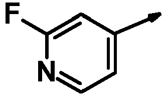 | 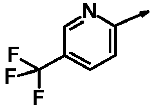 | 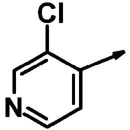 |
| Q-1.13 | Q-1.14 | Q-1.15 | Q-1.17 | Q-1.18 |
| 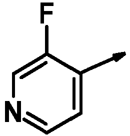 | 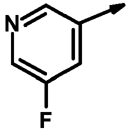 | 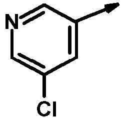 | 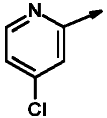 | 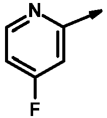 |
| Q-1.19 | Q-1.21 | Q-1.22 | Q-1.24 | Q-1.25 |
| 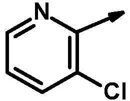 | 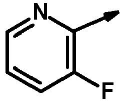 | 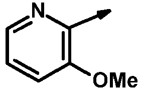 | | |
| Q-1.56 | Q-1.57 | Q-1.59 | | |

steht,
und (R²)ₘ-Phenyl für die Gruppen Q-2.1 bis Q-2.53

| | | | | |
|---|---|---|---|---|
| 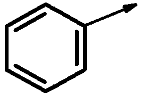 | 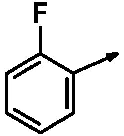 | 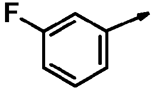 | 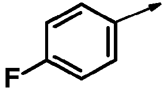 | 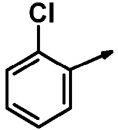 |
| Q-2.1 | Q-2.2 | Q-2.3 | Q-2.4 | Q-2.5 |
| 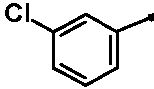 | 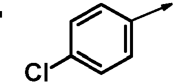 | 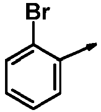 | 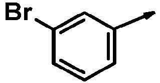 | 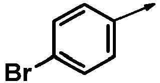 |
| Q-2.6 | Q-2.7 | Q-2.8 | Q-2.9 | Q-2.10 |
| 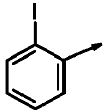 | 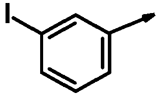 | 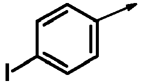 | 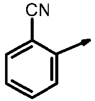 | 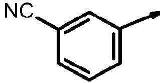 |
| Q-2.11 | Q-2.12 | Q-2.13 | Q-2.14 | Q-2.15 |
| 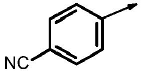 | 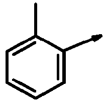 | 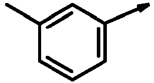 | 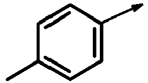 | 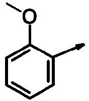 |
| Q-2.16 | Q-2.17 | Q-2.18 | Q-2.19 | Q-2.20 |
| 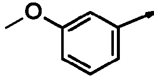 | 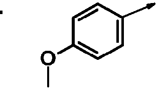 | 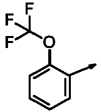 | 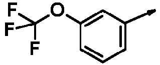 | 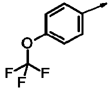 |
| Q-2.21 | Q-2.22 | Q-2.23 | Q-2.24 | Q-2.25 |
| 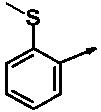 | 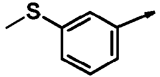 | 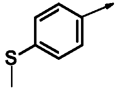 | 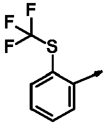 | 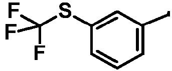 |
| Q-2.26 | Q-2.27 | Q-2.28 | Q-2.29 | Q-2.30 |
| 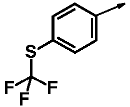 | 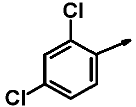 | 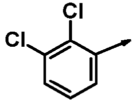 | 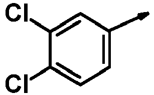 | 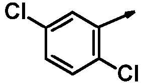 |
| Q-2.31 | Q-2.32 | Q-2.33 | Q-2.34 | Q-2.35 |
| 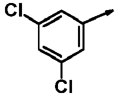 | 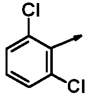 | 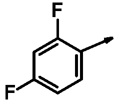 | 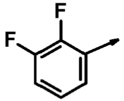 | 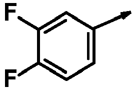 |
| Q-2.36 | Q-2.37 | Q-2.38 | Q-2.39 | Q-2.40 |
| 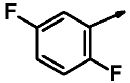 | 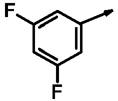 | 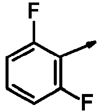 | 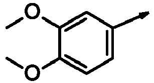 | 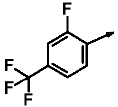 |
| Q-2.41 | Q-2.42 | Q-2.43 | Q-2.44 | Q-2.45 |
| 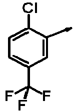 | 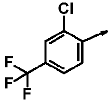 | 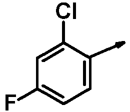 | 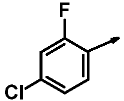 | 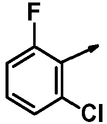 |
| Q-2.46 | Q-2.47 | Q-2.48 | Q-2.49 | Q-2.50 |
| 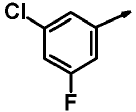 | 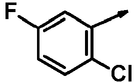 | | 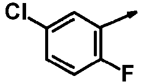 | |
| Q-2.51 | Q-2.52 | | Q-2.53 | |

steht.
- R³: für Wasserstoff steht,
- R⁴: für Wasserstoff, Methyl, Ethyl, n-Propyl, i-Propyl, n-Butyl, n-Pentyl, Phenyl, Benzyl, CH₂(4-Cl-Ph), CH₂(4-F-Ph), CH₂(4-MeO-Ph), 2-Methoxyethyl, Tetrahydrofuran-2-yl-methyl, Tetrahydrofuran-3-yl-methyl, Tetrahydropyran-2-yl-methyl, Tetrahydropyran-3-yl-methyl, Tetrahydropyran-4-yl-methyl, Methylpropionat-3-yl, Ethylpropionat-3-yl, Methylacetat-2-yl, Ethylacetat-2-yl, Methylpivalat-2-yl, Ethylpivalat-3-yl, Methyl-2-methylpropanoat-3-yl, Methyl-2,2-dimethylpropanoat-3-yl, Ethyl-2-methylpropanoat-3-yl, Methyl-2-propanoat-2-yl, Ethyl-2-propanoat-2-yl, Methyl-acetat-2-yl, Ethyl-acetat-2-yl, Methyl-1-methylcyclopropancarboxylat-2-yl, Ethyl-1-methylcyclopropancarboxylat-2-yl, 2-(Dimethylamino)ethyl, Oxetan-3-yl, (3-Methyloxetan-3-yl)methyl, 2,2,2-Trifluorethyl, 2,2-Difluorethyl, 2-Fluorethyl, 2,2,3,3,3-Pentafluorpropyl, Cyclopropylmethyl, 1-Cyclopropyl-ethyl, (1-Methyl-cyclopropyl)-methyl, (2,2-Dichlorcyclopropyl)-methyl, (2,2-Dimethyl-cyclopropyl)-methyl, Allyl, Propargyl (Prop-2-in-1-yl), 2-Chlorprop-2-en-1-yl, 3-Phenylprop-2-in-1-yl, 3,3-Dichlorprop-2-en-1-yl, 3,3-Dichlor-2-fluor-prop-2-en-1-yl, Methylprop-2-in-1-yl, 2-Methylprop-2-en-1-yl, But-2-en-1-yl, But-3-en-1-yl, But-2-in-1-yl, But-3-in-1-yl, 4-Chlor-but-2-in-1-yl, 3-Methyl-but-2-en-1-yl, 3-Methyl-but-1-en-1-yl, (2E)-1-Methylbut-2-en-1-yl, (E)-Pent-3-en-2-yl oder (Z)-Pent-3-en-2-yl, Cyclobutylmethyl, Cyclopentylmethyl, Cyclohexylmethyl, Heptan-2-yl, iso-Butyl, 1,3-Dioxolan-2-ylmethyl oder 1-Ethyl-5-methyl-1H-pyrazol-4-methyl steht.

Die oben aufgeführten allgemeinen oder in Vorzugsbereichen aufgeführten Restedefinitionen gelten sowohl für die Endprodukte der allgemeinen Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Vor allem aus den Gründen der höheren herbiziden Wirkung, besseren Selektivität und/oder besseren Herstellbarkeit sind erfindungsgemäße Verbindungen der genannten allgemeinen Formel (I) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.

Im Hinblick auf die erfindungsgemäßen Verbindungen werden die vorstehend und weiter unten verwendeten Bezeichnungen erläutert. Diese sind dem Fachmann geläufig und haben insbesondere die im Folgenden erläuterten Bedeutungen:
Sofern nicht anders definiert, gilt generell für die Bezeichnung von chemischen Gruppen, dass die Anbindung an das Gerüst bzw. den Rest des Moleküls über das zuletzt genannte Strukturelement der betreffenden chemischen Gruppe erfolgt, d.h. beispielsweise im Falle von (C₂-C₈)-Alkenyloxy über das Sauerstoffatom, und im Falle von Heterocyclyl-(C₁-C₈)-alkyl oder MeO(O)C-(C₁-C₈)-Alkyl jeweils über das C-Atom der Alkylgruppe.

Erfindungsgemäß steht "Alkylsulfonyl" - in Alleinstellung oder als Bestandteil einer chemischen Gruppe - für geradkettiges oder verzweigtes Alkylsulfonyl, vorzugsweise mit 1 bis 8, oder mit 1 bis 6 Kohlenstoffatomen, z.B. (aber nicht beschränkt auf) (C₁-C₆)-Alkylsulfonyl wie Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl.

Erfindungsgemäß steht "Alkylthio" - in Alleinstellung oder als Bestandteil einer chemischen Gruppe - für geradkettiges oder verzweigtes S-Alkyl, vorzugsweise mit 1 bis 8, oder mit 1 bis 6 Kohlenstoffatomen, wie (C₁-C₁₀)-, (C₁-C₆)- oder (C₁-C₄)-Alkylthio, z.B. (aber nicht beschränkt auf) (C₁-C₆)-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 1,1-Dimethylpropylthio, 1,2-Dimethylpropylthio, 2,2-Dimethylpropylthio, 1-Ethylpropylthio, Hexylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio.

"Alkylsulfinyl (Alkyl-S(=O)-)", soweit nicht an anderer Stelle anders definiert steht erfindungsgemäß für Alkylreste, die über -S(=O)- an das Gerüst gebunden sind, wie (C₁-C₁₀)-, (C₁-C₆)- oder (C₁-C₄)-Alkylsulfinyl, z. B. (aber nicht beschränkt auf) (C₁-C₆)-Alkylsulfinyl wie Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 1,1-Dimethylpropylsulfinyl, 1,2-Dimethylpropylsulfinyl, 2,2-Dimethylpropylsulfinyl, 1-Ethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl.

"Alkoxy" bedeutet ein über ein Sauerstoffatom gebundenen Alkylrest, z. B. (aber nicht beschränkt auf) (C₁-C₆)-Alkoxy wie Methoxy, Ethoxy, Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Alkenyloxy bedeutet ein über ein Sauerstoffatom gebundenen Alkenylrest, Alkinyloxy bedeutet ein über ein Sauerstoffatom gebundenen Alkinylrest wie (C₂-C₁₀)-, (C₂-C₆)- oder (C₂-C₄)-Alkenoxy bzw. (C₃-C₁₀)-, (C₃-C₆)- oder (C₃-C₄)-Alkinoxy.

"Alkoxycarbonyl (Alkyl-O-C(=O)-)", soweit nicht an anderer Stelle anders definiert: Alkylreste, die über -O-C(=O)- an das Gerüst gebunden sind, wie (C₁-C₁₀)-, (C₁-C₆)- oder (C₁-C₄)-Alkoxycarbonyl. Die Anzahl der C-Atome bezieht sich dabei auf den Alkylrest in der Alkoxycarbonylgruppe. Analog stehen "Alkenyloxycarbonyl" und "Alkinyloxycarbonyl", soweit nicht an anderer Stelle anders definiert, erfindungsgemäß für Alkenyl- bzw. Alkinylreste, die über -O-C(=O)- an das Gerüst gebunden sind, wie (C₂-C₁₀)-, (C₂-C₆)- oder (C₂-C₄)-Alkenyloxycarbonyl bzw. (C₃-C₁₀)-, (C₃-C₆)- oder (C₃-C₄)-Alkinyloxycarbonyl. Die Anzahl der C-Atome bezieht sich dabei auf den Alkenyl- bzw. Alkinylrest in der Alken- bzw. Alkinyloxycarbonylgruppe.

Der Begriff "Aryl" bedeutet ein gegebenenfalls substituiertes mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist "Aryl" in der Regel auch von dem Begriff "gegebenenfalls substituiertes Phenyl" umfasst. Bevorzugte Aryl-Substituenten sind hier zum Beispiel Wasserstoff, Halogen, Alkyl, Cycloalkyl, Cycloalkylalkyl, Cycloalkenyl, Halocycloalkyl, Alkenyl, Alkinyl, Aryl, Arylalkyl, Heteroaryl, Heteroarylalkyl, Heterocyclyl, Heterocyclylalkyl, Alkoxyalkyl, Alkylthio, Haloalkylthio, Haloalkyl, Alkoxy, Haloalkoxy, Cycloalkoxy, Cycloalkylalkoxy, Aryloxy, Heteroraryloxy, Alkoxyalkoxy, Alkinylalkoxy, Alkenyloxy, Bis-alkylaminoalkoxy, Tris-[alkyl]silyl, Bis-[alkyl]arylsilyl, Bis-[alkyl]alkylsilyl, Tris-[alkyl]silylalkinyl, Alkylalkinyl, Cycloalkylalkinyl, Haloalkylalkinyl, Heterocyclyl-N-alkoxy, Nitro, Cyano, Amino, Alkylamino, Bis-alkylamino, Alkylcarbonylamino, Cycloalkylcarbonylamino, Arylcarbonylamino, Alkoxycarbonylamino, Alkoxycarbonylalkylamino, Arylalkoxycarbonylalkylamino, Hydroxycarbonyl, Alkoxycarbonyl, Aminocarbonyl, Alkylaminocarbonyl, Cycloalkylaminocarbonyl, Bis-Alkylaminocarbonyl, Heteroarylalkoxy, Arylalkoxy

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P) der gesättigt, ungesättigt, teilgesättigt oder heteroaromatisch ist und dabei unsubstituiert oder substituiert sein kann, wobei die Bindungsstelle an einem Ringatom lokalisiert ist. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden auch mehrcyclische Systeme umfasst, wie beispielsweise 8-Aza-bicyclo[3.2.1]octanyl, 8-Aza-bicyclo[2.2.2]octanyl oder 1-Aza-bicyclo[2.2.1]heptyl. Im Falle von gegebenenfalls substituiertem Heterocyclyl werden auch spirocyclische Systeme umfasst, wie beispielsweise 1-Oxa-5-aza-spiro[2.3]hexyl. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen, wie beispielsweise mit einem Heteroatom aus der Gruppe N, O und S 1- oder 2- oder 3-Pyrrolidinyl, 3,4-Dihydro-2H-pyrrol-2- oder 3-yl, 2,3-Dihydro-1H-pyrrol-1- oder 2- oder 3- oder 4- oder 5-yl; 2,5-Dihydro-1H-pyrrol-1- oder 2- oder 3-yl, 1- oder 2- oder 3- oder 4-Piperidinyl; 2,3,4,5-Tetrahydropyridin-2- oder 3- oder 4- oder 5-yl oder 6-yl; 1,2,3,6-Tetrahydropyridin-1- oder 2- oder 3- oder 4- oder 5- oder 6-yl; 1,2,3,4-Tetrahydropyridin-1- oder 2- oder 3- oder 4- oder 5- oder 6-yl; 1,4-Dihydropyridin-1- oder 2- oder 3- oder 4-yl; 2,3-Dihydropyridin-2- oder 3- oder 4- oder 5- oder 6-yl; 2,5-Dihydropyridin-2- oder 3- oder 4- oder 5- oder 6-yl, 1- oder 2- oder 3- oder 4-Azepanyl; 2,3,4,5-Tetrahydro-1H-azepin-1- oder 2- oder 3- oder 4- oder 5- oder 6- oder 7-yl; 2,3,4,7-Tetrahydro-1H-azepin-1- oder 2- oder 3- oder 4- oder 5- oder 6- oder 7-yl; 2,3,6,7-Tetrahydro-1H-azepin-1- oder 2- oder 3- oder 4-yl; 3,4,5,6-Tetrahydro-2H-azepin-2- oder 3- oder 4- oder 5- oder 6- oder 7-yl; 4,5-Dihydro-1H-azepin-1- oder 2- oder 3- oder 4-yl; 2,5-Dihydro-1H-azepin-1- oder -2- oder 3- oder 4- oder 5- oder 6- oder 7-yl; 2,7-Dihydro-1H-azepin-1- oder -2- oder 3- oder 4-yl; 2,3-Dihydro-1H-azepin-1- oder -2- oder 3- oder 4- oder 5- oder 6- oder 7-yl; 3,4-Dihydro-2H-azepin-2- oder 3- oder 4- oder 5- oder 6- oder 7-yl; 3,6-Dihydro-2H-azepin-2- oder 3- oder 4- oder 5- oder 6- oder 7-yl; 5,6-Dihydro-2H-azepin-2- oder 3- oder 4- oder 5- oder 6- oder 7-yl; 4,5-Dihydro-3H-azepin-2- oder 3- oder 4- oder 5- oder 6- oder 7-yl; 1H-Azepin-1- oder -2- oder 3- oder 4- oder 5- oder 6- oder 7-yl; 2H-Azepin-2- oder 3- oder 4- oder 5- oder 6- oder 7-yl; 3H-Azepin-2- oder 3- oder 4- oder 5- oder 6- oder 7-yl; 4H-Azepin-2- oder 3- oder 4- oder 5- oder 6- oder 7-yl, 2- oder 3-Oxolanyl (= 2- oder 3-Tetrahydrofuranyl); 2,3-Dihydrofuran-2- oder 3- oder 4- oder 5-yl; 2,5-Dihydrofuran-2- oder 3-yl, 2- oder 3- oder 4-Oxanyl (= 2- oder 3- oder 4-Tetrahydropyranyl); 3,4-Dihydro-2H-pyran-2- oder 3- oder 4- oder 5- oder 6-yl; 3,6-Dihydro-2H-pyran-2- oder 3-oder 4- oder 5- oder 6-yl; 2H-Pyran-2- oder 3- oder 4- oder 5- oder 6-yl; 4H-Pyran-2- oder 3- oder 4-yl, 2- oder 3- oder 4-Oxepanyl; 2,3,4,5-Tetrahydrooxepin-2- oder 3- oder 4- oder 5- oder 6- oder 7-yl; 2,3,4,7-Tetrahydrooxepin-2- oder 3- oder 4- oder 5- oder 6- oder 7-yl; 2,3,6,7-Tetrahydrooxepin-2- oder 3- oder 4-yl; 2,3-Dihydrooxepin-2- oder 3- oder 4- oder 5- oder 6- oder 7-yl; 4,5-Dihydrooxepin-2- oder 3- oder 4-yl; 2,5-Dihydrooxepin-2- oder 3- oder 4- oder 5- oder 6- oder 7-yl; Oxepin-2- oder 3- oder 4- oder 5- oder 6- oder 7-yl; 2- oder 3-Tetrahydrothiophenyl; 2,3-Dihydrothiophen-2- oder 3- oder 4- oder 5-yl; 2,5-Dihydrothiophen-2- oder 3-yl; Tetrahydro-2H-thiopyran-2- oder 3- oder 4-yl; 3,4-Dihydro-2H-thiopyran-2- oder 3- oder 4- oder 5- oder 6-yl; 3,6-Dihydro-2H-thiopyran-2- oder 3- oder 4- oder 5- oder 6-yl; 2H-Thiopyran-2- oder 3- oder 4- oder 5- oder 6-yl; 4H-Thiopyran-2- oder 3- oder 4-yl. Bevorzugte 3-Ring und 4-Ring-Heterocyclen sind beispielsweise 1- oder 2-Aziridinyl, Oxiranyl, Thiiranyl, 1- oder 2- oder 3-Azetidinyl, 2- oder 3-Oxetanyl, 2- oder 3-Thietanyl, 1,3-Dioxetan-2-yl. Weitere Beispiele für "Heterocyclyl" sind ein partiell oder vollständig hydrierter heterocyclischer Rest mit zwei Heteroatomen aus der Gruppe N, O und S, wie beispielsweise 1- oder 2- oder 3- oder 4-Pyrazolidinyl; 4,5-Dihydro-3H-pyrazol- 3- oder 4- oder 5-yl; 4,5-Dihydro-1H-pyrazol-1- oder 3- oder 4- oder 5-yl; 2,3-Dihydro-1H-pyrazol-1- oder 2- oder 3- oder 4- oder 5-yl; 1- oder 2- oder 3- oder 4- Imidazolidinyl; 2,3-Dihydro-1H-imidazol-1- oder 2- oder 3- oder 4-yl; 2,5-Dihydro-1H-imidazol-1- oder 2- oder 4- oder 5-yl; 4,5-Dihydro-1H-imidazol-1- oder 2- oder 4- oder 5-yl; Hexahydropyridazin-1- oder 2- oder 3- oder 4-yl; 1,2,3,4-Tetrahydropyridazin-1- oder 2- oder 3- oder 4- oder 5- oder 6-yl; 1,2,3,6-Tetrahydropyridazin-1- oder 2- oder 3- oder 4- oder 5- oder 6-yl; 1,4,5,6-Tetrahydropyridazin-1- oder 3- oder 4- oder 5- oder 6-yl; 3,4,5,6-Tetrahydropyridazin-3- oder 4- oder 5-yl; 4,5-Dihydropyridazin-3- oder 4-yl; 3,4-Dihydropyridazin-3- oder 4- oder 5- oder 6-yl; 3,6-Dihydropyridazin-3- oder 4-yl; 1,6-Dihydropyriazin-1- oder 3- oder 4- oder 5- oder 6-yl; Hexahydropyrimidin-1- oder 2- oder 3- oder 4-yl; 1,4,5,6-Tetrahydropyrimidin-1- oder 2- oder 4- oder 5- oder 6-yl; 1,2,5,6-Tetrahydropyrimidin-1- oder 2- oder 4- oder 5- oder 6-yl; 1,2,3,4-Tetrahydropyrimidin-1- oder 2- oder 3- oder 4- oder 5- oder 6-yl; 1,6-Dihydropyrimidin-1- oder 2- oder 4- oder 5- oder 6-yl; 1,2-Dihydropyrimidin-1- oder 2- oder 4- oder 5- oder 6-yl; 2,5-Dihydropyrimidin-2- oder 4- oder 5-yl; 4,5-Dihydropyrimidin- 4- oder 5- oder 6-yl; 1,4-Dihydropyrimidin-1- oder 2- oder 4- oder 5- oder 6-yl; 1- oder 2- oder 3-Piperazinyl; 1,2,3,6-Tetrahydropyrazin-1- oder 2- oder 3- oder 5- oder 6-yl; 1,2,3,4-Tetrahydropyrazin-1- oder 2- oder 3- oder 4- oder 5- oder 6-yl; 1,2-Dihydropyrazin-1- oder 2- oder 3- oder 5- oder 6-yl; 1,4-Dihydropyrazin-1- oder 2- oder 3-yl; 2,3-Dihydropyrazin-2- oder 3- oder 5- oder 6-yl; 2,5-Dihydropyrazin-2- oder 3-yl; 1,3-Dioxolan-2- oder 4- oder 5-yl; 1,3-Dioxol-2- oder 4-yl; 1,3-Dioxan-2- oder 4- oder 5-yl; 4H-1,3-Dioxin-2- oder 4- oder 5- oder 6-yl; 1,4-Dioxan-2- oder 3- oder 5- oder 6-yl; 2,3-Dihydro-1,4-dioxin-2- oder 3- oder 5- oder 6-yl; 1,4-Dioxin-2- oder 3-yl; 1,2-Dithiolan-3- oder 4-yl; 3H-1,2-Dithiol-3- oder 4- oder 5-yl; 1,3-Dithiolan-2- oder 4-yl; 1,3-Dithiol-2- oder 4-yl; 1,2-Dithian-3- oder 4-yl; 3,4-Dihydro-1,2-dithiin-3- oder 4- oder 5- oder 6-yl; 3,6-Dihydro-1,2-dithiin-3- oder 4-yl; 1,2-Dithiin-3- oder 4-yl; 1,3-Dithian-2- oder 4- oder 5-yl; 4H-1,3-Dithiin-2- oder 4- oder 5- oder 6-yl; Isoxazolidin-2- oder 3- oder 4- oder 5-yl; 2,3-Dihydroisoxazol-2- oder 3- oder 4- oder 5-yl; 2,5-Dihydroisoxazol-2- oder 3- oder 4- oder 5-yl; 4,5-Dihydroisoxazol-3- oder 4- oder 5-yl; 1,3-Oxazolidin-2- oder 3- oder 4- oder 5-yl; 2,3-Dihydro-1,3-oxazol-2- oder 3- oder 4- oder 5-yl; 2,5-Dihydro-1,3-oxazol-2- oder 4- oder 5-yl; 4,5-Dihydro-1,3-oxazol-2- oder 4- oder 5-yl; 1,2-Oxazinan-2- oder 3- oder 4- oder 5- oder 6-yl; 3,4-Dihydro-2H-1,2-oxazin-2- oder 3- oder 4- oder 5- oder 6-yl; 3,6-Dihydro-2H-1,2-oxazin-2- oder 3- oder 4- oder 5- oder 6-yl; 5,6-Dihydro-2H-1,2-oxazin-2- oder 3- oder 4- oder 5- oder 6-yl; 5,6-Dihydro-4H-1,2-oxazin-3- oder 4- oder 5- oder 6-yl; 2H-1,2-Oxazin-2- oder 3- oder 4- oder 5- oder 6-yl; 6H-1,2-Oxazin-3- oder 4- oder 5- oder 6-yl; 4H-1,2-Oxazin-3- oder 4- oder 5- oder 6-yl; 1,3-Oxazinan-2- oder 3- oder 4- oder 5- oder 6-yl; 3,4-Dihydro-2H-1,3-oxazin-2- oder 3- oder 4- oder 5- oder 6-yl; 3,6-Dihydro-2H-1,3-oxazin-2- oder 3- oder 4- oder 5- oder 6-yl; 5,6-Dihydro-2H-1,3-oxazin-2- oder 4- oder 5- oder 6-yl; 5,6-Dihydro-4H-1,3-oxazin-2- oder 4- oder 5- oder 6-yl; 2H-1,3-Oxazin-2- oder 4- oder 5- oder 6-yl; 6H-1,3-Oxazin-2- oder 4- oder 5- oder 6-yl; 4H-1,3-Oxazin-2- oder 4- oder 5- oder 6-yl; Morpholin-2- oder 3- oder 4-yl; 3,4-Dihydro-2H-1,4-oxazin-2- oder 3- oder 4- oder 5- oder 6-yl; 3,6-Dihydro-2H-1,4-oxazin-2- oder 3- oder 5- oder 6-yl; 2H-1,4-oxazin-2- oder 3- oder 5- oder 6-yl; 4H-1,4-oxazin-2- oder 3-yl; 1,2-Oxazepan-2- oder 3- oder 4- oder 5- oder 6- oder 7-yl; 2,3,4,5-Tetrahydro-1,2-oxazepin-2- oder 3- oder 4- oder 5- oder 6- oder 7-yl; 2,3,4,7-Tetrahydro-1,2-oxazepin-2- oder 3- oder 4- oder 5- oder 6- oder 7-yl; 2,3,6,7-Tetrahydro-1,2-oxazepin-2- oder 3- oder 4- oder 5- oder 6- oder 7-yl; 2,5,6,7-Tetrahydro-1,2-oxazepin-2- oder 3- oder 4- oder 5- oder 6- oder 7-yl; 4,5,6,7-Tetrahydro-1,2-oxazepin-3- oder 4- oder 5- oder 6- oder 7-yl; 2,3-Dihydro-1,2-oxazepin-2- oder 3- oder 4- oder 5- oder 6- oder 7-yl; 2,5-Dihydro-1,2-oxazepin-2- oder 3- oder 4- oder 5- oder 6- oder 7-yl; 2,7-Dihydro-1,2-oxazepin-2- oder 3- oder 4- oder 5- oder 6- oder 7-yl; 4,5-Dihydro-1,2-oxazepin-3- oder 4- oder 5- oder 6- oder 7-yl; 4,7-Dihydro-1,2-oxazepin-3- oder 4- oder 5- oder 6- oder 7-yl; 6,7-Dihydro-1,2-oxazepin-3- oder 4- oder 5- oder 6- oder 7-yl; 1,2-Oxazepin-3- oder 4- oder 5- oder 6- oder 7-yl; 1,3-Oxazepan-2- oder 3- oder 4- oder 5- oder 6- oder 7-yl; 2,3,4,5-Tetrahydro-1,3-oxazepin-2- oder 3- oder 4- oder 5- oder 6- oder 7-yl; 2,3,4,7-Tetrahydro-1,3-oxazepin-2- oder 3- oder 4- oder 5- oder 6- oder 7-yl; 2,3,6,7-Tetrahydro-1,3-oxazepin-2- oder 3- oder 4- oder 5- oder 6- oder 7-yl; 2,5,6,7-Tetrahydro-1,3-oxazepin-2- oder 4- oder 5- oder 6- oder 7-yl; 4,5,6,7-Tetrahydro-1,3-oxazepin-2- oder 4- oder 5- oder 6- oder 7-yl; 2,3-Dihydro-1,3-oxazepin-2- oder 3- oder 4- oder 5- oder 6- oder 7-yl; 2,5-Dihydro-1,3-oxazepin-2- oder 4- oder 5- oder 6- oder 7-yl; 2,7-Dihydro-1,3-oxazepin-2- oder 4- oder 5- oder 6- oder 7-yl; 4,5-Dihydro-1,3-oxazepin-2- oder 4- oder 5- oder 6- oder 7-yl; 4,7-Dihydro-1,3-oxazepin-2- oder 4- oder 5- oder 6- oder 7-yl; 6,7-Dihydro-1,3-oxazepin-2- oder 4- oder 5- oder 6- oder 7-yl; 1,3-Oxazepin-2- oder 4- oder 5- oder 6- oder 7-yl; 1,4-Oxazepan-2- oder 3- oder 5- oder 6- oder 7-yl; 2,3,4,5-Tetrahydro-1,4-oxazepin-2- oder 3- oder 4- oder 5- oder 6- oder 7-yl; 2,3,4,7-Tetrahydro-1,4-oxazepin-2- oder 3- oder 4- oder 5- oder 6- oder 7-yl; 2,3,6,7-Tetrahydro-1,4-oxazepin-2- oder 3- oder 5- oder 6- oder 7-yl; 2,5,6,7-Tetrahydro-1,4-oxazepin-2- oder 3- oder 5- oder 6- oder 7-yl; 4,5,6,7-Tetrahydro-1,4-oxazepin-2- oder 3- oder 4- oder 5- oder 6- oder 7-yl; 2,3-Dihydro-1,4-oxazepin-2- oder 3- oder 5- oder 6- oder 7-yl; 2,5-Dihydro-1,4-oxazepin-2- oder 3- oder 5- oder 6- oder 7-yl; 2,7-Dihydro-1,4-oxazepin-2- oder 3- oder 5- oder 6- oder 7-yl; 4,5-Dihydro-1,4-oxazepin-2- oder 3- oder 4- oder 5- oder 6- oder 7-yl; 4,7-Dihydro-1,4-oxazepin-2- oder 3- oder 4- oder 5- oder 6- oder 7-yl; 6,7-Dihydro-1,4-oxazepin-2- oder 3- oder 5- oder 6- oder 7-yl; 1,4-Oxazepin-2- oder 3- oder 5- oder 6- oder 7-yl; Isothiazolidin-2- oder 3- oder 4- oder 5-yl; 2,3-Dihydroisothiazol-2- oder 3- oder 4- oder 5-yl; 2,5-Dihydroisothiazol-2- oder 3- oder 4- oder 5-yl; 4,5-Dihydroisothiazol-3- oder 4- oder 5-yl; 1,3-Thiazolidin-2- oder 3- oder 4- oder 5-yl; 2,3-Dihydro-1,3-thiazol-2- oder 3- oder 4- oder 5-yl; 2,5-Dihydro-1,3-thiazol-2- oder 4- oder 5-yl; 4,5-Dihydro-1,3-thiazol-2- oder 4- oder 5-yl; 1,3-Thiazinan-2- oder 3- oder 4- oder 5- oder 6-yl; 3,4-Dihydro-2H-1,3-thiazin-2- oder 3- oder 4- oder 5- oder 6-yl; 3,6-Dihydro-2H-1,3-thiazin-2- oder 3- oder 4- oder 5- oder 6-yl; 5,6-Dihydro-2H-1,3-thiazin-2- oder 4- oder 5- oder 6-yl; 5,6-Dihydro-4H-1,3-thiazin-2- oder 4- oder 5- oder 6-yl; 2H-1,3-Thiazin-2- oder 4- oder 5- oder 6-yl; 6H-1,3-Thiazin-2- oder 4- oder 5- oder 6-yl; 4H-1,3-Thiazin-2- oder 4- oder 5- oder 6-yl. Weitere Beispiele für "Heterocyclyl" sind ein partiell oder vollständig hydrierter heterocyclischer Rest mit 3 Heteroatomen aus der Gruppe N, O und S, wie beispielsweise 1,4,2-Dioxazolidin-2- oder 3- oder 5-yl; 1,4,2-Dioxazol-3- oder 5-yl; 1,4,2-Dioxazinan-2- oder -3- oder 5- oder 6-yl; 5,6-Dihydro-1,4,2-dioxazin-3- oder 5- oder 6-yl; 1,4,2-Dioxazin-3- oder 5- oder 6-yl; 1,4,2-Dioxazepan-2- oder 3- oder 5- oder 6- oder 7-yl; 6,7-Dihydro-5H-1,4,2-Dioxazepin-3- oder 5- oder 6- oder 7-yl; 2,3-Dihydro-7H-1,4,2-Dioxazepin-2- oder 3- oder 5- oder 6- oder 7-yl; 2,3-Dihydro-5H-1,4,2-Dioxazepin-2- oder 3- oder 5- oder 6- oder 7-yl; 5H-1,4,2-Dioxazepin-3- oder 5- oder 6- oder 7-yl; 7H-1,4,2-Dioxazepin-3- oder 5- oder 6- oder 7-yl. Strukturbeispiele für gegebenenfalls weiter substituierte Heterocyclen sind auch im Folgenden aufgeführt:

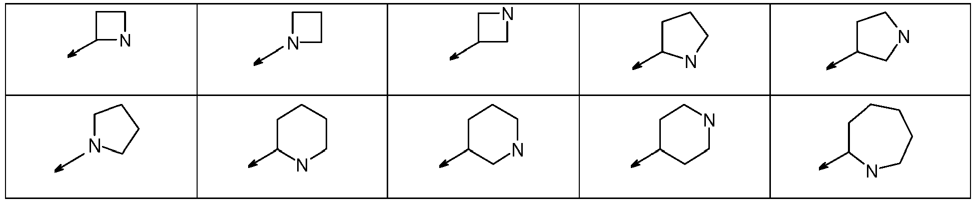

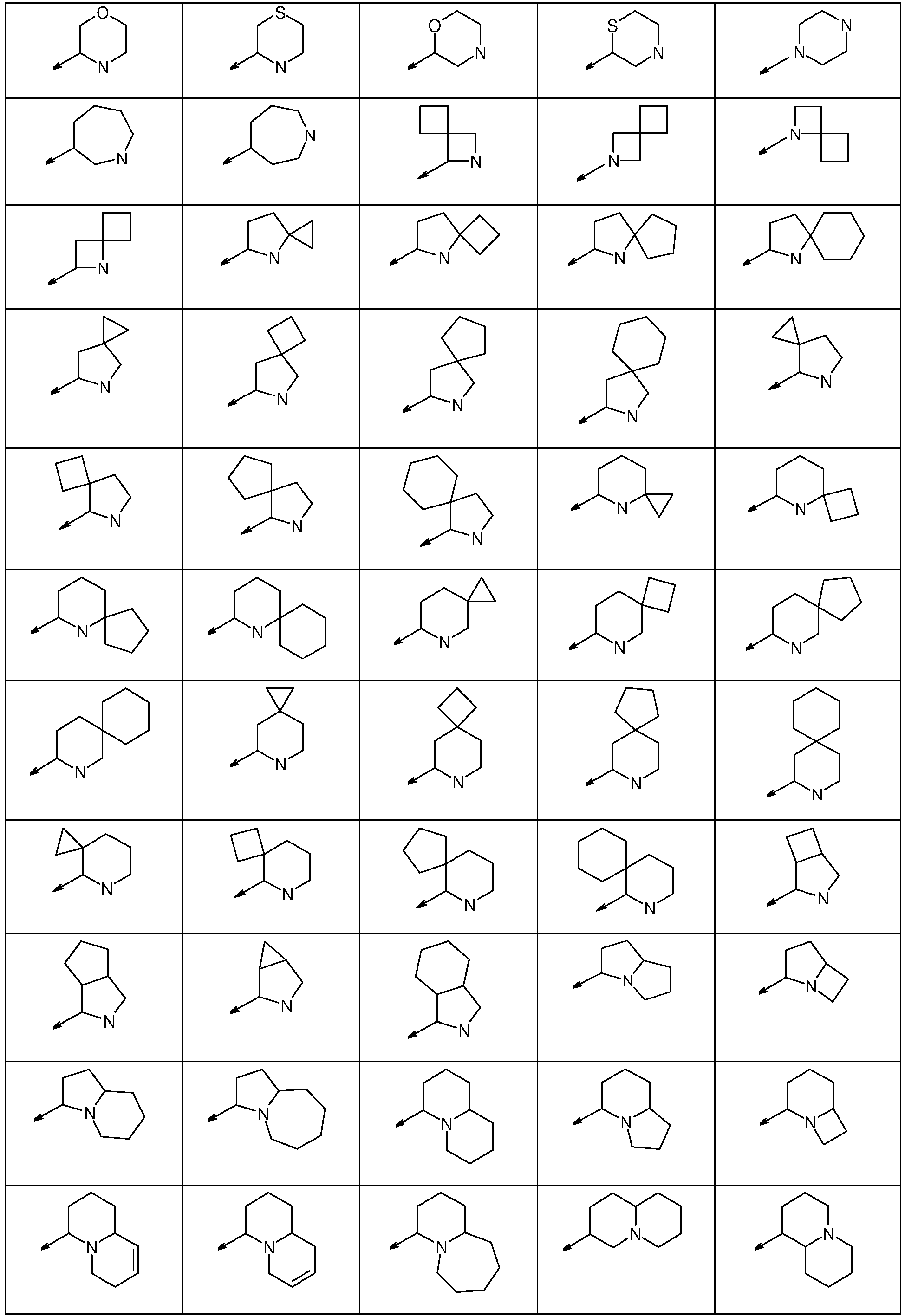

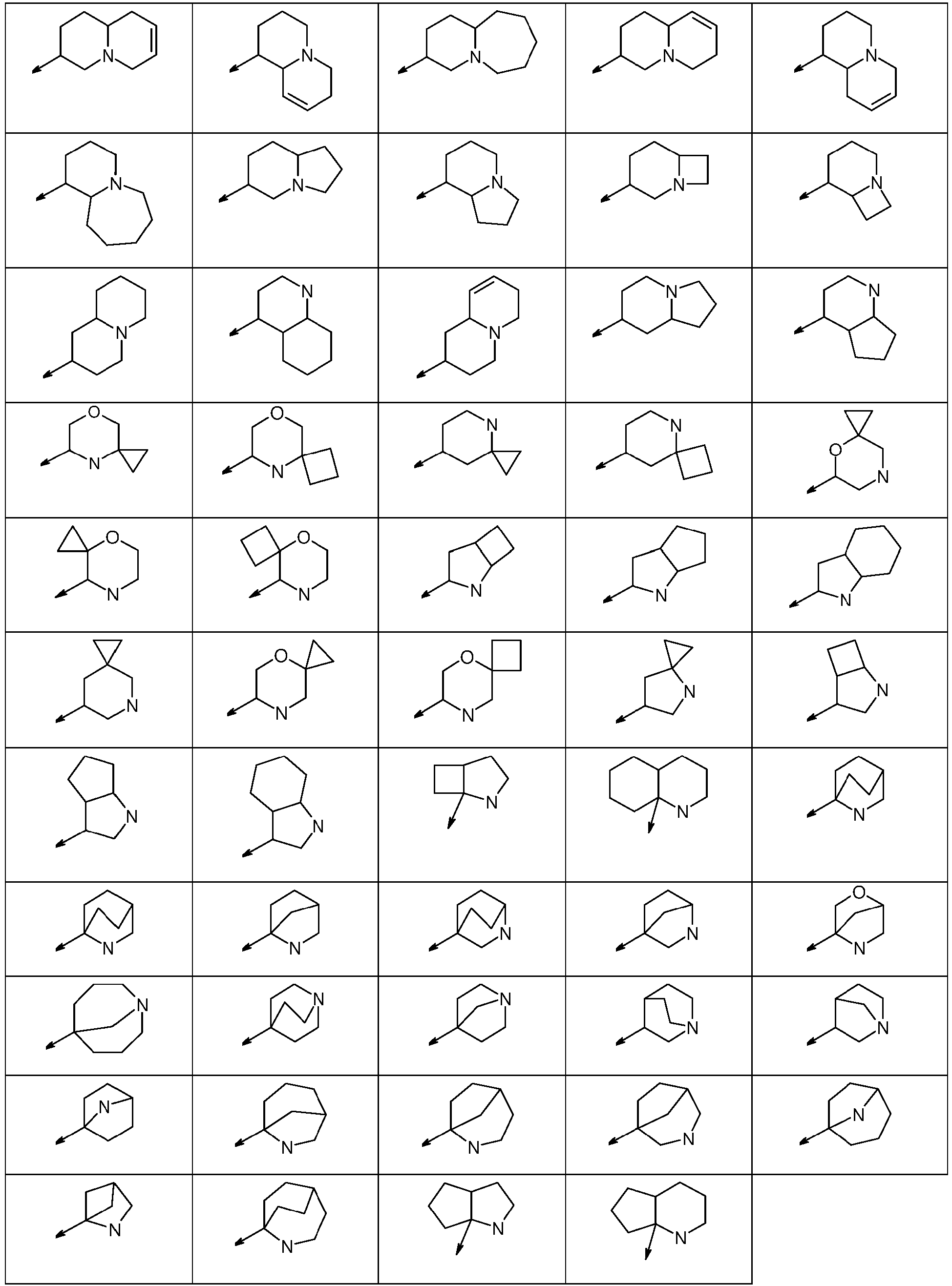

Die oben aufgeführten Heterocyclen sind bevorzugt beispielsweise durch Wasserstoff, Halogen, Alkyl, Haloalkyl, Hydroxy, Alkoxy, Cycloalkoxy, Aryloxy, Alkoxyalkyl, Alkoxyalkoxy, Cycloalkyl, Halocycloalkyl, Aryl, Arylalkyl, Heteroaryl, Heterocyclyl, Alkenyl, Alkylcarbonyl, Cycloalkylcarbonyl, Arylcarbonyl, Heteroarylcarbonyl, Alkoxycarbonyl, Hydroxycarbonyl, Cycloalkoxycarbonyl, Cycloalkylalkoxycarbonyl, Alkoxycarbonylalkyl, Arylalkoxycarbonyl, Arylalkoxycarbonylalkyl, Alkinyl, Alkinylalkyl, Alkylalkinyl, Tris-alkylsilylalkinyl, Nitro, Amino, Cyano, Haloalkoxy, Haloalkylthio, Alkylthio, Hydrothio, Hydroxyalkyl, Oxo, Heteroarylalkoxy, Arylalkoxy, Heterocyclylalkoxy, Heterocyclylalkylthio, Heterocyclyloxy, Heterocyclylthio, Heteroaryloxy, Bisalkylamino, Alkylamino, Cycloalkylamino, Hydroxycarbonylalkylamino, Alkoxycarbonylalkylamino, Arylalkoxycarbonylalkylamino, Alkoxycarbonylalkyl(alkyl)amino, Aminocarbonyl, Alkylaminocarbonyl, Bis-alkylaminocarbonyl, Cycloalkylaminocarbonyl, Hydroxycarbonylalkylaminocarbonyl, Alkoxycarbonylalkylaminocarbonyl, Arylalkoxycarbonylalkylaminocarbonyl substituiert.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Handelt es sich es sich um einen teilweise oder vollständig gesättigten Stickstoff-Heterocyclus, so kann dieser sowohl über Kohlenstoff als auch über den Stickstoff mit dem Rest des Moleküls verknüpft sein.

Als Substituenten für einen substituierten heterocyclischen Rest kommen die weiter unten genannten Substituenten in Frage, zusätzlich auch Oxo und Thioxo. Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen N(O) , S(O) (auch kurz SO) und S(O)₂ (auch kurz SO₂) im heterocyclischen Ring. Im Fall von -N(O)- und -S(O)-Gruppen sind jeweils beide Enantiomere umfasst.

Erfindungsgemäß steht der Ausdruck "Heteroaryl" für heteroaromatische Verbindungen, d. h. vollständig ungesättigte aromatische heterocyclische Verbindungen, vorzugsweise für 5- bis 7-gliedrige Ringe mit 1 bis 4, vorzugsweise 1, 2 oder 3 gleichen oder verschiedenen Heteroatomen, vorzugsweise O, S oder N. Erfindungsgemäße Heteroaryle sind beispielsweise 1H-Pyrrol-1-yl; 1H-Pyrrol-2-yl; 1H-Pyrrol-3-yl; Furan-2-yl; Furan-3-yl; Thien-2-yl; Thien-3-yl, 1H-Imidazol-1-yl; 1H-Imidazol-2-yl; 1H-Imidazol-4-yl; 1H-Imidazol-5-yl; 1H-Pyrazol-1-yl; 1H-Pyrazol-3-yl; 1H-Pyrazol-4-yl; 1H-Pyrazol-5-yl, 1H-1,2,3-Triazol-1-yl, 1H-1,2,3-Triazol-4-yl, 1H-1,2,3-Triazol-5-yl, 2H-1,2,3-Triazol-2-yl, 2H-1,2,3-Triazol-4-yl, 1H-1,2,4-Triazol-1-yl, 1H-1,2,4-Triazol-3-yl, 4H-1,2,4-Triazol-4-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, Azepinyl, Pyridin-2-yl, Pyridin-3-yl, Pyridin-4-yl, Pyrazin-2-yl, Pyrazin-3-yl, Pyrimidin-2-yl, Pyrimidin-4-yl, Pyrimidin-5-yl, Pyridazin-3-yl, Pyridazin-4-yl, 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl, 1,2,4-, 1,3,2-, 1,3,6- und 1,2,6-Oxazinyl, Isoxazol-3-yl, Isoxazol-4-yl, Isoxazol-5-yl, 1,3-Oxazol-2-yl, 1,3-Oxazol-4-yl, 1,3-Oxazol-5-yl, Isothiazol-3-yl, Isothiazol-4-yl, Isothiazol-5-yl, 1,3-Thiazol-2-yl, 1,3-Thiazol-4-yl, 1,3-Thiazol-5-yl, 1,3,4-Thiadiazol-2-yl, 1,3,4-Thiadiazol-5-yl, 1,2,4-Thiadiazol-5-yl, 1,2,4-Thiadiazol-3-yl, 1,2,5-Thiadiazol-3-yl, 1,2,5-Thiadiazol-3-yl, 1,2,3-Thiadiazol-4-yl, 1,2,3-Thiadiazol-5-yl, Oxepinyl, Thiepinyl, 1,2,4-Triazolonyl und 1,2,4-Diazepinyl, 2H-1,2,3,4-Tetrazol-5-yl, 1H-1,2,3,4-Tetrazol-5-yl, 1,2,3,4-Oxatriazol-5-yl, 1,2,3,4-Thiatriazol-5-yl, 1,2,3,5-Oxatriazol-4-yl, 1,2,3,5-Thiatriazol-4-yl. Die erfindungsgemäßen Heteroarylgruppen können ferner mit einem oder mehreren, gleichen oder verschiedenen Resten substituiert sein. Sind zwei benachbarte Kohlenstoffatome Bestandteil eines weiteren aromatischen Rings, so handelt es sich um annellierte heteroaromatische Systeme, wie benzokondensierte oder mehrfach annellierte Heteroaromaten. Bevorzugt sind beispielsweise Chinoline (z. B. Chinolin-2-yl, Chinolin-3-yl, Chinolin-4-yl, Chinolin-5-yl, Chinolin-6-yl, Chinolin-7-yl, Chinolin-8-yl); Isochinoline (z. B. Isochinolin-1-yl, Isochinolin-3-yl, Isochinolin-4-yl, Isochinolin-5-yl, Isochinolin-6-yl, Isochinolin-7-yl, Isochinolin-8-yl); Chinoxalin; Chinazolin; Cinnolin; 1,5-Naphthyridin; 1,6-Naphthyridin; 1,7-Naphthyridin; 1,8-Naphthyridin; 2,6-Naphthyridin; 2,7-Naphthyridin; Phthalazin; Pyridopyrazine; Pyridopyrimidine; Pyridopyridazine; Pteridine; Pyrimidopyrimidine. Beispiele für Heteroaryl sind auch 5- oder 6-gliedrige benzokondensierte Ringe aus der Gruppe 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 2H-Indazol-3-yl, 2H-Indazol-4-yl, 2H-Indazol-5-yl, 2H-Indazol-6-yl, 2H-Indazol-7-yl, 2H-Isoindol-2-yl, 2H-Isoindol-1-yl, 2H-Isoindol-3-yl, 2H-Isoindol-4-yl, 2H-Isoindol-5-yl, 2H-Isoindol-6-yl; 2H-Isoindol-7-yl, 1H-Benzimidazol-1-yl, 1H-Benzimidazol-2-yl, 1H-Benzimidazol-4-yl, 1H-Benzimidazol-5-yl, 1H-Benzimidazol-6-yl, 1H-Benzimidazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, 1,3-Benzthiazol-2-yl, 1,3-Benzthiazol-4-yl, 1,3-Benzthiazol-5-yl, 1,3-Benzthiazol-6-yl, 1,3-Benzthiazol-7-yl, 1,2-Benzisoxazol-3-yl, 1,2-Benzisoxazol-4-yl, 1,2-Benzisoxazol-5-yl, 1,2-Benzisoxazol-6-yl, 1,2-Benzisoxazol-7-yl, 1,2-Benzisothiazol-3-yl, 1,2-Benzisothiazol-4-yl, 1,2-Benzisothiazol-5-yl, 1,2-Benzisothiazol-6-yl, 1,2-Benzisothiazol-7-yl.

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder Iodatom.

Erfindungsgemäß bedeutet "Alkyl" einen geradkettigen oder verzweigten offenkettigen, gesättigten Kohlenwasserstoffrest, der gegebenenfalls ein- oder mehrfach substituiert ist und im letzteren Falle als "substituiertes Alkyl" bezeichnet wird. Bevorzugte Substituenten sind Halogenatome, Alkoxy-, Haloalkoxy-, Cyano-, Alkylthio, Haloalkylthio-, Cyloalkyl-, Alkoxycarbonyl-, Hydrocycarbonyl-, Heterocyclyl-, Hetaryl-, Aryl-, Amino- oder Nitrogruppen, besonders bevorzugt sind Methoxy, Methyl, Fluoralkyl, Cyano, Nitro, Fluor, Chlor, Brom oder Iod. Die Vorsilbe "Bis" schließt auch die Kombination unterschiedlicher Alkylreste ein, z. B. Methyl(Ethyl) oder Ethyl(Methyl).

"Haloalkyl", "-alkenyl" und "-alkinyl" bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie z. B. CH₂CH₂Cl, CH₂CH₂Br, CHClCH₃, CH₂Cl, CH₂F; Perhaloalkyl wie z. B. CCl₃, CClF₂, CFCl₂,CF₂CClF₂, CF₂CClFCF₃; Polyhaloalkyl wie z. B. CH₂CHFCl, CF₂CClFH, CF₂CBrFH, CH₂CF₃; Der Begriff Perhaloalkyl umfasst dabei auch den Begriff Perfluoralkyl.

"Haloalkoxy" ist z.B. OCF₃, OCHF₂, OCH₂F, OCF₂CF₃, OCH₂CF₃ und OCH₂CH₂Cl; Entsprechendes gilt für Haloalkenyl und andere durch Halogen substituierten Reste.

Der hier beispielhaft genannte Ausdruck "(C₁-C₄)-Alkyl" bedeutet eine Kurzschreibweise für geradkettiges oder verzweigtes Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)-Alkyl", umfassen entsprechend auch geradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, wobei mindestens eine Doppelbindung bzw. Dreifachbindung enthalten ist. Bevorzugt sind Reste mit einer Doppelbindung bzw. Dreifachbindung.

Der Begriff "Alkenyl" schließt insbesondere auch geradkettige oder verzweigte offenkettige Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl. Alkenyl bedeutet z.B. Vinyl, welches ggf. durch weitere Alkylreste substituiert sein kann, z B. (aber nicht beschränkt auf) (C₂-C₆)-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Der Begriff "Alkinyl" schließt insbesondere auch geradkettige oder verzweigte offenkettige Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl. (C₂-C₆)-Alkinyl bedeutet z.B. Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Di-methyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Der Begriff "Cycloalkyl" bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl, das gegebenenfalls weiter substituiert ist, bevorzugt durch Wasserstoff, Alkyl, Alkoxy, Cyano, Nitro, Alkylthio, Haloalkylthio, Halogen, Alkenyl, Alkinyl, Haloalkyl, AMino, Alkylamino, Bisalkylamino, Alkocycarbonyl, Hydroxycarbonyl, Arylalkoxycarbonyl, Aminocarbonyl, Alkylaminocarbonyl, Cycloalkylaminocarbonyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfasst, wie beispielsweise Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl und Adamantan-2-yl, aber auch Systeme wie z. B. 1,1'-Bi(cyclopropyl)-1-yl, 1,1'-Bi(cyclopropyl)-2-yl. Der Ausdruck "(C₃-C₇)-Cycloalkyl" bedeutet eine Kurzschreibweise für Cycloalkyl mit drei bis 7 Kohlenstoffatomen entsprechend der Bereichsangabe für C-Atome.

Im Falle von substituiertem Cycloalkyl werden auch spirocyclische aliphatische Systeme umfasst, wie beispielsweise Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl.

"Cycloalkenyl" bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Der Begriff "Alkyliden", z. B. auch in der Form (C₁-C₁₀)-Alkyliden, bedeutet den Rest eines geradkettigen oder verzweigten offenkettigen Kohlenwasserstoffrests, der über eine Zweifachbindung gebunden ist. Als Bindungsstelle für Alkyliden kommen naturgemäß nur Positionen am Grundkörper in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅. Cycloalkyliden bedeutet ein carbocyclischer Rest, der über eine Zweifachbindung gebunden ist.

"Teilfluoriertes Alkyl" bedeutet einen geradkettigen oder verzweigten, gesättigten Kohlenwasserstoff, der einfach oder mehrfach durch Fluor substituiert ist, wobei sich die entsprechenden Fluoratome als Substituenten an einem oder mehreren verschiedenen Kohlenstoffatomen der geradkettigen oder verzweigten Kohlenwasserstoffkette befinden können, wie z. B. CHFCH₃, CH₂CH₂F, CH₂CH₂CF₃, CHF₂, CH₂F, CHFCF₂CF₃.

"Alkoxyalkyl" steht für einen über eine Alkylgruppe gebundenen Alkoxyrest und "Alkoxyalkoxy" bedeutet einen über ein Sauerstoffatom gebundenen Alkoxyalkylrest, z.B. (aber nicht beschränkt auf) Methoxymethoxy, Methoxyethoxy, Ethoxyethoxy, Methoxy-n-propyloxy.

"Arylalkyl" steht für einen über eine Alkylgruppe gebundenen Arylrest, "Heteroarylalkyl" bedeutet einen über eine Alkylgruppe gebundenen Heteroarylrest, und "Heterocyclylalkyl" bedeutet einen über eine Alkylgruppe gebundenen Heterocyclylrest.

"Cycloalkylalkyl" steht für einen über eine Alkylgruppe gebundenen Cycloalkylrest, z. B. (aber nicht beschränkt auf) Cyclopropylmethyl, Cyclobutylmethyl, Cyclopentylmethyl, Cyclohexylmethyl, 1-Cyclopropyleth-1-yl, 2-Cyclopropyleth-1-yl, 1-Cyclopropylprop-1-yl, 3-Cyclopropylprop-1-yl.

"Halocycloalkyl" bedeutet durch gleiche oder verschiedene Halogenatome, wie z. B. F, Cl und Br, oder durch Haloalkyl, wie z. B. Trifluormethyl oder Difluormethyl teilweise oder vollständig substituiertes Cycloalkyl, z.B. 1-Fluorcycloprop-1-yl, 2-Fluorcycloprop-1-yl, 2,2-Difluorcycloprop-1-yl, 1-Fluorcyclobut-1-yl, 1-Trifluormethylcycloprop-1-yl, 2-Trifluormethylcycloprop-1-yl, 1-Chlorcycloprop-1-yl, 2-Chlorcycloprop-1-yl, 2,2-Dichlorcycloprop-1-yl, 3,3-Difluorcyclobutyl.

Erfindungsgemäß steht "Haloalkylthio" - in Alleinstellung oder als Bestandteil einer chemischen Gruppe - für geradkettiges oder verzweigtes S-Halogenalkyl, vorzugsweise mit 1 bis 8, oder mit 1 bis 6 Kohlenstoffatomen, wie (C₁-C₈)-, (C₁-C₆)- oder (C₁-C₄)-Haloalkylthio, z.B. (aber nicht beschränkt auf) Trifluormethylthio, Pentafluorethylthio, Difluormethyl, 2,2-Difluoreth-1-ylthio, 2,2,2-Difluoreth-1-ylthio, 3,3,3-prop-1-ylthio.

Wenn die Verbindungen durch Wasserstoffverschiebung Tautomere bilden können, welche strukturell formal nicht durch die allgemeine Formel (I) erfasst würden, so sind diese Tautomere gleichwohl von der Definition der erfindungsgemäßen Verbindungen der allgemeinen Formel (I) umfasst, sofern nicht ein bestimmtes Tautomer Gegenstand der Betrachtung ist. So können beispielsweise viele Carbonylverbindungen sowohl in der Ketoform wie auch in der Enolform vorliegen, wobei beide Formen durch die Definition der Verbindung der allgemeinen Formel (I) umfasst werden.

Die Verbindungen der allgemeinen Formel (I) können je nach Art und Verknüpfung der Substituenten als Stereoisomere vorliegen. Die durch ihre spezifische Raumform definierten möglichen Stereoisomere, wie Enantiomere, Diastereomere, Z- und E-Isomere sind alle von der allgemeinen Formel (I) umfasst. Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden erhalten. Die chromatographische Trennung kann sowohl im analytischen Maßstab zur Feststellung des Enantiomerenüberschusses bzw. des Diastereomerenüberschusses, wie auch im präparativen Maßstab zur Herstellung von Prüfmustern für die biologische Ausprüfung erfolgen. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfasst, jedoch nicht mit ihrer spezifischen Stereoform angegeben sind, sowie deren Gemische.

Sofern die Verbindungen als Feststoffe erhalten werden, kann die Reinigung auch durch Umkristallisieren oder Digerieren erfolgen. Sofern einzelne Verbindungen (I) nicht auf den nachstehend beschriebenen Wegen zufriedenstellend zugänglich sind, können sie durch Derivatisierung anderer Verbindungen (I) hergestellt werden.

Als Isolierungs-, Reinigungs- und Stereoisomerenauftrennungsverfahren von Verbindungen der allgemeinen Formel (I) kommen Methoden in Frage, die dem Fachmann aus analogen Fällen allgemein bekannt sind, z.B. durch physikalische Verfahren wie Kristallisation, Chromatographieverfahren, vor allem Säulenchromatographie und HPLC (Hochdruckflüssigchromatographie), Destillation, gegebenenfalls unter reduziertem Druck, Extraktion und andere Verfahren, können gegebenfalls verbleibende Gemische in der Regel durch chromatographische Trennung, z.B. an chiralen Festphasen, getrennt werden. Für präparative Mengen oder im industriellen Maßstab kommen Verfahren in Frage wie Kristallisation, z.B. diastereomerer Salze, die aus den Diastereomerengemischen mit optisch aktiven Säuren und gegebenenfalls bei vorhandenen sauren Gruppen mit optisch aktiven Basen erhalten werden können.

### Synthese von [(1-Phenyl-5-(Heteroaryl)-1H-1,2,4-triazol-3-yl)oxy]essigsäure-Derivaten der allgemeinen Formel (I)

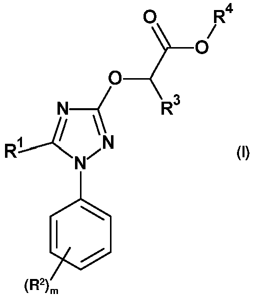

Die erfindungsgemäßen [(1-Phenyl-5-(Heteroaryl)-1H-1,2,4-triazol-3-yl)oxy]essigsäure-Derivate der allgemeinen Formel (I) können ausgehend von bekannten Verfahren hergestellt werden. Die eingesetzten und untersuchten Syntheserouten gehen dabei von kommerziell erhältlichen oder leicht herstellbaren substituierten Heteroarylcarbonsäuren, von entsprechend substituierten Heteroarylcarbonsäureamiden und von kommerziell erhältlichen Chemikalien wie substituierten Phenylhydrazinen und Diphenylcarbonat aus. Die Gruppierungen R¹, R², R³, R⁴, m und p der allgemeinen Formel (I) haben in den nachfolgenden Schemata die zuvor definierten Bedeutungen, sofern nicht beispielhafte, aber nicht einschränkende, Definitionen erfolgen.

Die Synthese der erfindungsgemäßen Verbindungen der allgemeinen Formel (Ia) erfolgt über eine Reaktion von der Verbindung der allgemeinen Formel (II) mit einer Verbindung der allgemeinen Formel (III) in Gegenwart einer Base, wie zum Beispiel Kaliumcarbonat. Die Reaktion findet bevorzugt in dem Temperaturbereich zwischen 0 °C und 120 °C, in einem adäquaten Lösungsmittel wie zum Beispiel Acetonitril statt (siehe Schema 1).

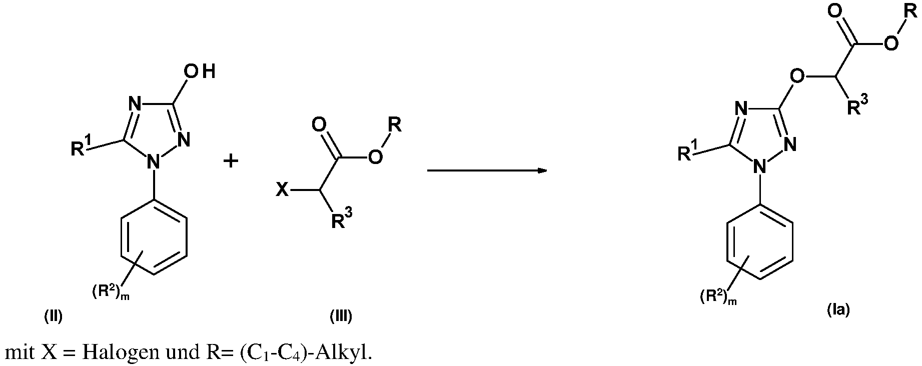

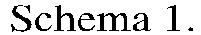

Die Synthese der Verbindungen der allgemeinen Formel (II) erfolgt über eine Zyklisierung einer Verbindung der allgemeinen Formel (IV) in Gegenwart eines Kondensationsreagenzes, wie zum Beispiel Polyphosphorsäure. Die Reaktion findet bevorzugt in dem Temperaturbereich zwischen 0 °C und 180 °C, in Substanz statt (siehe Schema 2).

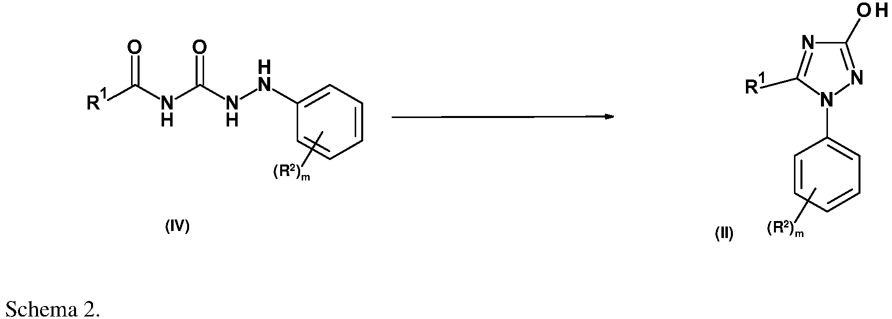

Die Synthese von Verbindungen der allgemeinen Formel (IV) lässt sich durch Reaktion der Verbindung der allgemeinen Formel (V) mit einem Phenylhydrazin der allgemeinen Formel (VI) in einem geeigneten Lösungsmittel, wie zum Beispiel Acetonitril, in einem Temperaturbereich zwischen -20 °C und 100 °C, vorzugsweise -5 °C und 50 °C herstellen. Die Reaktion findet in Gegenwart einer Base, wie zum Beispiel Triethylamin statt. Anstelle von Phenylhydrazinen der allgemeinen Formel (VI) können auch Phenylhydrazin Hydrohalogenide der allgemeinen Formel (VII) eingesetzt werden (Schema 3).

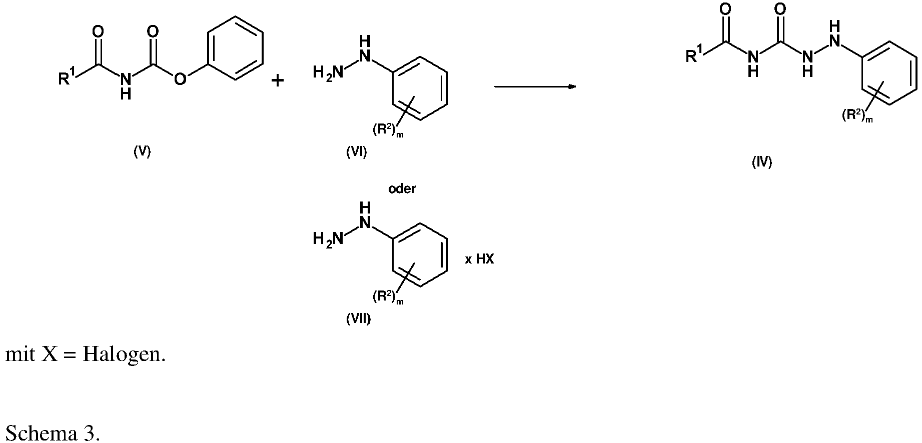

Die Synthese der Verbindung der allgemeinen Formel (V) lässt sich durch Reaktion der Verbindung der allgemeinen Formel (VIII) mit Diphenylcarbonat (IX) in Gegenwart einer Base, wie zum Beispiel Natriumhydrid, herstellen (siehe Schema 4). Die Reaktion findet bevorzugt in dem Temperaturbereich zwischen -20 °C und 150 °C in einem adäquaten Lösungsmittel wie zum Beispiel THF statt. Die Verbindungen der allgemeinen Formel (VIII) und (IX) sind kommerziell erhältlich oder lassen sich nach oder analog dem Fachmann bekannten Methoden herstellen.

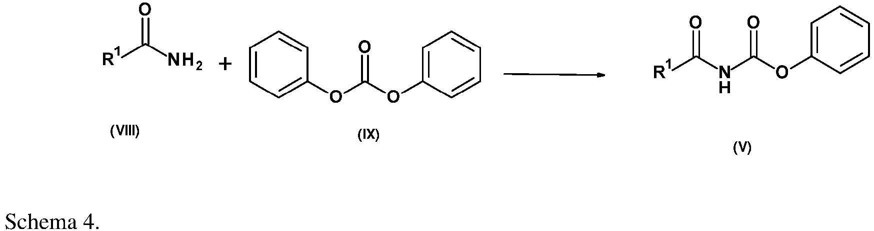

Die Synthese der Säure der allgemeinen Formel (X) lässt sich durch Verseifung der Verbindung der allgemeinen Formel (Ia) nach oder analog dem Fachmann bekannten Methoden herstellen.

Die Verseifung lässt sich in Gegenwart einer Base oder einer Lewis-Säure durchführen. Die Base kann ein Hydroxid-Salz von einem Alkali-Metall (wie zum Beispiel Lithium, Natrium oder Kalium; Schema 5) sein, und die Verseifungsreaktion findet bevorzugt in dem Temperaturbereich zwischen Raumtemperatur und 100 °C statt. Die Lewis-Säure kann Bortribromid sein, und die Reaktion in einem Temperaturbereich zwischen -20 °C und 100 °C, vorzugsweise -5 °C und 50 °C durchgeführt werden.

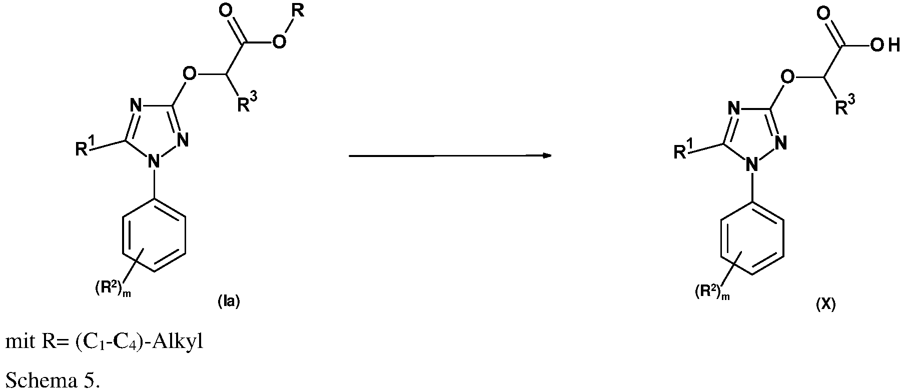

Die Synthese der erfindungsgemäßen Verbindungen der allgemeinen Formel (XI) erfolgt über eine Veresterung von einer Säure der allgemeinen Formel (X) mit einem Alkohol der allgemeinen Formel (XII) in Gegenwart eines Kupplungsreagenzes wie zum Beispiel T3P, Dicyclohexylcarbodiimid, *N*-(3-Dimethylaminopropyl)-N'-ethylcarbodiimid, *N*,*N*'-Cabonyldiimidazol, 2-Chlor-1,3-dimethyl-imidazolium chlorid oder 2-Chlor-1-methylpyridinium iodid (siehe Chemistry of Peptide Synthesis, Ed. N. Leo Benoiton, Taylor & Francis, 2006, ISBN-10: 1-57444-454-9). Polymergebundene Reagenzien, wie zum Beispiel polymergebundenes Dicyclohexylcarbodiimid, sind auch für diese Kupplungsreaktion geeignet. Die Reaktion findet bevorzugt in dem Temperaturbereich zwischen 0 °C und 80 °C, in einem adäquaten Lösungsmittel wie zum Beispiel Dichlormethan, Acetonitril, *N*,*N*-Dimethyl-formamid oder Ethylacetat und in Gegenwart eine Base wie zum Beispiel Triethylamin, N,N-Diisopropylethylamin oder 1,8-Diazabicyclo[5.4.0]undec-7-en statt (siehe Schema 6). Für die T3P Kupplungsbedingungen siehe Organic Process Research & Development 2009, 13, 900-906.

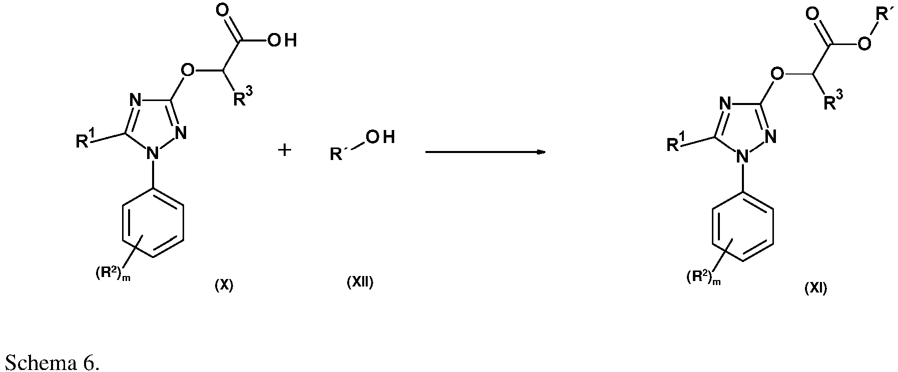

In Schema 7 wird die Synthese der Verbindung der allgemeinen Formel (II) dargestellt; die Synthese erfolgt durch Reaktion einer Verbindung der allgemeinen Formel (XIII) in Gegenwart einer Brönstedt-Säure, wie zum Beispiel 33%ige HBr in Essigsäure. Die Reaktion findet bevorzugt in dem Temperaturbereich zwischen 0 °C und 180 °C statt. Siehe Bioorganic & Medicinal Chemistry 2018, 26, 3321-3344.

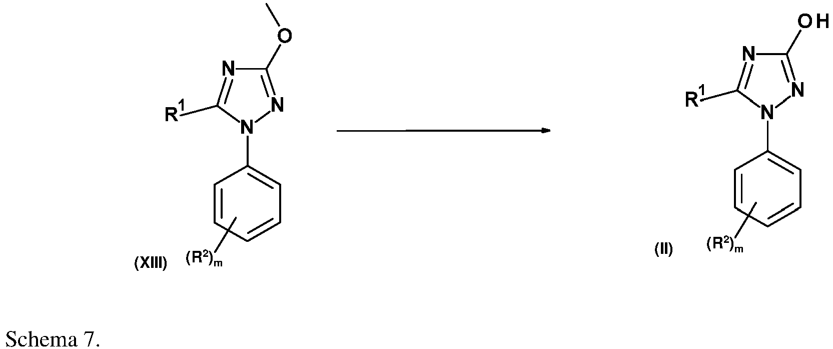

Verbindungen der allgemeinen Formel (XIII) lassen sich durch Reaktion einer Verbindung der allgemeinen Formel (XIV) und einem Phenylhydrazin der allgemeinen Formel (VI) in einem adäquaten Lösungsmittel wie zum Beispiel Ethanol herstellen (siehe Schema 8). Die Reaktion wird bevorzugt in dem Temperaturbereich zwischen 0 °C und 150 °C durchgeführt.

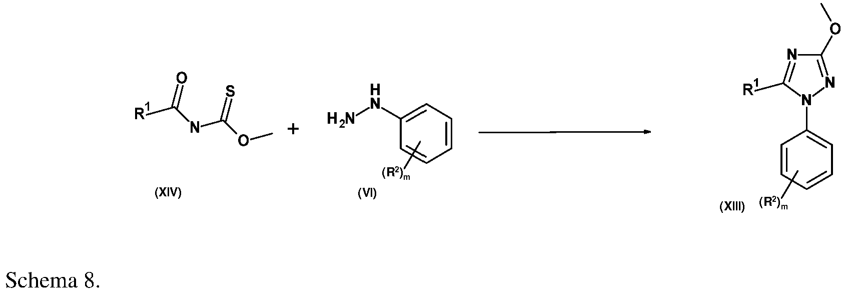

Die Verbindungen der allgemeinen Formel (XIV) lassen sich durch Reaktion von Heteroarylcarbonsäurecholriden der allgemeinen Formel (XV) mit einem Rhodanid-Salz der allgemeinen Formel (XVI) in Gegenwart von Methanol in einem adäquaten Lösungsmittel wie zum Beispiel Aceton herstellen (siehe Schema 9). Die Heteroarylcarbonsäurecholride sind entweder kommerziell erhältlich oder lassen sich nach oder analog dem Fachmann bekannten Methoden herstellen. Siehe Tetrahedron 1968, 24, 5205-5214; J. Chem. Soc. 1957, 1091; JP81 53,664 (1981); Justus Liebigs Ann. Chem. 1964, 675, 180 und J. heterocycl. Chem. 1983, 20, 1533.

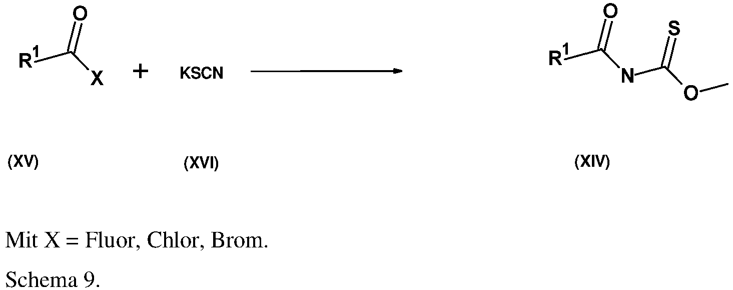

Ausgewählte detaillierte Synthesebeispiele für die erfindungsgemäßen Verbindungen der allgemeinen Formel (I) sind im Folgenden aufgeführt. Die angegebenen Beispielnummern entsprechen den in den nachstehenden Tabellen I.1 bis I.83 genannten Nummerierungen. Die ¹H-NMR-, ¹³C-NMR- und ¹⁹F-NMR-spektroskopischen Daten, die für die in den nachfolgenden Abschnitten beschriebenen chemischen Beispiele angegeben sind, (400 MHz bei ¹H-NMR und 150 MHz bei ¹³C-NMR und 375 MHz bei ¹⁹F-NMR, Lösungsmittel CDCl₃, CD₃OD oder d₆-DMSO, interner Standard: Tetramethylsilan δ = 0.00 ppm), wurden mit einem Gerät der Firma Bruker erhalten, und die bezeichneten Signale haben die nachfolgend aufgeführten Bedeutungen: br = breit(es); s = Singulett, d = Dublett, t = Triplett, dd = Doppeldublett, ddd = Dublett eines Doppeldubletts, m = Multiplett, q = Quartett, quint = Quintett, sext = Sextett, sept = Septett, dq = Doppelquartett, dt = Doppeltriplett. Bei Diastereomerengemischen werden entweder die jeweils signifikanten Signale beider Diastereomere oder das charakteristische Signal des Hauptdiastereomers angegeben. Die verwendeten Abkürzungen für chemische Gruppen haben beispielsweise die nachfolgenden Bedeutungen: Me = CH₃, Et = CH₂CH₃, t-Hex = C(CH₃)₂CH(CH₃)₂, t-Bu = C(CH₃)₃, n-Bu = unverzweigtes Butyl, n-Pr = unverzweigtes Propyl, i-Pr = verzweigtes Propyl, c-Pr = Cyclopropyl, c-Hex = Cyclohexyl.

### Synthesebeispiele:

### Synthesebeispiel No.: I.80-38

### Synthesestufe 1: 5-Chlorpyridin-2-carboxamid

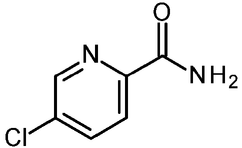

5-Chlorpyridin-2-carbonsäure (10 g, 63,47 mmol, 1,0 equiv) wurde in DCM (200 ml) suspendiert und bei Raumtemperatur tropfenweise mit Oxalylchlorid (11,07 ml, 126,94 mmol, 2,0 equiv) versetzt. Vorsicht Gasentwicklung! Anschließend erhitzte man das Reaktionsgemisch 1 h auf 40 °C, kühlte auf Raumtemperatur ab und entfernte das Lösungsmittel im Vakuum. Der Rückstand wurde in Ethylacetat (100 ml) gelöst und innerhalb von 15 min zu einer 35 %igen Ammoniumhydroxid-Lösung getropft (Vorsicht Gasentwicklung!). Danach wurde das Reaktionsgemisch 45 min bei Raumtemperatur gerührt. Der entstandene Feststoff wurde abgesaugt, mit Ethylacetat (100 ml) gewaschen und an der Luft getrocknet. Die vereinigten Muttelaugen wurden zweimal mit Ethylacetat extrahiert. Die vereinigten organischen Phasen wurden mit einer ges. Natriumchlorid-Lösung gewaschen, über Natriumsulfat getrocknet und das Lösungsmittel im Vakuum entfernt. Die beiden isolierten Feststoffe wurden vereinigt. 5-Chlorpyridin-2-carboxamid wurde in Form eines weißen Feststoffes isoliert (11,51 g, 79 % der Theorie). ¹H-NMR (400 MHz, DMSO-d⁶ δ, ppm) 8.68 (bs, 1H), 8.18-8.10 (m, 2H), 8.05 (m, 1H), 7.73 (bs, 1H).

### Synthesestufe 2: Phenyl-[(5-chlorpyridin-2-yl)carbonyl]carbamat

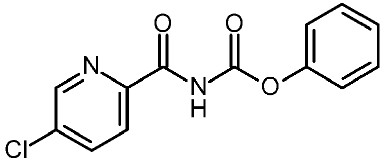

5-Chlorpyridin-2-carboxamid (2,50 g, 15,97 mmol, 1,0 equiv) und Diphenylcarbonat (5,13 g, 23,95 mmol, 1,5 equiv) wurden in THF (50 ml) unter Argonatmosphäre gelöst und mit einem Eisbad auf 0 °C abgekühlt. Die Lösung versetzte man portionsweise mit Natriumhydrid (60 %ig in Mineralöl, 0,64 g, 63,65 mmol, 1,0 equiv). Vorsicht Gasentwicklung! Anschließend entfernt man das Eisbad und rührt das Reaktionsgemisch 1h bei Raumtemperatur. Danach wurde das Reaktionsgemisch im Vakuum auf 1/3 konzentriert, wobei ein weißer Feststoff ausflockte. Der entstandene Feststoff wurde abgesaugt und an der Luft getrocknet. Phenyl-[(5-chlorpyridin-2-yl)carbonyl]carbamat wurde in Form eines weißen Feststoffes isoliert (3,94 g, 89 % der Theorie). ¹H-NMR (400 MHz, DMSO-d⁶ δ, ppm) 9.38 (bs, 1H), 8.68 (d, 1H), 8.13-8.03 (m, 2H), 7.17-7.13 (m, 2H), 6.77-6.74 (m, 3H).

### Synthesestufe 3: 5-Chlor-N-{[2-(2,4-difluorphenyl)hydrazino]carbonyl}pyridin-2-carboxamid

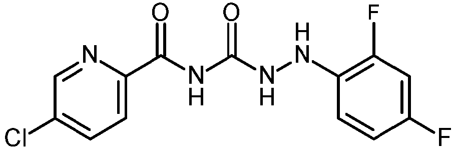

Phenyl-[(5-chlorpyridin-2-yl)carbonyl]carbamat (1,00 g, 3,61 mmol, 1,0 equiv) wurden in Acetonitril (50 ml) gelöst und anschließend bei Raumtemperatur mit (2,4-Difluorphenyl)hydrazinhydrochlorid (1:1) (0,72 g, 3,98 mmol, 1,1 equiv) und Triethylamin (1,51 ml, 10,84 mmol, 2,0 equiv) versetzt. Die Lösung verfärbte sich nach ca. 15-30 min rosa und es fiel ein beiger Feststoff aus. Das Reaktionsgemisch rührte man 1 h bei Raumtemperatur, filtrierte dann den entstandenen Niederschlag ab und trocknete diesen an der Luft. 5-Chlor-N-{ [2-(2,4-difluorphenyl)hydrazino]carbonyl}pyridin-2-carboxamid wurde in Form eines beigen Feststoffes isoliert (0,87 g, 66 % der Theorie, 90 %ig). ¹H-NMR (400 MHz, DMSO-d⁶ δ, ppm) 9.69 (bs, 1H), 8.80 (m, 1H), 8.25-8.10 (m, 4H), 7.83 (m, 1H), 7.16 (m, 1H), 6.92-6.86 (m, 1H).

### Synthesestufe 4: 5-(5-Chlorpyridin-2-yl)-1-(2,4-difluorphenyl)-1H-1,2,4-triazol-3-ol

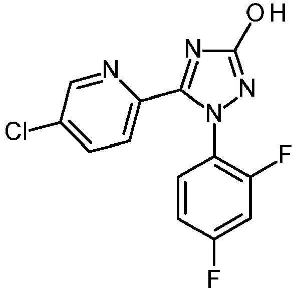

5-Chlor-N-{[2-(2,4-difluorphenyl)hydrazino]carbonyl}pyridin-2-carboxamid (0,4 g, 1,22 mmol, 1,0 equiv) wurden in Polyphosphorsäure (10 g) vermengt und anschließend 2 h bei 100 °C, wobei sich das Reaktionsgemisch verflüssigte. Nach dem Abkühlen auf Raumtemperatur tropfte man das Reaktionsgemisch in Eiswasser und saugte den hellbeigen Feststoff ab. Der abgesaugte Niederschlag trocknete man bei 55 °C im Vakuumtrockenschrank. 5-(5-Chlorpyridin-2-yl)-1-(2,4-difluorphenyl)-1H-1,2,4-triazol-3-ol wurde in Form eines hellbeigen Feststoffes isoliert (294 mg, 70 % der Theorie, 90 %ig). ¹H-NMR (400 MHz, CDCl₃ δ, ppm) 13.25 (bs, 1H), 8.32 (s, 1H), 8.05 (d, 1H), 7.82 (dd, 1H), 7.55 (m, 1H), 7.03 (m, 1H), 6.91 (m, 1H).

### Synthesestufe 5: Methyl-{[5-(5-chlorpyridin-2-yl)-1-(2,4-difluorphenyl)-1H-1,2,4-triazol-3-yl]oxy}acetat (Synthesebeispiel I.28-38)

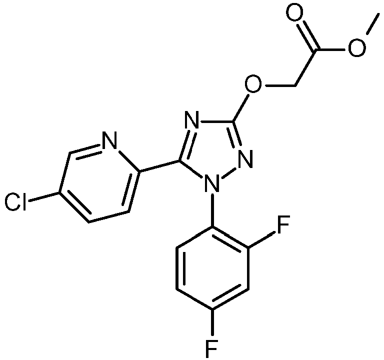

5-(5-Chlorpyridin-2-yl)-1-(2,4-difluorphenyl)-1H-1,2,4-triazol-3-ol (250 mg, 0,81 mmol, 1,0 equiv) und Kaliumcarbonat (336 mg, 2,43 mmol, 3 equiv) wurden in Acetonitril (25 ml) suspendiert und anschließend mit Bromessigsäuremethylester (149 mg, 0,97 mmol, 1,2 equiv) versetzt. Danach rührte man die Suspension über Nacht bei Raumtemperatur, filtrierte des Feststoff ab und engte das Reaktionsgemisch im Vakuum ein. Den Rückstand reinigte man durch Säulenchromatographie (Gradient Essigester/Heptan). Methyl-{[5-(5-chlorpyridin-2-yl)-1-(2,4-difluorphenyl)-1H-1,2,4-triazol-3-yl]oxy}acetat wurde in Form eines farblosen Öls isoliert (290 mg, 94 % der Theorie). ¹H-NMR (400 MHz, CDCl₃ δ, ppm) 8.26 (d, 1H), (.11 (dd, 1H), 7.76 (dd, 1H), 7.49 (m, 1H), 6.98 (m, 1H), 6.89 (m, 1H), 4.94 (s, 2H), 3.81 (s, 3H).

### Synthesestufe 6: {[5-(5-Chlorpyridin-2-yl)-1-(2,4-difluorphenyl)-1H-1,2,4-triazol-3-yl]oxy}essigsäure (Synthesebeispiel I.30-38)

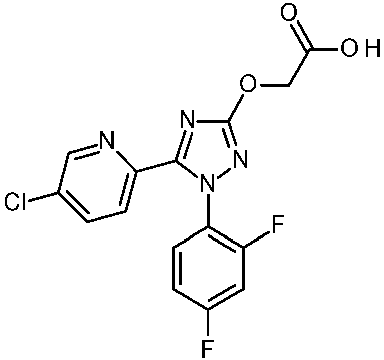

Methyl-{[5-(5-chlorpyridin-2-yl)-1-(2,4-difluorphenyl)-1H-1,2,4-triazol-3-yl]oxy}acetat (290 mg, 0,76 mmol, 1,0 equiv) und Lithiumhydroxid (55 mg, 2,29 mmol, 1 equiv) wurden in einem THF/Wasser Gemisch (7:2, 20 ml) gelöst und anschließend 1 h bei Raumtemperatur gerührt. Das Reaktionsgemisch engte man im Vakuum ein. Der Rückstand wurde in Wasser gelöst und mit 2 M Salzsäure auf pH = 2 eingestellt, wobei ein hellgelber Feststoff ausfällt. Der niederschlag wurde abgesaugt und bei 55 °C im Vakuumtrockenschrank getrocknet. { [5-(5-Chlorpyridin-2-yl)-1-(2,4-difluorphenyl)-1H-1,2,4-triazol-3-yl]oxy}essigsäure wurde in Form eines hellgelben Feststoffes isoliert (267 mg, 86 % der Theorie). ¹H-NMR (400 MHz, DMSO-d⁶ δ, ppm) 13.13 (bs, 1H), 8.45 (d, 1H), 8.14-8.07 (m, 2H), 7.69 (m, 1H), 7.50 (m, 1H), 7.25 (m, 1H), 4.88 (s, 2H).

### Synthesestufe 7: {[5-(5-Chlorpyridin-2-yl)-1-(2,4-difluorphenyl)-1H-1,2,4-triazol-3-yl]oxy } acetylchlorid

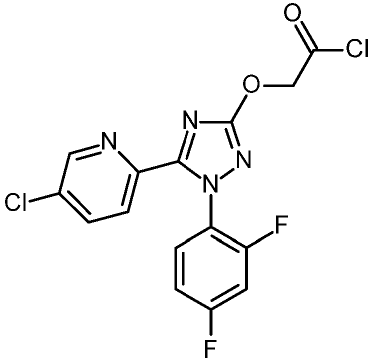

{[5-(5-Chlorpyridin-2-yl)-1-(2,4-difluorphenyl)-1H-1,2,4-triazol-3-yl]oxy}essigsäure (270 mg, 0,74 mmol, 1,0 equiv) und ein Tropfen DMF wurden in DCM (12 ml) gelöst und dann mit Oxalylchlorid (0,13 ml, 1,47 mmol, 2 equiv) versetzt (Vorsicht Gasentwicklung). Anschließend rührte man das Reaktionsgemisch bis zur Beendigung der Gasentwicklung bei 40 °C. Das Reaktionsgemisch engte man im Vakuum ein. Der Rückstand wurde ohne weitere Aufreinigung in der nächsten Synthesestufe eingesetzt. {[5-(5-Chlorpyridin-2-yl)-1-(2,4-difluorphenyl)-1H-1,2,4-triazol-3-yl]oxy}acetylchlorid wurde in Form eines gelben Öls isoliert (320 mg).

### Synthesestufe 8: Cyclopropylmethyl-{[5-(5-chlorpyridin-2-yl)-1-(2,4-difluorphenyl)-1H-1,2,4-triazol-3-yl]oxy}acetat (Synthesebeispiel I.80-38)

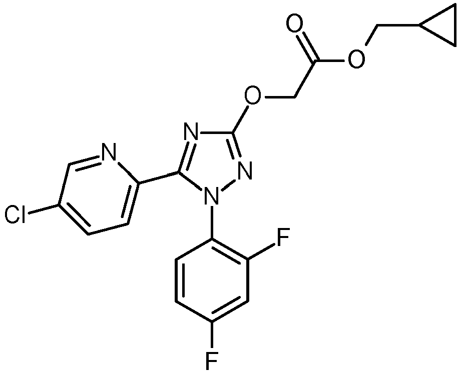

Cyclopropylmethanol (38 mg, 0,52 mmol, 2,50 equiv), Triethylamin (0,203 ml, 1,45 mmol, 7 equiv) und ein Körnchen DMAP wurden in DCM (6 ml) gelöst und anschließend mit {[5-(5-Chlorpyridin-2-yl)-1-(2,4-difluorphenyl)-1H-1,2,4-triazol-3-yl]oxy}acetylchlorid (250 mg, 0,81 mmol, 1,0 equiv) gelöst in DCM (1 ml) versetzt. Danach rührte man das Reaktionsgemisch über Nacht bei Raumtemperatur, versetzte mit Wasser (3 ml), trennte die organische Phase ab und trocknete diese über Natriumsulfat. Danach engte man die organische Phase im Vakuum ein. Den Rückstand reinigte man durch Säulenchromatographie (Gradient Essigester/Heptan). Cyclopropylmethyl-{ [5-(5-chlorpyridin-2-yl)-1-(2,4-difluorphenyl)-1H-1,2,4-triazol-3-yl]oxy}acetat wurde in Form eines farblosen Öls isoliert (42 mg, 45 % der Theorie). ¹H-NMR (400 MHz, CDCl₃ δ, ppm) 8.26 (s, 1H), 8.11 (d, 1H), 7.26 (dd, 1H), 7.48 (m, 1H), 6.98 (m, 1H), 6.89 (m, 1H), 4.95 (s, 2H), 4.05 (d, 2H), 1.15 (m, 1H), 0.57-0.52 (m, 2H), 0,30-0.26 (m, 2H).

In Analogie zu den oben angeführten und an entsprechender Stelle rezitierten Herstellungsbeispielen und unter Berücksichtigung der allgemeinen Angaben zur Herstellung von [(1-Phenyl-5-(Heteroaryl)-1H-1,2,4-triazol-3-yl)oxy]essigsäure-Derivaten erhält man die nachfolgend genannten Verbindungen:

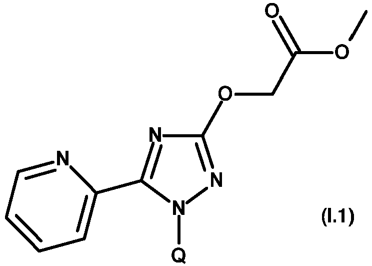

Tabelle I.1: Bevorzugte Verbindungen der Formel (I.1) sind die Verbindungen I.1-1 bis I.1-53, worin Q die in der jeweiligen Zeile angegebenen Bedeutungen der Tabelle 1 hat. Die Verbindungen I.1-1 bis I.1-53 der Tabelle 1.1 sind somit durch die Bedeutung der jeweiligen Einträge No. 1 bis 53 für Q der Tabelle 1 definiert.

**Tabelle 1:**

| No. | Q |
|---|---|
| 1 | Q-2.1 |
| 2 | Q-2.2 |
| 3 | Q-2.3 |
| 4 | Q-2.4 |
| 5 | Q-2.5 |
| 6 | Q-2.6 |
| 7 | Q-2.7 |
| 8 | Q-2.8 |
| 9 | Q-2.9 |
| 10 | Q-2.10 |
| 11 | Q-2.11 |
| 12 | Q-2.12 |
| 13 | Q-2.13 |
| 14 | Q-2.14 |
| 15 | Q-2.15 |
| 16 | Q-2.16 |
| 17 | Q-2.17 |
| 18 | Q-2.18 |
| 19 | Q-2.19 |
| 20 | Q-2.20 |
| 21 | Q-2.21 |
| 22 | Q-2.22 |
| 23 | Q-2.23 |
| 24 | Q-2.24 |
| 25 | Q-2.25 |
| 26 | Q-2.26 |
| 27 | Q-2.27 |
| 28 | Q-2.28 |
| 29 | Q-2.29 |
| 30 | Q-2.30 |
| 31 | Q-2.31 |
| 32 | Q-2.32 |
| 33 | Q-2.33 |
| 34 | Q-2.34 |
| 35 | Q-2.35 |
| 36 | Q-2.36 |
| 37 | Q-2.37 |
| 38 | Q-2.38 |
| 39 | Q-2.39 |
| 40 | Q-2.40 |
| 41 | Q-2.41 |
| 42 | Q-2.42 |
| 43 | Q-2.43 |
| 44 | Q-2.44 |
| 45 | Q-2.45 |
| 46 | Q-2.46 |
| 47 | Q-2.47 |
| 48 | Q-2.48 |
| 49 | Q-2.49 |
| 50 | Q-2.50 |
| 51 | Q-2.51 |
| 52 | Q-2.52 |
| 53 | Q-2.53 |

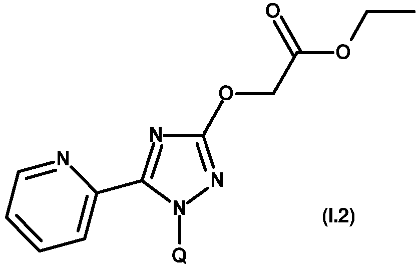

Tabelle I.2: Bevorzugte Verbindungen der Formel (I.2) sind die Verbindungen I.2-1 bis I.2-53, worin Q die in der jeweiligen Zeile angegebenen Bedeutungen der Tabelle 1 hat. Die Verbindungen I.2-1 bis I.2-53 der Tabelle I.2 sind somit durch die Bedeutung der jeweiligen Einträge No. 1 bis 53 für Q der Tabelle 1 definiert.

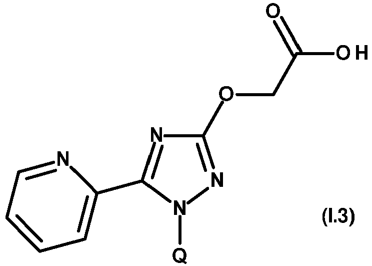

Tabelle I.3: Bevorzugte Verbindungen der Formel (I.3) sind die Verbindungen I.3-1 bis I.3-53, worin Q die in der jeweiligen Zeile angegebenen Bedeutungen der Tabelle 1 hat. Die Verbindungen I.3-1 bis I.3-53 der Tabelle I.3 sind somit durch die Bedeutung der jeweiligen Einträge No. 1 bis 53 für Q der Tabelle 1 definiert.

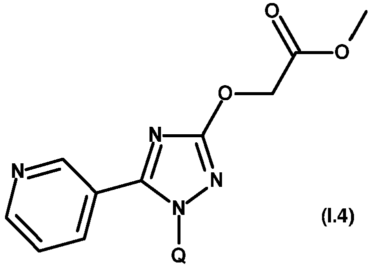

Tabelle I.4: Bevorzugte Verbindungen der Formel (I.4) sind die Verbindungen I.4-1 bis I.4-53, worin Q die in der jeweiligen Zeile angegebenen Bedeutungen der Tabelle 1 hat. Die Verbindungen I.4-1 bis I.4-53 der Tabelle I.4 sind somit durch die Bedeutung der jeweiligen Einträge No. 1 bis 53 für Q der Tabelle 1 definiert.

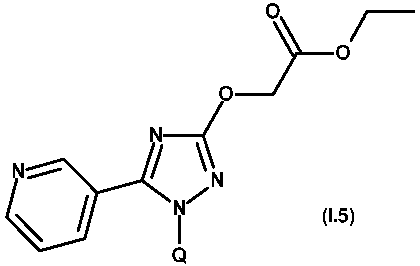

Tabelle I.5: Bevorzugte Verbindungen der Formel (I.5) sind die Verbindungen I.5-1 bis I.5-53, worin Q die in der jeweiligen Zeile angegebenen Bedeutungen der Tabelle 1 hat. Die Verbindungen I.5-1 bis I.5-53 der Tabelle I.5 sind somit durch die Bedeutung der jeweiligen Einträge No. 1 bis 53 für Q der Tabelle 1 definiert.

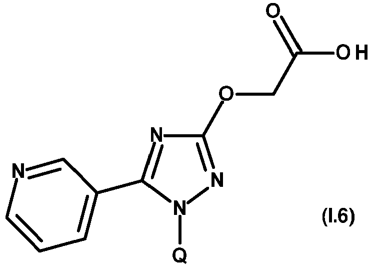

Tabelle I.6: Bevorzugte Verbindungen der Formel (I.6) sind die Verbindungen I.6-1 bis I.6-53, worin Q die in der jeweiligen Zeile angegebenen Bedeutungen der Tabelle 1 hat. Die Verbindungen I.6-1 bis I.6-53 der Tabelle I.6 sind somit durch die Bedeutung der jeweiligen Einträge No. 1 bis 53 für Q der Tabelle 1 definiert.

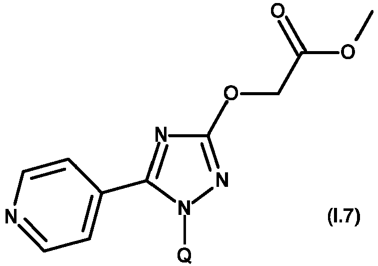

Tabelle I.7: Bevorzugte Verbindungen der Formel (I.7) sind die Verbindungen I.7-1 bis I.7-53, worin Q die in der jeweiligen Zeile angegebenen Bedeutungen der Tabelle 1 hat. Die Verbindungen I.7-1 bis I.7-53 der Tabelle I.7 sind somit durch die Bedeutung der jeweiligen Einträge No. 1 bis 53 für Q der Tabelle 1 definiert.

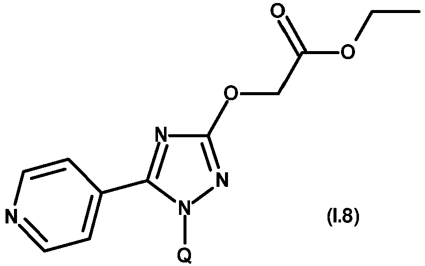

Tabelle I.8: Bevorzugte Verbindungen der Formel (I.8) sind die Verbindungen I.8-1 bis I.8-53, worin Q die in der jeweiligen Zeile angegebenen Bedeutungen der Tabelle 1 hat. Die Verbindungen 1.8-1 bis I.8-53 der Tabelle 1.8 sind somit durch die Bedeutung der jeweiligen Einträge No. 1 bis 53 für Q der Tabelle 1 definiert.

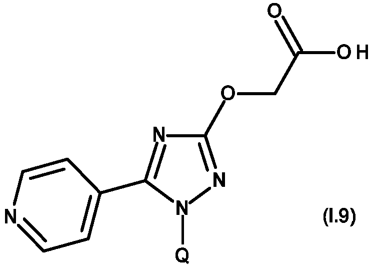

Tabelle I.9: Bevorzugte Verbindungen der Formel (I.9) sind die Verbindungen I.9-1 bis I.9-53, worin Q die in der jeweiligen Zeile angegebenen Bedeutungen der Tabelle 1 hat. Die Verbindungen I.9-1 bis I.9-53 der Tabelle I.9 sind somit durch die Bedeutung der jeweiligen Einträge No. 1 bis 53 für Q der Tabelle 1 definiert.

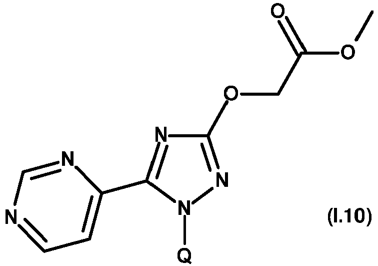

Tabelle I.10: Bevorzugte Verbindungen der Formel (I.10) sind die Verbindungen I.10-1 bis I.10-53, worin Q die in der jeweiligen Zeile angegebenen Bedeutungen der Tabelle 1 hat. Die Verbindungen I.10-1 bis 1.10-53 der Tabelle I.10 sind somit durch die Bedeutung der jeweiligen Einträge No. 1 bis 53 für Q der Tabelle 1 definiert.

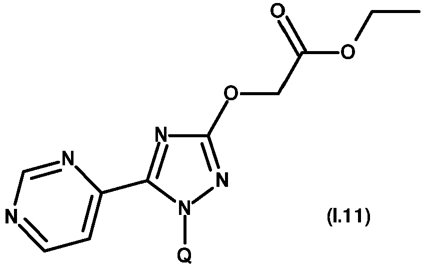

Tabelle 1.11: Bevorzugte Verbindungen der Formel (I.11) sind die Verbindungen I.11-1 bis I.11-53, worin Q die in der jeweiligen Zeile angegebenen Bedeutungen der Tabelle 1 hat. Die Verbindungen I.11-1 bis I.11-53 der Tabelle I.11 sind somit durch die Bedeutung der jeweiligen Einträge No. 1 bis 53 für Q der Tabelle 1 definiert.

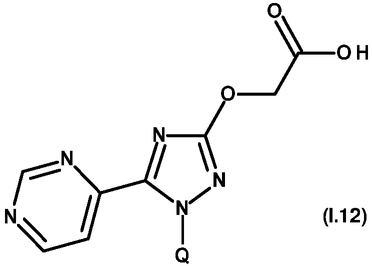

Tabelle I.12: Bevorzugte Verbindungen der Formel (I.12) sind die Verbindungen 1.12-1 bis 1.12-53, worin Q die in der jeweiligen Zeile angegebenen Bedeutungen der Tabelle 1 hat. Die Verbindungen I.12-1 bis 1.12-53 der Tabelle I.12 sind somit durch die Bedeutung der jeweiligen Einträge No. 1 bis 53 für Q der Tabelle 1 definiert.

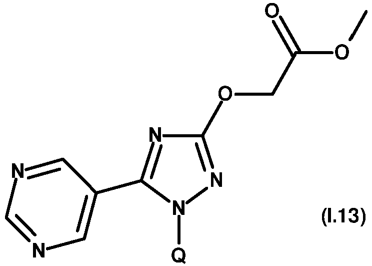

Tabelle I.13: Bevorzugte Verbindungen der Formel (I.13) sind die Verbindungen I.13-1 bis 1.13-53, worin Q die in der jeweiligen Zeile angegebenen Bedeutungen der Tabelle 1 hat. Die Verbindungen I.13-1 bis 1.13-53 der Tabelle I.13 sind somit durch die Bedeutung der jeweiligen Einträge No. 1 bis 53 für Q der Tabelle 1 definiert.

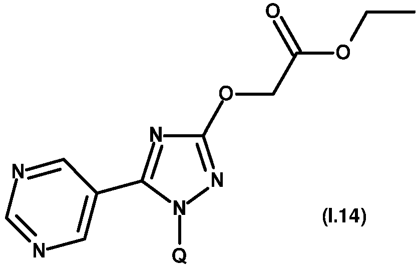

Tabelle I.14: Bevorzugte Verbindungen der Formel (I.14) sind die Verbindungen I.14-1 bis I.14-53, worin Q die in der jeweiligen Zeile angegebenen Bedeutungen der Tabelle 1 hat. Die Verbindungen I.14-1 bis I.14-53 der Tabelle I.14 sind somit durch die Bedeutung der jeweiligen Einträge No. 1 bis 53 für Q der Tabelle 1 definiert.

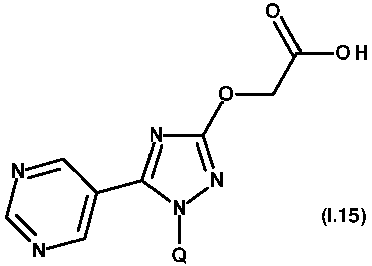

Tabelle I.15: Bevorzugte Verbindungen der Formel (I.15) sind die Verbindungen I.15-1 bis I.15-53, worin Q die in der jeweiligen Zeile angegebenen Bedeutungen der Tabelle 1 hat. Die Verbindungen I.15-1 bis I.15-53 der Tabelle I.15 sind somit durch die Bedeutung der jeweiligen Einträge No. 1 bis 53 für Q der Tabelle 1 definiert.

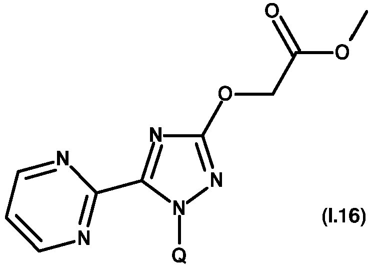

Tabelle I.16: Bevorzugte Verbindungen der Formel (I.16) sind die Verbindungen I.16-1 bis I.16-53, worin Q die in der jeweiligen Zeile angegebenen Bedeutungen der Tabelle 1 hat. Die Verbindungen I.16-1 bis I.16-53 der Tabelle I.16 sind somit durch die Bedeutung der jeweiligen Einträge No. 1 bis 53 für Q der Tabelle 1 definiert.

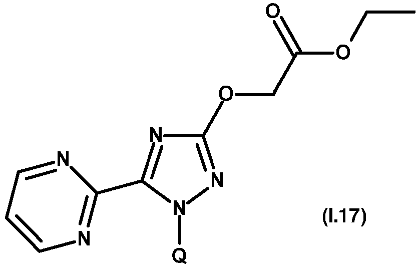

Tabelle I.17: Bevorzugte Verbindungen der Formel (I.17) sind die Verbindungen I.17-1 bis 1.17-53, worin Q die in der jeweiligen Zeile angegebenen Bedeutungen der Tabelle 1 hat. Die Verbindungen I.17-1 bis 1.17-53 der Tabelle I.17 sind somit durch die Bedeutung der jeweiligen Einträge No. 1 bis 53 für Q der Tabelle 1 definiert.

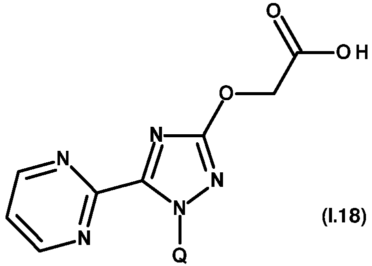

Tabelle 1.18: Bevorzugte Verbindungen der Formel (I.18) sind die Verbindungen 1.18-1 bis 1.18-53, worin Q die in der jeweiligen Zeile angegebenen Bedeutungen der Tabelle 1 hat. Die Verbindungen I.18-1 bis 1.18-53 der Tabelle 1.18 sind somit durch die Bedeutung der jeweiligen Einträge No. 1 bis 53 für Q der Tabelle 1 definiert.

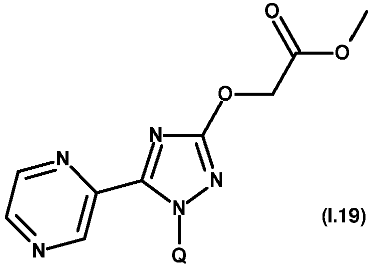

Tabelle 1.19: Bevorzugte Verbindungen der Formel (I.19) sind die Verbindungen 1.19-1 bis 1.19-53, worin Q die in der jeweiligen Zeile angegebenen Bedeutungen der Tabelle 1 hat. Die Verbindungen I.19-1 bis 1.19-53 der Tabelle 1.19 sind somit durch die Bedeutung der jeweiligen Einträge No. 1 bis 53 für Q der Tabelle 1 definiert.

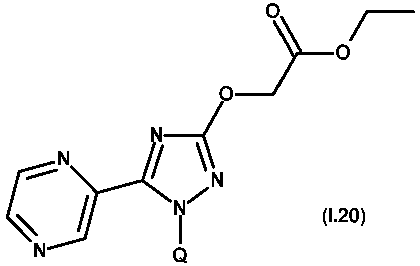

Tabelle I.20: Bevorzugte Verbindungen der Formel (I.20) sind die Verbindungen I.20-1 bis I.20-53, worin Q die in der jeweiligen Zeile angegebenen Bedeutungen der Tabelle 1 hat. Die Verbindungen I.20-1 bis I.20-53 der Tabelle I.20 sind somit durch die Bedeutung der jeweiligen Einträge No. 1 bis 53 für Q der Tabelle 1 definiert.

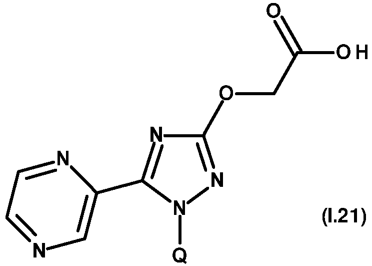

Tabelle I.21: Bevorzugte Verbindungen der Formel (I.21) sind die Verbindungen I.21-1 bis I.21-53, worin Q die in der jeweiligen Zeile angegebenen Bedeutungen der Tabelle 1 hat. Die Verbindungen I.21-1 bis I.21-53 der Tabelle I.21 sind somit durch die Bedeutung der jeweiligen Einträge No. 1 bis 53 für Q der Tabelle 1 definiert.

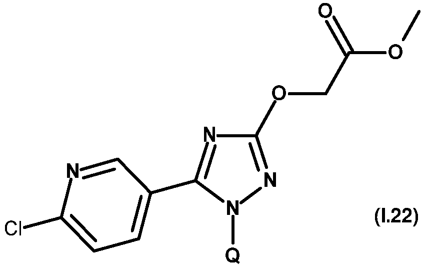

Tabelle I.22: Bevorzugte Verbindungen der Formel (I.22) sind die Verbindungen I.22-1 bis I.22-53, worin Q die in der jeweiligen Zeile angegebenen Bedeutungen der Tabelle 1 hat. Die Verbindungen I.22-1 bis I.22-53 der Tabelle I.22 sind somit durch die Bedeutung der jeweiligen Einträge No. 1 bis 53 für Q der Tabelle 1 definiert.

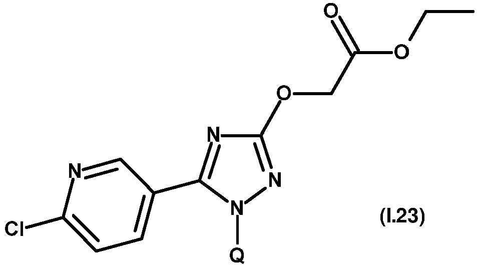

Tabelle I.23: Bevorzugte Verbindungen der Formel (I.23) sind die Verbindungen I.23-1 bis I.23-53, worin Q die in der jeweiligen Zeile angegebenen Bedeutungen der Tabelle 1 hat. Die Verbindungen I.23-1 bis I.23-53 der Tabelle I.23 sind somit durch die Bedeutung der jeweiligen Einträge No. 1 bis 53 für Q der Tabelle 1 definiert.

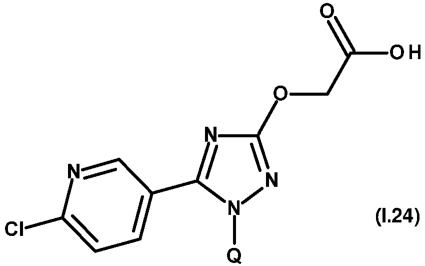

Tabelle I.24: Bevorzugte Verbindungen der Formel (I.24) sind die Verbindungen I.24-1 bis I.24-53, worin Q die in der jeweiligen Zeile angegebenen Bedeutungen der Tabelle 1 hat. Die Verbindungen I.24-1 bis I.24-53 der Tabelle I.24 sind somit durch die Bedeutung der jeweiligen Einträge No. 1 bis 53 für Q der Tabelle 1 definiert.

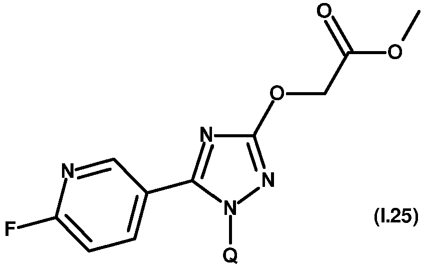

Tabelle I.25: Bevorzugte Verbindungen der Formel (I.25) sind die Verbindungen I.25-1 bis I.25-53, worin Q die in der jeweiligen Zeile angegebenen Bedeutungen der Tabelle 1 hat. Die Verbindungen I.25-1 bis I.25-53 der Tabelle I.25 sind somit durch die Bedeutung der jeweiligen Einträge No. 1 bis 53 für Q der Tabelle 1 definiert.

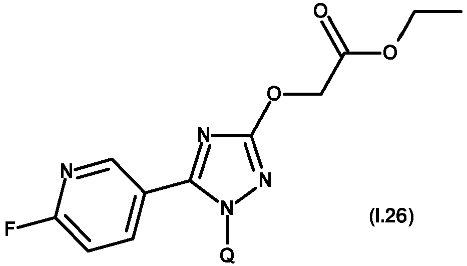

Tabelle I.26: Bevorzugte Verbindungen der Formel (I.26) sind die Verbindungen I.26-1 bis I.26-53, worin Q die in der jeweiligen Zeile angegebenen Bedeutungen der Tabelle 1 hat. Die Verbindungen I.26-1 bis I.26-53 der Tabelle I.26 sind somit durch die Bedeutung der jeweiligen Einträge No. 1 bis 53 für Q der Tabelle 1 definiert.

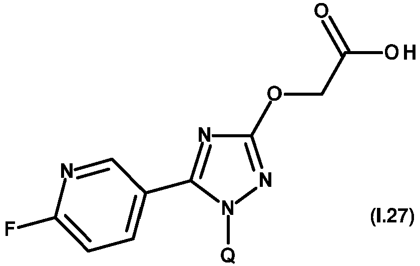

Tabelle I.27: Bevorzugte Verbindungen der Formel (I.27) sind die Verbindungen I.27-1 bis I.27-53, worin Q die in der jeweiligen Zeile angegebenen Bedeutungen der Tabelle 1 hat. Die Verbindungen I.27-1 bis I.27-53 der Tabelle I.27 sind somit durch die Bedeutung der jeweiligen Einträge No. 1 bis 53 für Q der Tabelle 1 definiert.

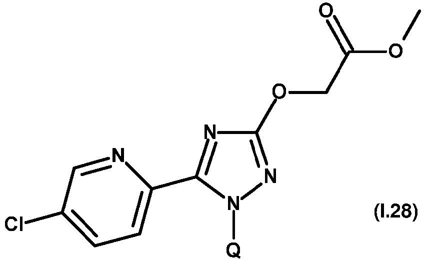

Tabelle I.28: Bevorzugte Verbindungen der Formel (I.28) sind die Verbindungen I.28-1 bis I.28-53, worin Q die in der jeweiligen Zeile angegebenen Bedeutungen der Tabelle 1 hat. Die Verbindungen I.28-1 bis I.28-53 der Tabelle I.28 sind somit durch die Bedeutung der jeweiligen Einträge No. 1 bis 53 für Q der Tabelle 1 definiert.

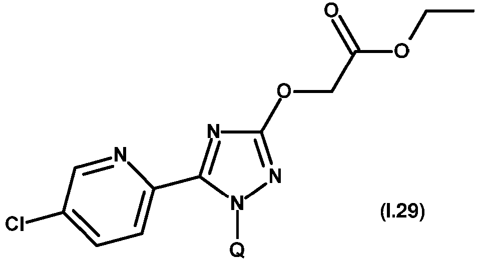

Tabelle I.29: Bevorzugte Verbindungen der Formel (I.29) sind die Verbindungen I.29-1 bis I.29-53, worin Q die in der jeweiligen Zeile angegebenen Bedeutungen der Tabelle 1 hat. Die Verbindungen I.29-1 bis I.29-53 der Tabelle I.29 sind somit durch die Bedeutung der jeweiligen Einträge No. 1 bis 53 für Q der Tabelle 1 definiert.

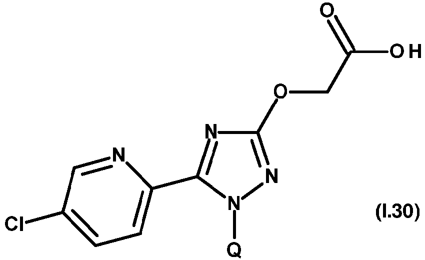

Tabelle I.30: Bevorzugte Verbindungen der Formel (I.30) sind die Verbindungen I.30-1 bis I.30-53, worin Q die in der jeweiligen Zeile angegebenen Bedeutungen der Tabelle 1 hat. Die Verbindungen I.30-1 bis I.30-53 der Tabelle I.30 sind somit durch die Bedeutung der jeweiligen Einträge No. 1 bis 53 für Q der Tabelle 1 definiert.

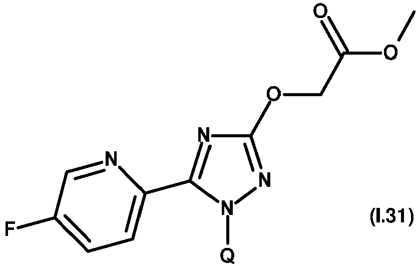

Tabelle I.31: Bevorzugte Verbindungen der Formel (I.31) sind die Verbindungen I.31-1 bis I.31-53, worin Q die in der jeweiligen Zeile angegebenen Bedeutungen der Tabelle 1 hat. Die Verbindungen I.31-1 bis I.31-53 der Tabelle I.31 sind somit durch die Bedeutung der jeweiligen Einträge No. 1 bis 53 für Q der Tabelle 1 definiert.

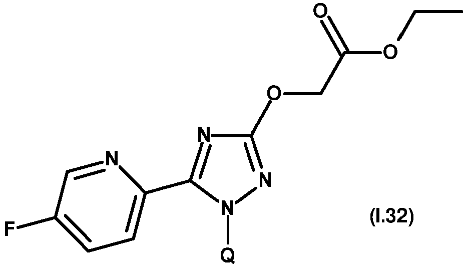

Tabelle I.32: Bevorzugte Verbindungen der Formel (I.32) sind die Verbindungen I.32-1 bis I.32-53, worin Q die in der jeweiligen Zeile angegebenen Bedeutungen der Tabelle 1 hat. Die Verbindungen I.32-1 bis I.32-53 der Tabelle I.32 sind somit durch die Bedeutung der jeweiligen Einträge No. 1 bis 53 für Q der Tabelle 1 definiert.

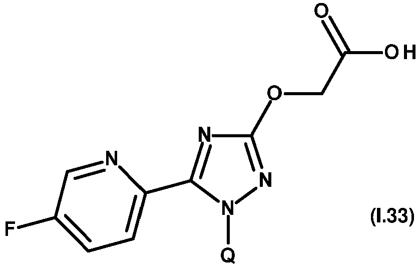

Tabelle I.33: Bevorzugte Verbindungen der Formel (I.33) sind die Verbindungen I.33-1 bis I.33-53, worin Q die in der jeweiligen Zeile angegebenen Bedeutungen der Tabelle 1 hat. Die Verbindungen I.33-1 bis I.33-53 der Tabelle I.33 sind somit durch die Bedeutung der jeweiligen Einträge No. 1 bis 53 für Q der Tabelle 1 definiert.

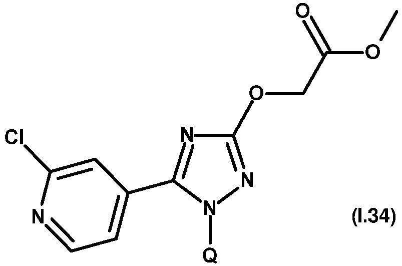

Tabelle I.34: Bevorzugte Verbindungen der Formel (I.34) sind die Verbindungen I.34-1 bis I.34-53, worin Q die in der jeweiligen Zeile angegebenen Bedeutungen der Tabelle 1 hat. Die Verbindungen I.34-1 bis I.34-53 der Tabelle I.34 sind somit durch die Bedeutung der jeweiligen Einträge No. 1 bis 53 für Q der Tabelle 1 definiert.

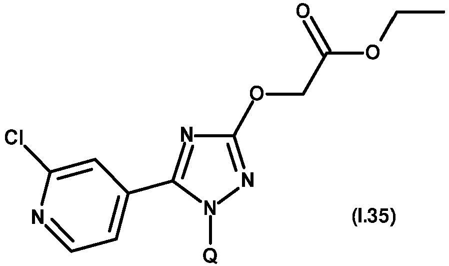

Tabelle I.35: Bevorzugte Verbindungen der Formel (I.35) sind die Verbindungen I.35-1 bis I.35-53, worin Q die in der jeweiligen Zeile angegebenen Bedeutungen der Tabelle 1 hat. Die Verbindungen I.35-1 bis I.35-53 der Tabelle I.35 sind somit durch die Bedeutung der jeweiligen Einträge No. 1 bis 53 für Q der Tabelle 1 definiert.

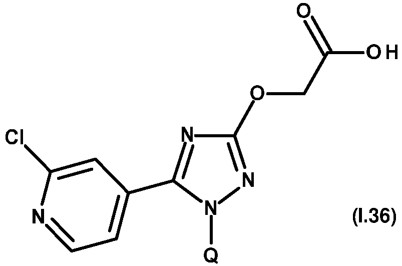

Tabelle I.36: Bevorzugte Verbindungen der Formel (I.36) sind die Verbindungen I.36-1 bis I.36-53, worin Q die in der jeweiligen Zeile angegebenen Bedeutungen der Tabelle 1 hat. Die Verbindungen I.36-1 bis I.36-53 der Tabelle I.36 sind somit durch die Bedeutung der jeweiligen Einträge No. 1 bis 53 für Q der Tabelle 1 definiert.

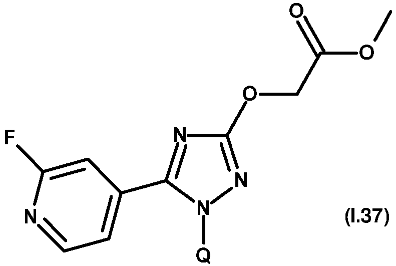

Tabelle I.37: Bevorzugte Verbindungen der Formel (I.37) sind die Verbindungen I.37-1 bis I.37-53, worin Q die in der jeweiligen Zeile angegebenen Bedeutungen der Tabelle 1 hat. Die Verbindungen I.37-1 bis I.37-53 der Tabelle I.37 sind somit durch die Bedeutung der jeweiligen Einträge No. 1 bis 53 für Q der Tabelle 1 definiert.

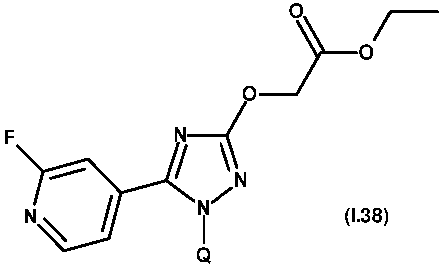

Tabelle I.38: Bevorzugte Verbindungen der Formel (I.38) sind die Verbindungen I.38-1 bis I.38-53, worin Q die in der jeweiligen Zeile angegebenen Bedeutungen der Tabelle 1 hat. Die Verbindungen I.38-1 bis I.38-53 der Tabelle I.38 sind somit durch die Bedeutung der jeweiligen Einträge No. 1 bis 53 für Q der Tabelle 1 definiert.

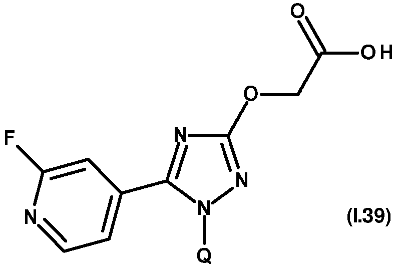

Tabelle I.39: Bevorzugte Verbindungen der Formel (I.39) sind die Verbindungen I.39-1 bis I.39-53, worin Q die in der jeweiligen Zeile angegebenen Bedeutungen der Tabelle 1 hat. Die Verbindungen I.39-1 bis I.39-53 der Tabelle I.39 sind somit durch die Bedeutung der jeweiligen Einträge No. 1 bis 53 für Q der Tabelle 1 definiert.

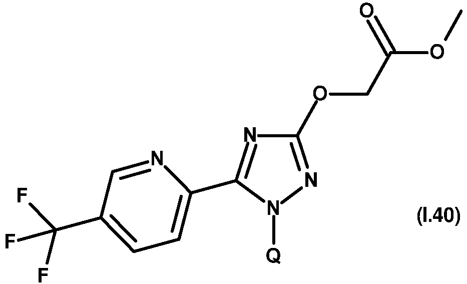

Tabelle I.40: Bevorzugte Verbindungen der Formel (I.40) sind die Verbindungen I.40-1 bis I.40-53, worin Q die in der jeweiligen Zeile angegebenen Bedeutungen der Tabelle 1 hat. Die Verbindungen I.40-1 bis I.40-53 der Tabelle I.40 sind somit durch die Bedeutung der jeweiligen Einträge No. 1 bis 53 für Q der Tabelle 1 definiert.

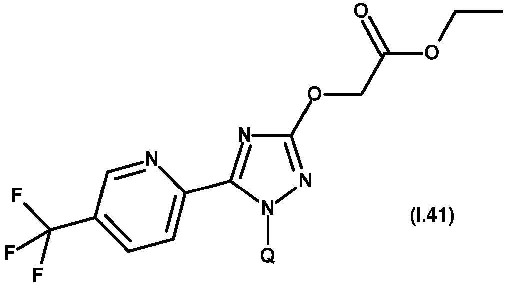

Tabelle I.41: Bevorzugte Verbindungen der Formel (I.41) sind die Verbindungen I.41-1 bis I.41-53, worin Q die in der jeweiligen Zeile angegebenen Bedeutungen der Tabelle 1 hat. Die Verbindungen I.41-1 bis I.41-53 der Tabelle I.41 sind somit durch die Bedeutung der jeweiligen Einträge No. 1 bis 53 für Q der Tabelle 1 definiert.

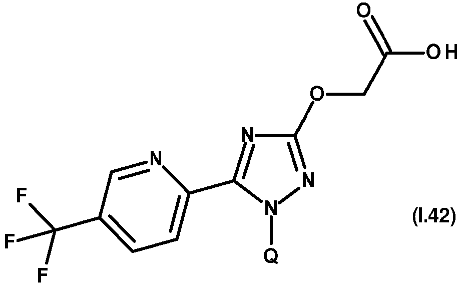

Tabelle I.42: Bevorzugte Verbindungen der Formel (I.42) sind die Verbindungen I.42-1 bis I.42-53, worin Q die in der jeweiligen Zeile angegebenen Bedeutungen der Tabelle 1 hat. Die Verbindungen I.42-1 bis I.42-53 der Tabelle I.42 sind somit durch die Bedeutung der jeweiligen Einträge No. 1 bis 53 für Q der Tabelle 1 definiert.

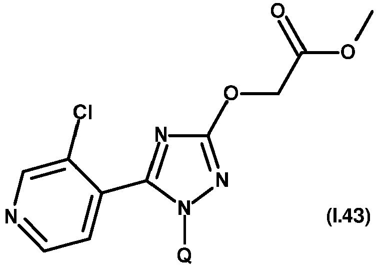

Tabelle I.43: Bevorzugte Verbindungen der Formel (I.4) sind die Verbindungen I.43-1 bis I.43-53, worin Q die in der jeweiligen Zeile angegebenen Bedeutungen der Tabelle 1 hat. Die Verbindungen I.43-1 bis I.43-53 der Tabelle I.43 sind somit durch die Bedeutung der jeweiligen Einträge No. 1 bis 53 für Q der Tabelle 1 definiert.

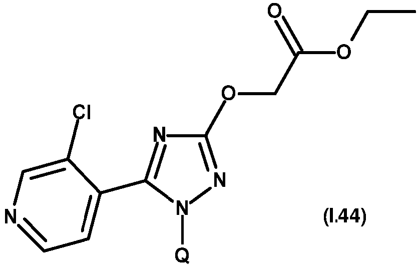

Tabelle I.44: Bevorzugte Verbindungen der Formel (I.44) sind die Verbindungen I.44-1 bis I.44-53, worin Q die in der jeweiligen Zeile angegebenen Bedeutungen der Tabelle 1 hat. Die Verbindungen I.44-1 bis I.44-53 der Tabelle I.44 sind somit durch die Bedeutung der jeweiligen Einträge No. 1 bis 53 für Q der Tabelle 1 definiert.

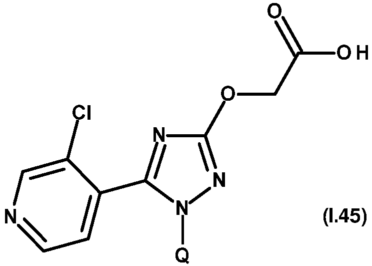

Tabelle I.45: Bevorzugte Verbindungen der Formel (I.45) sind die Verbindungen I.45-1 bis I.45-53, worin Q die in der jeweiligen Zeile angegebenen Bedeutungen der Tabelle 1 hat. Die Verbindungen I.45-1 bis I.45-53 der Tabelle I.45 sind somit durch die Bedeutung der jeweiligen Einträge No. 1 bis 53 für Q der Tabelle 1 definiert.

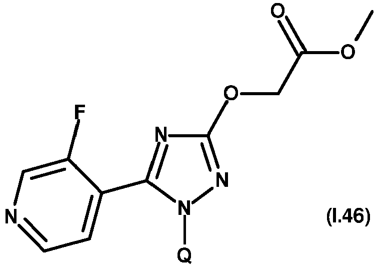

Tabelle I.46: Bevorzugte Verbindungen der Formel (I.46) sind die Verbindungen I.46-1 bis I.46-53, worin Q die in der jeweiligen Zeile angegebenen Bedeutungen der Tabelle 1 hat. Die Verbindungen I.46-1 bis I.46-53 der Tabelle I.46 sind somit durch die Bedeutung der jeweiligen Einträge No. 1 bis 53 für Q der Tabelle 1 definiert.

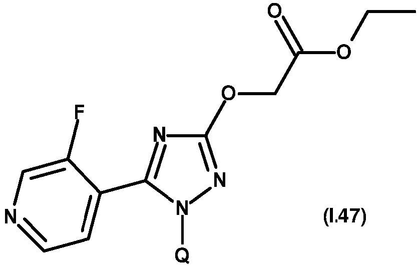

Tabelle I.47: Bevorzugte Verbindungen der Formel (I.47) sind die Verbindungen I.47-1 bis I.47-53, worin Q die in der jeweiligen Zeile angegebenen Bedeutungen der Tabelle 1 hat. Die Verbindungen I.47-1 bis I.47-53 der Tabelle I.47 sind somit durch die Bedeutung der jeweiligen Einträge No. 1 bis 53 für Q der Tabelle 1 definiert.

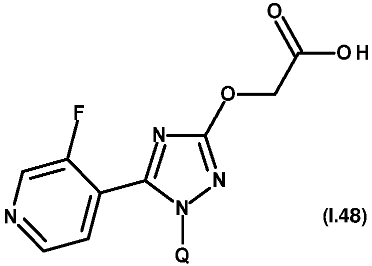

Tabelle I.48: Bevorzugte Verbindungen der Formel (I.48) sind die Verbindungen I.48-1 bis I.48-53, worin Q die in der jeweiligen Zeile angegebenen Bedeutungen der Tabelle 1 hat. Die Verbindungen I.48-1 bis I.48-53 der Tabelle I.48 sind somit durch die Bedeutung der jeweiligen Einträge No. 1 bis 53 für Q der Tabelle 1 definiert.

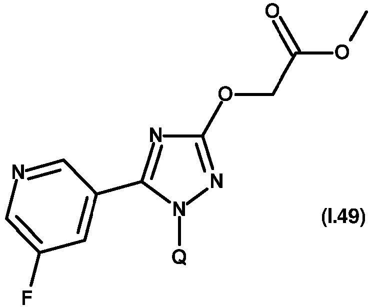

Tabelle I.49: Bevorzugte Verbindungen der Formel (I.49) sind die Verbindungen I.49-1 bis I.49-53, worin Q die in der jeweiligen Zeile angegebenen Bedeutungen der Tabelle 1 hat. Die Verbindungen I.49-1 bis I.49-53 der Tabelle I.49 sind somit durch die Bedeutung der jeweiligen Einträge No. 1 bis 53 für Q der Tabelle 1 definiert.

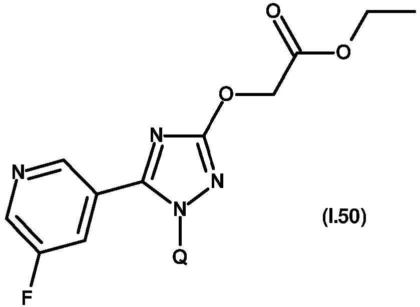

Tabelle I.50: Bevorzugte Verbindungen der Formel (I.50) sind die Verbindungen I.50-1 bis I.50-53, worin Q die in der jeweiligen Zeile angegebenen Bedeutungen der Tabelle 1 hat. Die Verbindungen I.50-1 bis I.50-53 der Tabelle I.50 sind somit durch die Bedeutung der jeweiligen Einträge No. 1 bis 53 für Q der Tabelle 1 definiert.

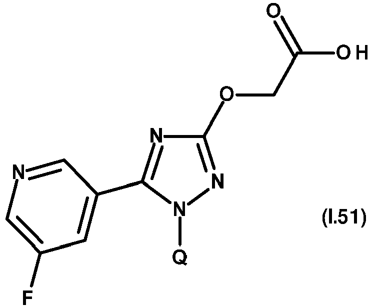

Tabelle I.51: Bevorzugte Verbindungen der Formel (I.51) sind die Verbindungen I.51-1 bis I.51-53, worin Q die in der jeweiligen Zeile angegebenen Bedeutungen der Tabelle 1 hat. Die Verbindungen I.51-1 bis I.5153 der Tabelle I.51 sind somit durch die Bedeutung der jeweiligen Einträge No. 1 bis 53 für Q der Tabelle 1 definiert.

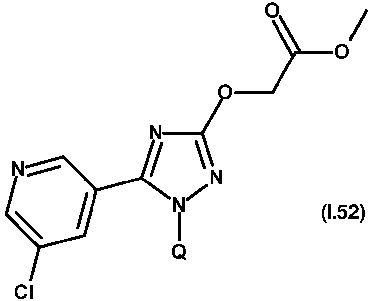

Tabelle I.52: Bevorzugte Verbindungen der Formel (I.52) sind die Verbindungen I.52-1 bis I.52-53, worin Q die in der jeweiligen Zeile angegebenen Bedeutungen der Tabelle 1 hat. Die Verbindungen I.52-1 bis I.52-53 der Tabelle I.52 sind somit durch die Bedeutung der jeweiligen Einträge No. 1 bis 53 für Q der Tabelle 1 definiert.

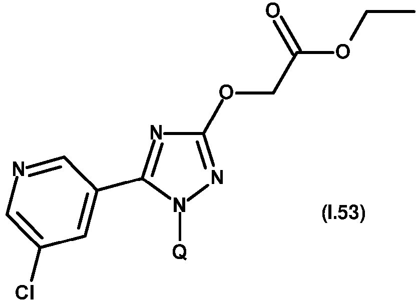

Tabelle I.53: Bevorzugte Verbindungen der Formel (I.53) sind die Verbindungen I.53-1 bis I.53-53, worin Q die in der jeweiligen Zeile angegebenen Bedeutungen der Tabelle 1 hat. Die Verbindungen I.53-1 bis I.53-53 der Tabelle I.53 sind somit durch die Bedeutung der jeweiligen Einträge No. 1 bis 53 für Q der Tabelle 1 definiert.

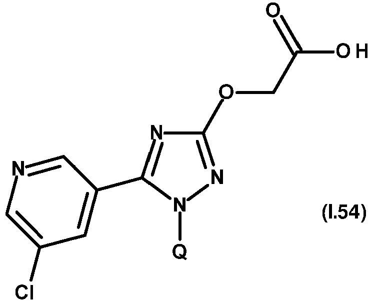

Tabelle I.54: Bevorzugte Verbindungen der Formel (I.54) sind die Verbindungen I.54-1 bis I.54-53, worin Q die in der jeweiligen Zeile angegebenen Bedeutungen der Tabelle 1 hat. Die Verbindungen I.54-1 bis I.54-53 der Tabelle I.54 sind somit durch die Bedeutung der jeweiligen Einträge No. 1 bis 53 für Q der Tabelle 1 definiert.

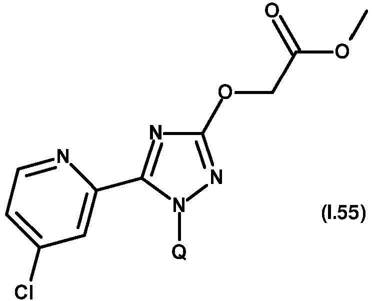

Tabelle I.55: Bevorzugte Verbindungen der Formel (I.55) sind die Verbindungen I.55-1 bis I.55-53, worin Q die in der jeweiligen Zeile angegebenen Bedeutungen der Tabelle 1 hat. Die Verbindungen I.55-1 bis I.55-53 der Tabelle I.55 sind somit durch die Bedeutung der jeweiligen Einträge No. 1 bis 53 für Q der Tabelle 1 definiert.

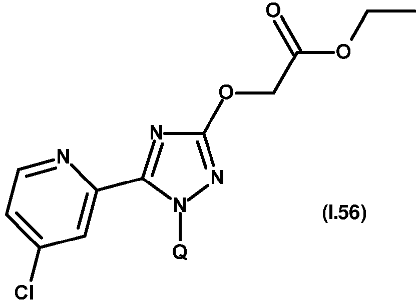

Tabelle I.56: Bevorzugte Verbindungen der Formel (I.56) sind die Verbindungen I.56-1 bis I.56-53, worin Q die in der jeweiligen Zeile angegebenen Bedeutungen der Tabelle 1 hat. Die Verbindungen I.56-1 bis I.56-53 der Tabelle I.56 sind somit durch die Bedeutung der jeweiligen Einträge No. 1 bis 53 für Q der Tabelle 1 definiert.

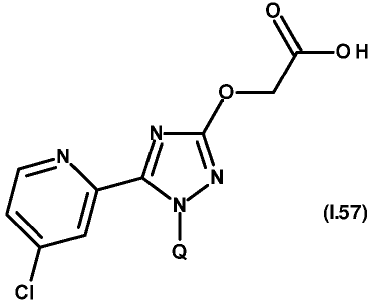

Tabelle I.57: Bevorzugte Verbindungen der Formel (I.57) sind die Verbindungen I.57-1 bis I.57-53, worin Q die in der jeweiligen Zeile angegebenen Bedeutungen der Tabelle 1 hat. Die Verbindungen I.57-1 bis I.57-53 der Tabelle I.57 sind somit durch die Bedeutung der jeweiligen Einträge No. 1 bis 53 für Q der Tabelle 1 definiert.

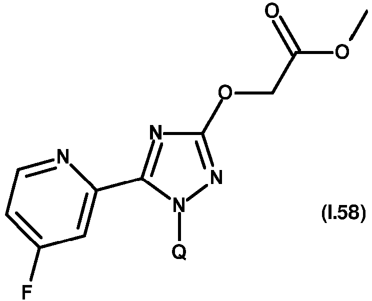

Tabelle I.58: Bevorzugte Verbindungen der Formel (I.58) sind die Verbindungen I.58-1 bis I.58-53, worin Q die in der jeweiligen Zeile angegebenen Bedeutungen der Tabelle 1 hat. Die Verbindungen I.58-1 bis I.58-53 der Tabelle I.58 sind somit durch die Bedeutung der jeweiligen Einträge No. 1 bis 53 für Q der Tabelle 1 definiert.

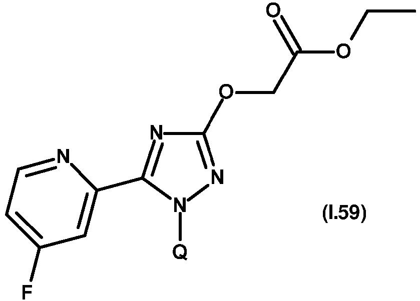

Tabelle I.59: Bevorzugte Verbindungen der Formel (I.59) sind die Verbindungen I.59-1 bis I.59-53, worin Q die in der jeweiligen Zeile angegebenen Bedeutungen der Tabelle 1 hat. Die Verbindungen I.59-1 bis I.59-53 der Tabelle I.59 sind somit durch die Bedeutung der jeweiligen Einträge No. 1 bis 53 für Q der Tabelle 1 definiert.

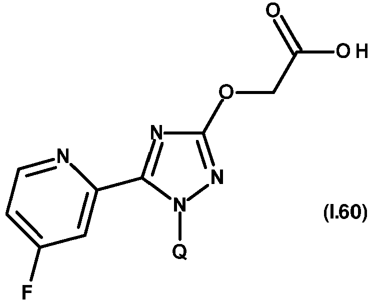

Tabelle I.60: Bevorzugte Verbindungen der Formel (I.60) sind die Verbindungen I.60-1 bis I.60-53, worin Q die in der jeweiligen Zeile angegebenen Bedeutungen der Tabelle 1 hat. Die Verbindungen I.60-1 bis I.60-53 der Tabelle I.60 sind somit durch die Bedeutung der jeweiligen Einträge No. 1 bis 53 für Q der Tabelle 1 definiert.

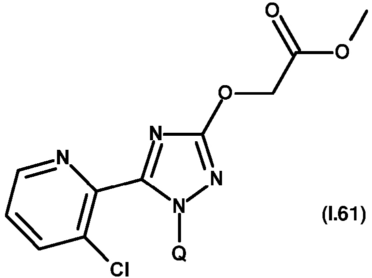

Tabelle I.61: Bevorzugte Verbindungen der Formel (I.61) sind die Verbindungen I.61-1 bis I.61-53, worin Q die in der jeweiligen Zeile angegebenen Bedeutungen der Tabelle 1 hat. Die Verbindungen I.61-1 bis I.61-53 der Tabelle I.61 sind somit durch die Bedeutung der jeweiligen Einträge No. 1 bis 53 für Q der Tabelle 1 definiert.

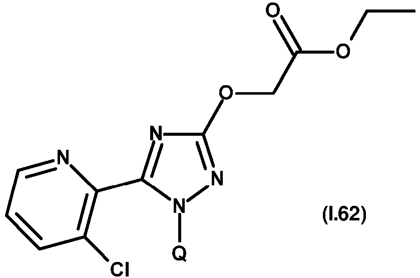

Tabelle I.62: Bevorzugte Verbindungen der Formel (1.62) sind die Verbindungen I.62-1 bis I.62-53, worin Q die in der jeweiligen Zeile angegebenen Bedeutungen der Tabelle 1 hat. Die Verbindungen I.62-1 bis I.62-53 der Tabelle I.62 sind somit durch die Bedeutung der jeweiligen Einträge No. 1 bis 53 für Q der Tabelle 1 definiert.

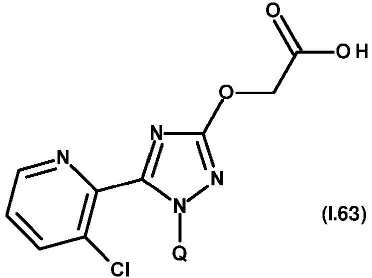

Tabelle I.63: Bevorzugte Verbindungen der Formel (I.63) sind die Verbindungen I.63-1 bis I.63-53, worin Q die in der jeweiligen Zeile angegebenen Bedeutungen der Tabelle 1 hat. Die Verbindungen I.63-1 bis I.63-53 der Tabelle I.63 sind somit durch die Bedeutung der jeweiligen Einträge No. 1 bis 53 für Q der Tabelle 1 definiert.

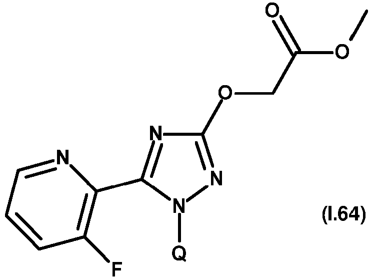

Tabelle I.64: Bevorzugte Verbindungen der Formel (I.64) sind die Verbindungen I.64-1 bis I.64-53, worin Q die in der jeweiligen Zeile angegebenen Bedeutungen der Tabelle 1 hat. Die Verbindungen I.64-1 bis I.64-53 der Tabelle I.64 sind somit durch die Bedeutung der jeweiligen Einträge No. 1 bis 53 für Q der Tabelle 1 definiert.

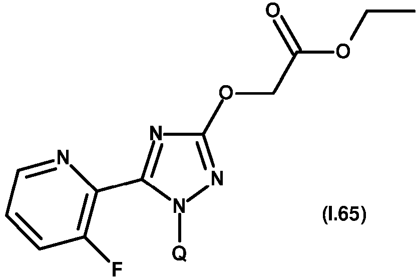

Tabelle I.65: Bevorzugte Verbindungen der Formel (I.65) sind die Verbindungen I.65-1 bis I.65-53, worin Q die in der jeweiligen Zeile angegebenen Bedeutungen der Tabelle 1 hat. Die Verbindungen I.65-1 bis I.65-53 der Tabelle I.65 sind somit durch die Bedeutung der jeweiligen Einträge No. 1 bis 53 für Q der Tabelle 1 definiert.

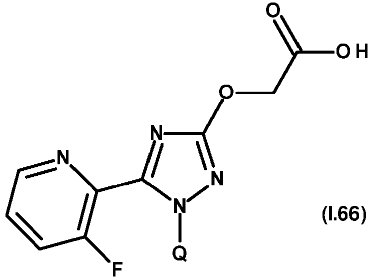

Tabelle I.66: Bevorzugte Verbindungen der Formel (I.66) sind die Verbindungen I.66-1 bis I.66-53, worin Q die in der jeweiligen Zeile angegebenen Bedeutungen der Tabelle 1 hat. Die Verbindungen I.66-1 bis I.66-53 der Tabelle I.66 sind somit durch die Bedeutung der jeweiligen Einträge No. 1 bis 53 für Q der Tabelle 1 definiert.

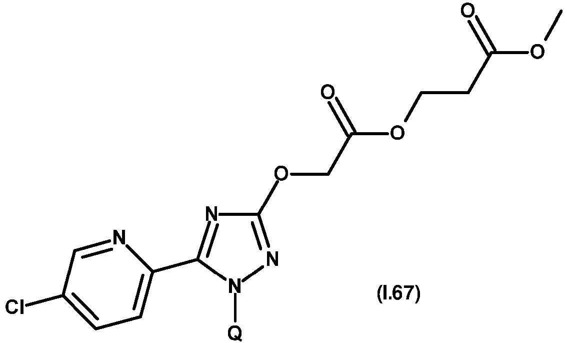

Tabelle I.67: Bevorzugte Verbindungen der Formel (I.67) sind die Verbindungen I.67-1 bis I.67-53, worin Q die in der jeweiligen Zeile angegebenen Bedeutungen der Tabelle 1 hat. Die Verbindungen I.67-1 bis I.67-53 der Tabelle I.67 sind somit durch die Bedeutung der jeweiligen Einträge No. 1 bis 53 für Q der Tabelle 1 definiert.

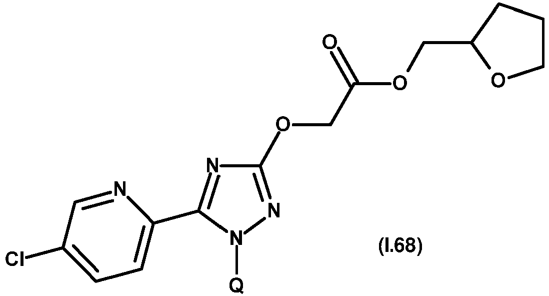

Tabelle I.62: Bevorzugte Verbindungen der Formel (I.62) sind die Verbindungen I.62-1 bis I.62-53, worin Q die in der jeweiligen Zeile angegebenen Bedeutungen der Tabelle 1 hat. Die Verbindungen I.62-1 bis I.62-53 der Tabelle I.62 sind somit durch die Bedeutung der jeweiligen Einträge No. 1 bis 53 für Q der Tabelle 1 definiert.

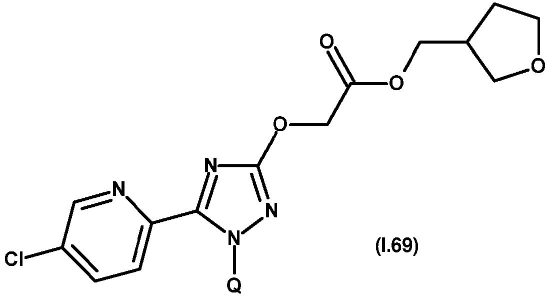

Tabelle I.69: Bevorzugte Verbindungen der Formel (I.69) sind die Verbindungen I.69-1 bis I.69-53, worin Q die in der jeweiligen Zeile angegebenen Bedeutungen der Tabelle 1 hat. Die Verbindungen I.69-1 bis I.69-53 der Tabelle I.69 sind somit durch die Bedeutung der jeweiligen Einträge No. 1 bis 53 für Q der Tabelle 1 definiert.

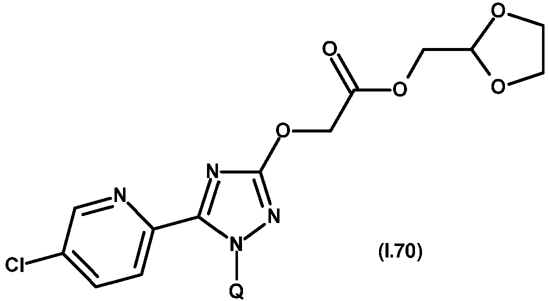

Tabelle I.70: Bevorzugte Verbindungen der Formel (I.70) sind die Verbindungen I.70-1 bis I.70-53, worin Q die in der jeweiligen Zeile angegebenen Bedeutungen der Tabelle 1 hat. Die Verbindungen I.70-1 bis I.70-53 der Tabelle I.70 sind somit durch die Bedeutung der jeweiligen Einträge No. 1 bis 53 für Q der Tabelle 1 definiert.

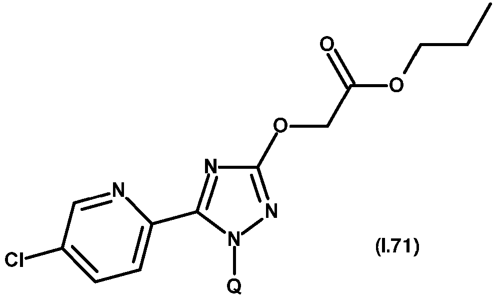

Tabelle I.71: Bevorzugte Verbindungen der Formel (I.71) sind die Verbindungen I.71-1 bis I.71-53, worin Q die in der jeweiligen Zeile angegebenen Bedeutungen der Tabelle 1 hat. Die Verbindungen I.71-1 bis I.71-53 der Tabelle I.71 sind somit durch die Bedeutung der jeweiligen Einträge No. 1 bis 53 für Q der Tabelle 1 definiert.

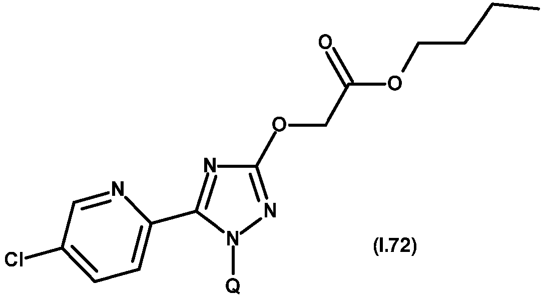

Tabelle I.72: Bevorzugte Verbindungen der Formel (I.72) sind die Verbindungen I.72-1 bis I.72-53, worin Q die in der jeweiligen Zeile angegebenen Bedeutungen der Tabelle 1 hat. Die Verbindungen I.72-1 bis I.72-53 der Tabelle I.72 sind somit durch die Bedeutung der jeweiligen Einträge No. 1 bis 53 für Q der Tabelle 1 definiert.

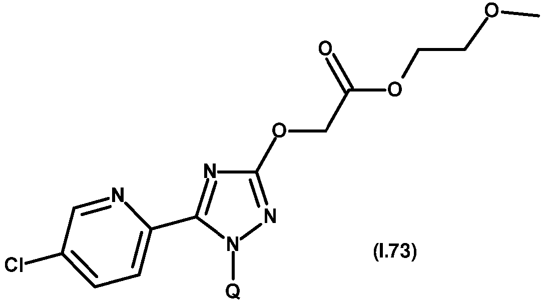

Tabelle I.73: Bevorzugte Verbindungen der Formel (I.73) sind die Verbindungen I.73-1 bis I.73-53, worin Q die in der jeweiligen Zeile angegebenen Bedeutungen der Tabelle 1 hat. Die Verbindungen I.73-1 bis I. 73-53 der Tabelle I.73 sind somit durch die Bedeutung der jeweiligen Einträge No. 1 bis 53 für Q der Tabelle 1 definiert.

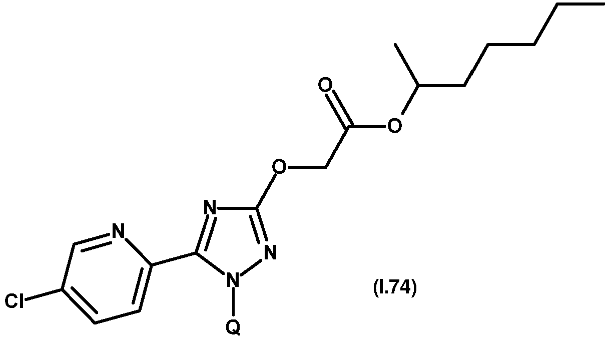

Tabelle I.74: Bevorzugte Verbindungen der Formel (I.74) sind die Verbindungen I.74-1 bis I.74-53, worin Q die in der jeweiligen Zeile angegebenen Bedeutungen der Tabelle 1 hat. Die Verbindungen I.74-1 bis I.74-53 der Tabelle I.74 sind somit durch die Bedeutung der jeweiligen Einträge No. 1 bis 53 für Q der Tabelle 1 definiert.

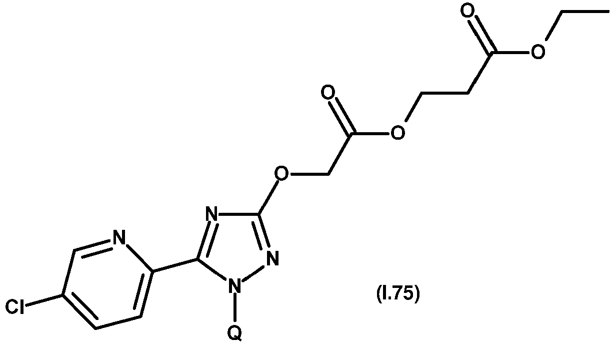

Tabelle I.75: Bevorzugte Verbindungen der Formel (I.75) sind die Verbindungen I.75-1 bis I.75-53, worin Q die in der jeweiligen Zeile angegebenen Bedeutungen der Tabelle 1 hat. Die Verbindungen I.75-1 bis I.75-53 der Tabelle I.75 sind somit durch die Bedeutung der jeweiligen Einträge No. 1 bis 53 für Q der Tabelle 1 definiert.

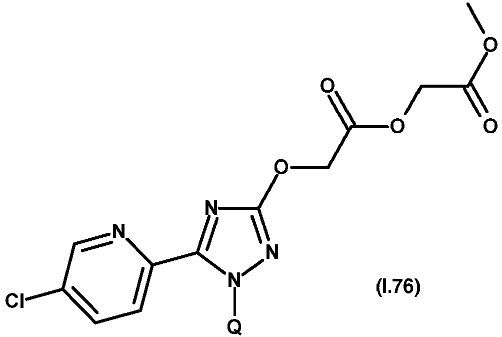

Tabelle I.76: Bevorzugte Verbindungen der Formel (I.76) sind die Verbindungen I.76-1 bis I.76-53, worin Q die in der jeweiligen Zeile angegebenen Bedeutungen der Tabelle 1 hat. Die Verbindungen I.76-1 bis I.76-53 der Tabelle I.76 sind somit durch die Bedeutung der jeweiligen Einträge No. 1 bis 53 für Q der Tabelle 1 definiert.

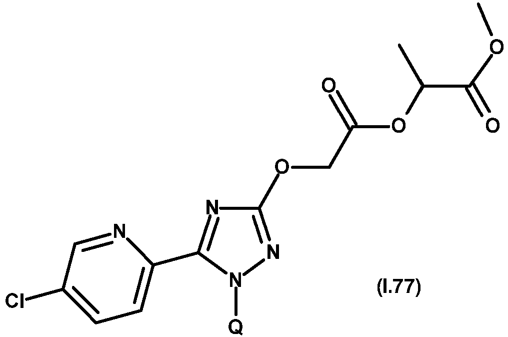

Tabelle I.77: Bevorzugte Verbindungen der Formel (I.77) sind die Verbindungen I.77-1 bis I.77-53, worin Q die in der jeweiligen Zeile angegebenen Bedeutungen der Tabelle 1 hat. Die Verbindungen I.77-1 bis I.77-53 der Tabelle I.77 sind somit durch die Bedeutung der jeweiligen Einträge No. 1 bis 53 für Q der Tabelle 1 definiert.

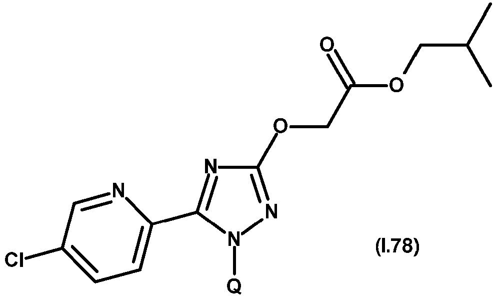

Tabelle I.78: Bevorzugte Verbindungen der Formel (I.78) sind die Verbindungen I.78-1 bis I.78-53, worin Q die in der jeweiligen Zeile angegebenen Bedeutungen der Tabelle 1 hat. Die Verbindungen I.78-1 bis I.78-53 der Tabelle I.78 sind somit durch die Bedeutung der jeweiligen Einträge No. 1 bis 53 für Q der Tabelle 1 definiert.

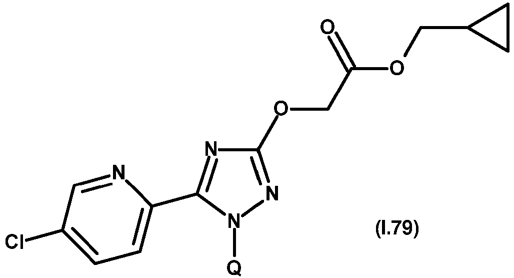

Tabelle I.79: Bevorzugte Verbindungen der Formel (I.79) sind die Verbindungen I.79-1 bis I.79-53, worin Q die in der jeweiligen Zeile angegebenen Bedeutungen der Tabelle 1 hat. Die Verbindungen I.79-1 bis I.79-53 der Tabelle I.79 sind somit durch die Bedeutung der jeweiligen Einträge No. 1 bis 53 für Q der Tabelle 1 definiert.

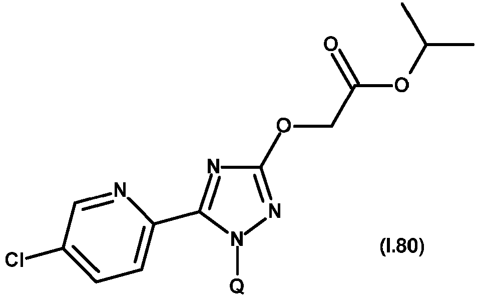

Tabelle I.80: Bevorzugte Verbindungen der Formel (I.80) sind die Verbindungen I.80-1 bis I.80-53, worin Q die in der jeweiligen Zeile angegebenen Bedeutungen der Tabelle 1 hat. Die Verbindungen I.80-1 bis I.80-53 der Tabelle I.80 sind somit durch die Bedeutung der jeweiligen Einträge No. 1 bis 53 für Q der Tabelle 1 definiert.

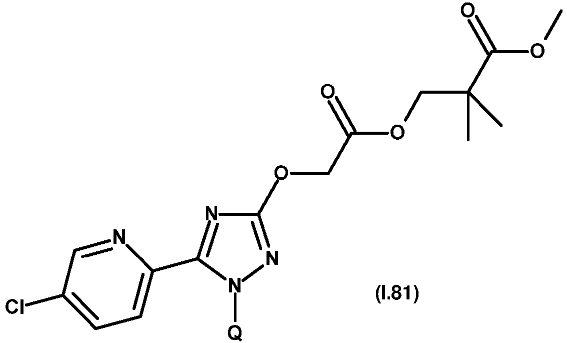

Tabelle I.81: Bevorzugte Verbindungen der Formel (I.81) sind die Verbindungen I.81-1 bis I.81-53, worin Q die in der jeweiligen Zeile angegebenen Bedeutungen der Tabelle 1 hat. Die Verbindungen I.81-1 bis I.81-53 der Tabelle I.81 sind somit durch die Bedeutung der jeweiligen Einträge No. 1 bis 53 für Q der Tabelle 1 definiert.

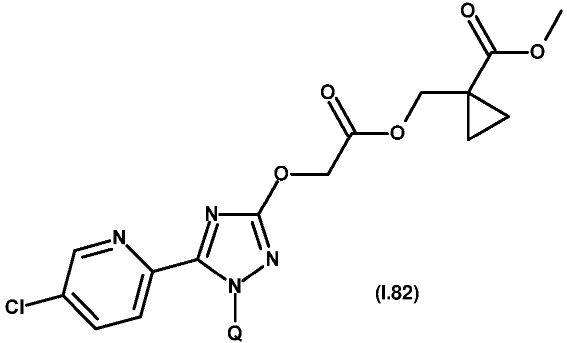

Tabelle I.82: Bevorzugte Verbindungen der Formel (I.82) sind die Verbindungen I.82-1 bis I.82-53, worin Q die in der jeweiligen Zeile angegebenen Bedeutungen der Tabelle 1 hat. Die Verbindungen I.82-1 bis I.82-53 der Tabelle I.82 sind somit durch die Bedeutung der jeweiligen Einträge No. 1 bis 53 für Q der Tabelle 1 definiert.

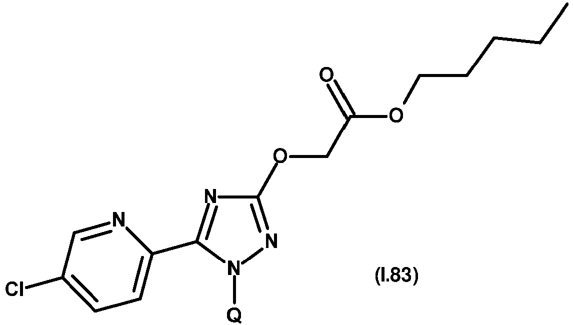

Tabelle I.83: Bevorzugte Verbindungen der Formel (I.83) sind die Verbindungen I.83-1 bis I.83-53, worin Q die in der jeweiligen Zeile angegebenen Bedeutungen der Tabelle 1 hat. Die Verbindungen I.83-1 bis I.83-53 der Tabelle I.83 sind somit durch die Bedeutung der jeweiligen Einträge No. 1 bis 53 für Q der Tabelle 1 definiert.

Spektroskopische Daten ausgewählter Tabellenbeispiele:
Ausgewählte detaillierte Synthesebeispiele für die erfindungsgemäßen Verbindungen der allgemeinen Formeln (I) sind im Folgenden aufgeführt. Die ¹H-NMR-, ¹³C-NMR- und ¹⁹F-NMR-spektroskopischen Daten, die für die in den nachfolgenden Abschnitten beschriebenen chemischen Beispiele angegeben sind, (400 MHz bei ¹H-NMR und 150 MHz bei ¹³C-NMR und 375 MHz bei ¹⁹F-NMR, Lösungsmittel CDCl₃, CD₃OD oder d₆-DMSO, interner Standard: Tetramethylsilan δ = 0.00 ppm), wurden mit einem Gerät der Firma Bruker erhalten, und die bezeichneten Signale haben die nachfolgend aufgeführten Bedeutungen: br = breit(es); s = Singulett, d = Dublett, t = Triplett, dd = Doppeldublett, ddd = Dublett eines Doppeldubletts, m = Multiplett, q = Quartett, quint = Quintett, sext = Sextett, sept = Septett, dq = Doppelquartett, dt = Doppeltriplett. Bei Diastereomerengemischen werden entweder die jeweils signifikanten Signale beider Diastereomere oder das charakteristische Signal des Hauptdiastereomers angegeben. Die verwendeten Abkürzungen für chemische Gruppen haben beispielsweise die nachfolgenden Bedeutungen: Me = CH₃, Et = CH₂CH₃, t-Hex = C(CH₃)₂CH(CH₃)₂, t-Bu = C(CH₃)₃, n-Bu = unverzweigtes Butyl, n-Pr = unverzweigtes Propyl, i-Pr = verzweigtes Propyl, c-Pr = Cyclopropyl, c-Hex = Cyclohexyl.

Die nachfolgend aufgeführten spektroskopischen Daten ausgewählter Tabellenbeispiele wurden über klassische ¹H-NMR-Interpretation oder über NMR-Peak-Listenverfahren ausgewertet.

### Klassische ¹H-NMR-Interpretation

### Beispiel No. I.29-48:

¹H-NMR (400 MHz, CDCl₃ δ, ppm) 8.22 (d, 1H), 8.11 (d, 1H), 7.75 (dd, 1H), 7.46 (dd, 1H), 7.21 (dd, 1H), 7.09 (m, 1H), 4.92 (s, 2H), 4.27 (q, 2H), 1.29 (t, 3H).

### Beispiel No. I.28-4:

¹H-NMR (400 MHz, CDCl₃ δ, ppm) 8.33 (d, 1H), 7.98 (dd, 1H), 7.75 (dd, 1H), 7.37-7.33 (m, 2H), 7.11-7.07 (m, 2H), 4.95 (s, 2H), 3.81 (s, 3H).

### Beispiel No. I.32-48:

¹H-NMR (400 MHz, CDCl₃ δ, ppm) 8.18 (dd, 1H), 8.12 (d, 1H), 7.50-7.45 (m, 2H), 7.20 (dd, 1H), 7.09 (m, 1H), 4.92 (s, 2H), 4.27 (q, 2H), 1.29 (t, 3H).

### Beispiel No. I.32-2:

¹H-NMR (400 MHz, CDCl₃ δ, ppm) 8.17-8.14 (m, 2H), 7.54-7.39 (m, 3H), 7.25 (m, 1H), 7.12 (m, 1H), 4.93 (s, 2H), 4.28 (q, 2H), 1.29 (t, 3H).

### Beispiel No. I.32-4:

¹H-NMR (400 MHz, CDCl₃ δ, ppm) 8.24 (d, 1H), 8.04 (dd, 1H), 7.48 (m, 1H), 7.36-7.33 (m, 2H), 7.11-7.06 (m, 2H), 4.93 (s, 2H), 4.27 (q, 2H), 1.30 (t, 3H).

### Beispiel No. I.30-49:

¹H-NMR (400 MHz, DMSO-d⁶ δ, ppm) 8.47 (d, 1H), 8.15-8.07 (m, 2H), 7.70-7.63 (m, 2H), 7.45 (m, 1H), 4.85 (s, 2H).

### Beispiel No. I.30-7:

¹H-NMR (400 MHz, DMSO-d⁶ δ, ppm) 13.12 (bs, 1H), 8.51 (d, 1H), 8.12 (dd, 1H), 7.99 (d, 1H), 7.52 (d, 2H), 7.42 (d, 2H), 4.87 (s, 2H).

### Beispiel No. I.29-2:

¹H-NMR (400 MHz, CDCl₃ δ, ppm) 8.25 (d, 1H), 8.08 (dd, 1H), 7.75 (dd, 1H), 7.52 (m, 1H), 7.43 (m, 1H), 7.24 (m, 1H), 7.12 (m, 1H), 4.93 (s, 2H), 4.28 (q, 2H), 1.29 (t, 3H).

### Beispiel No. I.33-48:

¹H-NMR (400 MHz, DMSO-d⁶ δ, ppm) 13.10 (bs, 1H), 8.37 (d, 1H), 8.15 (dd, 1H), 7.91 (m, 1H), 7.72-7.66 (m, 2H), 7.39 (m, 1H), 4.86 (s, 2H).

### Beispiel No. I.71-38:

¹H-NMR (400 MHz, CDCl₃ δ, ppm) 8.26 (d, 1H), 8.10 (dd, 1H), 7.76 (dd, 1H), 7.48 (m, 1H), 7.43 (m, 1H), 6.98 (m, 1H), 6.89 (m, 1H), 4.93 (s, 2H), 4.18 (t, 2H), 1.70-1.58 (m, 2H), 0.91 (t, 3H).

### Beispiel No. I.33-2:

¹H-NMR (400 MHz, DMSO-d⁶ δ, ppm) 13.11 (bs, 1H), 8.38 (d, 1H), 8.13 (dd, 1H), 7.92 (m, 1H), 7.61-7.53 (m, 2H), 7.40-7.32 (m, 2H), 4.88 (s, 2H).

### Beispiel No. I.31-49:

¹H-NMR (400 MHz, CDCl₃ δ, ppm) 8.20-8.17 (m, 2H), 7.52-7.44 (m, 2H), 7.25 (m, 1H), 7.16 (m, 1H), 4.94 (s, 2H), 3.81 (s, 3H).

### Beispiel No. I.31-38:

¹H-NMR (400 MHz, CDCl₃ δ, ppm) 8.20-8.16 (m, 2H), 7.52-7.46 (m, 2H), 6.99 (m, 1H), 6.88 (m, 1H), 4.94 (s, 2H), 3.81 (s, 3H).

### Beispiel No. I.29-4:

¹H-NMR (400 MHz, CDCl₃ δ, ppm) 8.20-8.17 (m, 2H), 7.52-7.43 (m, 2H), 7.25 (m, 1H), 7.16 (dd, 1H), 4.92 (s, 2H), 4.27 (q, 2H), 1.29 (t, 3H).

### Beispiel No. I.29-38:

¹H-NMR (400 MHz, CDCl₃ δ, ppm) 8.26 (d, 1H), 8.11 (dd, 1H), 7.76 (dd, 1H), 7.48 (m, 1H), 6.99 (m, 1H), 6.90 (m,1H), 4.92 (s, 2H), 4.27 (q, 2H), 1.29 (t, 3H).

### Beispiel No. I.30-38:

¹H-NMR (400 MHz, CDCl₃ δ, ppm) 9.52 (bs, 1H), 7.54 (m, 1H), 7.43 (m, 1H), 7.22 (m, 1H), 7.05 (dd, 1H), 6.96 (m, 1H), 6.88 (m, 1H), 4.98 (s, 2H).

### Beispiel No. I.28-49:

¹H-NMR (400 MHz, CDCl₃ δ, ppm) 8.28 (d, 1H), 8.10 (dd, 1H), 7.77 (dd, 1H), 7.45 (m, 1H), 7.24 (m, 1H), 7.16 (dd, 1H), 4.94 (s, 2H), 3.81 (s, 2H).

### Beispiel No. I.30-48:

¹H-NMR (400 MHz, DMSO-d⁶ δ, ppm) 13.10 (bs, 1H), 8.40 (m, 1H), 8.13-8.07 (m, 2H), 7.73-7.67 (m, 2H), 7.39 (m, 1H), 4.87 (s, 2H).

### Beispiel No. I.30-4:

¹H-NMR (400 MHz, DMSO-d⁶ δ, ppm) 13.11 (bs, 1H), 8.48 (d, 1H), 8.11 (dd, 1H), 7.98 (dd, 1H), 7.47-7.42 (m, 2H), 7.31-7.27 (m,2H), 4.87 (s, 2H).

### Beispiel No. I.28-7:

¹H-NMR (400 MHz, CDCl₃ δ, ppm) 8.35 (d, 1H), 7.98 (dd, 1H), 7.77 (dd, 1H), 7.39-7.36 (m, 1H), 7.32-7.29 (m, 2H), 4.95 (s, 2H), 3.81 (s, 3H).

### Beispiel No. I.31-48:

¹H-NMR (400 MHz, CDCl₃ δ, ppm) 8.18 (m, 1H), 8.13 (d, 1H), 7.50-7.45 (m, 2H), 7.21 (dd, 1H), 7.10 (m, 2H), 4.95 (s, 2H), 3.80 (s, 3H).

### Beispiel No. I.67-38:

¹H-NMR (400 MHz, CDCl₃ δ, ppm) 8.27 (d, 1H), 8.11 (dd, 1H), 7.16 (dd, 1H), 7.49 (m, 1H), 6.99 (m, 1H), 6.89 (m, 1H), 4.92 (s, 2H), 4.49 (t, 2H), 3.67 (s, 3H), 2.69 (t, 2H).

### Beispiel No. I.32-7:

¹H-NMR (400 MHz, CDCl₃ δ, ppm) 8.18 (d, 1H), 7.98 (dd, 1H), 7.42 (m, 1H), 7.31-7.28 (m, 2H), 7.24-7.19 (m, 2H), 4.85 (s, 2H), 4.20 (q, 2H), 1.22 (t, 3H).

### Beispiel No. I.31-7:

¹H-NMR (400 MHz, CDCl₃ δ, ppm) 8.25 (d, 1H), 8.05 (dd, 1H), 7.49 (m, 1H), 7.38-7.35 (m, 2H), 7.32-7.29 (m, 2H), 4.95 (s, 2H), 3.81 (s, 3H).

### Beispiel No. I.28-48:

¹H-NMR (400 MHz, CDCl₃ δ, ppm) 8.22 (d, 1H), 8.11 (dd, 1H), 7.75 (dd, 1H), 7.46 (dd, 1H), 7.21 (m, 1H), 7.10 (m, 1H), 4.95 (s, 2H), 3.80 (s, 3H).

### Beispiel No. I.33-49:

¹H-NMR (400 MHz, DMSO-d⁶ δ, ppm) 13.13 (bs, 1H), 8.43 (d, 1H), 8.16 (m, 1H), 7.94 (m, 1H), 7.70-7.63 (m, 2H), 7.45 (m, 1H), 4.88 (s, 2H).

### Beispiel No. I.33-38:

¹H-NMR (400 MHz, DMSO-d⁶ δ, ppm) 13.12 (bs, 1H), 8.41 (d, 1H), 8.15 (m, 1H), 7.93 (m, 1H), 7.67 (m, 1H), 7.50 (m, 1H), 7.25 (m, 1H), 4.87 (s, 2H).

### Beispiel No. I.31-2:

¹H-NMR (400 MHz, CDCl₃ δ, ppm) 8.17-8.14 (m, 2H), 7.54-7.41 (m, 3H), 7.27 (m, 1H), 7.12 (m, 1H), 4.95 (s, 2H), 3.81 (s, 3H).

### Beispiel No. I.32-49:

¹H-NMR (400 MHz, CDCl₃ δ, ppm) 8.20-8.17 (m, 2H), 7.52-7.43 (m, 2H), 7.24 (m, 1H), 7.16 (dd, 1H), 4.92 (s, 2H), 4.27 (q, 2H), 1.29 (t, 3H).

### Beispiel No. I.30-2:

¹H-NMR (400 MHz, DMSO-d⁶ δ, ppm) 13.12 (bs, 1H), 8.41 (d, 1H), 8.13 (dd, 1H), 8.06 (dd, 1H), 7.61-7.54 (m, 2H), 7.41-7.33 (m, 2H), 4.88 (s, 2H).

### Beispiel No. I.28-2:

¹H-NMR (400 MHz, CDCl₃ δ, ppm) 8.25 (d, 1H), 8.08 (dd, 1H), 7.75 (dd, 1H), 7.51 (m, 1H), 7.42 (m, 1H), 7.25 (m, 1H), 7.12 (m, 1H), 4.95 (s, 2H), 3.81 (s, 3H).

### Beispiel No. I.68-38:

¹H-NMR (400 MHz, CDCl₃ δ, ppm) 8.26 (d, 1H), 8.11 (dd, 1H), 7.76 (dd, 1H), 7.49 (m, 1H), 6.99 (m, 1H), 6.88 (m, 1H), 4.97 (s, 2H), 4.26 (m, 1H), 3.77 (m, 1H), 1.98-1.85 (m, 3H), 1.60 (m, 1H).

### Beispiel No. I.33-7:

¹H-NMR (400 MHz, DMSO-d⁶ δ, ppm) 13.10 (bs, 1H), 8.47 (d, 1H), 8.05 (m, 1H), 7.92 (m, 1H), 7.53-7.49 (m, 2H), 7.42-7.38 (m, 2H), 4.87 (s, 2H).

### Beispiel No. I.33-4:

¹H-NMR (400 MHz, DMSO-d⁶ δ, ppm) 13.10 (bs, 1H), 8.45 (d, 1H), 8.04 (m, 1H), 7.92 (m, 1H), 7.45-7.40 (m, 2H), 7.32-7.27 (m, 2H), 4.86 (s, 2H).

### Beispiel No. I.31-4:

¹H-NMR (400 MHz, CDCl₃ δ, ppm) 8.24 (d, 1H), 8.04 (dd, 1H), 7.49 (m, 1H), 7.36-7.33 (m, 2H), 7.11-7.07 (m, 2H), 4.95 (s, 2H), 3.81 (s, 3H).

### Beispiel No. I.29-7:

¹H-NMR (400 MHz, CDCl₃ δ, ppm) 8.36 (m, 1H), 7.99 (m, 1H), 7.76 (dd, 1H), 7.38-7.36 (m, 2H), 7.32-7.29 (m, 2H), 4.92 (s, 2H), 4.28 (q, 2H), 1.30 (t, 3H).

### Beispiel No. I.32-38:

¹H-NMR (400 MHz, CDCl₃ δ, ppm) 8.20-8.16 (m, 2H), 7.52-7.46 (m, 2H), 6.99 (m, 1H), 6.89 (m, 1H), 4.92 (s, 2H), 4.28 (q, 2H), 1.29 (t, 3H).

### NMR-Peak-Listenverfahren

### Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾;......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu ¹H-NMR-Peaklisten können der Research Disclosure Database Number 564025 entnommen werden.

| |
|---|
| I.64-1: ¹H-NMR(400.2 MHz, d₆-DMSO): |
| δ= 8.4662 (0.8); 8.4624 (1.3); 8.4590 (0.9); 8.4548 (1.0); 8.4508 (1.4); 8.4476 (0.8); 7.9534 (0.7); 7.9504 (0.8); 7.9319 (1.0); 7.9288 (1.5); 7.9256 (0.9); 7.9071 (0.9); 7.9041 (0.9); 7.6910 (0.9); 7.6800 (1.4); 7.6691 (1.4); 7.6584 (1.2); 7.6475 (0.7); 7.4316 (0.6); 7.4272 (0.5); 7.4198 (2.1); 7.4154 (1.5); 7.4131 (1.6); 7.4057 (3.7); 7.4016 (4.5); 7.3964 (0.9); 7.3942 (0.9); 7.3899 (0.6); 7.2950 (0.5); 7.2894 (2.7); 7.2827 (1.7); 7.2803 (1.2); 7.2779 (1.4); 7.2749 (1.0); 7.2696 (1.9); 7.2651 (1.7); 5.0148 (7.8); 3.7208 (16.0); 3.7105 (0.4); 3.3411 (38.8); 3.3174 (3.6); 2.5120 (4.8); 2.5076 (9.9); 2.5031 (13.6); 2.4985 (9.8); 2.4941 (4.7); 2.0766 (0.3); -0.0001 (0.8) |
| I.66-43: ¹H-NMR(400.2 MHz, d₆-DMSO): |
| δ= 8.2864 (3.1); 8.2751 (3.3); 7.9944 (1.7); 7.9716 (2.8); 7.9468 (1.9); 7.6758 (0.6); 7.6597 (1.3); 7.6544 (1.3); 7.6473 (2.0); 7.6371 (4.7); 7.6257 (3.5); 7.6156 (3.6); 7.6043 (1.7); 7.3404 (3.7); 7.3192 (6.6); 7.2984 (3.2); 4.8876 (16.0); 3.5078 (0.6); 3.3336 (31.0); 2.6721 (0.6); 2.5071 (55.8); 2.5028 (77.5); 2.4986 (61.0); 2.3298 (0.6); 2.0765 (0.8); 1.2343 (0.3); -0.0001 (3.5) |
| I.64-38: ¹H-NMR(300.1 MHz, d₆-DMSO): |
| δ= 8.3372 (1.0); 8.3326 (1.5); 8.3279 (1.0); 8.3221 (1.1); 8.3173 (1.6); 8.3130 (1.0); 7.9881 (0.8); 7.9842 (0.8); 7.9595 (1.1); 7.9549 (1.4); 7.9498 (0.9); 7.9250 (1.0); 7.9211 (0.9); 7.7196 (0.6); 7.6997 (0.7); 7.6902 (1.2); 7.6705 (1.2); 7.6608 (0.8); 7.6549 (1.0); 7.6408 (2.1); 7.6261 (1.5); 7.6119 (1.2); 7.5974 (0.7); 7.5085 (0.7); 7.4994 (0.7); 7.4783 (0.8); 7.4724 (0.9); 7.4699 (0.9); 7.4641 (0.8); 7.4431 (0.6); 7.4339 (0.7); 7.2644 (0.5); 7.2597 (0.5); 7.2554 (0.5); 7.2509 (0.5); 7.2323 (0.9); 7.2298 (0.9); 7.2075 (0.4); 7.2027 (0.5); 7.1984 (0.4); 7.1940 (0.4); 5.0122 (7.9); 3.7207 (16.0); 3.6743 (0.4); 3.3403 (7.6); 3.3170 (0.5); 2.5193 (1.6); 2.5136 (2.9); 2.5077 (3.8); 2.5019 (2.7); 2.0798 (1.5) |
| I.66-38: ¹H-NMR(300.1 MHz, d₆-DMSO): |
| δ= 8.3338 (2.3); 8.3292 (3.4); 8.3246 (2.4); 8.3187 (2.6); 8.3139 (3.6); 7.9852 (1.8); 7.9814 (1.8); 7.9566 (2.5); 7.9521 (3.1); 7.9470 (2.0); 7.9222 (2.3); 7.9183 (2.0); 7.7089 (1.3); 7.6890 (1.5); 7.6795 (2.7); 7.6598 (2.8); 7.6498 (3.5); 7.6353 (3.8); 7.6209 (3.5); 7.6067 (2.7); 7.5922 (1.6); 7.5073 (1.4); 7.4982 (1.5); 7.4769 (1.8); 7.4713 (2.2); 7.4628 (1.8); 7.4417 (1.5); 7.4326 (1.5); 7.2617 (1.1); 7.2570 (1.2); 7.2524 (1.1); 7.2298 (2.0); 7.2270 (2.0); 7.2047 (1.1); 7.1999 (1.1); 7.1957 (1.0); 4.8740 (16.0); 3.3363 (12.6); 3.1574 (0.4); 3.1171 (0.4); 3.0925 (0.6); 3.0678 (0.5); 2.7288 (0.3); 2.5085 (44.8); 2.5027 (57.1); 2.4970 (40.7); 2.2724 (0.4); 2.0759 (1.1); 1.2371 (0.4); 1.1906 (0.4); 1.1665 (0.9); 1.1422 (0.6); -0.0005 (1.6) |
| I.66-49: ¹H-NMR(300.1 MHz, de-DMSO): |
| δ= 13.0926 (1.1); 8.2738 (4.2); 8.2587 (4.5); 7.9648 (2.1); 7.9610 (2.2); 7.9320 (3.7); 7.9014 (2.7); 7.8978 (2.6); 7.7279 (3.1); 7.7092 (3.4); 7.6982 (3.9); 7.6797 (3.8); 7.6622 (4.0); 7.6530 (4.3); 7.6336 (4.2); 7.6242 (4.2); 7.6142 (2.8); 7.6000 (4.2); 7.5855 (4.4); 7.5713 (3.3); 7.5570 (2.0); 7.4050 (2.2); 7.3956 (2.2); 7.3774 (3.2); 7.3681 (3.1); 7.3486 (2.0); 7.3392 (1.9); 4.8661 (16.0); 3.3304 (1.6); 2.5081 (35.9); 2.5023 (46.9); 2.4965 (33.4); 2.3798 (0.4); 2.2711 (0.4); 2.0765 (1.6); 1.7527 (0.6); -0.0005 (12.0) |
| 1.66-1: ¹H-NMR(300.1 MHz, d₆-DMSO): |
| δ= 13.0504 (0.4); 13.0353 (0.4); 12.9713 (0.4); 8.4644 (4.7); 8.4525 (4.8); 7.9534 (2.2); 7.9233 (4.2); 7.8922 (2.6); 7.6941 (2.2); 7.6799 (3.6); 7.6660 (3.5); 7.6518 (3.0); 7.3993 (14.7); 7.2871 (7.8); 7.2805 (7.7); 7.2629 (6.6); 4.8860 (16.0); 3.3302 (4.5); 3.1683 (0.9); 2.7504 (0.4); 2.5021 (25.9); 2.0745 (7.8); -0.0007 (1.0) |
| 1.64-2: ¹H-NMR(400.1 MHz, de-DMSO): |
| δ= 8.3138 (1.6); 8.3024 (1.7); 7.9663 (0.8); 7.9643 (0.9); 7.9418 (1.5); 7.9193 (1.0); 7.9171 (1.0); 7.6372 (0.8); 7.6264 (1.4); 7.6156 (1.5); 7.6049 (1.3); 7.5991 (0.9); 7.5946 (1.4); 7.5797 (1.3); 7.5759 (1.6); 7.5597 (0.8); 7.5563 (1.0); 7.5506 (0.5); 7.5463 (0.5); 7.5372 (0.5); 7.5291 (0.8); 7.5173 (0.9); 7.5114 (0.9); 7.5068 (0.6); 7.4976 (0.6); 7.4939 (0.5); 7.3596 (1.0); 7.3420 (1.8); 7.3363 (1.5); 7.3247 (2.0); 7.3145 (1.0); 7.3120 (1.0); 7.3062 (1.0); 5.7564 (0.3); 5.0101 (8.8); 3.7187 (16.0); 3.3204 (4.2); 2.5071 (2.9); 2.5030 (3.9); 2.4988 (3.1); -0.0004 (0.6) |
| I.64-43: ¹H-NMR(300.1 MHz, de-DMSO): |
| δ= 8.2935 (0.9); 8.2888 (1.4); 8.2841 (0.9); 8.2784 (1.0); 8.2736 (1.4); 8.2690 (0.8); 8.0094 (0.8); 8.0054 (0.8); 7.9808 (1.0); 7.9763 (1.2); 7.9706 (0.8); 7.9459 (0.9); 7.9419 (0.8); 7.6712 (0.6); 7.6627 (0.6); 7.6587 (1.0); 7.6434 (2.2); 7.6300 (1.6); 7.6150 (1.6); 7.6013 (0.7); 7.5926 (0.4); 7.3514 (1.8); 7.3234 (2.9); 7.2956 (1.4); 7.2911 (0.8); 5.0152 (7.8); 3.7106 (16.0); 3.3373 (20.7); 3.3137 (2.8); 2.5158 (4.6); 2.5100 (9.0); 2.5040 (11.9); 2.4981 (8.2); 2.4924 (3.8); 2.0777 (0.6); -0.0003 (0.8) |
| I.66-2: ¹H-NMR(400.1 MHz, de-DMSO): |
| δ= 8.3117 (4.4); 8.3014 (4.8); 7.9586 (2.3); 7.9349 (4.1); 7.9117 (2.7); 7.6307 (1.9); 7.6203 (3.4); 7.6095 (3.5); 7.5987 (3.5); 7.5888 (3.8); 7.5697 (4.4); 7.5506 (2.8); 7.5227 (2.8); 7.5084 (2.9); 7.4904 (1.8); 7.3520 (3.0); 7.3397 (3.9); 7.3229 (7.0); 7.3035 (4.4); 5.7527 (14.0); 4.8638 (16.0); 3.5072 (1.0); 3.3312 (6.1); 3.0664 (0.4); 2.6724 (0.4); 2.5010 (27.5); 1.2348 (1.3); -0.0012 (0.4) |
| I.64-49: ¹H-NMR(300.1 MHz, d₆-DMSO): |
| δ= 8.2785 (0.9); 8.2735 (1.4); 8.2688 (0.9); 8.2633 (1.0); 8.2582 (1.4); 8.2537 (0.8); 7.9665 (0.8); 7.9625 (0.8); 7.9380 (1.0); 7.9336 (1.1); 7.9277 (0.8); 7.9032 (0.9); 7.8991 (0.9); 7.7317 (1.2); 7.7131 (1.3); 7.7021 (1.4); 7.6835 (1.4); 7.6671 (1.3); 7.6578 (1.4); 7.6385 (1.4); 7.6292 (1.4); 7.6170 (0.9); 7.6031 (1.3); 7.5884 (1.5); 7.5740 (1.0); 7.5598 (0.6); 7.4045 (0.8); 7.3951 (0.7); 7.3773 (1.1); 7.3753 (1.0); 7.3678 (0.9); 7.3659 (0.9); 7.3479 (0.7); 7.3385 (0.6); 4.9942 (7.3); 3.7090 (16.0); 3.3437 (84.2); 3.3209 (0.7); 2.5157 (5.5); 2.5098 (10.8); 2.5038 (14.4); 2.4978 (9.9); 2.4920 (4.5) |
| I.71-38: ¹H-NMR(400.6 MHz, CDCl3): |
| δ= 8.2696 (2.5); 8.2679 (2.7); 8.2635 (2.7); 8.2618 (2.6); 8.1166 (3.1); 8.1148 (3.0); 8.0954 (3.5); 8.0936 (3.4); 7.7759 (3.5); 7.7698 (3.3); 7.7547 (3.0); 7.7485 (3.0); 7.5051 (0.9); 7.4906 (0.9); 7.4834 (1.2); 7.4689 (1.2); 7.4624 (1.0); 7.4480 (0.9); 7.2625 (8.0); 7.0105 (0.6); 7.0068 (0.6); 7.0036 (0.7); 7.0000 (0.7); 6.9910 (0.7); 6.9876 (0.9); 6.9844 (1.1); 6.9810 (1.0); 6.9779 (0.7); 6.9689 (0.6); 6.9652 (0.6); 6.9620 (0.6); 6.9584 (0.6); 6.9165 (1.0); 6.9097 (0.8); 6.8953 (1.1); 6.8921 (1.1); 6.8886 (0.9); 6.8853 (0.9); 6.8710 (1.0); 6.8642 (0.8); 5.3000 (2.5); 4.9296 (16.0); 4.1922 (4.0); 4.1756 (8.5); 4.1590 (4.1); 1.7023 (1.2); 1.7006 (1.8); 1.6839 (3.7); 1.6823 (2.1); 1.6654 (3.7); 1.6487 (1.9); 1.6473 (1.4); 1.6304 (0.6); 1.5846 (2.3); 0.9314 (7.2); 0.9129 (15.2); 0.8943 (6.6); -0.0002 (12.2); -0.0028 (0.5) |
| I.53-49: ¹H-NMR(400.0 MHz, CDCl3): |
| δ= 8.5991 (3.7); 8.5933 (3.7); 8.4218 (3.6); 8.4172 (3.6); 7.9736 (2.6); 7.9682 (4.1); 7.9630 (2.5); 7.5168 (1.8); 7.4958 (2.9); 7.4761 (2.3); 7.3446 (1.5); 7.3414 (1.6); 7.3392 (1.7); 7.3362 (1.6); 7.3232 (1.1); 7.3200 (1.2); 7.3178 (1.4); 7.3148 (1.3); 7.2621 (29.6); 7.2539 (2.4); 7.2484 (2.0); 7.2303 (2.2); 7.2249 (2.0); 4.9177 (16.0); 4.3074 (2.1); 4.2895 (6.4); 4.2717 (6.4); 4.2538 (2.1); 1.6090 (0.8); 1.3169 (7.4); 1.2990 (14.9); 1.2812 (7.2); 0.8818 (0.6); 0.0078 (0.6); -0.0002 (17.9); -0.0085 (0.6) |
| I.52-49: ¹H-NMR(400.0 MHz, CDCl3): |
| δ= 8.6011 (1.5); 8.5963 (1.5); 8.4193 (1.7); 7.9738 (1.6); 7.9685 (2.4); 7.9633 (1.5); 7.5204 (1.2); 7.4994 (1.9); 7.4796 (1.4); 7.3457 (1.0); 7.3406 (1.3); 7.3376 (1.1); 7.3191 (1.0); 7.2625 (14.2); 7.2548 (1.8); 7.2492 (1.4); 7.2311 (1.4); 7.2258 (1.3); 5.3003 (1.3); 4.9419 (9.4); 3.8162 (16.0); 1.5845 (0.7); 0.0699 (0.9); 0.0079 (0.5); -0.0002 (8.6) |
| I.54-49: ¹H-NMR(400.6 MHz, d₆-DMSO): |
| δ= 13.1310 (1.5); 8.7643 (5.6); 8.7638 (5.6); 8.7583 (5.7); 8.7578 (5.7); 8.5261 (5.6); 8.5216 (5.7); 8.0226 (4.5); 8.0177 (5.5); 8.0168 (5.4); 8.0119 (4.4); 7.8009 (2.5); 7.7804 (6.3); 7.7756 (3.4); 7.7590 (3.4); 7.7560 (3.2); 7.7504 (2.9); 7.5598 (2.1); 7.5568 (2.2); 7.5540 (2.0); 7.5514 (1.9); 7.5384 (1.8); 7.5354 (1.9); 7.5325 (1.7); 7.5300 (1.6); 4.8804 (16.0); 3.3231 (14.1); 2.5247 (1.0); 2.5200 (1.6); 2.5112 (25.2); 2.5066 (55.1); 2.5021 (77.0); 2.4975 (54.4); 2.4930 (24.9); 0.0080 (1.8); -0.0002 (64.0); -0.0085 (2.0) |
| I.47-4: ¹H-NMR(400.6 MHz, CDCl3): |
| δ= 8.5511 (2.2); 8.5487 (2.1); 8.5389 (2.3); 8.5364 (2.1); 8.4700 (3.2); 8.4660 (3.1); 7.5556 (1.6); 7.5418 (2.2); 7.5289 (1.6); 7.2692 (2.7); 7.2630 (13.1); 7.2576 (2.8); 7.2522 (1.7); 7.2467 (3.4); 7.2407 (1.0); 7.2350 (3.2); 7.0991 (3.5); 7.0934 (1.0); 7.0819 (1.2); 7.0791 (3.9); 7.0766 (3.0); 7.0735 (1.1); 7.0621 (0.9); 7.0565 (2.6); 5.3006 (4.4); 4.9335 (15.7); 4.3112 (2.0); 4.2934 (6.3); 4.2756 (6.4); 4.2578 (2.1); 1.5923 (1.5); 1.3219 (7.8); 1.3041 (16.0); 1.2863 (7.7); 1.2563 (0.7); -0.0002 (18.0); -0.0085 (0.6) |
| I.47-38: ¹H-NMR(400.6 MHz, CDCl3): |
| δ= 8.5307 (2.2); 8.5281 (2.2); 8.5184 (2.3); 8.5159 (2.3); 8.4634 (3.3); 8.4592 (3.3); 7.5482 (1.7); 7.5345 (2.3); 7.5213 (1.7); 7.4926 (0.8); 7.4784 (0.8); 7.4711 (1.3); 7.4569 (1.3); 7.4497 (0.9); 7.4355 (0.8); 7.2633 (9.7); 7.0158 (0.6); 7.0120 (0.6); 7.0090 (0.7); 7.0053 (0.6); 6.9967 (0.6); 6.9932 (1.1); 6.9899 (1.2); 6.9865 (1.2); 6.9831 (0.6); 6.9745 (0.5); 6.9707 (0.6); 6.9677 (0.6); 6.9641 (0.6); 6.9075 (1.0); 6.9008 (0.9); 6.8869 (1.1); 6.8827 (1.2); 6.8803 (1.0); 6.8761 (1.0); 6.8622 (1.0); 6.8554 (0.9); 5.3007 (6.6); 4.9293 (16.0); 4.3087 (2.0); 4.2909 (6.2); 4.2731 (6.3); 4.2552 (2.1); 2.0454 (0.8); 1.5992 (1.1); 1.3153 (7.5); 1.2975 (15.3); 1.2797 (7.6); 1.2640 (1.4); 1.2597 (1.3); 0.8987 (0.6); 0.8818 (2.2); 0.8642 (0.9); -0.0002 (13.6) |
| I.44-48: ¹H-NMR(400.6 MHz, CDCl3): |
| δ= 8.6212 (5.1); 8.6202 (5.2); 8.5755 (4.3); 8.5632 (4.4); 7.4115 (1.7); 7.3917 (2.0); 7.3902 (2.5); 7.3844 (2.9); 7.3710 (4.2); 7.2639 (9.7); 7.2335 (1.4); 7.2304 (1.5); 7.2281 (1.5); 7.2250 (1.5); 7.2121 (1.1); 7.2090 (1.1); 7.2066 (1.2); 7.2036 (1.2); 7.1323 (2.1); 7.1269 (1.8); 7.1082 (2.0); 7.1028 (1.8); 5.3008 (6.0); 4.9312 (15.1); 4.3067 (2.0); 4.2889 (6.1); 4.2711 (6.2); 4.2532 (2.0); 1.6093 (1.4); 1.3145 (7.7); 1.2967 (16.0); 1.2789 (7.6); -0.0002 (14.2) |
| I.54-38: ¹H-NMR(400.6 MHz, d₆-DMSO): |
| δ= 13.1423 (0.6); 8.7570 (5.6); 8.7562 (5.7); 8.7511 (5.7); 8.7503 (5.8); 8.5232 (5.5); 8.5226 (5.3); 8.5185 (5.6); 8.5178 (5.4); 7.9892 (5.1); 7.9844 (5.8); 7.9833 (5.8); 7.9785 (5.0); 7.8760 (1.1); 7.8613 (1.2); 7.8540 (2.1); 7.8393 (2.1); 7.8320 (1.2); 7.8173 (1.2); 7.6136 (1.1); 7.6068 (1.1); 7.5912 (1.3); 7.5874 (1.4); 7.5845 (1.4); 7.5806 (1.3); 7.5651 (1.1); 7.5582 (1.1); 7.3817 (0.8); 7.3781 (0.9); 7.3746 (0.7); 7.3713 (0.7); 7.3612 (1.0); 7.3591 (1.2); 7.3578 (1.3); 7.3555 (1.2); 7.3520 (1.2); 7.3390 (0.8); 7.3354 (0.8); 7.3319 (0.7); 7.3287 (0.6); 5.7574 (16.0); 4.8772 (15.8); 2.5259 (0.6); 2.5212 (0.8); 2.5125 (11.2); 2.5079 (24.7); 2.5033 (34.6); 2.4987 (23.7); 2.4941 (10.4); 0.0079 (1.0); 0.0030 (0.8); 0.0022 (1.4); -0.0002 (39.2); -0.0028 (1.7); -0.0036 (1.2); -0.0044 (0.7); -0.0052 (0.5); -0.0085 (1.2) |
| I.44-38: ¹H-NMR(400.6 MHz, CDCl3): |
| δ= 8.6157 (5.4); 8.5685 (4.5); 8.5562 (4.6); 7.4635 (0.9); 7.4493 (0.9); 7.4418 (1.3); 7.4277 (1.3); 7.4206 (1.0); 7.4065 (1.0); 7.3802 (3.0); 7.3679 (2.9); 7.2634 (11.1); 6.9822 (0.6); 6.9785 (0.6); 6.9755 (0.7); 6.9718 (0.6); 6.9632 (0.7); 6.9597 (1.2); 6.9563 (1.2); 6.9530 (1.2); 6.9495 (0.6); 6.9410 (0.6); 6.9372 (0.6); 6.9342 (0.6); 6.9306 (0.6); 6.8679 (1.0); 6.8612 (0.9); 6.8472 (1.1); 6.8429 (1.2); 6.8407 (1.0); 6.8363 (0.9); 6.8224 (1.0); 6.8157 (0.8); 5.3007 (4.6); 4.9325 (15.9); 4.3074 (2.0); 4.2896 (6.3); 4.2718 (6.4); 4.2540 (2.1); 2.0453 (1.9); 1.5927 (1.8); 1.3150 (7.9); 1.2972 (16.0); 1.2793 (7.7); 1.2594 (1.2); 1.2416 (0.6); -0.0002 (16.6) |
| I.44-4: ¹H-NMR(400.6 MHz, CDCl3): |
| δ= 8.6421 (3.6); 8.6082 (2.7); 8.5959 (2.8); 7.4235 (2.6); 7.4220 (2.6); 7.4112 (2.5); 7.4097 (2.5); 7.2639 (9.0); 7.2204 (2.4); 7.2146 (0.8); 7.2088 (2.6); 7.2032 (1.6); 7.1975 (3.3); 7.1917 (1.0); 7.1892 (0.6); 7.1859 (3.2); 7.0563 (3.3); 7.0505 (0.9); 7.0391 (1.0); 7.0363 (3.5); 7.0335 (2.7); 7.0305 (1.0); 7.0192 (0.8); 7.0135 (2.5); 5.3007 (4.4); 4.9393 (14.1); 4.3106 (1.9); 4.2928 (5.7); 4.2750 (5.8); 4.2572 (1.9); 1.3204 (7.6); 1.3026 (16.0); 1.2977 (0.5); 1.2847 (7.5); -0.0002 (14.0) |
| I.43-38: ¹H-NMR(400.6 MHz, CDCl3): |
| δ= 8.6172 (3.1); 8.5691 (2.5); 8.5568 (2.6); 7.4670 (0.5); 7.4528 (0.5); 7.4452 (0.8); 7.4311 (0.7); 7.4241 (0.6); 7.4099 (0.6); 7.3816 (1.7); 7.3693 (1.7); 7.2635 (5.7); 6.9613 (0.7); 6.9579 (0.7); 6.9546 (0.7); 6.8694 (0.6); 6.8626 (0.5); 6.8487 (0.6); 6.8444 (0.7); 6.8421 (0.6); 6.8378 (0.5); 6.8239 (0.6); 6.8172 (0.5); 5.3008 (1.8); 4.9545 (8.7); 3.8179 (16.0); 1.5938 (2.1); 0.0700 (0.5); -0.0002 (8.4) |
| I.46-38: ¹H-NMR(400.6 MHz, CDCl3): |
| δ= 8.5323 (1.0); 8.5200 (1.0); 8.4646 (1.3); 8.4610 (1.3); 7.5511 (0.8); 7.5372 (1.2); 7.5242 (0.8); 7.4741 (0.7); 7.4599 (0.7); 7.4527 (0.5); 7.2635 (5.8); 6.9946 (0.6); 6.9912 (0.7); 6.9879 (0.7); 6.9089 (0.6); 6.8883 (0.6); 6.8841 (0.7); 6.8817 (0.6); 6.8775 (0.5); 6.8636 (0.6); 5.3008 (3.8); 4.9519 (8.7); 3.8182 (16.0); 1.5974 (2.6); 0.0701 (0.5); - 0.0002 (8.3) |
| I.46-4: ¹H-NMR(400.6 MHz, CDCl3): |
| δ= 8.5520 (0.9); 8.5399 (1.0); 8.4726 (1.3); 8.4692 (1.3); 7.5624 (0.8); 7.5491 (1.2); 7.5357 (0.8); 7.2717 (1.5); 7.2639 (5.6); 7.2601 (1.7); 7.2546 (1.0); 7.2490 (2.0); 7.2431 (0.6); 7.2374 (2.0); 7.1015 (2.0); 7.0958 (0.6); 7.0844 (0.6); 7.0815 (2.2); 7.0789 (1.9); 7.0759 (0.7); 7.0646 (0.5); 7.0588 (1.6); 4.9569 (8.6); 3.8198 (16.0); -0.0002 (7.8) |
| I.45-4: ¹H-NMR(400.6 MHz, d₆-DMSO): |
| δ= 13.1171 (0.9); 8.7606 (7.4); 8.7597 (7.4); 8.6974 (6.6); 8.6852 (7.1); 7.7467 (5.1); 7.7451 (5.0); 7.7344 (5.1); 7.7328 (5.0); 7.3695 (2.5); 7.3632 (1.1); 7.3571 (3.1); 7.3524 (1.6); 7.3512 (1.5); 7.3463 (5.7); 7.3401 (1.6); 7.3340 (5.4); 7.3262 (0.7); 7.3170 (0.7); 7.3095 (5.6); 7.3032 (1.3); 7.2963 (0.8); 7.2925 (1.2); 7.2883 (6.4); 7.2823 (1.4); 7.2715 (1.1); 7.2654 (2.8); 5.7572 (8.5); 4.8843 (16.0); 3.3266 (5.2); 2.5254 (0.6); 2.5206 (0.8); 2.5118 (14.2); 2.5073 (31.4); 2.5027 (44.4); 2.4981 (30.4); 2.4935 (13.3); 0.0080 (2.4); 0.0038 (0.8); 0.0021 (2.9); -0.0002 (97.9); - 0.0027 (3.6); -0.0043 (1.3); -0.0052 (1.0); -0.0060 (0.8); -0.0068 (0.7); -0.0085 (2.7); -0.0273 (0.5) |
| I.73-38: ¹H-NMR(400.6 MHz, CDCl3): |
| δ= 8.2690 (1.3); 8.2673 (1.2); 8.2629 (1.4); 8.2613 (1.2); 8.1169 (1.5); 8.1152 (1.3); 8.0957 (1.7); 8.0941 (1.4); 7.7756 (1.4); 7.7695 (1.4); 7.7545 (1.2); 7.7483 (1.2); 7.4932 (0.6); 7.4788 (0.6); 7.2637 (7.7); 6.9844 (0.5); 6.9812 (0.5); 6.8941 (0.5); 6.8909 (0.5); 4.9743 (7.3); 4.3757 (1.8); 4.3673 (0.9); 4.3641 (1.8); 4.3603 (0.9); 4.3523 (1.9); 3.6290 (2.1); 3.6221 (0.8); 3.6208 (1.0); 3.6172 (2.0); 3.6138 (1.0); 3.6055 (2.0); 3.3573 (16.0); 0.0709 (0.6); -0.0002 (4.8) |
| I.70-7: ¹H-NMR(400.6 MHz, CDCl3): |
| δ= 8.2579 (3.1); 8.2508 (3.1); 8.0705 (1.6); 8.0697 (1.6); 8.0595 (1.6); 8.0587 (1.6); 8.0487 (1.7); 8.0478 (1.8); 8.0377 (1.7); 8.0368 (1.7); 7.5205 (1.5); 7.5133 (1.4); 7.5005 (1.6); 7.4988 (1.6); 7.4933 (1.6); 7.4916 (1.5); 7.4788 (1.3); 7.4716 (1.2); 7.3851 (0.6); 7.3787 (4.2); 7.3731 (1.8); 7.3621 (2.3); 7.3564 (9.2); 7.3502 (1.5); 7.3216 (1.4); 7.3153 (9.2); 7.3096 (2.4); 7.2986 (1.8); 7.2930 (4.4); 7.2867 (0.5); 7.2634 (17.7); 5.3001 (3.4); 5.1807 (2.3); 5.1710 (4.9); 5.1614 (2.4); 4.9854 (16.0); 4.2588 (9.0); 4.2491 (8.8); 4.0037 (1.7); 3.9903 (2.2); 3.9859 (4.3); 3.9811 (3.0); 3.9759 (2.7); 3.9691 (3.5); 3.9454 (1.3); 3.9327 (1.2); 3.9091 (3.4); 3.9023 (2.6); 3.8971 (2.8); 3.8922 (4.1); 3.8879 (2.3); 3.8745 (1.6); 1.6052 (0.9); 0.0706 (1.0); -0.0002 (10.6) |
| I.48-4: ¹H-NMR(400.6 MHz, d₆-DMSO): |
| δ= 13.1240 (0.8); 8.6812 (4.5); 8.6768 (4.6); 8.5953 (3.2); 8.5925 (2.8); 8.5831 (3.3); 8.5803 (2.9); 7.6647 (2.3); 7.6524 (2.5); 7.6514 (2.3); 7.6495 (2.8); 7.6372 (2.4); 7.4527 (3.3); 7.4469 (1.1); 7.4406 (3.8); 7.4354 (1.9); 7.4299 (4.9); 7.4236 (1.5); 7.4213 (1.1); 7.4178 (4.6); 7.3474 (0.5); 7.3389 (5.0); 7.3330 (1.3); 7.3262 (0.7); 7.3218 (1.4); 7.3175 (6.6); 7.3118 (1.4); 7.3007 (1.2); 7.2949 (3.4); 5.7571 (7.3); 4.8854 (16.0); 3.3275 (7.2); 2.5250 (0.8); 2.5204 (1.2); 2.5116 (16.7); 2.5071 (36.4); 2.5025 (51.0); 2.4979 (34.7); 2.4933 (15.0); 1.3560 (0.7); 0.0080 (2.6); 0.0057 (0.5); 0.0049 (0.6); 0.0040 (0.9); 0.0031 (1.7); 0.0023 (3.1); -0.0002 (95.4); -0.0025 (3.5); -0.0041 (1.3); -0.0050 (0.9); -0.0058 (0.7); -0.0066 (0.6); -0.0085 (2.6) |
| I.43-4: ¹H-NMR(400.6 MHz, CDCl3): |
| δ= 8.6446 (2.6); 8.6092 (1.8); 8.5969 (1.8); 7.4288 (1.6); 7.4274 (1.7); 7.4165 (1.6); 7.4152 (1.7); 7.2637 (5.7); 7.2223 (1.5); 7.2166 (0.6); 7.2106 (1.6); 7.2051 (1.0); 7.1995 (2.2); 7.1936 (0.6); 7.1878 (2.1); 7.0589 (2.1); 7.0531 (0.6); 7.0417 (0.6); 7.0389 (2.2); 7.0362 (1.8); 7.0333 (0.7); 7.0219 (0.5); 7.0162 (1.6); 4.9620 (8.6); 3.8200 (16.0); - 0.0002 (8.4) |
| I.66-1: ¹H-NMR(300.1 MHz, d₆-DMSO): |
| δ= 13.0504 (0.4); 13.0353 (0.4); 12.9713 (0.4); 8.4644 (4.7); 8.4525 (4.8); 7.9534 (2.2); 7.9233 (4.2); 7.8922 (2.6); 7.6941 (2.2); 7.6799 (3.6); 7.6660 (3.5); 7.6518 (3.0); 7.3993 (14.7); 7.2871 (7.8); 7.2805 (7.7); 7.2629 (6.6); 4.8860 (16.0); 3.3302 (4.5); 3.1683 (0.9); 2.7504 (0.4); 2.5021 (25.9); 2.0745 (7.8); -0.0007 (1.0) |
| I.52-38: ¹H-NMR(400.6 MHz, CDCl3): |
| δ= 8.5938 (1.5); 8.5880 (1.5); 8.4329 (1.5); 8.4282 (1.5); 7.9529 (1.6); 7.9480 (2.1); 7.9473 (1.9); 7.9423 (1.6); 7.5616 (0.5); 7.5539 (0.7); 7.5398 (0.7); 7.5330 (0.6); 7.5188 (0.5); 7.2650 (6.6); 7.0776 (0.6); 7.0742 (0.7); 7.0709 (0.7); 6.9972 (0.6); 6.9767 (0.6); 6.9730 (0.6); 6.9701 (0.5); 6.9663 (0.5); 6.9525 (0.6); 5.3010 (2.2); 4.9446 (8.2); 3.8170 (16.0); 1.6105 (0.8); 0.0707 (1.7); -0.0002 (5.6) |
| I.50-49: ¹H-NMR(400.6 MHz, CDCl3): |
| δ= 8.5102 (1.0); 8.4124 (1.2); 7.6775 (1.0); 7.6731 (1.1); 7.6706 (1.1); 7.6663 (1.0); 7.6555 (1.0); 7.6510 (1.1); 7.6486 (1.1); 7.6442 (1.0); 7.5205 (1.8); 7.5006 (1.9); 7.4992 (2.3); 7.4797 (2.1); 7.3460 (1.4); 7.3428 (1.4); 7.3405 (1.5); 7.3374 (1.4); 7.3246 (1.1); 7.3215 (1.1); 7.3191 (1.2); 7.3160 (1.2); 7.2623 (36.6); 7.2573 (0.7); 7.2564 (0.6); 7.2539 (2.2); 7.2484 (1.8); 7.2303 (1.9); 7.2249 (1.8); 5.3005 (6.4); 4.9188 (14.5); 4.3076 (1.9); 4.2897 (5.9); 4.2719 (6.0); 4.2541 (1.9); 1.5821 (0.6); 1.3165 (7.8); 1.2987 (16.0); 1.2809 (7.5); 0.0698 (1.0); 0.0079 (0.6); -0.0002 (22.8); -0.0085 (0.8) |
| I.45-38: ¹H-NMR(400.6 MHz, d₆-DMSO): |
| δ= 13.1288 (0.7); 8.7719 (7.5); 8.7709 (7.4); 8.6513 (6.8); 8.6391 (7.2); 7.6961 (1.1); 7.6814 (1.2); 7.6740 (2.0); 7.6594 (2.1); 7.6521 (1.2); 7.6377 (5.6); 7.6363 (5.1); 7.6254 (4.5); 7.6239 (4.4); 7.5262 (1.1); 7.5193 (1.1); 7.5037 (1.2); 7.4995 (1.4); 7.4970 (1.3); 7.4928 (1.2); 7.4772 (1.1); 7.4704 (1.1); 7.2651 (0.8); 7.2614 (0.9); 7.2580 (0.7); 7.2547 (0.7); 7.2447 (1.0); 7.2424 (1.2); 7.2412 (1.3); 7.2388 (1.2); 7.2352 (1.2); 7.2224 (0.8); 7.2188 (0.8); 7.2153 (0.6); 7.2120 (0.6); 5.7573 (8.3); 4.8792 (16.0); 3.3270 (7.7); 2.5255 (1.0); 2.5209 (1.4); 2.5121 (16.1); 2.5076 (35.2); 2.5030 (49.3); 2.4983 (33.3); 2.4938 (14.2); 2.0865 (2.8); 1.3563 (1.5); 0.0080 (3.1); 0.0057 (0.7); 0.0048 (0.9); -0.0002 (113.8); -0.0085 (2.8) |
| I.43-49: ¹H-NMR(400.6 MHz, CDCl3): |
| δ= 8.6219 (3.2); 8.5761 (2.5); 8.5638 (2.5); 7.4145 (1.0); 7.3934 (1.6); 7.3858 (1.8); 7.3736 (3.0); 7.2631 (7.2); 7.2351 (0.9); 7.2320 (0.9); 7.2296 (0.9); 7.2265 (0.9); 7.2136 (0.6); 7.2105 (0.7); 7.2081 (0.7); 7.2051 (0.7); 7.1335 (1.2); 7.1281 (1.1); 7.1094 (1.2); 7.1039 (1.1); 5.3007 (1.4); 4.9530 (8.5); 3.8174 (16.0); 2.0454 (0.6); 1.5972 (1.0); 1.2642 (1.0); 1.2596 (1.1); 0.8985 (0.5); 0.8817 (1.8); 0.8639 (0.7); -0.0002 (10.2) |
| I.33-38: ¹H-NMR(400.6 MHz, d₆-DMSO): |
| δ= 13.1164 (0.9); 8.4178 (4.4); 8.4106 (4.5); 8.1708 (2.1); 8.1595 (2.2); 8.1487 (2.5); 8.1375 (2.5); 7.9495 (1.8); 7.9423 (1.7); 7.9278 (3.1); 7.9205 (2.8); 7.9060 (1.6); 7.8987 (1.4); 7.7082 (1.1); 7.6933 (1.2); 7.6863 (2.1); 7.6715 (2.2); 7.6645 (1.3); 7.6496 (1.1); 7.5246 (1.1); 7.5178 (1.1); 7.5019 (1.4); 7.4984 (1.5); 7.4952 (1.5); 7.4918 (1.4); 7.4760 (1.2); 7.4691 (1.1); 7.2735 (0.8); 7.2700 (0.9); 7.2664 (0.8); 7.2634 (0.8); 7.2494 (1.5); 7.2442 (1.4); 7.2307 (0.8); 7.2273 (0.8); 7.2237 (0.7); 7.2208 (0.6); 4.8749 (16.0); 2.5230 (0.6); 2.5142 (7.6); 2.5098 (16.0); 2.5052 (21.7); 2.5007 (15.2); 2.4962 (6.8); -0.0002 (13.7) |
| I.53-38: ¹H-NMR(400.6 MHz, CDCl3): |
| δ= 8.5928 (1.1); 8.5891 (1.1); 8.4312 (1.3); 7.9525 (2.3); 7.9476 (3.0); 7.9471 (2.9); 7.9419 (2.3); 7.5719 (0.8); 7.5578 (0.8); 7.5502 (1.1); 7.5360 (1.1); 7.5292 (0.9); 7.5151 (0.8); 7.2644 (12.7); 7.0987 (0.5); 7.0949 (0.6); 7.0919 (0.6); 7.0882 (0.6); 7.0798 (0.6); 7.0762 (1.0); 7.0728 (1.1); 7.0695 (1.1); 7.0660 (0.6); 7.0576 (0.5); 7.0538 (0.5); 7.0508 (0.5); 7.0471 (0.5); 6.9963 (0.9); 6.9896 (0.7); 6.9758 (0.9); 6.9721 (1.0); 6.9692 (0.9); 6.9654 (0.8); 6.9516 (0.9); 6.9449 (0.8); 4.9206 (14.1); 4.3085 (1.8); 4.2907 (5.8); 4.2728 (5.8); 4.2550 (1.9); 1.6154 (0.8); 1.3174 (7.6); 1.2995 (16.0); 1.2817 (7.4); -0.0002 (10.9) |
| I.51-49: ¹H-NMR(400.6 MHz, d₆-DMSO): |
| δ= 13.1516 (0.5); 8.7301 (5.6); 8.7232 (5.6); 8.4665 (3.0); 8.4626 (5.4); 8.4590 (2.8); 7.8527 (2.1); 7.8483 (2.2); 7.8457 (2.2); 7.8413 (2.0); 7.8293 (2.2); 7.8249 (2.4); 7.8223 (2.2); 7.8179 (2.0); 7.7973 (2.6); 7.7762 (5.0); 7.7709 (3.2); 7.7653 (3.1); 7.7554 (3.3); 7.7458 (2.9); 7.7402 (3.1); 7.5547 (2.3); 7.5517 (2.4); 7.5489 (2.1); 7.5462 (2.0); 7.5332 (1.9); 7.5302 (2.0); 7.5274 (1.8); 7.5248 (1.7); 5.7570 (10.4); 4.8744 (16.0); 3.3208 (3.6); 2.6705 (0.7); 2.6659 (0.5); 2.5242 (1.5); 2.5196 (2.4); 2.5109 (35.0); 2.5063 (77.4); 2.5017 (108.5); 2.4971 (76.2); 2.4925 (34.2); 2.4741 (0.7); 2.3288 (0.6); 1.2366 (0.9); 0.0079 (2.5); 0.0063 (0.8); 0.0054 (0.9); -0.0002 (86.0); -0.0052 (1.3); - 0.0060 (1.1); -0.0069 (0.9); -0.0085 (2.5) |
| I.69-38: ¹H-NMR(400.6 MHz, CDCl3): |
| δ= 8.2719 (3.1); 8.2702 (2.9); 8.2658 (3.3); 8.2641 (2.8); 8.1105 (3.5); 8.1087 (3.0); 8.0893 (4.0); 8.0876 (3.5); 7.7799 (3.3); 7.7738 (3.3); 7.7587 (3.0); 7.7526 (2.9); 7.5049 (0.9); 7.4904 (1.0); 7.4835 (1.4); 7.4691 (1.4); 7.4622 (1.0); 7.4478 (1.0); 7.2618 (45.5); 7.0145 (0.6); 7.0108 (0.7); 7.0077 (0.7); 7.0041 (0.7); 6.9950 (0.8); 6.9917 (1.0); 6.9885 (1.2); 6.9852 (1.2); 6.9820 (0.7); 6.9730 (0.6); 6.9692 (0.6); 6.9661 (0.6); 6.9625 (0.6); 6.9223 (1.0); 6.9156 (0.9); 6.9011 (1.2); 6.8979 (1.2); 6.8945 (1.0); 6.8912 (1.0); 6.8768 (1.0); 6.8701 (0.9); 5.3003 (2.0); 4.9369 (16.0); 4.2472 (1.4); 4.2308 (1.4); 4.2203 (2.2); 4.2039 (2.2); 4.1409 (2.4); 4.1209 (2.4); 4.1139 (1.5); 4.0940 (1.5); 3.8450 (0.7); 3.8312 (0.8); 3.8243 (1.5); 3.8105 (1.5); 3.8038 (1.2); 3.7989 (1.7); 3.7900 (1.1); 3.7811 (1.8); 3.7767 (2.1); 3.7589 (2.0); 3.7395 (1.2); 3.7218 (1.8); 3.7029 (1.5); 3.7009 (1.3); 3.6817 (0.8); 3.5492 (1.9); 3.5356 (2.0); 3.5270 (1.6); 3.5133 (1.7); 2.6108 (0.6); 2.5934 (0.8); 2.5758 (0.6); 2.0221 (0.6); 2.0096 (0.9); 2.0012 (0.5); 1.9966 (0.6); 1.9886 (0.8); 1.9768 (0.7); 1.9699 (0.5); 1.6438 (0.5); 1.6264 (0.7); 1.6118 (1.0); 1.6089 (0.8); 1.5923 (1.0); 1.5798 (0.6); 1.5775 (0.7); 1.5656 (2.8); 0.0697 (1.4); 0.0080 (0.7); -0.0002 (29.5); -0.0085 (0.8) |
| I.70-38: ¹H-NMR(400.6 MHz, CDCl3): |
| δ= 8.2704 (2.8); 8.2686 (3.0); 8.2642 (3.0); 8.2626 (3.0); 8.1166 (3.2); 8.1149 (3.2); 8.0955 (3.7); 8.0937 (3.6); 7.7758 (3.4); 7.7697 (3.2); 7.7546 (3.0); 7.7485 (3.0); 7.5179 (0.9); 7.5034 (0.9); 7.4963 (1.4); 7.4818 (1.4); 7.4753 (1.0); 7.4608 (1.0); 7.2616 (46.7); 7.0112 (0.6); 7.0075 (0.6); 7.0044 (0.7); 7.0008 (0.7); 6.9917 (0.7); 6.9883 (1.0); 6.9851 (1.2); 6.9818 (1.1); 6.9787 (0.7); 6.9696 (0.6); 6.9659 (0.6); 6.9628 (0.6); 6.9592 (0.6); 6.9154 (1.0); 6.9086 (0.9); 6.8942 (1.1); 6.8910 (1.2); 6.8876 (1.0); 6.8842 (1.0); 6.8698 (1.0); 6.8631 (0.9); 5.3003 (3.3); 5.1808 (2.3); 5.1711 (5.0); 5.1614 (2.4); 4.9832 (16.0); 4.2601 (8.9); 4.2504 (8.8); 3.9999 (1.6); 3.9865 (2.1); 3.9827 (3.7); 3.9816 (3.5); 3.9771 (3.0); 3.9719 (2.8); 3.9652 (3.6); 3.9417 (1.3); 3.9313 (1.3); 3.9077 (3.4); 3.9010 (2.6); 3.8958 (2.8); 3.8900 (3.7); 3.8866 (2.2); 3.8731 (1.6); 1.5636 (1.7); 0.0697 (1.9); 0.0079 (0.8); -0.0002 (29.7); -0.0052 (0.5); - 0.0085 (1.0) |
| I.48-38: ¹H-NMR(400.6 MHz, d₆-DMSO): |
| δ= 8.7056 (4.6); 8.7012 (4.7); 8.5792 (3.1); 8.5765 (2.9); 8.5670 (3.3); 8.5642 (3.0); 7.7367 (1.0); 7.7219 (1.1); 7.7146 (2.0); 7.7000 (2.0); 7.6927 (1.2); 7.6780 (1.1); 7.6312 (2.2); 7.6182 (2.5); 7.6161 (2.8); 7.6037 (2.3); 7.5748 (1.1); 7.5679 (1.1); 7.5524 (1.2); 7.5481 (1.4); 7.5458 (1.4); 7.5415 (1.2); 7.5259 (1.1); 7.5190 (1.1); 7.2857 (0.8); 7.2821 (0.8); 7.2787 (0.7); 7.2754 (0.7); 7.2620 (1.3); 7.2596 (1.3); 7.2561 (1.3); 7.2431 (0.8); 7.2395 (0.8); 7.2360 (0.7); 7.2328 (0.6); 4.8844 (16.0); 3.3354 (7.2); 2.5256 (0.8); 2.5208 (1.0); 2.5120 (17.6); 2.5075 (39.0); 2.5029 (54.9); 2.4983 (38.1); 2.4937 (17.1); 0.0080 (2.2); 0.0056 (0.5); -0.0002 (81.0); -0.0057 (1.0); -0.0066 (0.8); -0.0085 (2.3) |
| I.45-49: ¹H-NMR(400.6 MHz, d₆-DMSO): |
| δ= 8.7794 (8.1); 8.7787 (8.1); 8.6588 (6.7); 8.6466 (7.1); 7.7027 (2.7); 7.6971 (2.7); 7.6772 (2.7); 7.6716 (2.8); 7.6432 (4.7); 7.6418 (4.7); 7.6310 (5.2); 7.6295 (4.8); 7.6118 (4.3); 7.5911 (3.1); 7.4548 (2.2); 7.4518 (2.3); 7.4490 (2.0); 7.4464 (1.8); 7.4332 (1.7); 7.4303 (1.8); 7.4275 (1.6); 7.4249 (1.5); 4.8846 (16.0); 3.3386 (5.4); 2.5260 (0.8); 2.5213 (1.1); 2.5125 (16.3); 2.5080 (35.8); 2.5034 (50.2); 2.4988 (34.3); 2.4942 (15.0); 1.3563 (0.6); 0.0080 (1.8); 0.0048 (0.5); 0.0040 (0.7); -0.0002 (61.6); -0.0049 (0.7); -0.0058 (0.6); -0.0085 (1.6) |

Gegenstand der Erfindung ist auch das Verfahren zum Schützen von Kultur- oder Nutzpflanzen vor phytotoxischen Wirkungen von Agrochemikalien, wie Pestiziden, oder insbesondere Herbiziden, welche Schäden an den Kultur- oder Nutzpflanzen verursachen, dadurch gekennzeichnet, dass man Verbindungen der allgemeinen Formel (I) oder deren Salze als Safener anwendet, vorzugsweise eine effektive Menge der Verbindungen der allgemeinen Formel (I) oder deren Salzen auf die Pflanzen, Teile der Pflanzen oder deren Samen (oder Saatgut) appliziert.

Die Verbindungen der allgemeinen Formel (I) (= Safener) wie voranstehend genannt sind geeignet, zusammen mit Wirkstoffen (Pestiziden) zur selektiven Bekämpfung von Schadorganismen in einer Reihe von Pflanzenkulturen eingesetzt zu werden, beispielsweise in wirtschaftlich bedeutenden Kulturen wie Getreide (Weizen, Gerste, Triticale, Roggen, Reis, Mais, Hirse), Zuckerrübe, Zuckerrohr, Raps, Baumwolle, Sonnenblume, Erbsen, Bohnen und Soja. Die Herbizid-Safener-Kombinationen mit Safenern der allgemeinen Formel (I) sind auch geeignet zur Bekämpfung von Schadpflanzen auf Beeten und Flächen von Nutz- und Zierpflanzen, wie beispielsweise Rasenflächen mit Nutz- oder Zierrasen, speziell Weidelgras, Rispengras oder Bermudagras.

Bei den Nutz- und Kulturpflanzen, in denen die Herbizid-Safener-Kombinationen mit vorgenannten Verbindungen der allgemeinen Formel (I) verwendet werden können, sind auch gegenüber einigen Pestiziden ganz oder partiell tolerante Mutantenkulturen oder ganz oder partiell tolerante transgene Kulturen von Interesse, z. B. Maiskulturen, die gegenüber Glufosinate oder Glyphosate resistent sind, oder Sojakulturen, die gegen pflanzenschädigend wirkende Imidazolinone resistent sind. Der besondere Vorteil der neuartig eingesetzten Safener der allgemeinen Formel (I) ist jedoch ihre effektive Wirkung in Kulturen, welche normalerweise nicht ausreichend tolerant gegenüber den zur Anwendung zu bringenden Pestiziden sind.

Die Verbindungen der allgemeinen Formel (I) können zur gemeinsamen Anwendung mit Pestiziden gleichzeitig oder in beliebiger Reihenfolge mit den Wirkstoffen ausgebracht werden und sind dann in der Lage, schädliche Nebenwirkungen dieser Wirkstoffe bei Kulturpflanzen zu reduzieren oder völlig aufzuheben, ohne die Wirksamkeit dieser Wirkstoffe gegen unerwünschte Schadorganismen zu beeinträchtigen oder wesentlich zu reduzieren. Dabei können auch Schädigungen, welche durch die Anwendung mehrerer Pestizide entstehen, z.B. durch mehrere Herbizide oder durch Herbizide in Kombination mit Insektiziden oder Fungiziden, wesentlich reduziert oder völlig aufgehoben werden. Hierdurch kann das Einsatzgebiet herkömmlicher Pestizide ganz erheblich erweitert werden.

Für den Fall, dass die erfindungsgemäßen Mittel Pestizide enthalten, werden diese Mittel nach entsprechender Verdünnung entweder direkt auf die Anbaufläche, auf die bereits gekeimten Schadund/ oder Nutzpflanzen oder auf die bereits aufgelaufenen Schad- und/oder Nutzpflanzen appliziert. Für den Fall, dass die erfindungsgemäßen Mittel kein Pestizid enthalten, können diese Mittel im sogenannten Tankmix-Verfahren - d.h. unmittelbar vor dem Aufbringen auf die zu behandelnde Fläche erfolgt beim Anwender die Vermischung und Verdünnung der separat formulierten Produkte (= nutzpflanzenschützendes Mittel und Pestizid) - oder zeitlich vor der Anwendung eines Pestizids, oder zeitlich nach der Anwendung eines Pestizids, oder zur Saatgut-Vorbehandlung, d.h. beispielsweise zur Beizung des Nutzpflanzensaatguts verwendet werden. Bevorzugt ist die zeitnahe Anwendung des Safeners mit dem Pestizid, insbesondere, wenn der Safener nach dem Herbizid auf die Pflanzen appliziert wird. Die vorteilhaften Wirkungen der Verbindungen der allgemeinen Formel (I) werden beobachtet, wenn man sie zusammen mit den Pestiziden im Vorauflauf oder im Nachauflauf einsetzt, beispielsweise bei gleichzeitiger Applikation als Tankmix oder als Koformulierung oder bei einer separaten Applikation parallel oder nacheinander (Split-Applikation). Auch ist es möglich die Applikation mehrfach zu wiederholen. Manchmal kann es sinnvoll sein, eine Vorauflaufapplikation mit einer Nachauflaufapplikation zu kombinieren. Meist bietet sich die Anwendung als Nachauflaufapplikation auf die Nutz- oder Kulturpflanze mit gleichzeitiger oder späterer Applikation des Pestizids an. In Frage kommt auch die Anwendung der erfindungsgemäßen Verbindungen (I) bei der Saatgutbeizung, der (Tauch-)Behandlung von Keimpflanzen (z. B. Reis) oder Behandlung von anderem Vermehrungsgut (z.B. Kartoffelknollen).

Oftmals werden bei der Anwendung der Verbindungen der allgemeinen Formel (I) in Kombination mit Herbiziden neben der Safenerwirkung auch Wirkungsverstärkungen in der Herbizidwirkung gegenüber Schadpflanzen beobachtet. Weiterhin ist das Wachstum der Nutz- und Kulturpflanzen in vielen Fällen verbessert, und es können die Ernteerträge erhöht werden.

Die erfindungsgemäßen Mittel können ein oder mehrere Pestizide enthalten. Als Pestizide kommen beispielsweise Herbizide, Insektizide, Fungizide, Akarizide und Nematizide, welche jeweils bei alleiniger Anwendung phytotoxische Schäden an den Kulturpflanzen ergeben würden oder bei denen eine Schädigung wahrscheinlich wäre, in Frage. Von besonderem Interesse sind entsprechende pestizide Wirkstoffe aus den Gruppen der Herbizide, Insektizide, Akarizide, Nematizide und Fungizide, insbesondere Herbizide.

Das Gewichtsverhältnis Safener (der allgemeinen Formel (I)) zu Pestizid kann innerhalb weiter Grenzen variiert werden und liegt in der Regel im Bereich von 1:100 bis 100:1, vorzugsweise 1:20 bis 20:1, insbesondere 1:10 bis 10:1. Das optimale Gewichtsverhältnis Safener zu Pestizid hängt in der Regel sowohl von dem jeweils eingesetzten Safener und dem jeweiligen Pestizid als auch von der Art der zu schützenden Nutz- oder Kulturpflanze ab. Die erforderliche Aufwandmenge an Safener kann je nach verwendetem Pestizid und Art der zu schützenden Nutzpflanze innerhalb weiter Grenzen variiert werden und liegt in der Regel im Bereich von 0,001 bis 10 kg, vorzugsweise 0,01 bis 1 kg, insbesondere 0,01 bis 0,2 kg Safener je Hektar. Die für eine erfolgreiche Behandlung notwendigen Mengen und Gewichtsverhältnisse können durch einfache Vorversuche ermittelt werden.

Im Falle einer Saatbeizung werden beispielsweise 0,005 bis 20 g Safener (der allgemeinen Formel (I)) pro Kilogramm Saatgut, vorzugsweise 0,01 bis 10 g Safener pro Kilogramm Saatgut, insbesondere 0,05 bis 5 g Safener pro Kilogramm Saatgut eingesetzt.

Wenn Lösungen von Safener (der allgemeinen Formel (I)) in der Saatbehandlung benutzt werden und das Saatgut oder Keimlinge mit den Lösungen benetzt werden, so liegt die geeignete Konzentration in der Regel im Bereich von 1 bis 10000 ppm, vorzugsweise 100 bis 1000 ppm bezogen auf das Gewicht. Die für eine erfolgreiche Behandlung notwendigen Mengen und Gewichtsverhältnisse können durch einfache Vorversuche ermittelt werden.

Die Safener der allgmeinen Formel (I) können in üblicher Weise separat oder zusammen mit den Pestiziden formuliert werden. Gegenstand sind daher auch die nutzpflanzen- oder kulturpflanzenschützenden Mittel.

Bevorzugt ist die gemeinsame Anwendung von Safener und Pestizid, insbesondere die von Safener und Herbizid als Fertigformulierung oder die Anwendung im Tankmix-Verfahren.

Ebenso bevorzugt ist die Anwendung des Safeners der allgemeinen Formel (I) in der Saatgutbehandlung mit späterer Applikation von Pestiziden, vorzugsweise Herbiziden, nach der Aussaat im Vor- oder Nachauflaufverfahren.

Die Verbindungen der allgemeinen Formel (I) oder deren Salze können als solche oder in Form ihrer Zubereitungen (Formulierungen) mit anderen pestizid wirksamen Stoffen, wie z.B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Als Kombinationspartner für die erfindungsgemäßen Verbindungen der allgemeinen Formel (I) in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II oder Protoporphyrinogen-Oxidase beruhen, einsetzbar, wie sie z.B. aus Weed Research 26 (1986) 441-445 oder "The Pesticide Manual", 16th edition, The British Crop Protection Council und the Royal Soc. of Chemistry, 2006 und dort zitierter Literatur beschrieben sind. Nachfolgend werden beispielhaft bekannte Herbizide oder Pflanzenwachstumsregulatoren genannt, die mit den erfindungsgemäßen Verbindungen kombiniert werden können, wobei diese Wirkstoffe entweder mit ihrem "common name" in der englischsprachigen Variante gemäß International Organization for Standardization (ISO) oder mit dem chemischen Namen bzw. mit der Codenummer bezeichnet sind. Dabei sind stets sämtliche Anwendungsformen wie beispielsweise Säuren, Salze, Ester sowie auch alle isomeren Formen wie Stereoisomere und optische Isomere umfasst, auch wenn diese nicht explizit erwähnt sind.

Beispiele für solche herbiziden Mischungspartner sind:
Acetochlor, acifluorfen, acifluorfen-sodium, aclonifen, alachlor, allidochlor, alloxydim, alloxydimsodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlorpotassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenacsodium, chlorfenprop, chlorflurenol, chlorflurenol-methyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyrimorate, cyclosulfamuron, cycloxydim, cyhalofop, cyhalofop-butyl, cyprazine, 2,4-D, 2,4-D-butotyl, -butyl, - dimethylammonium, -diolamin, -ethyl, 2-ethylhexyl, -isobutyl, -isooctyl, -isopropylammonium, - potassium, -triisopropanolammonium und -trolamine, 2,4-DB, 2,4-DB-butyl, -dimethylammonium, isooctyl, -potassium und -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyrsodium, dimefuron, dimepiperate, dimethachlor, dimethametryn, dimethenamid, dimethenamid-P, dimetrasulfuron, dinitramine, dinoterb, diphenamid, diquat, diquat-dibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, - dimethylammonium und -methyl, fluoroglycofen, fluoroglycofen-ethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium, - potassium, -sodium und -trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuronmethyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(Dimethoxyphosphoryl)-ethyl-(2,4-dichlorphenoxy)acetat, imazamethabenz, Imazamethabenz-methyl, imazamox, imazamox-ammonium, imazapic, imazapic-ammonium, imazapyr, imazapyr-isopropylammonium, imazaquin, imazaquinammonium, imazethapyr, imazethapyr-immonium, imazosulfuron, indanofan, indaziflam, iodosulfuron, iodosulfuron-methyl-sodium, ioxynil, ioxynil-octanoate, -potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl] methyl } sulfonyl)-5,5-dimethyl-4,5-dihydro-1,2-oxazol, ketospiradox, lactofen, lenacil, linuron, MCPA, MCPA-butotyl, -dimethylammonium, -2-ethylhexyl, - isopropylammonium, -potassium und -sodium, MCPB, MCPB-methyl, -ethyl und -sodium, mecoprop, mecoprop-sodium, und -butotyl, mecoprop-P, mecoprop-P-butotyl, -dimethylammonium, -2-ethylhexyl und -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, neburon, nicosulfuron, nonanoic acid (Pelargonsäure), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuronethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrion, sulfentrazone, sulfometuron, sulfometuron-methyl, sulfosulfuron, SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trifluoressigsäure), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazonemethyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-Dichlor-N-{2-[(4,6-dimethoxypyrimidin-2-yl)oxy]benzyl}anilin, sowie die folgenden Verbindungen:

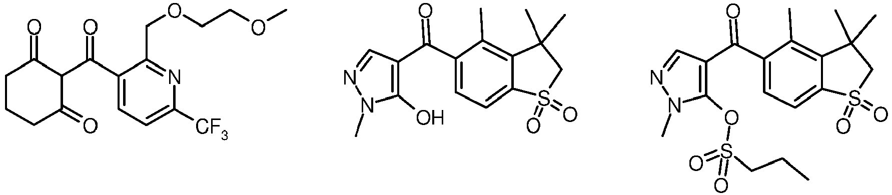

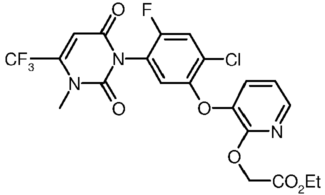

Beispiele für Pflanzenwachstumsregulatoren als mögliche Mischungspartner sind:
Acibenzolar, acibenzolar-S-methyl, 5-Aminolävulinsäure, ancymidol, 6-benzylaminopurine, Brassinolid, Catechin, chlormequat chloride, cloprop, cyclanilide, 3-(Cycloprop-1-enyl)propionsäure, daminozide, dazomet, n-decanol, dikegulac, dikegulac-sodium, endothal, endothaldipotassium, -disodium, und mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, Jasmonsäure, Jasmonsäuremethylester, maleic hydrazide, mepiquat chloride, 1-methylcyclopropene, 2-(1-naphthyl)acetamide, 1-naphthylacetic acid, 2-naphthyloxyacetic acid, nitrophenolate-mixture, 4-Oxo-4[(2-phenylethyl)amino]buttersäure, paclobutrazol, N-phenylphthalamic acid, prohexadione, prohexadione-calcium, prohydrojasmone, Salicylsäure, Strigolacton, tecnazene, thidiazuron, triacontanol, trinexapac, trinexapac-ethyl, tsitodef, uniconazole, uniconazole-P.

Im Falle der Anwendung als Wirkstofformulierungen oder Coformulierungen enthalten diese gegebenenfalls in der Regel die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Die Verbindungen der allgemeinen Formel (I) und deren Kombinationen mit einem oder mehreren der genannten Pestizide können in Abhängigkeit von den vorgegebenen chemisch-physikalischen und biologischen Parametern auf verschiedene Arten formuliert werden. Als Formulierungsarten sind beispielsweise geeignet:
- Emulgierbare Konzentrate, die durch Auflösen der Wirkstoffe in einem organischen Lösungsmittel, z.B. Butanol, Cyclohexanon, Dimethylformamid, Xylol oder auch höher siedenden Kohlenwasserstoffen oder Mischungen der organischen Lösungsmittel unter Zusatz von einem oder mehreren Tensiden ionischer und/oder nichtionischer Art (Emulgatoren) hergestellt werden. Geeignete Emulgatoren sind beispielsweise alkylarylsulfonsaure CalciumSalze, Fettsäurepolyglykolester, Alkylarylpolyglykolether, Fettalkoholpolyglykol-ether, Propylenoxid-Ethylenoxid-Kondensationsprodukte, Alkylpolyether, Sorbitanester und Polyoxyethylensorbitanfettsäureester;
- Stäubemittel, die durch Vermahlen der Wirkstoffe mit fein-verteilten festen anorganischen oder organischen Stoffen, z.B. Talkum, natürlichen Tonen, wie Kaolin, Bentonit und Pyrophyllit, Diatomeenerde oder Mehlen erhalten werden.
- auf Wasser oder Öl basierende Suspensionskonzentrate, die beispielsweise durch Nassvermahlung mittels Perlmühlen hergestellt werden können;
- wasserlösliche Pulver;
- wasserlösliche Konzentrate;
- Granulate, wie wasserlösliche Granulate, wasserdispergierbare Granulate sowie Granulate für die Streu- und Bodenapplikation;
- Spritzpulver, die neben Wirkstoff noch Verdünnungs- oder Inertstoffe und Tenside enthalten;
- Kapselsuspensionen und Mikrokapseln;
- Ultra-Low-Volume-Formulierungen.

Die oben genannten Formulierungsarten sind dem Fachmann bekannt und werden beispielsweise beschrieben in: K. Martens, "Spray Drying Handbook", 3rd Ed., G. Goodwin Ltd., London. 1979; W. van Valkenburg, "Pesticide Formulations", Marcel Dekker, N.Y. 1973; Winacker-Küchler, "Chemische Technologie", Band 7, C. Hanser Verlag München, 4. Auflage 1986; "Perry's Chemical Engineer's Handbook", 5th Ed., McGraw-Hill, N.Y. 1973, Seiten 8-57.

Die notwendigen Formulierungshilfsmittel wie Inertmaterialien, Tenside, Lösungsmittel und weitere Zusatzstoffe sind ebenfalls bekannt und werden beispielsweise beschrieben in: McCutcheon's "Detergents and Emulsifiers Annual", MC Publ. Corp., Ridgewood N.J.; C. Marsden, "Solvents Guide", 2nd Ed., Interscience, N.Y. 1963; H. von Olphen, "Introduction to Clay Colloid Chemistry", 2nd Ed., J. Wiley & Sons, N.Y.; Schönfeldt, "Grenzflächenaktive Äthylenoxidaddukte", Wiss. Verlagsgesellschaft, Stuttgart 1976; Sisley and Wood, "Encyclopedia of Surface Active Agents", Chem. Publ. Co. Inc., N.Y. 1964; Watkins, "Handbook of Insecticide Dust Diluents and Carriers", 2nd Ed., Darland Books, Caldwell N.J.; Winnacker-Küchler, "Chemische Technologie", Band 7, C. Hanser Verlag München, 4. Auflage 1986.

Außer den vorstehend genannten Formulierungshilfsmitteln können die nutzpflanzenschützenden Mittel gegebenenfalls übliche Haft-, Netz-, Dispergier-, Penetrations-, Emulgier-, Konservierungs-, Frostschutz-, Füll-, Träger- und Farbstoffe, Entschäumer, Verdunstungshemmer sowie den pH-Wert oder die Viskosität beeinflussende Mittel enthalten.

Je nach Art der Formulierung enthalten die nutzpflanzenschützenden Mittel in der Regel 0,1 bis 99 Gew.-%, insbesondere 0,2 bis 95 Gew.-%, eines oder mehrerer Safener der allgemeinen Formel (I) oder eine Kombination von Safener und Pestizid. Weiterhin enthalten sie 1 bis 99,9, insbesondere 4 bis 99,5 Gew.-%, eines oder mehrerer fester oder flüssiger Zusatzstoffe und 0 bis 25, insbesondere 0,1 bis 25 Gew.-% eines Tensids. In emulgierbaren Konzentraten beträgt die Wirkstoffkonzentration, d.h. die Konzentration von Safener und/oder Pestizid, in der Regel 1 bis 90, insbesondere 5 bis 80 Gew.-%. Stäubemittel enthalten üblicherweise 1 bis 30, vorzugsweise 5 bis 20 Gew.-% Wirkstoff. In Spritzpulvern beträgt die Wirkstoffkonzentration in der Regel 10 bis 90 Gew.-%. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Granulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids u. a. variiert die erforderliche Aufwandmenge der Safener der allgemeinen Formel (I).

In den nachfolgenden Beispielen, die die Erfindung erläutern aber nicht limitieren, beziehen sich die Mengenangaben auf das Gewicht, wenn nicht Näheres definiert ist.

### BEISPIELE

### 1. Formulierungsbeispiele

### 1.1 Stäubemittel

Ein Stäubemittel wird erhalten, indem man 10 Gew.-Teile einer Verbindung der allgemeinen Formel (I)(Safener) oder eines Wirkstoffgemischs aus einem Pestizid (z. B. ein Herbizid) und einem Safener der allgemeinen Formel (I) und 90 Gew.-Teile Talkum als Inertstoff mischt und in einer Schlagmühle zerkleinert.

### 1.2 Wasserdispergierbares Pulver

Ein in Wasser leicht dispergierbares, benetzbares Pulver wird erhalten, indem man 25 Gewichtsteile einer Verbindung der allgemeinen Formel (I) oder eines Wirkstoffgemischs aus einem Pestizid (z. B. einem Herbizid) und einem Safener der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 1.3 Wasserdispergierbares Konzentrat

Ein in Wasser leicht dispergierbares Dispersionskonzentrat wird erhalten, indem man 20 Gewichtsteile einer Verbindung der allgemeinen Formel (I) oder eines Wirkstoffgemischs aus einem Pestizid (z. B. einem Herbizid) und einem Safener der Formel (I) mit 6 Gew.-Teilen Alkylphenolpolyglykolether (^{®}Triton X 207), 3 Gew.-Teilen Isotridecanolpolyglykolether und 71 Gew.-Teilen paraffinischem Mineralöl mischt und in einer Reibkugelmühle auf eine Feinheit von unter 5 Mikron vermahlt.

### 1.4 Emulgierbares Konzentrat

Ein emulgierbares Konzentrat wird erhalten aus 15 Gew.-Teilen einer Verbindung der allgemeinen Formel (I) oder eines Wirkstoffgemischs aus einem Pestizid (z. B. Herbizid) und einem Safener der allgemeinen Formel (I), 75 Gew.-Teilen Cyclohexanon als Lösungsmittel und 10 Gew.-Teilen oxethyliertes Nonylphenol als Emulgator.

### 1.5 Wasserdispergierbares Granulat

Ein in Wasserdispergierbares Granulat wird erhalten, indem man

| | | |
|---|---|---|
| 75 | Gewichtsteile | eines Safeners der allgemeinen Formel (I) oder eines Gemischs eines Pestizids und eines Safeners der allgemeinen Formel (I), |
| 10 | " | ligninsulfonsaures Calcium, |
| 5 | " | Natriumlaurylsulfat, |
| 3 | " | Polyvinylalkohol und |
| 7 | " | Polyvinylalkohol und |
| 7 | " | Kaolin |

mischt, in einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man

| | | |
|---|---|---|
| 25 | Gewichtsteile | eines Safeners der allgemeinen Formel (I) oder eines Gemischs eines Pestizids und eines Safeners der allgemeinen Formel (I), |
| 5 | " | 2,2'-dinaphthylmethan-6,6'-disulfonsaures Natrium, |
| 2 | " | oleoylmethyltaurinsaures Natrium, |
| 17 | " | Calciumcarbonat, |
| 50 | " | Wasser und |
| 1 | Gewichtsteil | Polyvinylalkohol |

auf einer Kolloidmühle homogenisiert, zerkleinert, dann in einer Perlmühle mahlt und die so erhaltene Suspension in einem Sprühturm mittels einer Einstoffdüse zerstäubt und trocknet.

### 2. Biologische Beispiele:

### 2.1 Relative Wirkung ausgewählter erfindungsgemäßer Verbindungen am Beispiel der Schadredultion von Mesosulfuron-methyl an Sommerweizen (TRZAS)

Die Samen der zu behandelnden Kulturpflanzen wurden in Plastiktöpfen (Durchmesser ~4cm) in sandiger Lehmerde ausgelegt, mit Erde abgedeckt und im Gewächshaus unter guten Bedingungen für Keimung und Wachstum angezogen. Die Behandlung der Versuchspflanzen erfolgte im frühen Laubblattstadium (BBCH10 - BBCH12). Dabei wurden die in Form von benetzbaren Pulvern (WP) formulierten erfindungsgemäßen Verbindungen der allgemeinen Formel (I) als wässrige Suspension mit einer Wasseraufwandmenge entsprechend 800 l/ha unter Zusatz von Netzmittel (z.B. 0,2% Genapol-LRO oder 0,2% Mero) in der angegebenen Dosis auf die oberirdischen Pflanzenteile gesprüht.

Danach erfolgte die Applikation des Herbizids. Dazu wurde Mesosulfuron-methyl, formuliert als wasserdispergierbares Granulat (WG), als wässrige Dispersion mit einer Wasseraufwandmenge entsprechend 800 l/ha unter Zusatz von Netzmittel (z.B. 0,2% Genapol-LRO oder 1 l/ha Biopower) in einer Dosis von 40-60 g/ha auf die oberirdischen Pflanzenteile gesprüht. Die Dosis des Herbizids war dabei so gewählt, dass sie zum Auswertezeitpunkt an einer im gleichen Versuch mitgeführten Kontrollgruppe von Kulturpflanzen ohne Safenerbehandlung einen mittleren, visuell deutlich erkennbaren Schaden (min. 30%, max. 75%) im Vergleich zu unbehandelten Kulturpflanzen verursacht.

Nach Applikation wurden die Pflanzen unter guten Wachstumsbedingungen in einem Gewächshaus kultiviert. 9-13 Tage nach Applikation erfolgte eine visuelle Bonitur der Wirksamkeit der Testverbindungen. Dazu wurde das Erscheinungsbild der mit Testverbindung und Herbizid behandelten Pflanzen verglichen mit den entsprechenden Herbizidkontrollen (ohne Safener; mit deutlich erkennbaren Schäden) sowie den unbehandelten Kontrollen (ohne Schäden). Die schadreduzierende Wirkung der Testverbindungen wurde dabei in "Efficacy Codes" nach folgendem Schema abgestuft erfasst:
0: keine Schadreduktion (Erscheinungsbild entsprechend der Herbizidkontrolle)
1: schwache Schadreduktion
2: deutliche Schadreduktion
3: starke Schadreduktion
4: komplette Schadreduktion (Erscheinungsbild entsprechend der unbehandelten Kontrolle)

Die Versuche zeigen eine deutliche Wirksamkeit beispielhaft ausgewählter erfindungsgemäßer Verbindungen hinsichtlich der Reduktion einer durch das Herbizid Mesosulfuron-methyl verursachten Schädigung der Kulturpflanze Sommerweizen (TRZAS; cv. Triso):

| Beispiel Nr. | Dosierung des Safeners der Formel (I) (g/ha) | Kulturpflanze | Wirksamkeit des Safeners (Efficacy Code) |
|---|---|---|---|
| I.29-38 | 100 | TRZAS | 2 |
| I.30-38 | 100 | TRZAS | 2 |
| I.28-49 | 100 | TRZAS | 2 |
| I.64-1 | 500 | TRZAS | 2 |
| I.64-1 | 100 | TRZAS | 2 |
| I.64-49 | 500 | TRZAS | 2 |
| I.29-2 | 100 | TRZAS | 2 |
| I.31-48 | 100 | TRZAS | 2 |
| I.31-48 | 100 | TRZAS | 2 |
| I.32-4 | 100 | TRZAS | 2 |
| I.31-38 | 100 | TRZAS | 2 |
| I.32-38 | 100 | TRZAS | 2 |
| I.31-49 | 100 | TRZAS | 2 |
| I.32-49 | 100 | TRZAS | 2 |
| I.32-2 | 100 | TRZAS | 2 |
| I.31-7 | 100 | TRZAS | 2 |
| I.32-7 | 100 | TRZAS | 2 |
| I.33-4 | 100 | TRZAS | 2 |
| I.33-49 | 100 | TRZAS | 2 |
| I.80-38 | 100 | TRZAS | 2 |
| I.68-38 | 100 | TRZAS | 2 |
| I.67-38 | 100 | TRZAS | 2 |

### 2.2 Relative Wirkung ausgewählter erfindungsgemäßer Verbindungen am Beispiel der Schadredultion von Mesosulfuron-methyl an Sommergerste (HORVS)

Die Samen der zu behandelnden Kulturpflanzen wurden in Plastiktöpfen (Durchmesser ~4cm) in sandiger Lehmerde ausgelegt, mit Erde abgedeckt und im Gewächshaus unter guten Bedingungen für Keimung und Wachstum angezogen. Die Behandlung der Versuchspflanzen erfolgte im frühen Laubblattstadium (BBCH10 - BBCH12). Dabei wurden die in Form von benetzbaren Pulvern (WP) formulierten erfindungsgemäßen Verbindungen der allgemeinen Formel (I) als wässrige Suspension mit einer Wasseraufwandmenge entsprechend 800 l/ha unter Zusatz von Netzmittel (z.B. 0,2% Genapol-LRO oder 0,2% Mero) in der angegebenen Dosis auf die oberirdischen Pflanzenteile gesprüht.

Danach erfolgte die Applikation des Herbizids. Dazu wurde Mesosulfuron-methyl, formuliert als wasserdispergierbares Granulat (WG), als wässrige Dispersion mit einer Wasseraufwandmenge entsprechend 800 l/ha unter Zusatz von Netzmittel (z.B. 0,2% Genapol-LRO oder 1 l/ha Biopower) in einer Dosis von 40-60 g/ha auf die oberirdischen Pflanzenteile gesprüht. Die Dosis des Herbizids war dabei so gewählt, dass sie zum Auswertezeitpunkt an einer im gleichen Versuch mitgeführten Kontrollgruppe von Kulturpflanzen ohne Safenerbehandlung einen mittleren, visuell deutlich erkennbaren Schaden (min. 30%, max. 75%) im Vergleich zu unbehandelten Kulturpflanzen verursacht.

Nach Applikation wurden die Pflanzen unter guten Wachstumsbedingungen in einem Gewächshaus kultiviert. 9-13 Tage nach Applikation erfolgte eine visuelle Bonitur der Wirksamkeit der Testverbindungen. Dazu wurde das Erscheinungsbild der mit Testverbindung und Herbizid behandelten Pflanzen verglichen mit den entsprechenden Herbizidkontrollen (ohne Safener; mit deutlich erkennbaren Schäden) sowie den unbehandelten Kontrollen (ohne Schäden). Die schadreduzierende Wirkung der Testverbindungen wurde dabei für 2 Replikate separat in "Efficacy Codes" nach folgendem Schema abgestuft erfasst:
0: keine Schadreduktion (Erscheinungsbild entsprechend der Herbizidkontrolle)
1: schwache Schadreduktion
2: deutliche Schadreduktion
3: starke Schadreduktion
4: komplette Schadreduktion (Erscheinungsbild entsprechend der unbehandelten Kontrolle)

Die Versuche zeigen eine deutliche Wirksamkeit beispielhaft ausgewählter erfindungsgemäßer Verbindungen hinsichtlich der Reduktion einer durch das Herbizid Mesosulfuron-methyl verursachten Schädigung der Kulturpflanze Sommergerste (HORVS; cv. Montoya):

| Beispiel Nr. | Dosierung des Safeners der Formel (I) (g/ha) | Kulturpflanze | Wirksamkeit des Safeners (Efficacy Code) |
|---|---|---|---|
| I.28-38 | 100 | HORVS | 2 |
| I.29-38 | 100 | HORVS | 2 |
| I.30-38 | 100 | HORVS | 2 |
| I.28-49 | 100 | HORVS | 2 |
| I.64-1 | 500 | HORVS | 2 |
| I.64-49 | 500 | HORVS | 2 |
| I.31-48 | 100 | HORVS | 2 |
| I.32-38 | 100 | HORVS | 2 |
| I.31-49 | 100 | HORVS | 2 |
| I.32-49 | 100 | HORVS | 2 |
| I.31-7 | 100 | HORVS | 2 |
| I.32-7 | 100 | HORVS | 2-3 |
| I.68-38 | 100 | HORVS | 2 |
| I.69-38 | 100 | HORVS | 2-3 |
| I.70-38 | 100 | HORVS | 2 |
| I.73-38 | 100 | HORVS | 2-3 |
| I.43-49 | 100 | HORVS | 2 |

## Patentansprüche

1. Verbindungen der allgemeinen Formel (I) oder deren Salze 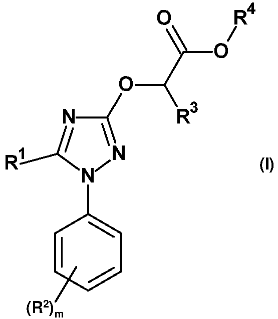 worin
R¹ für Heteroaryl steht, wobei der Heteroaryl-Rest unsubstituiert oder durch Halogen, Cyano, Nitro, (C₁-C₆)Alkyl, (C₂-C₆)Alkenyl, (C₂-C₆)Alkinyl, (C₃-C₈)Cycloalkyl, (C₃-C₈)Cycloalkenyl, (C₁-C₆)Alkoxy und (C₁-C₆)AlkylS(O)ₚ, wobei die letztgenannten sieben Reste unsubstituiert sind, oder durch einen oder mehrere Reste aus der Gruppe Halogen, Cyano, (C₁-C₆)Alkoxy und (C₁-C₆)AlkylS(O)ₚ substituiert sind, bedeuten,
R² für Wasserstoff, Halogen, Cyano, Nitro, (C₁-C₆)Alkyl, (C₂-C₆)Alkenyl, (C₂-C₆)Alkinyl, (C₃-C₈)Cycloalkyl, (C₃-C₈)Cycloalkenyl, (C₁-C₆)Alkoxy und (C₁-C₆)AlkylS(O)ₚ, wobei die letztgenannten sieben Reste unsubstituiert oder durch einen schon mehrere Reste aus der Gruppe Halogen, Cyano, (C₁-C₆)Alkoxy und (C₁-C₆)AlkylS(O)ₚ substituiert sind, bedeuten,
R³ für Wasserstoff und (C₁-C₆)Alkyl steht,
R⁴ für Wasserstoff, (C₁-C₁₈)Alkyl, (C₁-C₁₈)Haloalkyl, (C₁-C₁₈)Cyanoalkyl, (C₂-C₁₈)Alkenyl, (C₂-C₁₈)Alkinyl, (C₃-C₁₂)Cycloalkyl, (C₃-C₁₂)Cycloalkenyl, Aryl, Heteroaryl, (C₁-C₁₈)Alkoxy-(C₁-C₁₈)alkyl, (Ci-Cis)Haloalkoxy-(Ci-Cis)alkyl, (Ci-C₁₈)Alkoxy-(C₁-C₁₈)haloalkyl, (C₁-C₁₈)Alkylthio-(C₁-C₁₈)alkyl, (C₁-C₁₈)Haloalkylthio-(C₁-C₁₈)alkyl, (C₂-C₁₈)Haloalkenyl, (C₂-C₁₈)Haloalkinyl, Heterocyclyl-(C₁-C₁₈)alkyl, Aryl-(C₁-C₁₈)alkyl, (C₃-C₁₂)Cycloalkyl-(C₁-C₁₈)alkyl, (C₁-C₁₈)Alkoxycarbonyl-(C₁-C₁₈)alkyl, und (C₁-C₁₈)Alkoxycarbonyl-(C₃-C₁₂)cycloalkyl-(C₁-C₁₈)alkyl, steht, oder einen Rest der Formel -NR^{a}R^{b} oder -N=CR^{c}R^{d},
wobei in den vorgenannten 2 Resten jeder der Reste R^{a}, R^{b}, R^{c} und R^{d} unabhängig voneinander Wasserstoff, (C₁-C₄)Alkyl, (C₂-C₄)Alkenyl, (C₂-C₄)Alkinyl, Benzyl, substituiertes Benzyl, Phenyl oder substituiertes Phenyl bedeutet,
oder R^{a} und R^{b} zusammen mit dem N-Atom einen 3-bis 8-gliedrigen Heterocyclus, welcher zusätzlich zum N-Atom ein oder zwei weitere Heteroringatome aus der Gruppe N, O und S enthalten kann, und welcher unsubstituiert oder durch einen oder mehrere Reste aus der Gruppe (C₁-C₄)Alkyl und (C₁-C₄)Haloalkyl substituiert ist, bilden können, oder R^{c} und R^{d} zusammen mit dem C-Atom einen 3- bis 8-gliedrigen carbocyclischen oder heterocyclischen Rest, welcher 1 bis 3 Heteroringatome aus der Gruppe N, O und S enthalten kann, wobei der carbocyclische oder heterocyclische Rest unsubstituiert oder durch einen oder mehrere Reste aus der Gruppe (C₁-C₄)Alkyl und (C₁-C₄)Haloalkyl substituiert ist, bedeutet,
m für eine Zahl von 0 bis 5 steht,
und
p 0, 1 oder 2 bedeutet.

2. Verbindungen der allgemeinen Formel (I) gemäß Anspruch 1 oder deren Salze, worin
R' für Heteroaryl steht, wobei der Heteroaryl-Rest unsubstituiert oder durch Halogen, Cyano, Nitro, (C₁-C₆)Alkyl, (C₂-C₆)Alkenyl, (C₂-C₄)Alkinyl, (C₃-C₇)Cycloalkyl, (C₃-C₇)Cycloalkenyl, (C₁-C₄)Alkoxy und (C₁-C₄)AlkylS(O)ₚ, wobei die letztgenannten sieben Reste unsubstituiert sind, oder durch einen oder mehrere Reste aus der Gruppe Halogen, Cyano, (C₁-C₄)Alkoxy und (C₁-C₄)AlkylS(O)ₚ substituiert sind, bedeuten,
R² für Wasserstoff, Halogen, Cyano, Nitro, (C₁-C₄)Alkyl, (C₂-C₄)Alkenyl, (C₂-C₄)Alkinyl, (C₃-C₆)Cycloalkyl, (C₃-C₆)Cycloalkenyl, (C₁-C₄)Alkoxy und (C₁-C₄)AlkylS(O)ₚ,wobei die letztgenannten sieben Reste unsubstituiert sind, oder durch einen schon mehrere Reste aus der Gruppe Halogen, Cyano, (C₁-C₄)Alkoxy und (C₁C₄)AlkylS(O)ₚ substituiert sind, bedeuten,
R³ für Wasserstoff und (C₁-C₄)Alkyl steht,
R⁴ für Wasserstoff, (C₁-C₁₆)Alkyl, (C₁-C₁₆)Haloalkyl, (C₁-C₁₆)Cyanoalkyl, (C₂-C₁₆)Alkenyl, (C₂-C₁₆)Alkinyl, (C₃-C₁₂)Cycloalkyl, (C₃-C₁₂)Cycloalkenyl, Aryl, Heteroaryl, (C₁-C₁₆)Alkoxy-(C₁-C₁₆)alkyl, (C₁-C₁₆)Haloalkoxy-(C₁-C₁₆)alkyl, (C₁-C₁₆)Alkoxy-(C₁-C₁₆)haloalkyl, (C₁-C₁₆)Alkylthio-(C₁-C₁₆)alkyl, (C₁-C₁₆)Haloalkylthio-(C₁-C₁₆)alkyl, (C₂-C₁₆)Haloalkenyl, (C₂-C₁₆)Haloalkinyl, Heterocyclyl-(C₁-C₁₆)alkyl, Aryl-(C₁-C₁₆)alkyl, (C₃-C₁₂)Cycloalkyl-(C₁-C₁₆)alkyl, (C₁-C₁₆)Alkoxycarbonyl-(C₁-C₁₆)alkyl, und (C₁-C₁₆)Alkoxycarbonyl-(C₃-C₁₂)cycloalkyl-(C₁-C₁₆)alkyl, steht,
m für eine Zahl von 0 bis 4 steht,
und
p 0,1 oder 2 bedeutet.

3. Verbindungen der allgemeinen Formel (I) gemäß Anspruch 1 oder deren Salze, worin
R¹ für Heteroaryl steht, wobei der Heteroaryl-Rest unsubstituiert ist, oder einfach oder mehrfach durch Halogen, Cyano, Methyl, Ethyl, CF₃, CF₂Cl, CH₂F, CHF₂, OCH₃, OCF₃, SCH₃, SOCH₃, SO₂CH₃ und SCF₃ substituiert ist,
R² für Wasserstoff, Halogen, Cyano, Methyl, Ethyl, CF₃, CF₂Cl, CH₂F, CHF₂, OCH₃, OCF₃, SCH₃, SOCH₃, SO₂CH₃ und SCF₃ steht,
R³ für Wasserstoff, CH₂CH₃ und CH₃ steht,
R⁴ für Wasserstoff, (C₁-C₁₂)Alkyl, (C₁-C₁₂)Haloalkyl, (C₁-C₁₂)Cyanoalkyl, (C₂-C₁₂)Alkenyl, (C₂-C₁₂)Alkinyl, (C₃-C₁₂)Cycloalkyl, (C₃-C₁₂)Cycloalkenyl, Aryl, Heteroaryl, (C₁-C₁₂)Alkoxy-(C₁-C₁₂)alkyl, (C₁-C₁₂)Haloalkoxy-(C₁-C₁₂)alkyl, (C₁-C₁₂)Alkoxy-(C₁-C₁₂)haloalkyl, (C₁-C₁₂)Alkylthio-(C₁-C₁₂)alkyl, (C₁-C₁₂)Haloalkylthio-(C₁-C₁₂)alkyl, (C₂-C₁₂)Haloalkenyl, (C₂-C₁₂)Haloalkinyl, Heterocyclyl-(C₁-C₁₂)alkyl, Aryl-(C₁-C₁₂)alkyl, (C₃-C₁₂)Cycloalkyl-(C₁-C₁₂)alkyl, (C₁-C₁₂)Alkoxycarbonyl-(C₁-C₁₂)alkyl, und (C₁-C₁₂)Alkoxycarbonyl-(C₃-C₁₂)cycloalkyl-(C₁-C₁₂)alkyl steht.
und
m für 0, 1, 2 oder 3 steht.

4. Verbindungen der allgemeinen Formel (I) gemäß Anspruch 1 oder deren Salze, worin
R¹ für Pyridin-2-yl, Pyridin-3-yl, Pyridin-4-yl, Pyrazin-2-yl, Pyrazin-3-yl, Pyrimidin-2-yl, Pyrimidin-4-yl, Pyrimidin-5-yl, Pyridazin-3-yl, Pyridazin-4-yl, 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl, 1,2,4-, 1,3,2-, 1,3,6- und 1,2,6-Oxdiazinyl, Isoxazol-3-yl, Isoxazol-4-yl, Isoxazol-5-yl, 1,3-Oxazol-2-yl, 1,3-Oxazol-4-yl, 1,3-Oxazol-5-yl, Isothiazol-3-yl, Isothiazol-4-yl, Isothiazol-5-yl, 1,3-Thiazol-2-yl, 1,3-Thiazol-4-yl, 1,3-Thiazol-5-yl, 1H-Pyrrol-1-yl, 1H-Pyrrol-2-yl, 1H-Pyrrol-3-yl, Furan-2-yl, Furan-3-yl, Thien-2-yl; Thien-3-yl, 1H-Imidazol-1-yl, 1H-Imidazol-2-yl, 1H-Imidazol-4-yl, 1H-Imidazol-5-yl, 1H-Pyrazol-1-yl, 1H-Pyrazol-3-yl, 1H-Pyrazol-4-yl, 1H-Pyrazol-5-yl, 1H-1,2,3-Triazol-1-yl, 1H-1,2,3-Triazol-4-yl, 1H-1,2,3-Triazol-5-yl, 2H-1,2,3-Triazol-2-yl, 2H-1,2,3-Triazol-4-yl, 1H-1,2,4-Triazol-1-yl, 1H-1,2,4-Triazol-5-yl, 4H-1,2,4-Triazol-3-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, 1,3,4-Thiadiazol-2-yl, 1,3,4-Thiadiazol-2-yl, 1,2,4-Thiadiazol-5-yl, 1,2,4-Thiadiazol-3-yl, 1,2,5-Thiadiazol-3-yl, 1,2,5-Thiadiazol-3-yl, 1,2,3-Thiadiazol-4-yl, 1,2,3-Thiadiazol-3-yl, welcher unsubstituiert ist, oder einfach oder mehrfach durch Halogen, Cyano, Methyl, CF₃, CF₂Cl, CH₂F, CHF₂, OCH₃, OCF₃, SCH₃, SOCH₃, SO₂CH₃ und SCF₃ substituiert ist, steht,
R² für Wasserstoff, Fluor, Chlor, Brom, Iod, CN, Methyl, CF₃, CF₂Cl, CH₂F, CHF₂, OCH₃, OCF₃, SCH₃, SOCH₃, SO₂CH₃ und SCF₃ steht,
R³ für Wasserstoff und CH₃ steht,
R⁴ für Wasserstoff, (C₁-C₁₀)Alkyl, (C₁-C₁₀)Haloalkyl, (C₁-C₁₀)Cyanoalkyl, (C₂-C₁₀)Alkenyl, (C₂-C₁₀)Alkinyl, (C₃-C₉)Cycloalkyl, (C₃-C₉)Cycloalkenyl, Aryl, Heteroaryl, (C₁-C₁₀)Alkoxy-(C₁-C₁₀)alkyl, (C₁-C₁₀)Haloalkoxy-(C₁-C₁₀)alkyl, (C₁-C₁₀)Alkoxy-(C₁-C₁₀)haloalkyl, (C₁-C₁₀)Alkylthio-(C₁-C₁₀)alkyl, (C₁-C₁₀)Haloalkylthio-(C₁-C₁₀)alkyl, (C₂-C₁₈)Haloalkenyl, (C₂-C₁₈)Haloalkinyl, Heterocyclyl-(C₁-C₁₀)alkyl, Aryl-(C₁-C₁₀)alkyl, (C₃-C₉)Cycloalkyl-(C₁-C₁₀)alkyl, (C₁-C₁₀)Alkoxycarbonyl-(C₁-C₁₀)alkyl und (C₁-C₁₀)Alkoxycarbonyl-(C₃-C₉)cycloalkyl-(C₁-C₁₀)alkyl steht,
und
m für 0, 1, 2 oder 3 steht.

5. Verbindungen der allgemeinen Formel (I) gemäß Anspruch 1 oder deren Salze, worin
R¹ für die Gruppen Q-1.1 bis Q-1.59
| | | | | |
|---|---|---|---|---|
| 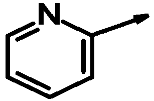 | 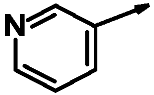 | 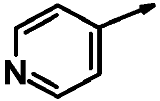 | 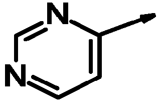 | 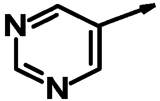 |
| Q-1.1 | Q-1.2 | Q-1.3 | Q-1.4 | Q-1.5 |
| 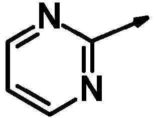 | 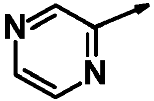 | 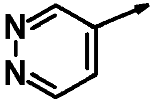 | 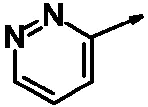 | 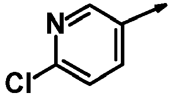 |
| Q-1.6 | Q-1.7 | Q-1.8 | Q-1.9 | Q-1.10 |
| 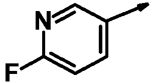 | 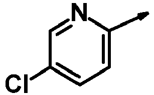 | 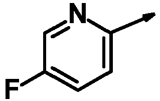 | 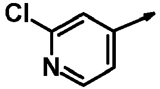 | 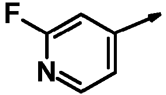 |
| Q-1.11 | Q-1.12 | Q-1.13 | Q-1.14 | Q-1.15 |
| 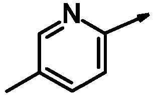 | 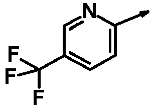 | 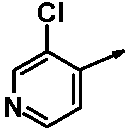 | 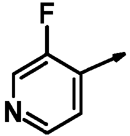 | 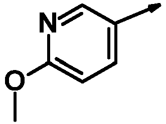 |
| Q-1.16 | Q-1.17 | Q-1.18 | Q-1.19 | Q-1.20 |
| 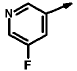 | 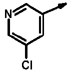 | 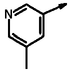 | 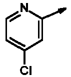 | 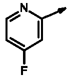 |
| Q-1.21 | Q-1.22 | Q-1.23 | Q-1.24 | Q-1.25 |
| 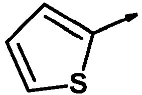 | 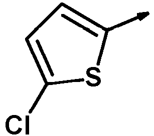 | 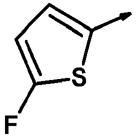 | 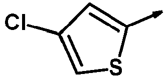 | 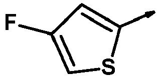 |
| Q-1.26 | Q-1.27 | Q-1.28 | Q-1.29 | Q-1.30 |
| 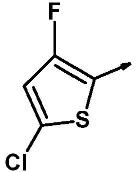 | 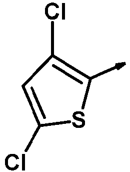 | 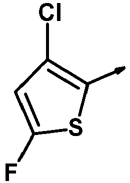 | 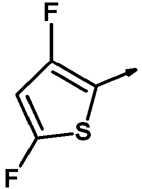 | 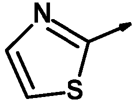 |
| Q-1.31 | Q-1.32 | Q-1.33 | Q-1.34 | Q-1.35 |
| 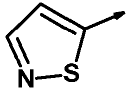 | 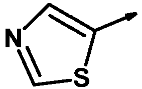 | 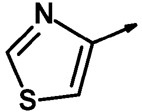 | 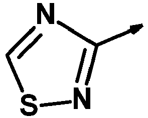 | 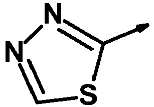 |
| Q-1.36 | Q-1.37 | Q-1.38 | Q-1.39 | Q-1.40 |
| 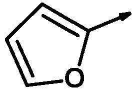 | 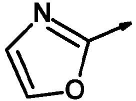 | 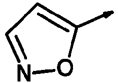 | 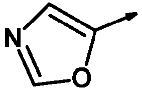 | 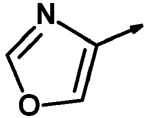 |
| Q-1.41 | Q-1.42 | Q-1.43 | Q-1.44 | Q-1.45 |
| 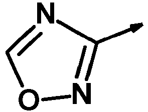 | 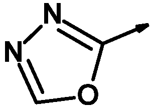 | 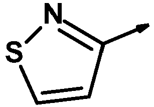 | 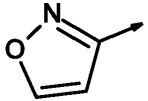 | 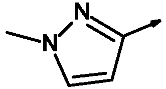 |
| Q-1.46 | Q-1.47 | Q-1.48 | Q-1.49 | Q-1.50 |
| 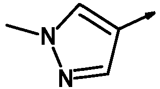 | 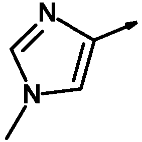 | 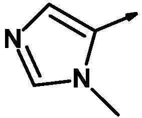 | 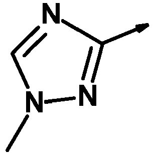 | 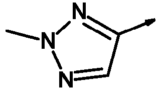 |
| Q-1.51 | Q-1.52 | Q-1.53 | Q-1.54 | Q-1.55 |
| 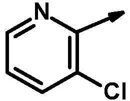 | 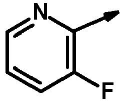 | 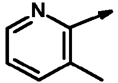 | 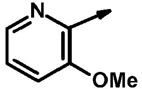 | |
| Q-1.56 | Q-1.57 | Q-1.58 | Q-1.59 | |
R² für Wasserstoff, Fluor, Chlor, Brom, Iod, Cyano, Methyl, CF₃, CH₂F, CHF₂, OCH₃, OCF₃, SCH₃, SOCH₃, SO₂CH₃ und SCF₃ steht,
R³ für Wasserstoff steht,
R⁴ für Wasserstoff, Methyl, Ethyl, n-Propyl, i-Propyl, n-Butyl, n-Pentyl, Phenyl, Benzyl, CH₂(4-Cl-Ph), CH₂(4-F-Ph), CH₂(4-MeO-Ph), 2-Methoxyethyl, Tetrahydrofuran-2-yl-methyl, Tetrahydrofuran-3-yl-methyl, Tetrahydropyran-2-yl-methyl, Tetrahydropyran-3-yl-methyl, Tetrahydropyran-4-yl-methyl, Methylpropionat-3-yl, Ethylpropionat-3-yl, Methylacetat-2-yl, Ethylacetat-2-yl, Methylpivalat-2-yl, Ethylpivalat-3-yl, Methyl-2-methylpropanoat-3-yl, Methyl-2,2-dimethylpropanoat-3-yl, Ethyl-2-methylpropanoat-3-yl, Methyl-2-propanoat-2-yl, Ethyl-2-propanoat-2-yl, Methyl-acet-2-yl, Ethyl-acetat-2-yl, Methyl-1-methylcyclopropancarboxylat-2-yl, Ethyl-1-methylcyclopropancarboxylat-2-yl, 2-(Dimethylamino)ethyl, Oxetan-3-yl, (3-Methyloxetan-3-yl)methyl, 2,2,2-Trifluorethyl, 2,2-Difluorethyl, 2-Fluorethyl, 2,2,3,3,3-Pentafluorpropyl, Cyclopropylmethyl, 1-Cyclopropyl-ethyl, (1-Methyl-cyclopropyl)-methyl, (2,2-Dichlorcyclopropyl)-methyl, (2,2-Dimethyl-cyclopropyl)-methyl, Allyl, Propargyl, (Prop-2-in-1-yl), 2-Chlorprop-2-en-1-yl, 3-Phenylprop-2-in-1-yl, 3,3-Dichlorprop-2-en-1-yl, 3,3-Dichlor-2-fluor-prop-2-en-1-yl, Methylprop-2-in-1-yl, 2-Methylprop-2-en-1-yl, But-2-en-1-yl, But-3-en-1-yl, But-2-in-1-yl, But-3-in-1-yl, 4-Chlor-but-2-in-1-yl, 3-Methyl-but-2-en-1-yl, 3-Methyl-but-1-en-1-yl, (2E)-1-Methylbut-2-en-1-yl, (E)-Pent-3-en-2-yl oder (Z)-Pent-3-en-2-yl, Cyclobutylmethyl, Cyclopentylmethyl, Cyclohexylmethyl, Heptan-2-yl, iso-Butyl, 1,3-Dioxolan-2-ylmethyl oder 1-Ethyl-5-methyl-1H-pyrazol-4-methyl steht,
m für 0, 1, 2 oder 3 steht.

6. Verbindungen der allgemeinen Formel (I) gemäß Anspruch 1 oder deren Salze, worin
R¹ für die Gruppen Q-1.1 bis Q-1.59
| | | | | |
|---|---|---|---|---|
| 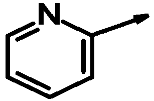 | 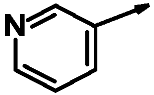 | 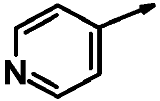 | 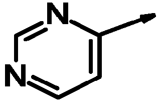 | 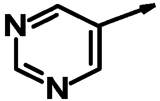 |
| Q-1.1 | Q-1.2 | Q-1.3 | Q-1.4 | Q-1.5 |
| 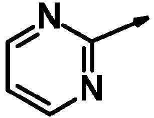 | 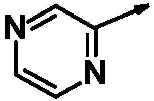 | 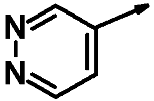 | 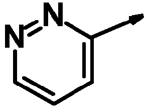 | 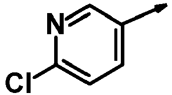 |
| Q-1.6 | Q-1.7 | Q-1.8 | Q-1.9 | Q-1.10 |
| 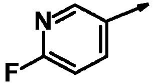 | 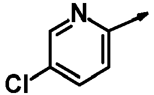 | 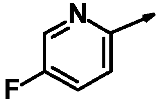 | 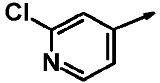 | 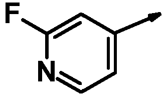 |
| Q-1.11 | Q-1.12 | Q-1.13 | Q-1.14 | Q-1.15 |
| 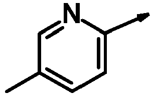 | 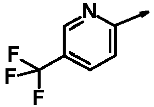 | 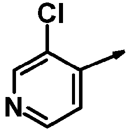 | 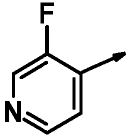 | 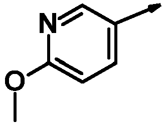 |
| Q-1.16 | Q-1.17 | Q-1.18 | Q-1.19 | Q-1.20 |
| 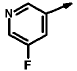 | 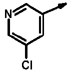 | 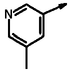 | 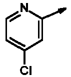 | 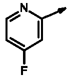 |
| Q-1.21 | Q-1.22 | Q-1.23 | Q-1.24 | Q-1.25 |
| 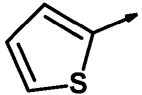 | 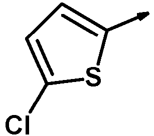 | 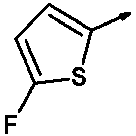 | 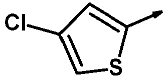 | 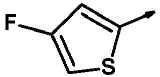 |
| Q-1.26 | Q-1.27 | Q-1.28 | Q-1.29 | Q-1.30 |
| 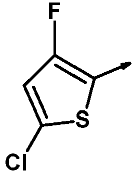 | 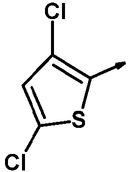 | 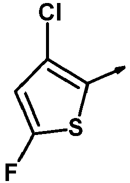 | 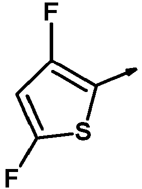 | 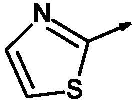 |
| Q-1.31 | Q-1.32 | Q-1.33 | Q-1.34 | Q-1.35 |
| 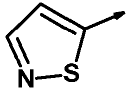 | 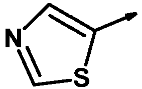 | 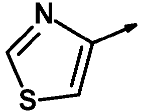 | 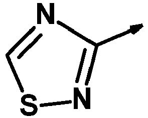 | 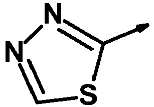 |
| Q-1.36 | Q-1.37 | Q-1.38 | Q-1.39 | Q-1.40 |
| 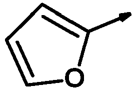 | 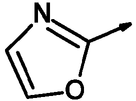 | 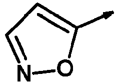 | 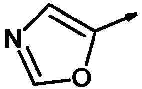 | 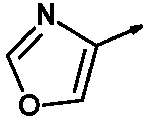 |
| Q-1.41 | Q-1.42 | Q-1.43 | Q-1.44 | Q-1.45 |
| 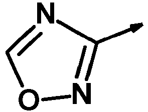 | 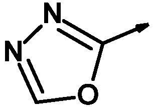 | 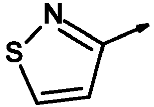 | 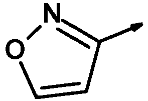 | 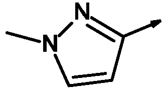 |
| Q-1.46 | Q-1.47 | Q-1.48 | Q-1.49 | Q-1.50 |
| 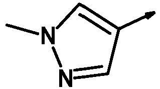 | 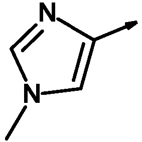 | 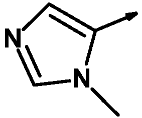 | 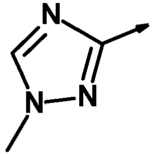 | 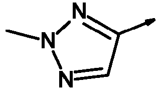 |
| Q-1.51 | Q-1.52 | Q-1.53 | Q-1.54 | Q-1.55 |
| 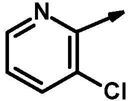 | 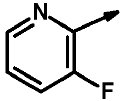 | 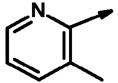 | 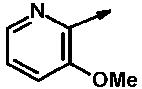 | |
| Q-1.56 | Q-1.57 | Q-1.58 | Q-1.59 | |
steht,
und (R²)ₘ-Phenyl für die Gruppen Q-2.1 bis Q-2.53
| | | | | |
|---|---|---|---|---|
| 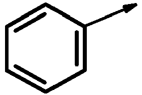 | 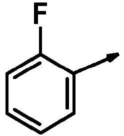 | 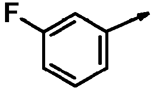 | 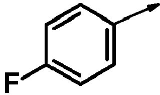 | 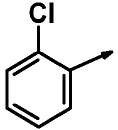 |
| Q-2.1 | Q-2.2 | Q-2.3 | Q-2.4 | Q-2.5 |
| 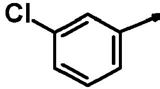 | 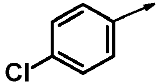 | 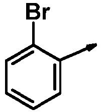 | 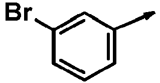 | 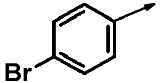 |
| Q-2.6 | Q-2.7 | Q-2.8 | Q-2.9 | Q-2.10 |
| 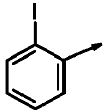 | 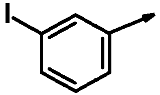 | 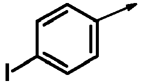 | 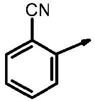 | 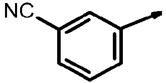 |
| Q-2.11 | Q-2.12 | Q-2.13 | Q-2.14 | Q-2.15 |
| 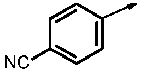 | 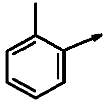 | 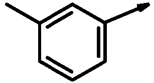 | 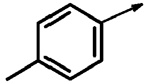 | 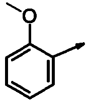 |
| Q-2.16 | Q-2.17 | Q-2.18 | Q-2.19 | Q-2.20 |
| 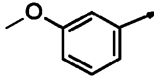 | 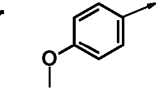 | 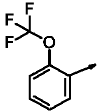 | 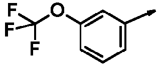 | 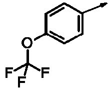 |
| Q-2.21 | Q-2.22 | Q-2.23 | Q-2.24 | Q-2.25 |
| 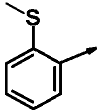 | 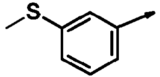 | 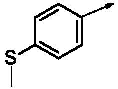 | 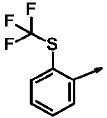 | 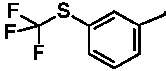 |
| Q-2.26 | Q-2.27 | Q-2.28 | Q-2.29 | Q-2.30 |
| 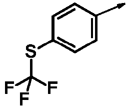 | 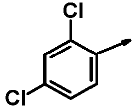 | 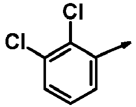 | 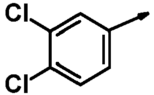 | 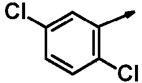 |
| Q-2.31 | Q-2.32 | Q-2.33 | Q-2.34 | Q-2.35 |
| 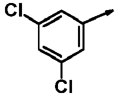 | 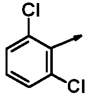 | 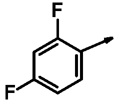 | 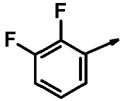 | 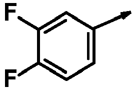 |
| Q-2.36 | Q-2.37 | Q-2.38 | Q-2.39 | Q-2.40 |
| 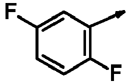 | 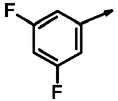 | 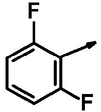 | 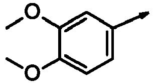 | 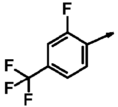 |
| Q-2.41 | Q-2.42 | Q-2.43 | Q-2.44 | Q-2.45 |
| 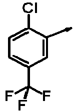 | 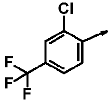 | 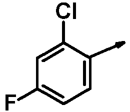 | 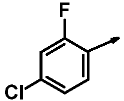 | 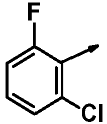 |
| Q-2.46 | Q-2.47 | Q-2.48 | Q-2.49 | Q-2.50 |
| 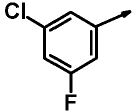 | 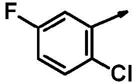 | 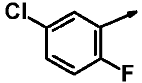 | | |
| Q-2.51 | Q-2.52 | Q-2.53 | | |
steht,
R³ für Wasserstoff steht,
R⁴ für Wasserstoff, Methyl, Ethyl, n-Propyl, i-Propyl, n-Butyl, n-Pentyl, Phenyl, Benzyl, CH₂(4-Cl-Ph), CH₂(4-F-Ph), CH₂(4-MeO-Ph), 2-Methoxyethyl, Tetrahydrofuran-2-yl-methyl, Tetrahydrofuran-3-yl-methyl, Tetrahydropyran-2-yl-methyl, Tetrahydropyran-3-yl-methyl, Tetrahydropyran-4-yl-methyl, Methylpropionat-3-yl, Ethylpropionat-3-yl, Methylacetat-2-yl, Ethylacetat-2-yl, Methylpivalat-2-yl, Ethylpivalat-3-yl, Methyl-2-methylpropanoat-3-yl, Methyl-2,2-dimethylpropanoat-3-yl, Ethyl-2-methylpropanoat-3-yl, Methyl-2-propanoat-2-yl, Ethyl-2-propanoat-2-yl, Methyl-acetat-2-yl, Ethyl-acetat-2-yl, Methyl-1-methylcyclopropancarboxylat-2yl, Ethyl-1-methylcyclopropancarboxylat-2-yl, 2-(Dimethylamino)ethyl, Oxetan-3-yl, (3-Methyloxetan-3-yl)methyl, 2,2,2-Trifluorethyl, 2,2-Difluorethyl, 2-Fluorethyl, 2,2,3,3,3-Pentafluorpropyl, Cyclopropylmethyl, 1-Cyclopropyl-ethyl, (1-Methyl-cyclopropyl)-methyl, (2,2-Dichlorcyclopropyl)-methyl, (2,2-Dimethyl-cyclopropyl)-methyl, Allyl, Propargyl (Prop-2-in-1-yl), 2-Chlorprop-2-en-1-yl, 3-Phenylprop-2-in-1-yl, 3,3-Dichlorprop-2-en-1-yl, 3,3-Dichlor-2-fluor-prop-2-en-1-yl, Methylprop-2-in-1-yl, 2-Methylprop-2-en-1-yl, But-2-en-1-yl, But-3-en-1-yl, But-2-in-1-yl, But-3-in-1-yl, 4-Chlor-but-2-in-1-yl, 3-Methyl-but-2-en-1-yl, 3-Methyl-but-1-en-1-yl, (2E)-1-Methylbut-2-en-1-yl, (E)-Pent-3- en-2-yl oder (Z)-Pent-3-en-2-yl, Cyclobutylmethyl, Cyclopentylmethyl, Cyclohexylmethyl, Heptan-2-yl, iso-Butyl, 1,3-Dioxolan-2-ylmethyl oder 1-Ethyl-5-methyl-1H-pyrazol-4-methyl steht.

7. Verbindungen der allgemeinen Formel (I) gemäß Anspruch 1 oder deren Salze, worin
R¹ für Q-1.1 bis Q-1.59
| | | | | |
|---|---|---|---|---|
| 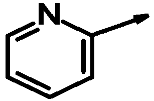 | 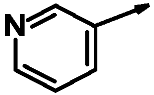 | 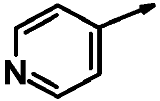 | 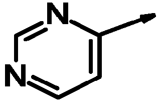 | 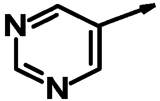 |
| Q-1.1 | Q-1.2 | Q-1.3 | Q-1.4 | Q-1.5 |
| 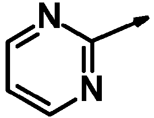 | 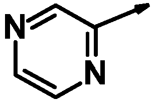 | 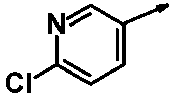 | 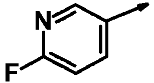 | 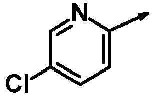 |
| Q-1.6 | Q-1.7 | Q-1.10 | Q-1.11 | Q-1.12 |
| 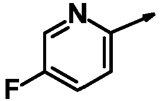 | 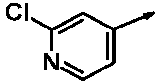 | 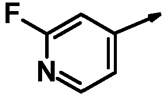 | 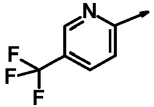 | 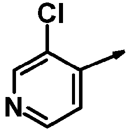 |
| Q-1.13 | Q-1.14 | Q-1.15 | Q-1.17 | Q-1.18 |
| 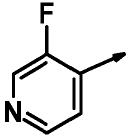 | 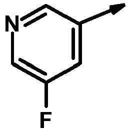 | 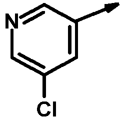 | 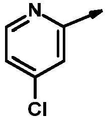 | 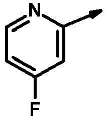 |
| Q-1.19 | Q-1.21 | Q-1.22 | Q-1.24 | Q-1.25 |
| 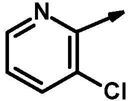 | 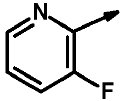 | 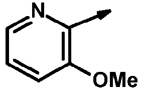 | | |
| Q-1.56 | Q-1.57 | Q-1.59 | | |
steht,
und (R²)ₘ-Phenyl für die Gruppen Q-2.1 bis Q-2.53
| | | | | |
|---|---|---|---|---|
| 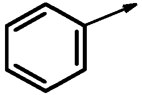 | 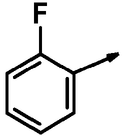 | 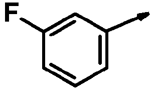 | 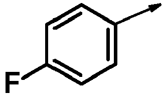 | 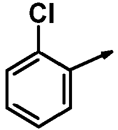 |
| Q-2.1 | Q-2.2 | Q-2.3 | Q-2.4 | Q-2.5 |
| 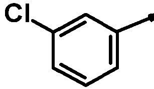 | 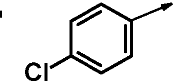 | 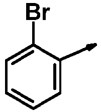 | 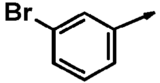 | 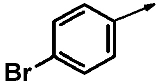 |
| Q-2.6 | Q-2.7 | Q-2.8 | Q-2.9 | Q-2.10 |
| 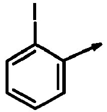 | 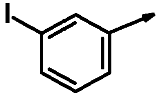 | 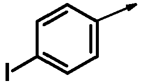 | 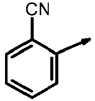 | 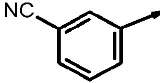 |
| Q-2.11 | Q-2.12 | Q-2.13 | Q-2.14 | Q-2.15 |
| 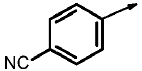 | 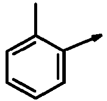 | 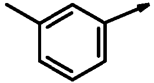 | 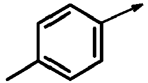 | 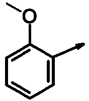 |
| Q-2.16 | Q-2.17 | Q-2.18 | Q-2.19 | Q-2.20 |
| 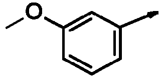 | 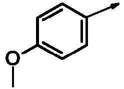 | 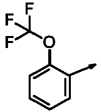 | 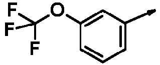 | 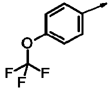 |
| Q-2.21 | Q-2.22 | Q-2.23 | Q-2.24 | Q-2.25 |
| 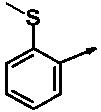 | 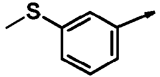 | 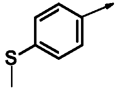 | 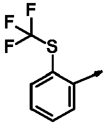 | 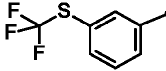 |
| Q-2.26 | Q-2.27 | Q-2.28 | Q-2.29 | Q-2.30 |
| 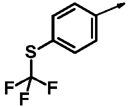 | 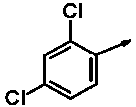 | 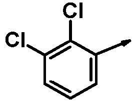 | 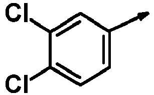 | 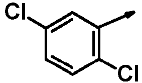 |
| Q-2.31 | Q-2.32 | Q-2.33 | Q-2.34 | Q-2.35 |
| 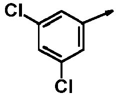 | 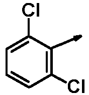 | 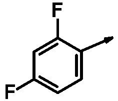 | 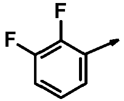 | 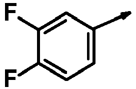 |
| Q-2.36 | Q-2.37 | Q-2.38 | Q-2.39 | Q-2.40 |
| 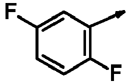 | 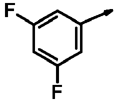 | 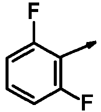 | 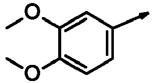 | 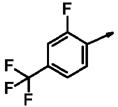 |
| Q-2.41 | Q-2.42 | Q-2.43 | Q-2.44 | Q-2.45 |
| 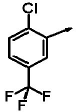 | 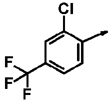 | 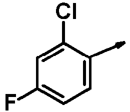 | 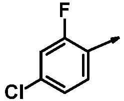 | 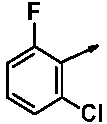 |
| Q-2.46 | Q-2.47 | Q-2.48 | Q-2.49 | Q-2.50 |
| 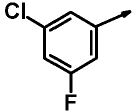 | 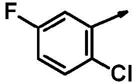 | 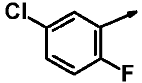 | | |
| Q-2.51 | Q-2.52 | Q-2.53 | | |
steht,
R³ für Wasserstoff steht,
R⁴ für Wasserstoff, Methyl, Ethyl, n-Propyl, i-Propyl, n-Butyl, n-Pentyl, Phenyl, Benzyl, CH₂(4-Cl-Ph), CH₂(4-F-Ph), CH₂(4-MeO-Ph), 2-Methoxyethyl, Tetrahydrofuran-2-yl-methyl, Tetrahydrofuran-3-yl-methyl, Tetrahydropyran-2-yl-methyl, Tetrahydropyran-3-yl-methyl, Tetrahydropyran-4-yl-methyl, Methylpropionat-3-yl, Ethylpropionat-3-yl, Methylacetat-2-yl, Ethylacetat-2-yl, Methylpivalat-2-yl, Ethylpivalat-3-yl, Methyl-2-methylpropanoat-3-yl, Methyl-2,2-dimethylpropanoat-3-yl, Ethyl-2-methylpropanoat-3- yl, Methyl-2-propanoat-2-yl, Ethyl-2-propanoat-2-yl, Methyl-acetat-2-yl, Ethyl-acetat-2-yl, Methyl-1-methylcyclopropancarboxylat-2-yl, Ethyl-1-methylcyclopropancarboxylat-2-yl, 2-(Dimethylamino)ethyl, Oxetan-3-yl, (3-Methyloxetan-3-yl)methyl, 2,2,2-Trifluorethyl, 2,2-Difluorethyl, 2-Fluorethyl, 2,2,3,3,3-Pentafluorpropyl, Cyclopropylmethyl, 1-Cyclopropyl-ethyl, (1-Methyl-cyclopropyl)-methyl, (2,2-Dichlorcyclopropyl)-methyl, (2,2-Dimethyl-cyclopropyl)-methyl, Allyl, Propargyl (Prop-2-in-1-yl), 2-Chlorprop-2-en-1-yl, 3-Phenylprop-2-in-1-yl, 3,3-Dichlorprop-2-en-1-yl, 3,3-Dichlor-2-fluor-prop-2-en-1-yl, Methylprop-2-in-1-yl, 2-Methylprop-2-en-1-yl, But-2-en-1-yl, But-3-en-1-yl, But-2-in-1-yl, But-3-in-1-yl, 4-Chlor-but-2-in-1-yl, 3-Methyl-but-2-en-1-yl, 3-Methyl-but-1-en-1-yl, (2E)-1-Methylbut-2-en-1-yl, (E)-Pent-3-en-2-yl oder (Z)-Pent-3-en-2-yl, Cyclobutylmethyl, Cyclopentylmethyl, Cyclohexylmethyl, Heptan-2-yl, iso-Butyl, 1,3-Dioxolan-2-ylmethyl oder 1-Ethyl-5-methyl-1H-pyrazol-4-methyl steht.

8. Nutz- oder kulturpflanzenschützendes Mittel, **gekennzeichnet durch** einen Gehalt mindestens einer Verbindung der allgemeinen Formel (I) oder deren Salze gemäß einem der Ansprüche 1 bis 7, in Kombination mit weiteren Agrochemikalien und gegebenenfalls Formulierungshilfsmitteln.

9. Mittel gemäß Anspruch 8, enthaltend mindestens ein Herbizid.

10. Verfahren zur Reduktion phytotoxischer Wirkungen von Pestiziden auf Nutz-oder Kulturpflanzen durch Verwendung einer oder mehrer Verbindungen der Ansprüche 1-7 oder der Mittel gemäß der Ansprüche 8 und 9.

11. Verfahren zur Reduktion phytotoxischer Wirkungungen von Pestiziden auf Nutz-oder Kulturpflanzen, **dadurch gekennzeichnet, dass** eine oder mehrere erfindungsgemäße Verbindungen der allgemeinen Formel (I) gemäß der Ansprüche 1 bis 7 zur gemeinsamen Anwendung mit Pestiziden gleichzeitig oder in beliebiger Reihenfolge mit den Pesitziden ausgebracht werden.

12. Verfahren gemäß Anspruch 11, wobei es sich bei den Pestiziden um ein oder mehrere Herbizide handelt.

13. Verfahren gemäß der Ansprüche 11 oder 12, dadurch gekennzeichent, dass Verbindungen der Formel (I) oder deren Salze gemäß den Anprüchen 1 bis 7 auf die Pflanzen, Teile der Pflanzen, deren Samen oder Saatgut appliziert werden.

## Claims

1. Compounds of the general formula (I) or salts thereof 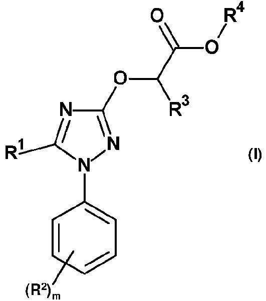 in which
R¹ is heteroaryl, where the heteroaryl radical is unsubstituted or substituted by halogen, cyano, nitro, (C₁-C₆)alkyl, (C₂-C₆)alkenyl, (C₂-C₆)alkynyl, (C₃-C₈)cycloalkyl, (C₃-C₈)cycloalkenyl, (C₁-C₆)alkoxy and (C₁-C₆)alkylS(O)ₚ, where the latter seven radicals are unsubstituted or are substituted by one or more radicals from the group of halogen, cyano, (C₁-C₆)alkoxy and (C₁-C₆)alkylS(O)ₚ,
R² is hydrogen, halogen, cyano, nitro, (C₁-C₆)alkyl, (C₂-C₆)alkenyl, (C₂-C₆)alkynyl, (C₃-C₈)cycloalkyl, (C₃-C₈)cycloalkenyl, (C₁-C₆)alkoxy and (C₁-C₆)alkyls(O)ₚ, where the latter seven radicals are unsubstituted or substituted by one already more radicals from the group of halogen, cyano, (C₁-C₆)alkoxy and (C₁-C₆)alkylS(O)ₚ,
R³ is hydrogen and (C₁-C₆)alkyl,
R⁴ is hydrogen, (C₁-C₁₈)alkyl, (C₁-C₁₈)haloalkyl, (C₁-C₁₈)cyanoalkyl, (C₂-C₁₈)alkenyl, (C₂-C₁₈)alkynyl, (C₃-C₁₂)cycloalkyl, (C₃-C₁₂)cycloalkenyl, aryl, heteroaryl, (C₁-C₁₈)alkoxy- (C₁-C₁₈)alkyl, (C₁-C₁₈)haloalkoxy- (C₁-C₁₈)alkyl, (C₁-C₁₈)alkoxy- (C₁-C₁₈)haloalkyl, (C₁-C₁₈)alkylthio- (C₁-C₁₈)alkyl, (C₁-C₁₈)haloalkylthio- (C₁-C₁₈)alkyl, (C₂-C₁₈)haloalkenyl, (C₂-C₁₈)haloalkynyl, heterocyclyl-(C₁-C₁₈)alkyl, aryl-(C₁-C₁₈)alkyl, (C₃-C₁₂)cycloalkyl - (C₁-C₁₈)alkyl, (C₁-C₁₈)alkoxycarbonyl - (C₁-C₁₈)alkyl and (C₁-C₁₈)alkoxycarbonyl- (C₃-C₁₂)cycloalkyl-(C₁-C₁₈)alkyl, or
a radical of the formula -NR^{a}R^{b} or -N=CR^{c}R^{d},
where, in the former 2 radicals, each of the R^{a}, R^{b}, R^{c} and R^{d} radicals is independently hydrogen, (C₁-C₄)alkyl, (C₂-C₄)alkenyl, (C₂-C₄)alkynyl, benzyl, substituted benzyl, phenyl or substituted phenyl,
or R^{a} and R^{b} together with the nitrogen atom may form a 3- to 8-membered heterocycle which, in addition to the nitrogen atom, may contain one or two further ring heteroatoms from the group of N, O and S and which is unsubstituted or substituted by one or more radicals from the group of (C₁-C₄)alkyl and (C₁-C₄)haloalkyl,
or R^{c} and R^{d} together with the carbon atom are a 3- to 8-membered carbocyclic or heterocyclic radical which may contain 1 to 3 ring heteroatoms from the group of N, O and S, where the carbocyclic or heterocyclic radical is unsubstituted or substituted by one or more radicals from the group of (C₁-C₄)alkyl and (C₁-C₄)haloalkyl,
m is a number from 0 to 5,
and
p is 0, 1 or 2.

2. Compounds of the general formula (I) according to Claim 1 or salts thereof,
in which
R¹ is heteroaryl, where the heteroaryl radical is unsubstituted or substituted by halogen, cyano, nitro, (C₁-C₆)alkyl, (C₂-C₆)alkenyl, (C₂-C₄)alkynyl, (C₃-C₇)cycloalkyl, (C₃-C₇)cycloalkenyl, (C₁-C₄)alkoxy and (C₁-C₄)alkylS(O)ₚ, where the latter seven radicals are unsubstituted or are substituted by one or more radicals from the group of halogen, cyano, (C₁-C₄)alkoxy and (C₁-C₄) alkylS(O)ₚ,
R² is hydrogen, halogen, cyano, nitro, (C₁-C₄)alkyl, (C₂-C₄)alkenyl, (C₂-C₄)alkynyl, (C₃-C₆)cycloalkyl, (C₃-C₆)cycloalkenyl, (C₁-C₄)alkoxy and (C₁-C₄)alkylS(O)ₚ, where the latter seven radicals are unsubstituted or are substituted by one already more radicals from the group of halogen, cyano, (C₁-C₄)alkoxy and (C₁-C₄)alkylS(O)ₚ,
R³ is hydrogen and (C₁-C₄)alkyl,
R⁴ is hydrogen, (C₁-C₁₆)alkyl, (C₁-C₁₆)haloalkyl, (C₁-C₁₆)cyanoalkyl, (C₂-C₁₆)alkenyl, (C₂-C₁₆)alkynyl, (C₃-C₁₂)cycloalkyl, (C₃-C₁₂)cycloalkenyl, aryl, heteroaryl, (C₁-C₁₆)alkoxy- (C₁-C₁₆)alkyl, (C₁-C₁₆)haloalkoxy-(C₁-C₁₆)alkyl, (C₁-C₁₆)alkoxy-(C₁-C₁₆)haloalkyl, (C₁-C₁₆)alkylthio- (C₁-C₁₆)alkyl, (C₁-C₁₆)haloalkylthio-(C₁-C₁₆)alkyl, (C₂-C₁₆)haloalkenyl, (C₂-C₁₆)haloalkynyl, heterocyclyl- (C₁-C₁₆)alkyl, aryl-(C₁-C₁₆)alkyl, (C₃-C₁₂)cycloalkyl - (C₁-C₁₆)alkyl, (C₁-C₁₆)alkoxycarbonyl-(C₁-C₁₆)alkyl and (C₁-C₁₆)alkoxycarbonyl-(C₃-C₁₂)cycloalkyl-(C₁-C₁₆)alkyl,
m is a number from 0 to 4,
and
p is 0, 1 or 2.

3. Compounds of the general formula (I) according to Claim 1 or salts thereof, in which
R¹ is heteroaryl, where the heteroaryl radical is unsubstituted or mono- or polysubstituted by halogen, cyano, methyl, ethyl, CF₃, CF₂Cl, CH₂F, CHF₂, OCH₃, OCF₃, SCH₃, SOCH₃, SO₂CH₃ and SCF₃,
R² is hydrogen, halogen, cyano, methyl, ethyl, CF₃, CF₂Cl, CH₂F, CHF₂, OCH₃, OCF₃, SCH₃, SOCH₃, SO₂CH₃ and SCF₃,
R³ is hydrogen, CH₂CH₃ and CH₃,
R⁴ is hydrogen, (C₁-C₁₂)alkyl, (C₁-C₁₂)haloalkyl, (C₁-C₁₂)cyanoalkyl, (C₂-C₁₂)alkenyl, (C₂-C₁₂)alkynyl, (C₃-C₁₂)cycloalkyl, (C₃-C₁₂)cycloalkenyl, aryl, heteroaryl, (C₁-C₁₂)alkoxy- (C₁-C₁₂)alkyl, (C₁-C₁₂)haloalkoxy- (C₁-C₁₂)alkyl, (C₁-C₁₂)alkoxy- (C₁-C₁₂)haloalkyl, (C₁-C₁₂)alkylthio- (C₁-C₁₂)alkyl, (C₁-C₁₂)haloalkylthio- (C₁-C₁₂)alkyl, (C₂-C₁₂)haloalkenyl, (C₂-C₁₂)haloalkynyl, heterocyclyl- (C₁-C₁₂)alkyl, aryl- (C₁-C₁₂)alkyl, (C₃-C₁₂)cycloalkyl-(C₁-C₁₂)alkyl, (C₁-C₁₂)alkoxycarbonyl-(C₁-C₁₂)alkyl and (C₁-C₁₂)alkoxycarbonyl- (C₃-C₁₂)cycloalkyl-(C₁-C₁₂)alkyl,
and
m is 0, 1, 2 or 3.

4. Compounds of the general formula (I) according to Claim 1 or salts thereof, in which
R¹ is pyridin-2-yl, pyridin-3-yl, pyridin-4-yl, pyrazin-2-yl, pyrazin-3-yl, pyrimidin-2-yl, pyrimidin-4-yl, pyrimidin-5-yl, pyridazin-3-yl, pyridazin-4-yl, 1,3,5-triazin-2-yl, 1,2,4-triazin-3-yl, 1,2,4-triazin-5-yl, 1,2,4-triazin-6-yl, 1,2,3-triazin-4-yl, 1,2,3-triazin-5-yl, 1,2,4-, 1,3,2-, 1,3,6- and 1,2,6-oxazinyl, isoxazol-3-yl, isoxazol-4-yl, isoxazol-5-yl, 1,3-oxazol-2-yl, 1,3-oxazol-4-yl, 1,3-oxazol-5-yl, isothiazol-3-yl, isothiazol-4-yl, isothiazol-5-yl, 1,3-thiazol-2-yl, 1,3-thiazol-4-yl, 1,3-thiazol-5-yl, 1H-pyrrol-1-yl, 1H-pyrrol-2-yl, 1H-pyrrol-3-yl, furan-2-yl, furan-3-yl, thien-2-yl; thien-3-yl, 1H-imidazol-1-yl, 1H-imidazol-2-yl, 1H-imidazol-4-yl, 1H-imidazol-5-yl, 1H-pyrazol-1-yl, 1H-pyrazol-3-yl, 1H-pyrazol-4-yl, 1H-pyrazol-5-yl, 1H-1,2,3-triazol-1-yl, 1H-1,2,3-triazol-4-yl, 1H-1,2,3-triazol-5-yl, 2H-1,2,3-triazol-2-yl, 2H-1,2,3-triazol-4-yl, 1H-1,2,4-triazol-1-yl, 1H-1,2,4-triazol-5-yl, 4H-1,2,4-triazol-3-yl, 1,2,4-oxadiazol-3-yl, 1,2,4-oxadiazol-5-yl, 1,3,4-oxadiazol-2-yl, 1,2,3-oxadiazol-4-yl, 1,2,3-oxadiazol-5-yl, 1,2,5-oxadiazol-3-yl, 1,3,4-thiadiazol-2-yl, 1,3,4-thiadiazol-2-yl, 1,2,4-thiadiazol-5-yl, 1,2,4-thiadiazol-3-yl, 1,2,5-thiadiazol-3-yl, 1,2,5-thiadiazol-3-yl, 1,2,3-thiadiazol-4-yl, 1,2,3-thiadiazol-3-yl, which is unsubstituted or mono- or polysubstituted by halogen, cyano, methyl, CF₃, CF₂Cl, CH₂F, CHF₂, OCH₃, OCF₃, SCH₃, SOCH₃, SO₂CH₃ and SCF₃,
R² is hydrogen, fluorine, chlorine, bromine, iodine, CN, methyl, CF₃,CF₂Cl, CH₂F, CHF₂, OCH₃, OCF₃, SCH₃, SOCH₃, SO₂CH₃ and SCF₃,
R³ is hydrogen and CH₃,
R⁴ is hydrogen, (C₁-C₁₀)alkyl, (C₁-C₁₀)haloalkyl, (C₁-C₁₀)cyanoalkyl, (C₂-C₁₀)alkenyl, (C₂-C₁₀)alkynyl, (C₃-C₉)cycloalkyl, (C₃-C₉)cycloalkenyl, aryl, heteroaryl, (C₁-C₁₀)alkoxy-(C₁-C₁₀)alkyl, (C₁-C₁₀)haloalkoxy- (C₁-C₁₀)alkyl, (C₁-C₁₀)alkoxy- (C₁-C₁₀)haloalkyl, (C₁-C₁₀)alkylthio -(C₁-C₁₀)alkyl, (C₁-C₁₀)haloalkylthio-(C₁-C₁₀)alkyl, (C₂-C₁₈)haloalkenyl, (C₂-C₁₈)haloalkynyl, heterocyclyl- (C₁-C₁₀)alkyl, aryl - (C₁-C₁₀)alkyl, (C₃-C₉)cycloalkyl -(C₁-C₁₀)alkyl, (C₁-C₁₀)alkoxycarbonyl- (C₁-C₁₀)alkyl and (C₁-C₁₀)alkoxycarbonyl-(C₃-C₉)cycloalkyl- (C₁-C₁₀)alkyl,
and
m is 0, 1, 2 or 3.

5. Compounds of the general formula (I) according to Claim 1 or salts thereof, in which
R¹ represents the Q-1.1 to Q-1.59 groups
| | | | | |
|---|---|---|---|---|
| 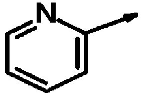 | 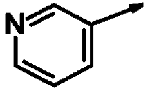 | 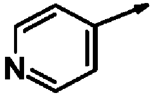 | 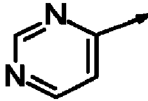 | 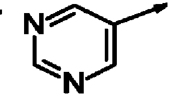 |
| Q-1.1 | Q-1.2 | Q-1.3 | Q-1.4 | Q-1.5 |
| 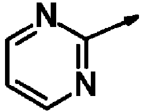 | 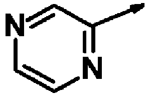 | 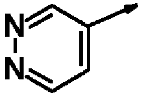 | 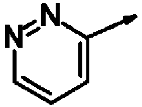 | 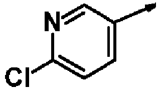 |
| Q-1.6 | Q-1.7 | Q-1.8 | Q-1.9 | Q-1.10 |
| 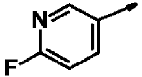 | 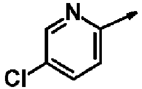 | 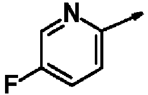 | 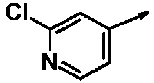 | 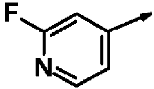 |
| Q-1.11 | Q-1.12 | Q-1.13 | Q-1.14 | Q-1.15 |
| 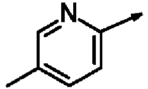 | 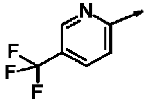 | 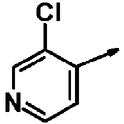 | 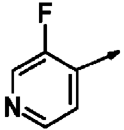 | 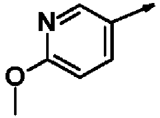 |
| Q-1.16 | Q-1.17 | Q-1.18 | Q-1.19 | Q-1.20 |
| 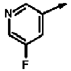 | 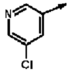 | 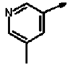 | 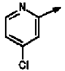 | 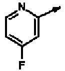 |
| Q-1.21 | Q-1.22 | Q-1.23 | Q-1.24 | Q-1.25 |
| 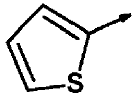 | 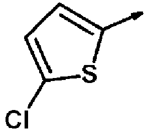 | 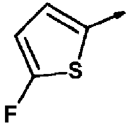 | 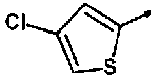 | 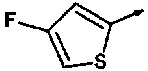 |
| Q-1.26 | Q-1.27 | Q-1.28 | Q-1.29 | Q-1.30 |
| 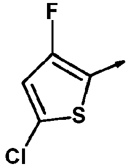 | 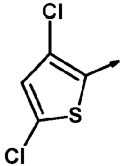 | 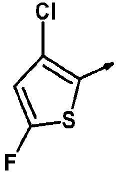 | 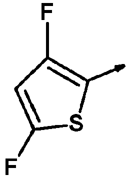 | 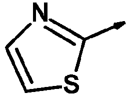 |
| Q-1.31 | Q-1.32 | Q-1.33 | Q-1.34 | Q-1.35 |
| 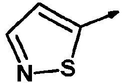 | 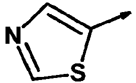 | 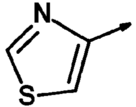 | 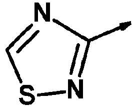 | 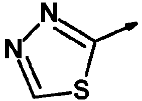 |
| Q-1.36 | Q-1.37 | Q-1.38 | Q-1.39 | Q-1.40 |
| 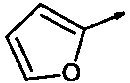 | 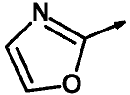 | 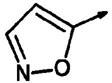 | 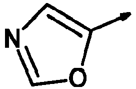 | 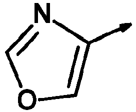 |
| Q-1.41 | Q-1.42 | Q-1.43 | Q-1.44 | Q-1.45 |
| 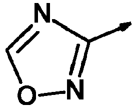 | 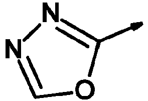 | 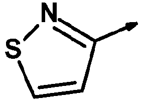 | 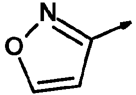 | 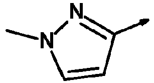 |
| Q-1.46 | Q-1.47 | Q-1.48 | Q-1.49 | Q-1.50 |
| 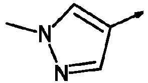 | 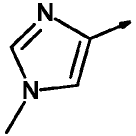 | 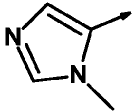 | 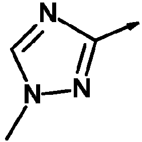 | 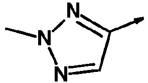 |
| Q-1.51 | Q-1.52 | Q-1.53 | Q-1.54 | Q-1.55 |
| 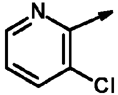 | 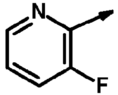 | 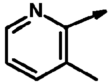 | 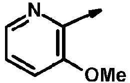 | |
| Q-1.56 | Q-1.57 | Q-1.58 | Q-1.59 | |
R² is hydrogen, fluorine, chlorine, bromine, iodine, cyano, methyl, CF₃, CH₂F, CHF₂, OCH₃, OCF₃, SCH₃, SOCH₃, SO₂CH₃ and SCF₃,
R³ is hydrogen,
R⁴ hydrogen, methyl, ethyl, n-propyl, i-propyl, n-butyl, n-pentyl, phenyl, benzyl, CH₂(4-Cl-Ph), CH₂(4-F-Ph), CH₂(4-MeO-Ph), 2-methoxyethyl, tetrahydrofuran-2-ylmethyl, tetrahydrofuran-3-ylmethyl, tetrahydropyran-2-ylmethyl, tetrahydropyran-3-ylmethyl, tetrahydropyran-4-ylmethyl, methylpropionate-3-yl, ethylpropionate-3-yl, methylacetate-2-yl, ethylacetate-2-yl, methylpivalate-2-yl, ethylpivalate-3-yl, methyl-2-methylpropanoate-3-yl, methyl-2,2-dimethylpropanoate-3-yl, ethyl-2-methylpropanoate-3-yl, methyl-2-propanoate-2-yl, ethyl-2-propanoate-2-yl, methylacet-2-yl, ethylacetate-2-yl, methyl-1-methylcyclopropanecarboxylate-2-yl, ethyl-1-methylcyclopropanecarboxylate-2-yl, 2-(dimethylamino)ethyl, oxetan-3-yl, (3-methyloxetan-3-yl)methyl, 2,2,2-trifluoroethyl, 2,2-difluoroethyl, 2-fluoroethyl, 2,2,3,3,3-pentafluoropropyl, cyclopropylmethyl, 1-cyclopropylethyl, (1-methylcyclopropyl)methyl, (2,2-dichlorocyclopropyl)methyl, (2,2-dimethylcyclopropyl)methyl, allyl, propargyl (prop-2-yn-1-yl), 2-chloroprop-2-en-1-yl, 3-phenylprop-2-yn-1-yl, 3,3-dichloroprop-2-en-1-yl, 3,3-dichloro-2-fluoroprop-2-en-1-yl, methylprop-2-yn-1-yl, 2-methylprop-2-en-1-yl, but-2-en-1-yl, but-3-en-1-yl, but-2-yn-1-yl, but-3-yn-1-yl, 4-chlorobut-2-yn-1-yl, 3-methylbut-2-en-1-yl, 3-methylbut-1-en-1-yl, (2E)-1-methylbut-2-en-1-yl, (E)-pent- 3 -en- 2-yl or (Z) -pent-3-en-2-yl, cyclobutylmethyl, cyclopentylmethyl, cyclohexylmethyl, heptan-2-yl, isobutyl, 1,3-dioxolan-2-ylmethyl or 1-ethyl-5-methyl-1H-pyrazole-4-methyl,
m is 0, 1, 2 or 3.

6. Compounds of the general formula (I) according to Claim 1 or salts thereof,
in which
R¹ represents the Q-1.1 to Q-1.59 groups
| | | | | |
|---|---|---|---|---|
| 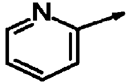 | 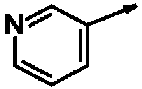 | 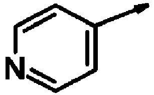 | 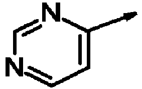 | 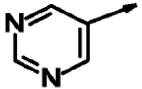 |
| Q-1.1 | Q-1.2 | Q-1.3 | Q-1.4 | Q-1.5 |
| 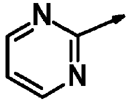 | 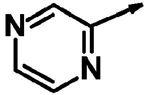 | 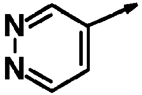 | 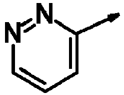 | 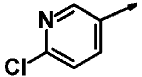 |
| Q-1.6 | Q-1.7 | Q-1.8 | Q-1.9 | Q-1.10 |
| 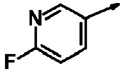 | 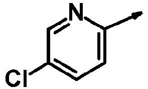 | 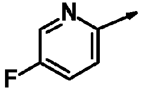 | 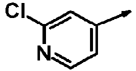 | 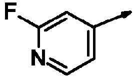 |
| Q-1.11 | Q-1.12 | Q-1.13 | Q-1.14 | Q-1.15 |
| 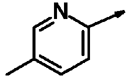 | 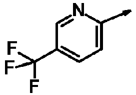 | 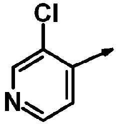 | 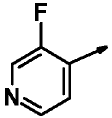 | 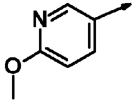 |
| Q-1.16 | Q-1.17 | Q-1.18 | Q-1.19 | Q-1.20 |
| 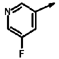 | 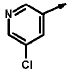 | 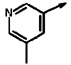 | 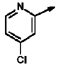 | 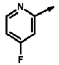 |
| Q-1.21 | Q-1.22 | Q-1.23 | Q-1.24 | Q-1.25 |
| 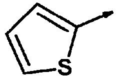 | 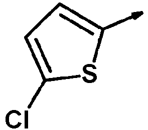 | 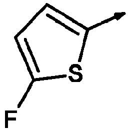 | 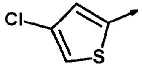 | 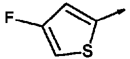 |
| Q-1.26 | Q-1.27 | Q-1.28 | Q-1.29 | Q-1.30 |
| 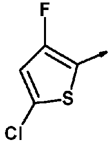 | 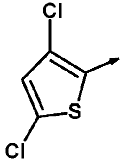 | 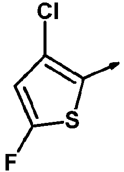 | 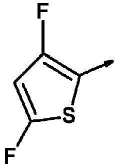 | 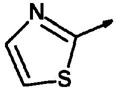 |
| Q-1.31 | Q-1.32 | Q-1.33 | Q-1.34 | Q-1.35 |
| 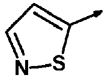 | 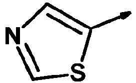 | 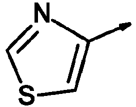 | 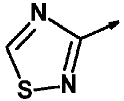 | 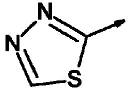 |
| Q-1.36 | Q-1.37 | Q-1.38 | Q-1.39 | Q-1.40 |
| 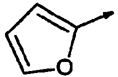 | 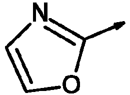 | 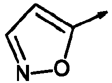 | 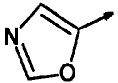 | 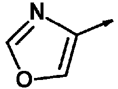 |
| Q-1.41 | Q-1.42 | Q-1.43 | Q-1.44 | Q-1.45 |
| 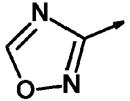 | 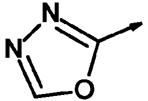 | 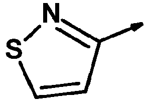 | 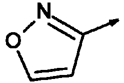 | 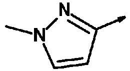 |
| Q-1.46 | Q-1.47 | Q-1.48 | Q-1.49 | Q-1.50 |
| 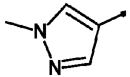 | 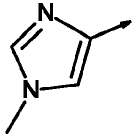 | 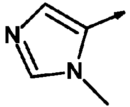 | 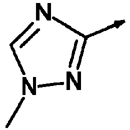 | 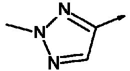 |
| Q-1.51 | Q-1.52 | Q-1.53 | Q-1.54 | Q-1.55 |
| 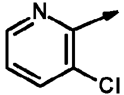 | 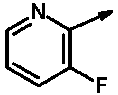 | 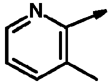 | 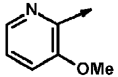 | |
| Q-1.56 | Q-1.57 | Q-1.58 | Q-1.59 | |
and (R²)ₘ-phenyl represents the Q-2.1 to Q-2.53 groups
| | | | | |
|---|---|---|---|---|
| 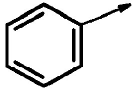 | 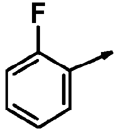 | 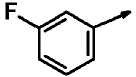 | 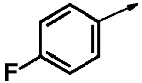 | 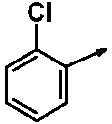 |
| Q-2.1 | Q-2.2 | Q-2.3 | Q-2.4 | Q-2.5 |
| 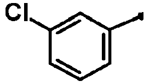 | 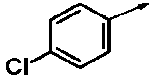 | 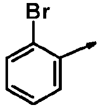 | 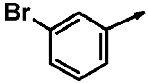 | 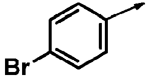 |
| Q-2.6 | Q-2.7 | Q-2.8 | Q-2.9 | Q-2.10 |
| 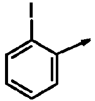 | 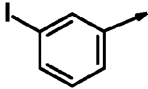 | 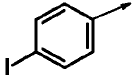 | 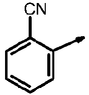 | 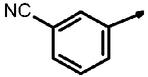 |
| Q-2.11 | Q-2.12 | Q-2.13 | Q-2.14 | Q-2.15 |
| 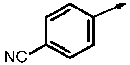 | 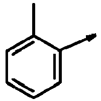 | 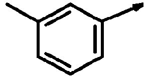 | 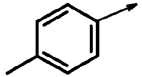 | 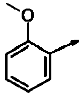 |
| Q-2.16 | Q-2.17 | Q-2.18 | Q-2.19 | Q-2.20 |
| 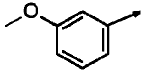 | 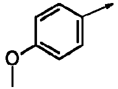 | 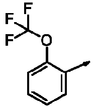 | 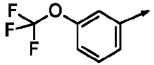 | 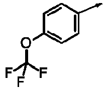 |
| Q-2.21 | Q-2.22 | Q-2.23 | Q-2.24 | Q-2.25 |
| 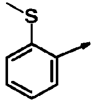 | 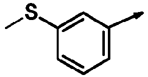 | 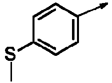 | 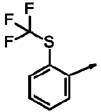 | 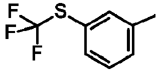 |
| Q-2.26 | Q-2.27 | Q-2.28 | Q-2.29 | Q-2.30 |
| 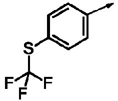 | 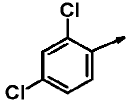 | 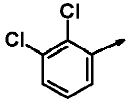 | 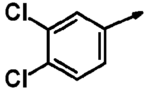 | 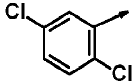 |
| Q-2.31 | Q-2.32 | Q-2.33 | Q-2.34 | Q-2.35 |
| 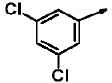 | 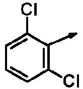 | 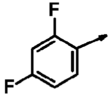 | 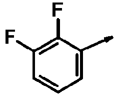 | 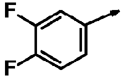 |
| Q-2.36 | Q-2.37 | Q-2.38 | Q-2.39 | Q-2.40 |
| 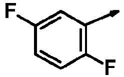 | 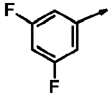 | 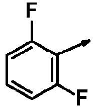 | 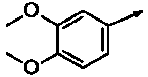 | 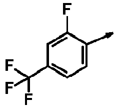 |
| Q-2.41 | Q-2.42 | Q-2.43 | Q-2.44 | Q-2.45 |
| 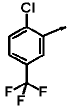 | 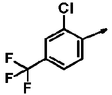 | 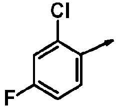 | 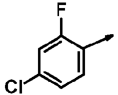 | 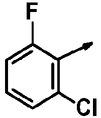 |
| Q-2.46 | Q-2.47 | Q-2.48 | Q-2.49 | Q-2.50 |
| 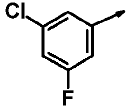 | 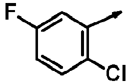 | 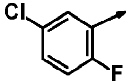 | | |
| Q-2.51 | Q-2.52 | Q-2.53 | | |
R³ is hydrogen,
R⁴ is hydrogen, methyl, ethyl, n-propyl, i-propyl, n-butyl, n-pentyl, phenyl, benzyl, CH₂(4-Cl-Ph), CH₂(4-F-Ph), CH₂(4-MeO-Ph), 2-methoxyethyl, tetrahydrofuran-2-ylmethyl, tetrahydrofuran-3-ylmethyl, tetrahydropyran-2-ylmethyl, tetrahydropyran-3-ylmethyl, tetrahydropyran-4-ylmethyl, methylpropionate-3-yl, ethylpropionate-3-yl, methylacetate-2-yl, ethylacetate-2-yl, methylpivalate-2-yl, ethylpivalate-3-yl, methyl-2-methylpropanoate-3-yl, methyl-2,2-dimethylpropanoate-3-yl, ethyl-2-methylpropanoate-3-yl, methyl-2-propanoate-2-yl, ethyl-2-propanoate-2-yl, methylacetate-2-yl, ethylacetate-2-yl, methyl-1-methylcyclopropanecarboxylate-2-yl, ethyl-1-methylcyclopropanecarboxylate-2-yl, 2-(dimethylamino)ethyl, oxetan-3-yl, (3-methyloxetan-3-yl)methyl, 2,2,2-trifluoroethyl, 2,2-difluoroethyl, 2-fluoroethyl, 2,2,3,3,3-pentafluoropropyl, cyclopropylmethyl, 1-cyclopropylethyl, (1-methylcyclopropyl)methyl, (2,2-dichlorocyclopropyl)methyl, (2,2-dimethylcyclopropyl)methyl, allyl, propargyl (prop-2-yn-1-yl), 2-chloroprop-2-en-1-yl, 3-phenylprop-2-yn-1-yl, 3,3-dichloroprop-2-en-1-yl, 3,3-dichloro-2-fluoroprop-2-en-1-yl, methylprop-2-yn-1-yl, 2-methylprop-2-en-1-yl, but-2-en-1-yl, but-3-en-1-yl, but-2-yn-1-yl, but-3-yn-1-yl, 4-chlorobut-2-yn-1-yl, 3-methylbut-2-en-1-yl, 3-methylbut-1-en-1-yl, (2E) -1-methylbut-2-en-1-yl, (E)-pent-3-en-2-yl or (Z) -pent-3-en-2-yl, cyclobutylmethyl, cyclopentylmethyl, cyclohexylmethyl, heptan-2-yl, isobutyl, 1,3-dioxolan-2-ylmethyl or 1-ethyl-5-methyl-1H-pyrazole-4-methyl.

7. Compounds of the general formula (I) according to Claim 1 or salts thereof,
in which
R¹ is Q-1.1 to Q-1.59
| | | | | |
|---|---|---|---|---|
| 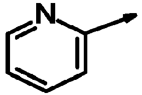 | 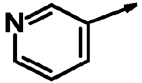 | 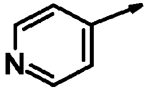 | 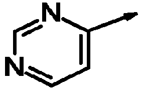 | 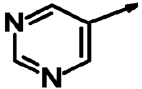 |
| Q-1.1 | Q-1.2 | Q-1.3 | Q-1.4 | Q-1.5 |
| 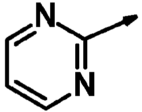 | 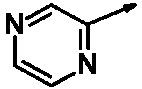 | 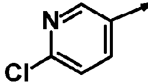 | 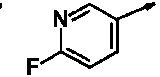 | 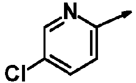 |
| Q-1.6 | Q-1.7 | Q-1.10 | Q-1.11 | Q-1.12 |
| 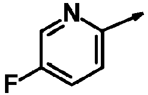 | 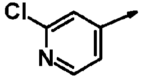 | 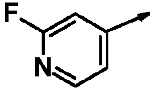 | 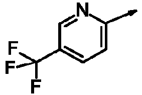 | 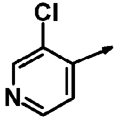 |
| Q-1.13 | Q-1.14 | Q-1.15 | Q-1.17 | Q-1.18 |
| 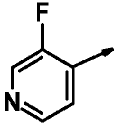 | 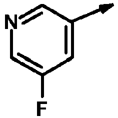 | 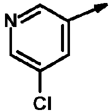 | 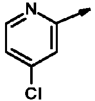 | 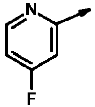 |
| Q-1.19 | Q-1.21 | Q-1.22 | Q-1.24 | Q-1.25 |
| 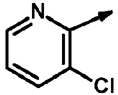 | 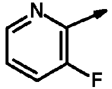 | 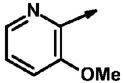 | | |
| Q-1.56 | Q-1.57 | Q-1.59 | | |
and (R²)ₘ-phenyl represents the Q-2.1 to Q-2.53 groups
| | | | | |
|---|---|---|---|---|
| 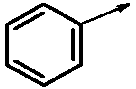 | 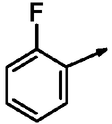 | 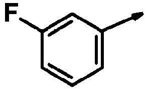 | 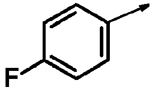 | 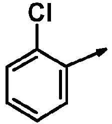 |
| Q-2.1 | Q-2.2 | Q-2.3 | Q-2.4 | Q-2.5 |
| 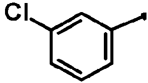 | 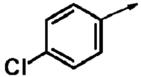 | 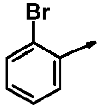 | 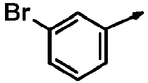 | 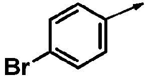 |
| Q-2.6 | Q-2.7 | Q-2.8 | Q-2.9 | Q-2.10 |
| 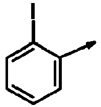 | 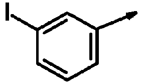 | 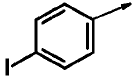 | 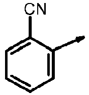 | 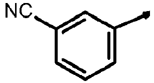 |
| Q-2.11 | Q-2.12 | Q-2.13 | Q-2.14 | Q-2.15 |
| 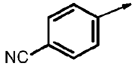 | 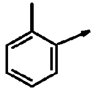 | 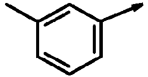 | 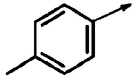 | 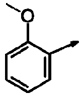 |
| Q-2.16 | Q-2.17 | Q-2.18 | Q-2.19 | Q-2.20 |
| 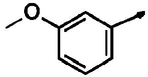 | 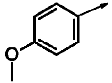 | 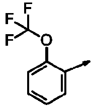 | 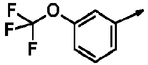 | 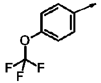 |
| Q-2.21 | Q-2.22 | Q-2.23 | Q-2.24 | Q-2.25 |
| 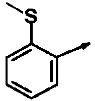 | 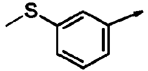 | 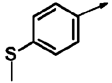 | 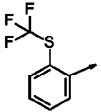 | 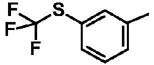 |
| Q-2.26 | Q-2.27 | Q-2.28 | Q-2.29 | Q-2.30 |
| 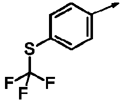 | 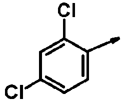 | 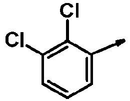 | 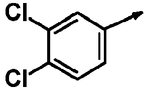 | 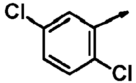 |
| Q-2.31 | Q-2.32 | Q-2.33 | Q-2.34 | Q-2.35 |
| 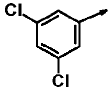 | 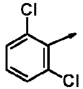 | 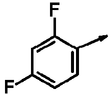 | 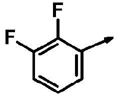 | 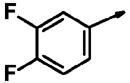 |
| Q-2.36 | Q-2.37 | Q-2.38 | Q-2.39 | Q-2.40 |
| 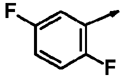 | 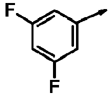 | 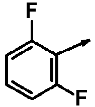 | 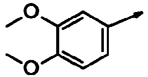 | 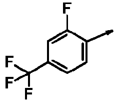 |
| Q-2.41 | Q-2.42 | Q-2.43 | Q-2.44 | Q-2.45 |
| 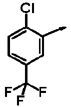 | 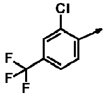 | 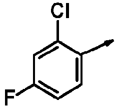 | 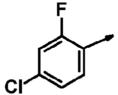 | 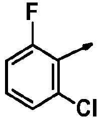 |
| Q-2.46 | Q-2.47 | Q-2.48 | Q-2.49 | Q-2.50 |
| 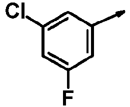 | 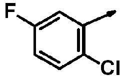 | 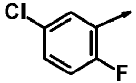 | | |
| Q-2.51 | Q-2.52 | Q-2.53 | | |
R³ is hydrogen,
R⁴ is hydrogen, methyl, ethyl, n-propyl, i-propyl, n-butyl, n-pentyl, phenyl, benzyl, CH₂(4-Cl-Ph), CH₂(4-F-Ph), CH₂(4-MeO-Ph), 2-methoxyethyl, tetrahydrofuran-2-ylmethyl, tetrahydrofuran-3-ylmethyl, tetrahydropyran-2-ylmethyl, tetrahydropyran-3-ylmethyl, tetrahydropyran-4-ylmethyl, methylpropionate-3-yl, ethylpropionate-3-yl, methylacetate-2-yl, ethylacetate-2-yl, methylpivalate-2-yl, ethylpivalate-3-yl, methyl-2-methylpropanoate-3-yl, methyl-2,2-dimethylpropanoate-3-yl, ethyl-2-methylpropanoate-3-yl, methyl-2-propanoate-2-yl, ethyl-2-propanoate-2-yl, methylacetate-2-yl, ethylacetate-2-yl, methyl-1-methylcyclopropanecarboxylate-2-yl, ethyl-1-methylcyclopropanecarboxylate-2-yl, 2-(dimethylamino)ethyl, oxetan-3-yl, (3-methyloxetan-3-yl)methyl, 2,2,2-trifluoroethyl, 2,2-difluoroethyl, 2-fluoroethyl, 2,2,3,3,3-pentafluoropropyl, cyclopropylmethyl, 1-cyclopropylethyl, (1-methylcyclopropyl)methyl, (2,2-dichlorocyclopropyl)methyl, (2,2-dimethylcyclopropyl)methyl, allyl, propargyl (prop-2-yn-1-yl), 2-chloroprop-2-en-1-yl, 3-phenylprop-2-yn-1-yl, 3,3-dichloroprop-2-en-1-yl, 3,3-dichloro-2-fluoroprop-2-en-1-yl, methylprop-2-yn-1-yl, 2-methylprop-2-en-1-yl, but-2-en-1-yl, but-3-en-1-yl, but-2-yn-1-yl, but-3-yn-1-yl, 4-chlorobut-2-yn-1-yl, 3-methylbut-2-en-1-yl, 3-methylbut-1-en-1-yl, (2E) -1-methylbut-2-en-1-yl, (E)-pent- 3 -en- 2-yl or (Z) -pent-3-en-2-yl, cyclobutylmethyl, cyclopentylmethyl, cyclohexylmethyl, heptan-2-yl, isobutyl, 1,3-dioxolan-2-ylmethyl or 1-ethyl-5-methyl-1H-pyrazole-4-methyl.

8. Useful plant- or crop plant-protecting composition, **characterized by** a content of at least one compound of the general formula (I) or salts thereof according to any of Claims 1 to 7 in combination with further agrochemicals and optionally formulation auxiliaries.

9. Composition according to Claim 8, comprising at least one herbicide.

10. Method of reducing phytotoxic effects of pesticides on useful plants or crop plants by the use of one or more compounds of Claims 1-7 or of the compositions according to Claim 8 or 9.

11. Method of reducing phytotoxic effects of pesticides on useful plants or crop plants, **characterized in that** one or more inventive compounds of the general formula (I) according to any of Claims 1 to 7 for joint use with pesticides is deployed simultaneously or in any sequence with the pesticides.

12. Method according to Claim 11, wherein the pesticides are one or more herbicides.

13. Method according to either of Claims 11 and 12, **characterized in that** compounds of the formula (I) or salts thereof according to Claims 1 to 7 are applied to the plants, parts of the plants, or the seeds or seed material thereof.

## Revendications

1. Composés de formule générale (I) ou leurs sels 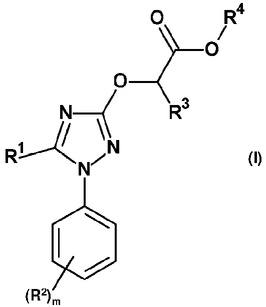 dans laquelle
R¹ représente hétéroaryle, le radical hétéroaryle étant non substitué ou substitué par halogène, cyano, nitro, (C₁-C₆)alkyle, (C₂-C₆)alcényle, (C₂-C₆)alcynyle, (C₃-C₈)cycloalkyle, (C₃-C₈)cycloalcényle, (C₁-C₆)alcoxy et (C₁-C₆)alkyl-S(O)ₚ, les sept radicaux cités en dernier étant non substitués ou substitués par un ou plusieurs radicaux du groupe halogène, cyano, (C₁-C₆)alcoxy et (C₁-C₆)alkyl-S(O)ₚ,
R² représente hydrogène, halogène, cyano, nitro, (C₁-C₆)alkyle, (C₂-C₆)alcényle, (C₂-C₆)alcynyle, (C₃-C₈)cycloalkyle, (C₃-C₈)cycloalcényle, (C₁-C₆)alcoxy et (C₁-C₆)alkyl-S(O)ₚ, les sept radicaux cités en dernier étant non substitués ou substitués par un ou plusieurs radicaux du groupe halogène, cyano, (C₁-C₆)alcoxy et (C₁-C₆)alkyl-S(O)ₚ,
R³ représente hydrogène et (C₁-C₆)alkyle,
R⁴ représente hydrogène, (C₁-C₁₈)alkyle, (C₁-C₁₈)halogénoalkyle, (C₁-C₁₈)cyanoalkyle, (C₂-C₁₈)alcényle, (C₂-C₁₈)alcynyle, (C₃-C₁₂)cycloalkyle, (C₃-C₁₂)cycloalcényle, aryle, hétéroaryle, (C₁-C₁₈)alcoxy- (C₁-C₁₈)alkyle, (C₁-C₁₈)halogénoalcoxy-(C₁-C₁₈)alkyle, (C₁-C₁₈)alcoxy- (C₁-C₁₈)halogénoalkyle, (C₁-C₁₈)alkyl- thio -(C₁-C₁₈)alkyle, (C₁-C₁₈)halogénoalkyl-thio - (C₁-C₁₈)alkyle, (C₂-C₁₈)halogénoalcényle, (C₂-C₁₈)halogénoalcynyle, hétérocyclyl- (C₁-C₁₈)alkyle, aryl- (C₁-C₁₈)alkyle, (C₃-C₁₂)cycloalkyl- (C₁-C₁₈)alkyle, (C₁-C₁₈)alcoxy-carbonyl-(C₁-C₁₈)alkyle et (C₁-C₁₈)alcoxy-carbonyl-(C₃-C₁₂)cycloalkyl- (C₁-C₁₈)alkyle ou signifie un radical de formule -NR^{a}R^{b} ou -N=CR^{c}R^{d},
où, dans les 2 radicaux susmentionnés, chacun des radicaux R^{a}, R^{b}, R^{c} et R^{d} signifie, indépendamment les uns des autres, hydrogène, (C₁-C₄)alkyle, (C₂-C₄)alcényle, (C₂-C₄)alcynyle, benzyle, benzyle substitué, phényle ou phényle substitué,
ou R^{a} et R^{b} peuvent former, ensemble avec l'atome de N, un hétérocycle de 3 à 8 chaînons, qui peut contenir, en plus de l'atome de N, un ou deux autres hétéroatomes de cycle du groupe N, O et S et qui est non substitué ou substitué par un ou plusieurs radicaux du groupe (C₁-C₄)alkyle et (C₁-C₄)halogénoalkyle,
ou R^{c} et R^{d} forment, ensemble avec l'atome de C, un radical carbocyclique ou hétérocyclique de 3 à 8 chaînons, qui peut contenir 1 à 3 hétéroatomes de cycle du groupe N, O et S, le radical carbocyclique ou hétérocyclique étant non substitué ou substitué par un ou plusieurs radicaux du groupe (C₁-C₄)alkyle et (C₁-C₄)halogénoalkyle,
m représente un nombre de 0 à 5
et
p signifie 0, 1 ou 2.

2. Composés de formule générale (I) selon la revendication 1 ou leurs sels, dans laquelle
R¹ représente hétéroaryle, le radical hétéroaryle étant non substitué ou substitué par halogène, cyano, nitro, (C₁-C₆)alkyle, (C₂-C₆)alcényle, (C₂-C₄)alcynyle, (C₃-C₇)cycloalkyle, (C₃-C₇)cycloalcényle, (C₁-C₄)alcoxy et (C₁-C₄)alkyl-S(O)ₚ, les sept radicaux cités en dernier étant non substitués ou substitués par un ou plusieurs radicaux du groupe halogène, cyano, (C₁-C₄)alcoxy et (C₁-C₄)alkyl -S(O)ₚ,
R² représente hydrogène, halogène, cyano, nitro, (C₁-C₄)alkyle, (C₂-C₄)alcényle, (C₂- C₄)alcynyle, (C₃-C₆)cycloalkyle, (C₃-C₆)cycloalcényle, (C₁-C₄)alcoxy et (C₁-C₄)alkyl-S(O)ₚ, les sept radicaux cités en dernier étant non substitués ou substitués par un ou plusieurs radicaux du groupe halogène, cyano, (C₁-C₄)alcoxy et (C₁-C₄)alkyl-S(O)ₚ,
R³ représente hydrogène et (C₁-C₄)alkyle,
R⁴ représente hydrogène, (C₁-C₁₆)alkyle, (C₁-C₁₆)halogénoalkyle, (C₁-C₁₆)cyanoalkyle, (C₂-C₁₆)alcényle, (C₂-C₁₆)alcynyle, (C₃-C₁₂)cycloalkyle, (C₃-C₁₂)cycloalcényle, aryle, hétéroaryle, (C₁-C₁₆)alcoxy- (C₁-C₁₆)alkyle, (C₁-C₁₆)halogénoalcoxy-(C₁-C₁₆)alkyle, (C₁-C₁₆)alcoxy- (C₁-C₁₆)halogénoalkyle, (C₁-C₁₆)alkyl- thio- (C₁-C₁₆)alkyle, (C₁-C₁₆)halogénoalkyl-thio -(C₁-C₁₆)alkyle, (C₂-C₁₆)halogénoalcényle, (C₂-C₁₆)halogénoalcynyle, hétérocyclyl- (C₁-C₁₆)alkyle, aryl-(C₁-C₁₆)alkyle, (C₃-C₁₂)cycloalkyl- (C₁-C₁₆)alkyle, (C₁-C₁₆)alcoxy-carbonyl-(C₁-C₁₆)alkyle, et (C₁-C₁₆)alcoxy-carbonyl-(C₃-C₁₂)cycloalkyl- (C₁-C₁₆)alkyle,
m représente un nombre de 0 à 4
et
p signifie 0, 1 ou 2.

3. Composés de formule générale (I) selon la revendication 1 ou leurs sels,
dans laquelle
R¹ représente hétéroaryle, le radical hétéroaryle étant non substitué ou monosubstitué ou polysubstitué par halogène, cyano, méthyle, éthyle, CF₃, CF₂Cl, CH₂F, CHF₂, OCH₃, OCF₃, SCH₃, SOCH₃, SO₂CH₃ et SCF₃,
R² représente hydrogène, halogène, cyano, méthyle, éthyle, CF₃, CF₂Cl, CH₂F, CHF₂, OCH₃, OCF₃, SCH₃, SOCH₃, SO₂CH₃ et SCF₃,
R³ représente hydrogène, CH₂CH₃ et CH₃,
R⁴ représente hydrogène, (C₁-C₁₂)alkyle, (C₁-C₁₂)halogénoalkyle, (C₁-C₁₂)cyanoalkyle, (C₂-C₁₂)alcényle, (C₂-C₁₂)alcynyle, (C₃-C₁₂)cycloalkyle, (C₃-C₁₂)cycloalcényle, aryle, hétéroaryle, (C₁-C₁₂)alcoxy- (C₁-C₁₂)alkyle, (C₁-C₁₂)halogénoalcoxy-(C₁-C₁₂)alkyle, (C₁-C₁₂)alcoxy- (C₁-C₁₂)halogénoalkyle, (C₁-C₁₂)alkyl -thio - (C₁-C₁₂)alkyle, (C₁-C₁₂)halogénoalkyl-thio - (C₁-C₁₂)alkyle, (C₂-C₁₂)halogénoalcényle, (C₂-C₁₂)halogénoalcynyle, hétérocyclyl- (C₁-C₁₂)alkyle, aryl- (C₁-C₁₂)alkyle, (C₃-C₁₂)cycloalkyl- (C₁-C₁₂)alkyle, (C₁-C₁₂)alcoxy-carbonyl-(C₁-C₁₂)alkyle, et (C₁-C₁₂)alcoxy-carbonyl-(C₃-C₁₂)cycloalkyl- (C₁-C₁₂)alkyle.
et
m représente 0, 1, 2 ou 3.

4. Composés de formule générale (I) selon la revendication 1 ou leurs sels,
dans laquelle
R¹ représente pyridin-2-yle, pyridin-3-yle, pyridin-4-yle, pyrazin-2-yle, pyrazin-3-yle, pyrimidin-2-yle, pyrimidin-4-yle, pyrimidin-5-yle, pyridazin-3-yle, pyridazin-4-yle, 1,3,5-triazin-2-yle, 1,2,4-triazin-3-yle, 1,2,4-triazin-5-yle, 1,2,4-triazin-6-yle, 1,2,3-triazin-4-yle, 1,2,3-triazin-5-yle, 1,2,4-oxdiazinyle, 1,3,2-oxdiazinyle, 1,3,6-oxdiazinyle et 1,2,6-oxdiazinyle, isoxazol-3-yle, isoxazol-4-yle, isoxazol-5-yle, 1,3-oxazol-2-yle, 1,3-oxazol-4-yle, 1,3-oxazol-5-yle, isothiazol-3-yle, isothiazol-4-yle, isothiazol-5-yle, 1,3-thiazol-2-yle, 1,3-thiazol-4-yle, 1,3-thiazol-5-yle, 1H-pyrrol-1-yle, 1H-pyrrol-2-yle, 1H-pyrrol-3-yle, furann-2-yle, furann-3-yle, thién-2-yl ; thién-3-yle, 1H-imidazol-1-yle, 1H-imidazol-2-yle, 1H-imidazol-4-yle, 1H-imidazol-5-yle, 1H-pyrazol-1-yle, 1H-pyrazol-3-yle, 1H-pyrazol-4-yle, 1H-pyrazol-5-yle, 1H-1,2,3-triazol-1-yle, 1H-1,2,3-triazol-4-yle, 1H-1,2,3-triazol-5-yle, 2H-1,2,3-triazol-2-yle, 2H-1,2,3-triazol-4-yle, 1H-1,2,4-triazol-1-yle, 1H-1,2,4-triazol-5-yle, 4H-1,2,4-triazol-3-yle, 1,2,4-oxadiazol-3-yle, 1,2,4-oxadiazol-5-yle, 1,3,4-oxadiazol-2-yle, 1,2,3-oxadiazol-4-yle, 1,2,3-oxadiazol-5-yle, 1,2,5-oxadiazol-3-yle, 1,3,4-thiadiazol-2-yle, 1,3,4-thiadiazol-2-yle, 1,2,4-thiadiazol-5-yle, 1,2,4-thiadiazol-3-yle, 1,2,5-thiadiazol-3-yle, 1,2,5-thiadiazol-3-yle, 1,2,3-thiadiazol-4-yle, 1,2,3-thiadiazol-3-yle, qui est non substitué ou monosubstitué ou polysubstitué par halogène, cyano, méthyle, CF₃, CF₂Cl, CH₂F, CHF₂, OCH₃, OCF₃, SCH₃, SOCH₃, SO₂CH₃ et SCF₃,
R² représente hydrogène, fluor, chlore, brome, iode, CN, méthyle, CF₃, CF₂Cl, CH₂F, CHF₂, OCH₃, OCF₃, SCH₃, SOCH₃, SO₂CH₃ et SCF₃,
R³ représente hydrogène et CH₃,
R⁴ représente hydrogène, (C₁-C₁₀)alkyle, (C₁-C₁₀)halogénoalkyle, (C₁-C₁₀)cyanoalkyle, (C₂-C₁₀)alcényle, (C₂-C₁₀)alcynyle, (C₃-C₉)cycloalkyle, (C₃-C₉)cycloalcényle, aryle, hétéroaryle, (C₁-C₁₀)alcoxy- (C₁-C₁₀)alkyle, (C₁-C₁₀)halogénoalcoxy-(C₁-C₁₀)alkyle, (C₁-C₁₀)alcoxy- (C₁-C₁₀)halogénoalkyle, (C₁-C₁₀)alkyl-thio - (C₁-C₁₀)alkyle, (C₁-C₁₀)halogénoalkyl-thio - (C₁-C₁₀)alkyle, (C₂-C₁₈)halogénoalcényle, (C₂-C₁₈)halogénoalcynyle, hétérocyclyl- (C₁-C₁₀)alkyle, aryl- (C₁-C₁₀)alkyle, (C₃-C₉)cycloalkyl- (C₁-C₁₀)alkyle, (C₁-C₁₀)alcoxy-carbonyl- (C₁-C₁₀)alkyle et (C₁-C₁₀)alcoxy -carbonyl- (C₃-C₉)cycloalkyl-(C₁-C₁₀)alkyle,
et
m représente 0, 1, 2 ou 3.

5. Composés de formule générale (I) selon la revendication 1 ou leurs sels,
dans laquelle
R¹ représente les groupes Q-1.1 à Q-1.59
| | | | | |
|---|---|---|---|---|
| 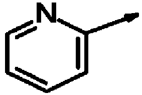 | 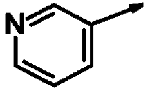 | 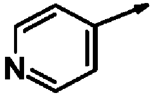 | 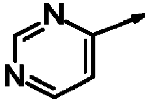 | 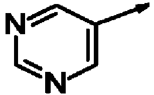 |
| Q-1.1 | Q-1.2 | Q-1.3 | Q-1.4 | Q-1.5 |
| 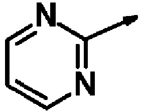 | 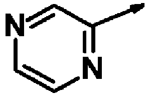 | 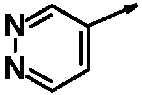 | 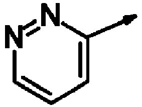 | 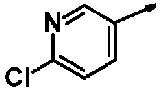 |
| Q-1.6 | Q-1.7 | Q-1.8 | Q-1.9 | Q-1.10 |
| 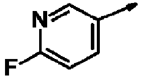 | 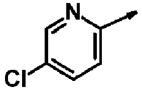 | 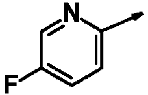 | 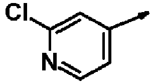 | 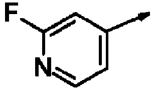 |
| Q-1.11 | Q-1.12 | Q-1.13 | Q-1.14 | Q-1.15 |
| 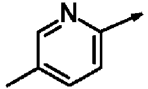 | 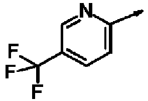 | 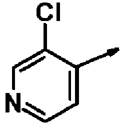 | 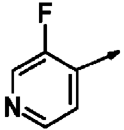 | 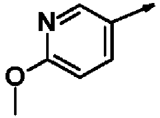 |
| Q-1.16 | Q-1.17 | Q-1.18 | Q-1.19 | Q-1.20 |
| 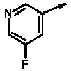 | 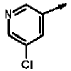 | 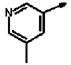 | 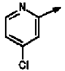 | 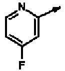 |
| Q-1.21 | Q-1.22 | Q-1.23 | Q-1.24 | Q-1.25 |
| 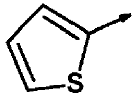 | 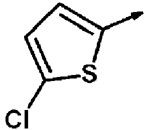 | 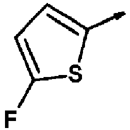 | 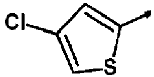 | 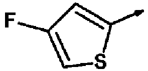 |
| Q-1.26 | Q-1.27 | Q-1.28 | Q-1.29 | Q-1.30 |
| 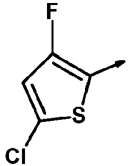 | 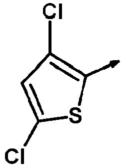 | 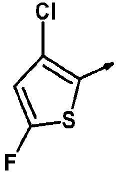 | 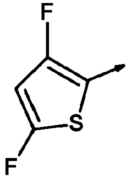 | 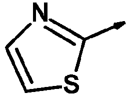 |
| Q-1.31 | Q-1.32 | Q-1.33 | Q-1.34 | Q-1.35 |
| 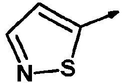 | 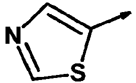 | 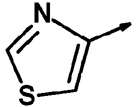 | 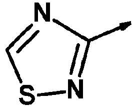 | 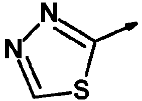 |
| Q-1.36 | Q-1.37 | Q-1.38 | Q-1.39 | Q-1.40 |
| 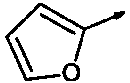 | 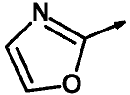 | 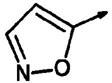 | 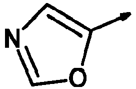 | 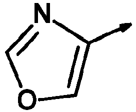 |
| Q-1.41 | Q-1.42 | Q-1.43 | Q-1.44 | Q-1.45 |
| 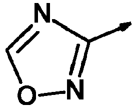 | 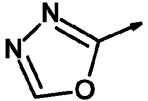 | 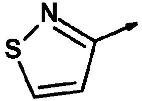 | 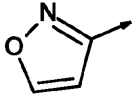 | 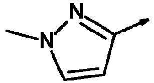 |
| Q-1.46 | Q-1.47 | Q-1.48 | Q-1.49 | Q-1.50 |
| 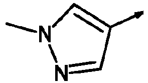 | 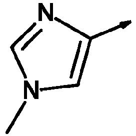 | 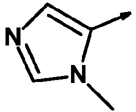 | 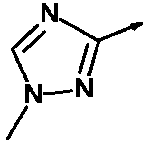 | 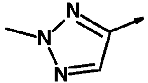 |
| Q-1.51 | Q-1.52 | Q-1.53 | Q-1.54 | Q-1.55 |
| 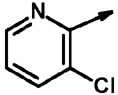 | 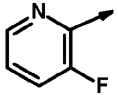 | 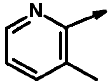 | 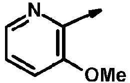 | |
| Q-1.56 | Q-1.57 | Q-1.58 | Q-1.59 | |
R² représente hydrogène, fluor, chlore, brome, iode, cyano, méthyle, CF₃, CH₂F, CHF₂, OCH₃, OCF₃, SCH₃, SOCH₃, SO₂CH₃ et SCF₃,
R³ représente hydrogène,
R⁴ représente hydrogène, méthyle, éthyle, n-propyle, i-propyle, n-butyle, n-pentyle, phényle, benzyle, CH₂(4-Cl-Ph), CH₂(4-F-Ph), CH₂(4-MeO-Ph), 2-méthoxyéthyle, tétrahydrofurann-2-yl-méthyle, tétrahydrofurann-3-yl-méthyle, tétrahydropyran-2-yl-méthyle, tétrahydropyran-3-yl-méthyle, tétrahydropyran-4-yl-méthyle, méthylpropionat-3-yle, éthylpropionat-3-yle, méthylacétat-2-yle, éthylacétat-2-yle, méthylpivalat-2-yle, éthylpivalat-3-yle, méthyl-2-méthylpropanoat-3-yle, méthyl-2,2-diméthylpropanoat-3-yle, éthyl-2-méthylpropanoat-3-yle, méthyl-2-propanoat-2-yle, éthyl-2-propanoat-2-yle, méthyl-acét-2-yle, éthyl-acétat-2-yle, méthyl-1-méthylcyclopropanecarboxylat-2-yle, éthyl-1-méthylcyclopropanecarboxylat-2-yle, 2-(diméthylamino)éthyle, oxétan-3-yle, (3-méthyloxétan-3-yl)méthyle, 2,2,2-trifluoroéthyle, 2,2-difluoroéthyle, 2-fluoroéthyle, 2,2,3,3,3-pentafluoropropyle, cyclopropylméthyle, 1-cyclopropyl-éthyle, (1-méthyl-cyclopropyl)-méthyle, (2,2-dichlorocyclopropyl)-méthyle, (2,2-diméthyl-cyclopropyl)-méthyle, allyle, propargyle, (prop-2-yn-1-yle), 2-chloroprop-2-én-1-yle, 3-phénylprop-2-yn-1-yle, 3,3-dichloroprop-2-én-1-yle, 3,3-dichloro-2-fluoro-prop-2-én-1-yle, méthylprop-2-yn-1-yle, 2-méthylprop-2-én-1-yle, but-2-én-1-yle, but-3-én-1-yle, but-2-yn-1-yle, but-3-yn-1-yle, 4-chloro-but-2-yn-1-yle, 3-méthyl-but-2-én-1-yle, 3-méthyl-but-1-én-1-yle, (2E)-1-méthylbut-2-én-1-yle, (E)-pent-3-én-2-yle ou (Z)-pent-3-én-2-yle, cyclobutylméthyle, cyclopentylméthyle, cyclohexylméthyle, heptan-2-yle, iso-butyle, 1,3-dioxolan-2-ylméthyle ou 1-éthyl-5-méthyl-1H-pyrazole-4-méthyle,
n représente 0, 1, 2 ou 3.

6. Composés de formule générale (I) selon la revendication 1 ou leurs sels,
dans laquelle
R¹ représente les groupes Q-1.1 à Q-1.59
| | | | | |
|---|---|---|---|---|
| 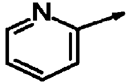 | 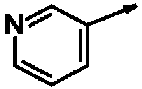 | 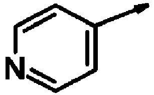 | 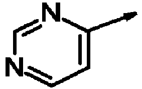 | 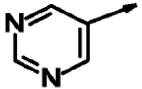 |
| Q-1.1 | Q-1.2 | Q-1.3 | Q-1.4 | Q-1.5 |
| 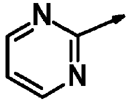 | 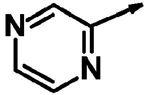 | 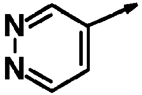 | 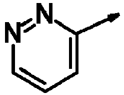 | 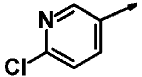 |
| Q-1.6 | Q-1.7 | Q-1.8 | Q-1.9 | Q-1.10 |
| 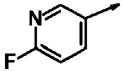 | 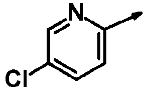 | 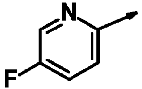 | 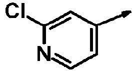 | 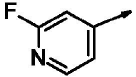 |
| Q-1.11 | Q-1.12 | Q-1.13 | Q-1.14 | Q-1.15 |
| 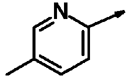 | 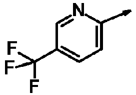 | 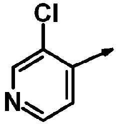 | 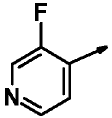 | 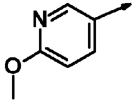 |
| Q-1.16 | Q-1.17 | Q-1.18 | Q-1.19 | Q-1.20 |
| 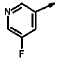 | 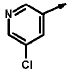 | 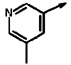 | 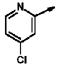 | 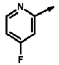 |
| Q-1.21 | Q-1.22 | Q-1.23 | Q-1.24 | Q-1.25 |
| 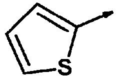 | 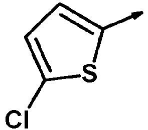 | 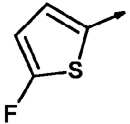 | 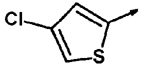 | 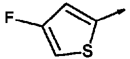 |
| Q-1.26 | Q-1.27 | Q-1.28 | Q-1.29 | Q-1.30 |
| 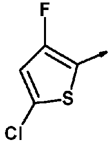 | 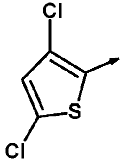 | 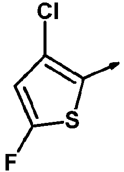 | 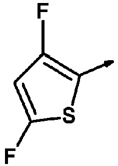 | 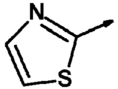 |
| Q-1.31 | Q-1.32 | Q-1.33 | Q-1.34 | Q-1.35 |
| 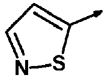 | 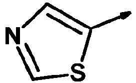 | 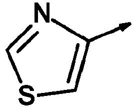 | 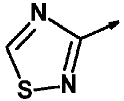 | 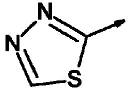 |
| Q-1.36 | Q-1.37 | Q-1.38 | Q-1.39 | Q-1.40 |
| 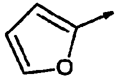 | 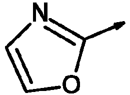 | 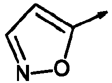 | 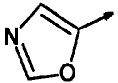 | 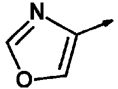 |
| Q-1.41 | Q-1.42 | Q-1.43 | Q-1.44 | Q-1.45 |
| 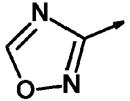 | 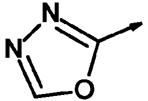 | 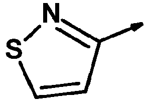 | 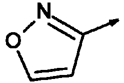 | 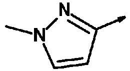 |
| Q-1.46 | Q-1.47 | Q-1.48 | Q-1.49 | Q-1.50 |
| 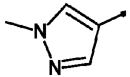 | 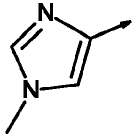 | 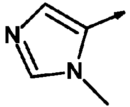 | 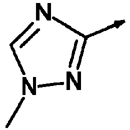 | 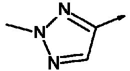 |
| Q-1.51 | Q-1.52 | Q-1.53 | Q-1.54 | Q-1.55 |
| 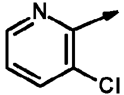 | 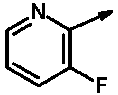 | 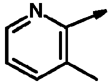 | 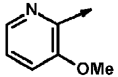 | |
| Q-1.56 | Q-1.57 | Q-1.58 | Q-1.59 | |
,
et (R²)ₘ-phényle représente les groupes Q-2.1 à Q-2.53
| | | | | |
|---|---|---|---|---|
| 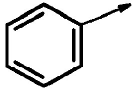 | 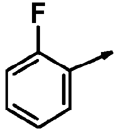 | 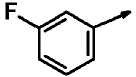 | 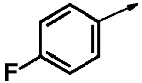 | 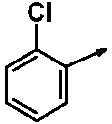 |
| Q-2.1 | Q-2.2 | Q-2.3 | Q-2.4 | Q-2.5 |
| 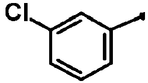 | 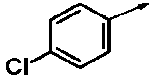 | 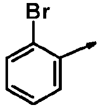 | 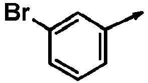 | 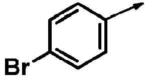 |
| Q-2.6 | Q-2.7 | Q-2.8 | Q-2.9 | Q-2.10 |
| 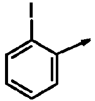 | 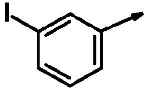 | 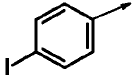 | 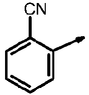 | 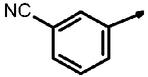 |
| Q-2.11 | Q-2.12 | Q-2.13 | Q-2.14 | Q-2.15 |
| 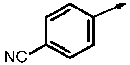 | 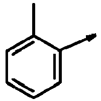 | 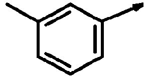 | 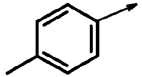 | 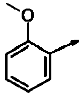 |
| Q-2.16 | Q-2.17 | Q-2.18 | Q-2.19 | Q-2.20 |
| 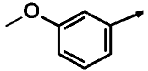 | 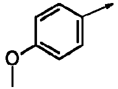 | 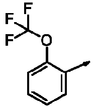 | 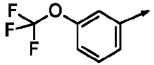 | 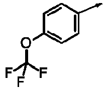 |
| Q-2.21 | Q-2.22 | Q-2.23 | Q-2.24 | Q-2.25 |
| 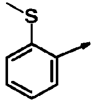 | 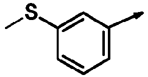 | 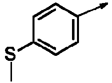 | 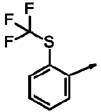 | 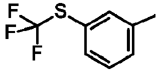 |
| Q-2.26 | Q-2.27 | Q-2.28 | Q-2.29 | Q-2.30 |
| 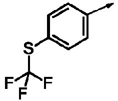 | 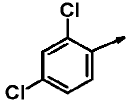 | 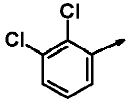 | 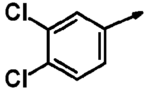 | 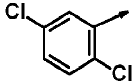 |
| Q-2.31 | Q-2.32 | Q-2.33 | Q-2.34 | Q-2.35 |
| 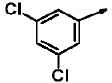 | 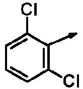 | 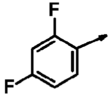 | 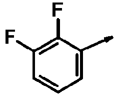 | 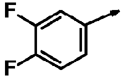 |
| Q-2.36 | Q-2.37 | Q-2.38 | Q-2.39 | Q-2.40 |
| 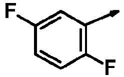 | 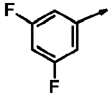 | 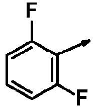 | 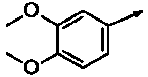 | 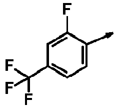 |
| Q-2.41 | Q-2.42 | Q-2.43 | Q-2.44 | Q-2.45 |
| 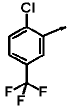 | 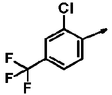 | 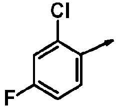 | 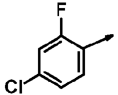 | 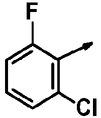 |
| Q-2.46 | Q-2.47 | Q-2.48 | Q-2.49 | Q-2.50 |
| 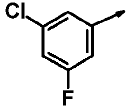 | 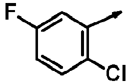 | 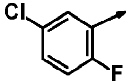 | | |
| Q-2.51 | Q-2.52 | Q-2.53 | | |
,
R³ représente hydrogène,
R⁴ représente hydrogène, méthyle, éthyle, n-propyle, i-propyle, n-butyle, n-pentyle, phényle, benzyle, CH₂(4-Cl-Ph), CH₂(4-F-Ph), CH₂(4-MeO-Ph), 2-méthoxyéthyle, tétrahydrofurann-2-yl-méthyle, tétrahydrofurann-3-yl-méthyle, tétrahydropyran-2-yl-méthyle, tétrahydropyran-3-yl-méthyle, tétrahydropyran-4-yl-méthyle, méthylpropionat-3-yle, éthylpropionat-3-yle, méthylacétat-2-yle, éthylacétat-2-yle, méthylpivalat-2-yle, éthylpivalat-3-yle, méthyl-2-méthylpropanoat-3-yle, méthyl-2,2-diméthylpropanoat-3-yle, éthyl-2-méthylpropanoat-3-yle, méthyl-2-propanoat-2-yle, éthyl-2-propanoat-2-yle, méthyl-acétat-2-yle, éthyl-acétat-2-yle, méthyl-1-méthylcyclopropanecarboxylat-2yle, éthyl-1-méthylcyclopropanecarboxylat-2-yle, 2-(diméthylamino)éthyle, oxétan-3-yle, (3-méthyloxétan-3-yl)méthyle, 2,2,2-trifluoroéthyle, 2,2-difluoroéthyle, 2-fluoroéthyle, 2,2,3,3,3-pentafluoropropyle, cyclopropylméthyle, 1-cyclopropyl-éthyle, (1-méthyl-cyclopropyl)-méthyle, (2,2-dichlorocyclopropyl)-méthyle, (2,2-diméthyl-cyclopropyl)-méthyle, allyle, propargyle (prop-2-yn-1-yle), 2-chloroprop-2-én-1-yle, 3-phénylprop-2-yn-1-yle, 3,3-dichloroprop-2-én-1-yle, 3,3-dichloro-2-fluoro-prop-2-én-1-yle, méthylprop-2-yn-1-yle, 2-méthylprop-2-én-1-yle, but-2-én-1-yle, but-3-én-1-yle, but-2-yn-1-yle, but-3-yn-1-yle, 4-chloro-but-2-yn-1-yle, 3-méthyl-but-2-én-1-yle, 3-méthyl-but-1-én-1-yle, (2E)-1-méthylbut-2-én-1-yle, (E)-pent-3-én-2-yle ou (Z)-pent-3-én-2-yle, cyclobutylméthyle, cyclopentylméthyle, cyclohexylméthyle, heptan-2-yle, iso-butyle, 1,3-dioxolan-2-ylméthyle ou 1-éthyl-5-méthyl-1H-pyrazole-4-méthyle.

7. Composés de formule générale (I) selon la revendication 1 ou leurs sels,
dans laquelle
R¹ représente Q-1.1 à 0-1.59
| | | | | |
|---|---|---|---|---|
| 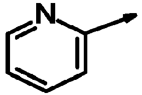 | 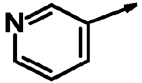 | 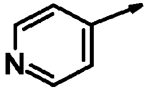 | 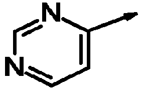 | 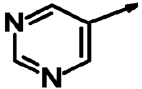 |
| Q-1.1 | Q-1.2 | Q-1.3 | Q-1.4 | Q-1.5 |
| 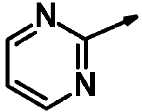 | 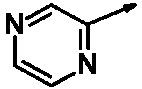 | 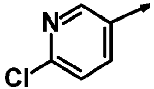 | 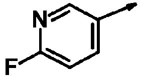 | 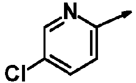 |
| Q-1.6 | Q-1.7 | Q-1.10 | Q-1.11 | Q-1.12 |
| 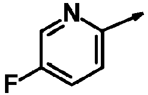 | 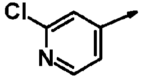 | 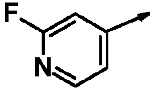 | 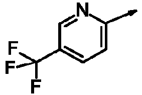 | 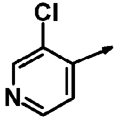 |
| Q-1.13 | Q-1.14 | Q-1.15 | Q-1.17 | Q-1.18 |
| 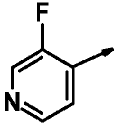 | 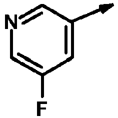 | 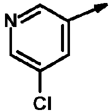 | 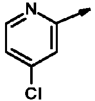 | 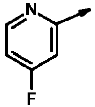 |
| Q-1.19 | Q-1.21 | Q-1.22 | Q-1.24 | Q-1.25 |
| 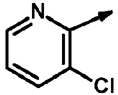 | 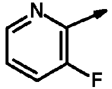 | 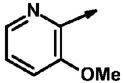 | | |
| Q-1.56 | Q-1.57 | Q-1.59 | | |
,
et (R²)ₘ-phényle représente les groupes Q-2.1 à Q-2.53
| | | | | |
|---|---|---|---|---|
| 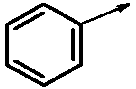 | 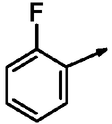 | 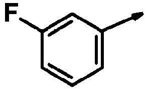 | 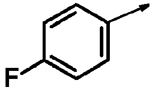 | 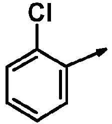 |
| Q-2.1 | Q-2.2 | Q-2.3 | Q-2.4 | Q-2.5 |
| 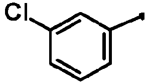 | 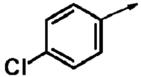 | 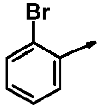 | 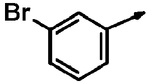 | 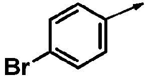 |
| Q-2.6 | Q-2.7 | Q-2.8 | Q-2.9 | Q-2.10 |
| 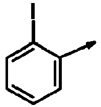 | 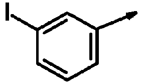 | 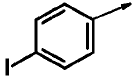 | 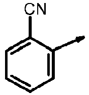 | 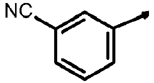 |
| Q-2.11 | Q-2.12 | Q-2.13 | Q-2.14 | Q-2.15 |
| 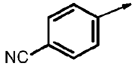 | 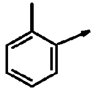 | 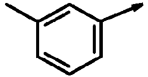 | 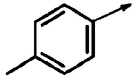 | 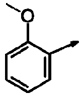 |
| Q-2.16 | Q-2.17 | Q-2.18 | Q-2.19 | Q-2.20 |
| 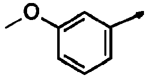 | 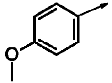 | 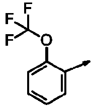 | 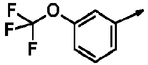 | 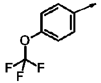 |
| Q-2.21 | Q-2.22 | Q-2.23 | Q-2.24 | Q-2.25 |
| 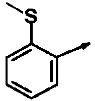 | 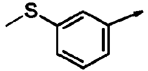 | 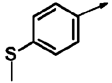 | 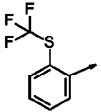 | 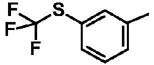 |
| Q-2.26 | Q-2.27 | Q-2.28 | Q-2.29 | Q-2.30 |
| 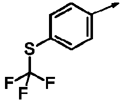 | 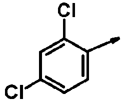 | 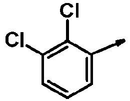 | 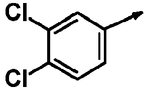 | 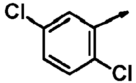 |
| Q-2.31 | Q-2.32 | Q-2.33 | Q-2.34 | Q-2.35 |
| 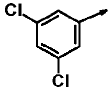 | 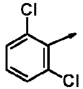 | 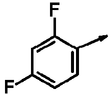 | 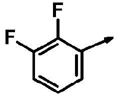 | 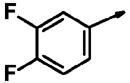 |
| Q-2.36 | Q-2.37 | Q-2.38 | Q-2.39 | Q-2.40 |
| 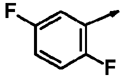 | 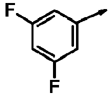 | 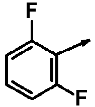 | 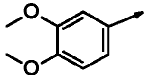 | 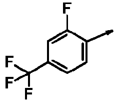 |
| Q-2.41 | Q-2.42 | Q-2.43 | Q-2.44 | Q-2.45 |
| 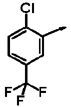 | 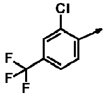 | 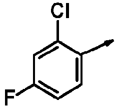 | 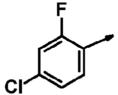 | 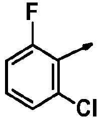 |
| Q-2.46 | Q-2.47 | Q-2.48 | Q-2.49 | Q-2.50 |
| 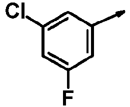 | 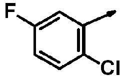 | 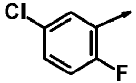 | | |
| Q-2.51 | Q-2.52 | Q-2.53 | | |
,
R³ représente hydrogène,
R⁴ représente hydrogène, méthyle, éthyle, n-propyle, i-propyle, n-butyle, n-pentyle, phényle, benzyle, CH₂(4-Cl-Ph), CH₂(4-F-Ph), CH₂ (4-MeO-Ph), 2-méthoxyéthyle, tétrahydrofurann-2-yl-méthyle, tétrahydrofurann-3-yl-méthyle, tétrahydropyran-2-yl-méthyle, tétrahydropyran-3-yl-méthyle, tétrahydropyran-4-yl-méthyle, méthylpropionat-3-yle, éthylpropionat-3-yle, méthylacétat-2-yle, éthylacétat-2-yle, méthylpivalat-2-yle, éthylpivalat-3-yle, méthyl-2-méthylpropanoat-3-yle, méthyl-2,2-diméthylpropanoat-3-yle, éthyl-2-méthylpropanoat-3-yle, méthyl-2-propanoat-2-yle, éthyl-2-propanoat-2-yle, méthyl-acétat-2-yle, éthyl-acétat-2-yle, méthyl-1-méthylcyclopropanecarboxylat-2-yle, éthyl-1-méthylcyclopropanecarboxylat-2-yle, 2-(diméthylamino)éthyle, oxétan-3-yle, (3-méthyloxétan-3-yl)méthyle, 2,2,2-trifluoroéthyle, 2,2-difluoroéthyle, 2-fluoroéthyle, 2,2,3,3,3-pentafluoropropyle, cyclopropylméthyle, 1-cyclopropyl-éthyle, (1-méthyl-cyclopropyl)-méthyle, (2,2-dichlorocyclopropyl)-méthyle, (2,2-diméthyl-cyclopropyl)-méthyle, allyle, propargyle (prop-2-yn-1-yle), 2-chloroprop-2-én-1-yle, 3-phénylprop-2-yn-1-yle, 3,3-dichloroprop-2-én-1-yle, 3,3-dichloro-2-fluoro-prop-2-én-1-yle, méthylprop-2-yn-1-yle, 2-méthylprop-2-én-1-yle, but-2-én-1-yle, but-3-én-1-yle, but-2-yn-1-yle, but-3-yn-1-yle, 4-chloro-but-2-yn-1-yle, 3-méthyl-but-2-én-1-yle, 3-méthyl-but-1-én-1-yle, (2E)-1-méthylbut-2-én-1-yle, (E)-pent-3-én-2-yle ou (Z)-pent-3-én-2-yle, cyclobutylméthyle, cyclopentylméthyle, cyclohexylméthyle, heptan-2-yle, iso-butyle, 1,3-dioxolan-2-ylméthyle ou 1-éthyl-5-méthyl-1H-pyrazole-4-méthyle.

8. Agent protecteur de plantes utiles ou cultivées, **caractérisé par** une teneur en au moins un composé de formule générale (I) ou ses sels selon l'une des revendications 1 à 7, en combinaison avec d'autres composés agrochimiques et le cas échéant des adjuvants de formulation.

9. Agent selon la revendication 8, contenant au moins un herbicide.

10. Procédé pour la réduction d'effets phytotoxiques de pesticides sur des plantes utiles ou cultivées, par utilisation d'un ou de plusieurs composés selon les revendications 1-7 ou de l'agent selon les revendications 8 et 9.

11. Procédé pour la réduction d'effets phytotoxiques de pesticides sur des plantes utiles ou cultivées, **caractérisé en ce qu'**un ou plusieurs composés selon l'invention de formule générale (I) selon les revendications 1 à 7 sont épandus, pour l'application conjointe avec des pesticides, simultanément ou dans un ordre quelconque avec les pesticides.

12. Procédé selon la revendication 11, dans lequel il s'agit, pour les pesticides, d'un ou de plusieurs herbicides.

13. Procédé selon les revendications 11 ou 12, **caractérisé en ce que** des composés de formule (I) ou leurs sels selon les revendications 1 à 7 sont appliqués sur les plantes, des parties des plantes, leurs graines ou semences.
